# NEUE EUROPÄISCHE PATENTSCHRIFT

(11) **EP 0 463 488 B2**
(45) Veröffentlichungstag und Bekanntmachung des Hinweises auf die Entscheidung über den Einspruch: **21.04.2004**
(45) Hinweis auf die Patenterteilung: 10.12.1997
(21) Anmeldenummer: 91109684.0
(22) Anmeldetag: 13.06.1991
(51) Int. Cl.: C07C 251/60, A01N 37/52, A01N 43/00, A01N 37/34, A01N 37/50, A01N 37/42, A01N 37/36, A01N 37/32, C07C 255/64, C07C 323/47, C07C 257/06

(54) **O-Benzyl-Oximether und diese Verbindungen enthaltende Pflanzenschutzmittel**
O-Benzyl oxime ethers and fungicides containing them
Ethers d'oximes o-benziliques et fongicides les contenants

(30) Priorität: 27.06.1990 DE 4020384; 27.06.1990 DE 4020388
(43) Veröffentlichungstag der Anmeldung: 02.01.1992
(60) Teilanmeldung: 95106324.7
(73) Patentinhaber: BASF Aktiengesellschaft, 67063 Ludwigshafen (DE)
(72) Erfinder: Brand, Siegbert, Dr., W-6701 Birkenheide (DE); Kardorff, Uwe, Dr., W-6800 Mannheim 1 (DE); Kirstgen, Reinhard, Dr., W-6730 Neustadt (DE); Müller, Bernd, Dr., W-6710 Frankenthal (DE); Oberdorf, Klaus, Dr., W-6904 Eppelheim (DE); Sauter, Hubert, Dr., W-6800 Mannheim 1 (DE); Lorenz, Gisela, Dr., W-6730 Neustadt (DE); Ammermann, Eberhard, Dr., W-6700 Ludwigshafen (DE); Kuenast, Christoph, Dr., W-6701 Otterstadt (DE); Harreus, Albrecht, Dr., W-6700 Ludwigshafen (DE)

(56) Entgegenhaltungen:
- EP-A- 310 954
- EP-A- 460 575
- EP-A- 0 243 012
- EP-A- 0 244 077
- EP-A- 0 244 786
- EP-A- 0 253 213
- EP-A- 0 254 426
- EP-A- 0 274 825
- EP-A- 0 370 629
- EP-A- 0 414 153
- EP-A- 0 460 575
- WO-A-90/07493
- WO-A-97/19912
- CH-D- 120 891
- CH-D- 189 190
- DE-A- 4 020 384

## Beschreibung

Die vorliegende Erfindung betrifft neue O-Benzyl-Oximether und ein Verfahren zur Bekämpfung von Schädlingen, insbesondere von Pilzen, Insekten, Nematoden und Spinnmilben mit diesen Verbindungen.

Es ist bekannt, substituierte Phenylessigsäureoximetherderivate als Fungizide (EP-A 253 213) zu verwenden. Ihre Wirkung ist jedoch unbefriedigend.

Außerdem werden in der EP-A 310 954 Phenylmethoxyacrylsäureester mit fungizider Wirksamkeit beschrieben. Aus EP-A 370 629 sind des weiteren Phenylmethoxyacrylsäureester bekannt, die in der ortho-Position des Phenylrings eine Imino-oxymethylengruppe tragen. Ähnliche Verbindungen werden in den älteren Anmeldungen EP-A 414 153, EP-A 460 575 und WO-A 90/07,493 beschrieben.

EP-A 244 786 beschreibt in allgemeiner Form die Herstellung der für die erfindungsgemäßen Verbindungen erforderlichen Zwischenprodukte.

Es wurde überraschend gefunden, daß O-Benzyl-Oximether der allgemeinen Formel I 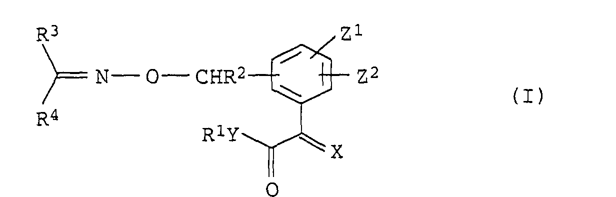 in der die Substituenten die folgende Bedeutung haben:
a)
- X: CH-C₁-C₄-Alkyl, CH-C₁-C₄-Alkylthio oder N-C₁-C₄-Alkoxy,
- Y: Sauerstoff, Schwefel oder NR⁵,
- R¹,R²,R⁵: unabhängig voneinander Wasserstoff oder C₁-C₄-Alkyl,
- Z¹, Z²: unabhängig voneinander
Wasserstoff, Halogen, Methyl, Methoxy oder Cyano,
- R³, R⁴: unabhängig voneinander Wasserstoff, Cyano, ggf. verzweigte C₁-C₁₀-Alkyl, C₁-C₄-Halogenalkyl, C₃-C₆-Cycloalkyl, C₃-C₆-Halogencycloalkyl, C₃-C₆-Cycloalkyl-C₁-C₄-alkyl, C₁-C₄-Alkoxy-C₁-C₄-alkyl, C₁-C₄-Alkylthio-C₁-C₄-alkyl, Arylthio-C₁-C₄-alkyl, C₂-C₆-Alkenyl, C₂-C₅-Halogenalkenyl, C₃-C₆-Cycloalkenyl, C₃-C₆-Halogencycloalkenyl, C₂-C₆-Alkinyl, C₁-C₆-Alkoxy, C₁-C₆-Halogenalkoxy, C₁-C₄-Alkylthio, Benzylthio, C₁-C₄-Alkylcarbonyl, C₁-C₄-Alkoxycarbonyl, ggf. subst. Phenylcarbonyl, ggf. subst. Benzylcarbonyl, ggf. subst. Phenoxycarbonyl, ggf. subst. Benzyloxycarbonyl, ggf. subst. Aryl, ggf. subst. Aryloxy, ggf. subst. Arylthio, ggf. subst. Aryl-C₁-C₄-alkyl, ggf. subst. Aryl-C₂-C₄-alkenyl, ggf. subst. Aryloxy-C₁-C₄-alkyl, ggf. subst. Arylthio-C₁-C₄-alkyl, ggf. subst. Hetaryl, ggf. subst. Hetaryloxy, ggf. subst. Hetarylthio, ggf. subst. Hetaryl-C₁-C₄-alkyl, ggf. subst. Hetaryl-C₂-C₄-alkenyl, ggf. subst. Hetaryl-C₁-C₄-alkoxy, ggf. subst. Heterocyclyl, ggf. subst. Heterocyclyloxy,
N(R⁶)₂, wobei die Bedeutungen von R⁶ gleich oder verschieden sind,
CON(R⁷)₂, wobei die Bedeutungen von R⁷ gleich oder verschieden sind,
- R³ und R⁴ zusammen: ein carbocyclischer oder heterocyclischer Ring, der durch die unter "ggf. subst." genannten Reste substituiert sein kann, oder
einer der Reste R³ oder R⁴ Halogen,
- R⁶: Wasserstoff, C₁-C₆-Alkyl oder ggf. subst. Phenyl,
- R⁷: Wasserstoff oder C₁-C₄-Alkyl,
- ggf.: subst. neben Wasserstoff die Reste Halogen, Cyano, Nitro, C₁-C₄-Alkyl, C₁-C₄-Alkoxy, C₁-C₄-Halogenalkyl, C₁-C₄-Halogenalkoxy, C₁-C₁₀-Alkoximino-C₁-C₂-alkyl, Aryl, Aryloxy, Benzyloxy, Hetaryl, Hetaryloxy, C₃-C₆-Cycloalkyl, Heterocyclyl oder Heterocyclyloxy,
außer den Verbindungen, in denen
- X: CH-C₁-C₂-Alkylthio oder N-C₁-C₂-Alkoxy,
- Y: Sauerstoff,
- Z¹, Z² und R²: Wasserstoff,
- R¹: C₁-C₄-Alkyl,
- R³: Wasserstoff, C₁-C₄-Alkyl, C₁-C₄-Halogenalkyl oder C₃-C₆-Cycloalkyl,
- R⁴: C₁-C₆-Alkyl, ggf. subst. Aryl-C₁-C₄-alkyl, ggf. subst. Hetaryl-C₁-C₄-alkyl, C₂-C₆-Alkenyl, ggf. subst. Aryl-C₂-C₄-alkenyl, ggf. subst. Hetaryl-C₂-C₄-alkenyl, C₃-C₆-Cycloalkyl, ggf. subst. Aryl, ggf. subst. Hetaryl, C₁-C₃-Alkylcarbonyl, ggf. subst. Phenylcarbonyl oder
- R³ und R⁴: zusammen mit dem C-Atom, an das sie gebunden sind, einen gegebenenfalls substutierten, gegebenenfalls ein Sauerstoff- oder Schwefelatom enthaltenden vier- bis siebengliedrigen gesättigten Ring bilden, der zudem einen gegebenenfalls substituierten ankondensierten Benzolring aufweisen kann,
wobei von den ausgenommenen Verbindungen, die folgenden Einzelverbindungen der Formel Ia

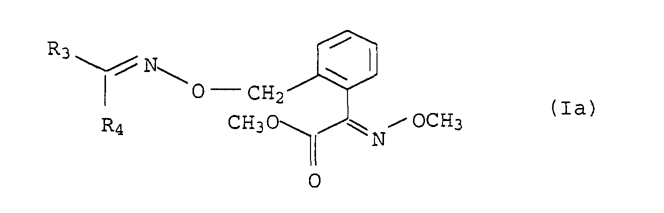

| | R³ | R⁴ |
|---|---|---|
| 8 | CCl₃ | Phenyl |
| 9 | CH₂Cl | Phenyl |
| 10 | CF₂CF₃ | Phenyl |
| 11 | CF₂Cl | Phenyl |
| 12 | CHCl₂ | Phenyl |
| 13 | Cyclopropyl | Cyclopropyl |
| 18 | Cyclopropyl | 4-Ethoxyphenyl |
| 20 | Cyclopropyl | Pentachlorphenyl |
| 21 | Cyclopropyl | Pentafluorphenyl |
| 22 | Cyclopentyl | Phenyl |
| 23 | Cyclohexyl | Phenyl |
| 497 | CH₃ | Methyl |
| 498 | CH₃ | Ethyl |
| 499 | CH₃ | n-Propyl |
| 500 | CH₃ | iso-Propyl |
| 501 | CH₃ | n-Butyl |
| 502 | CH₃ | iso-Butyl |
| 503 | CH₃ | sec.-Butyl |
| 504 | CH₃ | tert.-Butyl |
| 505 | CH₃ | n-Hexyl |
| 507 | CH₃ | Cyclopropyl |
| 508 | CH₃ | Cyclohexyl |
| 542 | CH₃ | Acetyl |
| 543 | CH₃ | Propion-1-yl |
| 544 | CH₃ | Butyr-1-yl |
| 545 | CH₃ | iso-Butyr-1-yl |
| 546 | CH₃ | Pivaloyl |
| 572 | CH₃ | Pentafluorphenyl |
| 576 | CH₃ | Pentachlorphenyl |
| 583 | CH₃ | 2,3,4-Trichlorphenyl |
| 584 | CH₃ | 2,3,5-Trichlorphenyl |
| 585 | CH₃ | 2,3,6-Trichlorphenyl |
| 586 | CH₃ | 2,4,5-Trichlorphenyl |
| 587 | CH₃ | 2,4,6-Trichlorphenyl |
| 588 | CH₃ | 3,4,5-Trichlorphenyl |
| 589 | CH₃ | 2,3,4,6-Tetrachlorphenyl |
| 590 | CH₃ | 2,3,5,6-Tetrachlorphenyl |
| 609 | CH₃ | 2-Cyanophenyl |
| 610 | CH₃ | 3-Cyanophenyl |
| 611 | CH₃ | 4-Cyanophenyl |
| 618 | CH₃ | 2,4-Dimethylphenyl |
| 619 | CH₃ | 2,6-Dimethylphenyl |
| 620 | CH₃ | 3,4-Dimethylphenyl |
| 621 | CH₃ | 3,5-Dimethylphenyl |
| 622 | CH₃ | 2,3,4-Trimethylphenyl |
| 623 | CH₃ | 2,3,5-Trimethylphenyl |
| 624 | CH₃ | 2,3,6-Trimethylphenyl |
| 625 | CH₃ | 2,4,5-Trimethylphenyl |
| 626 | CH₃ | 2,4,6-Trimethylphenyl |
| 627 | CH₃ | 3,4,5-Trimethylphenyl |
| 628 | CH₃ | Pentamethylphenyl |
| 629 | CH₃ | 2-Ethylphenyl |
| 630 | CH₃ | 3-Ethylphenyl |
| 631 | CH₃ | 4-Ethylphenyl |
| 632 | CH₃ | 3,5-Diethylphenyl |
| 633 | CH₃ | 2-n-Propylphenyl |
| 634 | CH₃ | 3-n-Propylphenyl |
| 635 | CH₃ | 4-n-Propylphenyl |
| 636 | CH₃ | 2-iso-Propylphenyl |
| 637 | CH₃ | 3-iso-Propylphenyl |
| 638 | CH₃ | 4-iso-Propylphenyl |
| 639 | CH₃ | 2,4-Di-iso-Propylphenyl |
| 640 | CH₃ | 3,5-Di-iso-Propylphenyl |
| 641 | CH₃ | 4-n-Butylphenyl |
| 642 | CH₃ | 4-sec.-Butylphenyl |
| 643 | CH₃ | 4-iso-Butylphenyl |
| 644 | CH₃ | 4-tert.-Butylphenyl |
| 645 | CH₃ | 3-tert.-Butylphenyl |
| 646 | CH₃ | 2-tert.-Butylphenyl |
| 647 | CH₃ | 2,4-Di-tert.-butylphenyl |
| 648 | CH₃ | 3,5-Di-tert.-butylphenyl |
| 650 | CH₃ | 4-n-Dodecylphenyl |
| 651 | CH₃ | 2-Methyl-4-tert.-butylphenyl |
| 652 | CH₃ | 2-Methyl-6-tert.-butylphenyl |
| 653 | CH₃ | 2-Methyl-4-iso-propylphenyl |
| 654 | CH₃ | 2-Methyl-4-cyclohexylphenyl |
| 655 | CH₃ | 2-Methyl-4-phenylphenyl |
| 656 | CH₃ | 2-Methyl-4-benzylphenyl |
| 657 | CH₃ | 2-Methyl-4-phenoxyphenyl |
| 658 | CH₃ | 2-Methyl-4-benzyloxyphenyl |
| 666 | CH₃ | 2-Methyl-3-methoxyphenyl |
| 667 | CH₃ | 2-Methyl-4-methoxyphenyl |
| 668 | CH₃ | 2-Methyl-5-methoxyphenyl |
| 669 | CH₃ | 2-Methyl-6-methoxyphenyl |
| 670 | CH₃ | 2-Methyl-4-iso-propoxyphenyl |
| 671 | CH₃ | 2-Methyl-2,5-dimethoxyphenyl |
| 688 | CH₃ | 2-Ethoxyphenyl |
| 689 | CH₃ | 3-Ethoxyphenyl |
| 690 | CH₃ | 4-Ethoxyphenyl |
| 691 | CH₃ | 2-iso-Propoxyphenyl |
| 692 | CH₃ | 3-iso-Propoxyphenyl |
| 693 | CH₃ | 4-iso-Propoxyphenyl |
| 694 | CH₃ | 3-tert.-Butoxyphenyl |
| 695 | CH₃ | 4-tert.-Butoxyphenyl |
| 699 | CH₃ | 3-(1',1',2',2'-Tetrafluor)ethoxyphenyl |
| 700 | CH₃ | 4-(1',1',2',2'-Tetrafluor)ethoxyphenyl |
| 701 | CH₃ | 2-Chlormethylphenyl |
| 702 | CH₃ | 3-Chlormethylphenyl |
| 703 | CH₃ | 4-Chlormethylphenyl |
| 707 | CH₃ | 2-(Methoxyiminomethyl)phenyl |
| 708 | CH₃ | 3-(Methoxyiminomethyl)phenyl |
| 709 | CH₃ | 4-(Methoxyiminomethyl)phenyl |
| 710 | CH₃ | 2-(Ethoxyiminomethyl)phenyl |
| 711 | CH₃ | 3-(Ethoxyiminomethyl)phenyl |
| 712 | CH₃ | 4-(Ethoxyiminomethyl)phenyl |
| 713 | CH₃ | 2-(n-Propoxyiminomethyl)phenyl |
| 714 | CH₃ | 3-(n-Propoxyiminomethyl)phenyl |
| 715 | CH₃ | 4-(n-Propoxyiminomethyl)phenyl |
| 716 | CH₃ | 2-(iso-Propoxyiminomethyl)phenyl |
| 717 | CH₃ | 3-(iso-Propoxyiminomethyl)phenyl |
| 718 | CH₃ | 4-(iso-Propoxyiminomethyl)phenyl |
| 719 | CH₃ | 2-(n-Butoxyiminomethyl)phenyl |
| 720 | CH₃ | 3-(n-Butoxyiminomethyl)phenyl |
| 721 | CH₃ | 4-(n-Butoxyiminomethyl)phenyl |
| 722 | CH₃ | 2-(iso-Butoxyiminomethyl)phenyl |
| 723 | CH₃ | 3-(iso-Butoxyiminomethyl)phenyl |
| 724 | CH₃ | 4-(iso-Butoxyiminomethyl)phenyl |
| 725 | CH₃ | 2-(tert.-Butoxyiminomethyl)phenyl |
| 726 | CH₃ | 3-(tert.-Butoxyiminomethyl)phenyl |
| 727 | CH₃ | 4-(tert.-Butoxyiminomethyl)phenyl |
| 728 | CH₃ | 2-(n-Pentoxyiminomethyl)phenyl |
| 729 | CH₃ | 3-(n-Pentoxyiminomethyl)phenyl |
| 730 | CH₃ | 4-(n-Pentoxyiminomethyl)phenyl |
| 731 | CH₃ | 2-(n-Hexoxyiminomethyl)phenyl |
| 732 | CH₃ | 3-(n-Hexoxyiminomethyl)phenyl |
| 733 | CH₃ | 4-(n-Hexoxyiminomethyl)phenyl |
| 740 | CH₃ | 2-(Methoxyimino-1'-ethyl)phenyl |
| 741 | CH₃ | 3-(Methoxyimino-1'-ethyl)phenyl |
| 742 | CH₃ | 4-(Methoxyimino-1'-ethyl)phenyl |
| 743 | CH₃ | 2-(Ethoxyimino-1'-ethyl)phenyl |
| 744 | CH₃ | 3-(Ethoxyimino-1'-ethyl)phenyl |
| 745 | CH₃ | 4-(Ethoxyimino-1'-ethyl)phenyl |
| 746 | CH₃ | 2-(n-Propoxyimino-1'-ethyl)phenyl |
| 747 | CH₃ | 3-(n-Propoxyimino-1'-ethyl)phenyl |
| 748 | CH₃ | 4-(n-Propoxyimino-1'-ethyl)phenyl |
| 749 | CH₃ | 2-(n-Butoxyamino-1'-ethyl)phenyl |
| 750 | CH₃ | 3-(n-Butoxyamino-1'-ethyl)phenyl |
| 751 | CH₃ | 4-(n-Butoxyamino-1'-ethyl)phenyl |
| 752 | CH₃ | 2-(n-Pentoxyimino-1'-ethyl)phenyl |
| 753 | CH₃ | 3-(n-Pentoxyimino-1'-ethyl)phenyl |
| 754 | CH₃ | 4-(n-Pentoxyimino-1'-ethyl)phenyl |
| 755 | CH₃ | 2-(n-Hexoxyimino-1'-ethyl)phenyl |
| 756 | CH₃ | 3-(n-Hexoxyimino-1'-ethyl)phenyl |
| 757 | CH₃ | 4-(n-Hexoxyimino-1'-ethyl)phenyl |
| 764 | CH₃ | 2-Phenylphenyl |
| 765 | CH₃ | 3-Phenylphenyl |
| 766 | CH₃ | 4-Phenylphenyl |
| 767 | CH₃ | 2-Phenoxyphenyl |
| 768 | CH₃ | 3-Phenoxyphenyl |
| 769 | CH₃ | 4-Phenoxyphenyl |
| 770 | CH₃ | 2-Benzyloxyphenyl |
| 771 | CH₃ | 3-Benzyloxyphenyl |
| 772 | CH₃ | 4-Benzyloxyphenyl |
| 773 | CH₃ | 4-(Imidazol-1'-yl)phenyl |
| 774 | CH₃ | 4-(Piperazin-1'-yl)phenyl |
| 777 | CH₃ | 4-(Pyridyl-2'-oxy)phenyl |
| 778 | CH₃ | 2-Cyclopropylphenyl |
| 779 | CH₃ | 3-Cyclopropylphenyl |
| 780 | CH₃ | 4-Cyclopropylphenyl |
| 781 | CH₃ | 3-Cyclohexylphenyl |
| 782 | CH₃ | 4-Cyclohexylphenyl |
| 783 | CH₃ | 4-Oxiranylphenyl |
| 784 | CH₃ | 4-(1',3'-Dioxan-2'-yl)phenyl |
| 785 | CH₃ | 4-(Tetrahydropyran-2-yloxy)phenyl |
| 788 | CH₃ | 9-Anthryl |
| 804 | CH₃ | Benzyl |
| 805 | CH₃ | 2-Methylbenzyl |
| 806 | CH₃ | 3-Methylbenzyl |
| 807 | CH₃ | 4-Methylbenzyl |
| 808 | CH₃ | 4-tert.-Butylbenzyl |
| 818 | CH₃ | 2-Methoxybenzyl |
| 819 | CH₃ | 4-Methoxybenzyl |
| 820 | CH₃ | 4-tert.-Butoxybenzyl |
| 821 | CH₃ | 4-Phenoxybenzyl |
| 822 | CH₃ | 1-Phenethyl |
| 823 | CH₃ | 2-Phenethyl |
| 824 | CH₃ | 1-Phenylpropyl |
| 825 | CH₃ | 2-Phenylpropyl |
| 826 | CH₃ | 3-Phenylpropyl |
| 827 | CH₃ | 2-Methyl-2-phenylpropyl |
| 828 | CH₃ | 2-Methyl-3-phenylpropyl |
| 829 | CH₃ | 4-Phenylbutyl |
| 830 | CH₃ | 2-Phenyl-1-ethenyl |
| 831 | CH₃ | 1-Phenyl-1-ethenyl |
| 832 | CH₃ | 1-Phenyl-1-propenyl |
| 833 | CH₃ | 1-Phenyl-1-propen-2-yl |
| 839 | CH₃ | 2,6-Pyrimidinyl |
| 840 | CH₃ | 1,5-Pyrimidinyl |
| 845 | CH₃ | 1-Pyrrolyl |
| 846 | CH₃ | 1-Imidazolyl |
| 847 | CH₃ | 1,2,4-Triazolyl |
| 848 | CH₃ | 1,3,4-Triazolyl |
| 849 | CH₃ | 4-Thiazolyl |
| 857 | CH₃ | 2-Pyridylmethyl |
| 858 | CH₃ | 3-Pyridylmethyl |
| 864 | CH₃ | 2'-Furyl-2-ethenyl |
| 865 | CH₃ | 2'-Thienyl-2-ethenyl |
| 866 | CH₃ | 3'-Pyridyl-2-ethenyl |
| 891 | CH₃ | Cyclopropyl |
| 892 | CH₃ | Cyclobutyl |
| 893 | CH₃ | Cyclopentyl |
| 894 | CH₃ | Cyclohexyl |
| 903 | CH₃ | Ethenyl |
| 904 | CH₃ | 1-Propenyl |
| 905 | CH₃ | 2-Methyl-1-propenyl |
| 906 | CH₃ | 4-Methylpent-3-en-1-yl |
| 907 | CH₃ | 2-Propenyl |
| 908 | CH₃ | 2-Butenyl |
| 909 | CH₃ | 1-Methyl-2-propenyl |
| 910 | CH₃ | 3-Methyl-2-butenyl |
| 948 | Ethyl | Ethyl |
| 949 | Ethyl | n-Propyl |
| 950 | Ethyl | iso-Propyl |
| 951 | Ethyl | n-Butyl |
| 952 | Ethyl | iso-Butyl |
| 953 | Ethyl | 2-Methyl-butyl |
| 954 | Ethyl | Benzyl |
| 955 | n-Propyl | n-Propyl |
| 956 | iso-Propyl | iso-Propyl |
| 957 | n-Butyl | n-Butyl |
| 958 | iso-Butyl | iso-Butyl |
| 959 | tert.-Butyl | tert.-Butyl |
| 966 | n-Butyl | Phenyl |

| | R³ | R⁴ |
|---|---|---|
| 17 | H | Methyl |
| 18 | H | Ethyl |
| 19 | H | n-Propyl |
| 20 | H | iso-Propyl |
| 21 | H | n-Butyl |
| 22 | H | iso-Butyl |
| 23 | H | sec.-Butyl |
| 24 | H | tert.-Butyl |
| 25 | H | n-Hexyl |
| 27 | H | Cyclopropyl |
| 28 | H | Cyclohexyl |
| 62 | H | Acetyl |
| 63 | H | Proption-1-yl |
| 64 | H | Butyr-1-yl |
| 65 | H | iso-Butyr-1-yl |
| 66 | H | Pivaloyl |
| 88 | H | Phenyl |
| 89 | H | 2-Fluorphenyl |
| 90 | H | 3-Fluorphenyl |
| 91 | H | 4-Fluorphenyl |
| 92 | H | Pentafluorphenyl |
| 93 | H | 2-Chlorphenyl |
| 94 | H | 3-Chlorphenyl |
| 95 | H | 4-Chlorphenyl |
| 97 | H | 2,3-Dichlorphenyl |
| 98 | H | 2,4-Dichlorphenyl |
| 99 | H | 2,5-Dichlorphenyl |
| 100 | H | 2,6-Dichlorphenyl |
| 101 | H | 3,4-Dichlorphenyl |
| 102 | H | 3,5-Dichlorphenyl |
| 103 | H | 2,3,4-Trichlorphenyl |
| 104 | H | 2,3,5-Trichlorphenyl |
| 105 | H | 2,3,6-Trichlorphenyl |
| 106 | H | 2,4,5-Trichlorphenyl |
| 107 | H | 2,4,6-Trichlorphenyl |
| 108 | H | 3,4,5-Trichlorphenyl |
| 111 | H | 2-Bromphenyl |
| 112 | H | 3-Bromphenyl |
| 113 | H | 4-Bromphenyl |
| 114 | H | 2,4-Dibromphenyl |
| 115 | H | 3-Brom-4-fluorphenyl |
| 116 | H | 3-Brom-4-methoxyphenyl |
| 117 | H | 2-Jodphenyl |
| 118 | H | 3-Jodphenyl |
| 119 | H | 4-Jodphenyl |
| 120 | H | 2-Chlor-4-fluorphenyl |
| 121 | H | 2-Chlor-5-fluorphenyl |
| 122 | H | 2-Chlor-6-fluorphenyl |
| 123 | H | 2-Chlor-4-bromphenyl |
| 124 | H | 2-Brom-4-chlorphenyl |
| 125 | H | 2-Brom-4-fluorphenyl |
| 126 | H | 3-Brom-4-chlorphenyl |
| 127 | H | 3-Chlor-4-fluorphenyl |
| 128 | H | 3-Fluor-4-chlorphenyl |
| 129 | H | 2-Cyanophenyl |
| 130 | H | 3-Cyanophenyl |
| 131 | H | 4-Cyanophenyl |
| 132 | H | 2-Nitrophenyl |
| 133 | H | 3-Nitrophenyl |
| 134 | H | 4-Nitrophenyl |
| 135 | H | 2-Methylphenyl |
| 136 | H | 3-Methylphenyl |
| 137 | H | 4-Methylphenyl |
| 138 | H | 2,4-Dimethylphenyl |
| 139 | H | 2,6-Dimethylphenyl |
| 140 | H | 3,4-Dimethylphenyl |
| 141 | H | 3,5-Dimethylphenyl |
| 142 | H | 2,3,4-Trimethylphenyl |
| 143 | H | 2,3,5-Trimethylphenyl |
| 144 | H | 2,3,6-Trimethylphenyl |
| 145 | H | 2,4,5-Trimethylphenyl |
| 146 | H | 2,4,6-Trimethylphenyl |
| 147 | H | 3,4,5-Trimethylphenyl |
| 148 | H | Pentamethylphenyl |
| 149 | H | 2-Ethylphenyl |
| 150 | H | 3-Ethylphenyl |
| 151 | H | 4-Ethylphenyl |
| 152 | H | 3,5-Diethylphenyl |
| 153 | H | 2-n-Propylphenyl |
| 154 | H | 3-n-Propylphenyl |
| 155 | H | 4-n-Propylphenyl |
| 156 | H | 2-iso-Propylphenyl |
| 157 | H | 3-iso-Propylphenyl |
| 158 | H | 4-iso-Propylphenyl |
| 159 | H | 2,4-Di-iso-propylphenyl |
| 160 | H | 3,5-Di-iso-propylphenyl |
| 161 | H | 4-n-Butylphenyl |
| 162 | H | 4-sec.-Butylphenyl |
| 163 | H | 4-iso-Butylphenyl |
| 164 | H | 4-tert.-Butylphenyl |
| 165 | H | 3-tert.-Butylphenyl |
| 166 | H | 2-tert.-Butylphenyl |
| 167 | H | 2,4-Di-tert.-butylphenyl |
| 168 | H | 3,5-Di-tert.-butylphenyl |
| 171 | H | 2-Methyl-4-tert.-butylphenyl |
| 172 | H | 2-Methyl-6-tert.-butylphenyl |
| 173 | H | 2-Methyl-4-iso-propylphenyl |
| 174 | H | 2-Methyl-4-cyclohexylphenyl |
| 175 | H | 2-Methyl-4-phenylphenyl |
| 177 | H | 2-Methyl-4-phenoxyphenyl |
| 178 | H | 2-Methyl-4-benzyloxyphenyl |
| 179 | H | 2-Methyl-3-chlorphenyl |
| 180 | H | 2-Methyl-4-chlorphenyl |
| 181 | H | 2-Methyl-5-chlorphenyl |
| 182 | H | 2-Methyl-6-chlorphenyl |
| 183 | H | 2-Methyl-4-fluorphenyl |
| 184 | H | 2-Methyl-3-bromphenyl |
| 185 | H | 2-Methyl-4-bromphenyl |
| 186 | H | 2-Methyl-3-methoxyphenyl |
| 187 | H | 2-Methyl-4-methoxyphenyl |
| 188 | H | 2-Methyl-5-methoxyphenyl |
| 189 | H | 2-Methyl-6-methoxyphenyl |
| 190 | H | 2-Methyl-4-iso-propoxyphenyl |
| 191 | H | 2-Methyl-2,5-dimethoxyphenyl |
| 192 | H | 2-Methoxyphenyl |
| 193 | H | 3-Methoxyphenyl |
| 194 | H | 4-Methoxyphenyl |
| 195 | H | 2,3-Dimethoxyphenyl |
| 196 | H | 2,4-Dimethoxyphenyl |
| 197 | H | 2,5-Dimethoxyphenyl |
| 198 | H | 2,6-Dimethoxyphenyl |
| 199 | H | 3,4-Dimethoxyphenyl |
| 200 | H | 3,5-Dimethoxyphenyl |
| 201 | H | 3,6-Dimethoxyphenyl |
| 202 | H | 2,3,4-Trimethoxyphenyl |
| 203 | H | 2,3,5-Trimethoxyphenyl |
| 204 | H | 2,3,6-Trimethoxyphenyl |
| 205 | H | 2,4,5-Trimethoxyphenyl |
| 206 | H | 2,4,6-Trimethoxyphenyl |
| 207 | H | 3,4,5-Trimethoxyphenyl |
| 208 | H | 2-Ethoxyphenyl |
| 209 | H | 3-Ethoxyphenyl |
| 210 | H | 4-Ethoxyphenyl |
| 211 | H | 2-iso-Propoxyphenyl |
| 212 | H | 3-iso-Propoxyphenyl |
| 213 | H | 4-iso-Propoxyphenyl |
| 214 | H | 3-tert.-Butoxyphenyl |
| 215 | H | 4-tert.-Butoxyphenyl |
| 216 | H | 2-Trifluormethoxyphenyl |
| 217 | H | 3-Trifluormethoxyphenyl |
| 218 | H | 4-Trifluormethoxyphenyl |
| 219 | H | 3-(1',1',2',2'-Tetrafluor)ethoxyphenyl |
| 220 | H | 4-(1',1',2',2'-Tetrafluor)ethoxyphenyl |
| 221 | H | 2-Chlormethylphenyl |
| 222 | H | 3-Chlormethylphenyl |
| 223 | H | 4-Chlormethylphenyl |
| 224 | H | 2-Trifluormethylphenyl |
| 225 | H | 3-Trifluormethylphenyl |
| 226 | H | 4-Trifluormethylphenyl |
| 227 | H | 2-(Methoxyiminomethyl)phenyl |
| 228 | H | 3-(Methoxyiminomethyl)phenyl |
| 229 | H | 4-(Methoxyiminomethyl)phenyl |
| 230 | H | 2-(Ethoxyiminomethyl)phenyl |
| 231 | H | 3-(Ethoxyiminomethyl)phenyl |
| 232 | H | 4-(Ethoxyiminomethyl)phenyl |
| 233 | H | 2-(n-Propoxyiminomethyl)phenyl |
| 234 | H | 3-(n-Propoxyiminomethyl)phenyl |
| 235 | H | 4-(n-Propoxyiminomethyl)phenyl |
| 236 | H | 2-(iso-Propoxyiminomethyl)phenyl |
| 237 | H | 3-(iso-Propoxyiminomethyl)phenyl |
| 238 | H | 4-(iso-Propoxyiminomethyl)phenyl |
| 239 | H | 2-(n-Butoxyiminomethyl)phenyl |
| 240 | H | 3-(n-Butoxyiminomethyl)phenyl |
| 241 | H | 4-(n-Butoxyiminomethyl)phenyl |
| 242 | H | 2-(iso-Butoxyiminomethyl)phenyl |
| 243 | H | 3-(iso-Butoxyiminomethyl)phenyl |
| 244 | H | 4-(iso-Butoxyiminomethyl)phenyl |
| 245 | H | 2-(tert.-Butoxyiminomethyl)phenyl |
| 246 | H | 3-(tert.-Butoxyiminomethyl)phenyl |
| 247 | H | 4-(tert.-Butoxyiminomethyl)phenyl |
| 248 | H | 2-(n-Pentoxyimonomethyl)phenyl |
| 249 | H | 3-(n-Pentoxyimonomethyl)phenyl |
| 250 | H | 4-(n-Pentoxyimonomethyl)phenyl |
| 251 | H | 2-(n-Hexoxyimonomethyl)phenyl |
| 252 | H | 3-(n-Hexoxyimonomethyl)phenyl |
| 253 | H | 4-(n-Hexoxyimonomethyl)phenyl |
| 260 | H | 2-(Methoxyimino-1'-ethyl)phenyl |
| 261 | H | 3-(Methoxyimino-1'-ethyl)phenyl |
| 262 | H | 4-(Methoxyimino-1'-ethyl)phenyl |
| 263 | H | 2-(Ethoxyimino-1'-ethyl)phenyl |
| 264 | H | 3-(Ethoxyimino-1'-ethyl)phenyl |
| 265 | H | 4-(Ethoxyimino-1'-ethyl)phenyl |
| 266 | H | 2-(n-Propoxyimino-1'-ethyl)phenyl |
| 267 | H | 3-(n-Propoxyimino-1'-ethyl)phenyl |
| 268 | H | 4-(n-Propoxyimino-1'-ethyl)phenyl |
| 269 | H | 2-(n-Butoxyamino-1'-ethyl)phenyl |
| 270 | H | 3-(n-Butoxyamino-1'-ethyl)phenyl |
| 271 | H | 4-(n-Butoxyamino-1'-ethyl)phenyl |
| 272 | H | 2-(n-Pentoxyimino-1'-ethyl)phenyl |
| 273 | H | 3-(n-Pentoxyimino-1'-ethyl)phenyl |
| 274 | H | 4-(n-Pentoxyimino-1'-ethyl)phenyl |
| 275 | H | 2-(n-Hexoxyimino-1'-ethyl)phenyl |
| 276 | H | 3-(n-Hexoxyimino-1'-ethyl)phenyl |
| 277 | H | 4-(n-Hexoxyimino-1'-ethyl)phenyl |
| 284 | H | 2-Phenylphenyl |
| 285 | H | 3-Phenylphenyl |
| 286 | H | 4-Phenylphenyl |
| 287 | H | 2-Phenoxyphenyl |
| 288 | H | 3-Phenoxyphenyl |
| 289 | H | 4-Phenoxyphenyl |
| 293 | H | 4-(Imidazol-1'-yl)phenyl |
| 294 | H | 4-(Piperazin-1'-yl)phenyl |
| 295 | H | 4-(Morpholin-1'-yl)phenyl |
| 296 | H | 4-(Piperidin-1'-yl)phenyl |
| 297 | H | 4-(Pyridyl-2'-oxy)phenyl |
| 298 | H | 2-Cyclopropylphenyl |
| 299 | H | 3-Cyclopropylphenyl |
| 300 | H | 4-Cyclopropylphenyl |
| 301 | H | 3-Cyclohexylphenyl |
| 302 | H | 4-Cyclohexylphenyl |
| 303 | H | 4-Oxiranylphenyl |
| 304 | H | 4-(1',3'-Dioxan-2'-yl)phenyl |
| 305 | H | 4-(Tetrahydropyran-2yloxy)phenyl |
| 306 | H | 1-Naphthyl |
| 307 | H | 2-Naphthyl |
| 308 | H | 9-Anthryl |
| 324 | H | Benzyl |
| 325 | H | 2-Methylbenzyl |
| 326 | H | 3-Methylbenzyl |
| 327 | H | 4-Methylbenzyl |
| 328 | H | 4-tert.-Butylbenzyl |
| 329 | H | 2-Chlorbenzyl |
| 330 | H | 3-Chlorbenzyl |
| 331 | H | 4-Chlorbenzyl |
| 332 | H | 2,4-Dichlorbenzyl |
| 333 | H | 2,6-Dichlorbenzyl |
| 334 | H | 2,4,6-Trichlorbenzyl |
| 335 | H | 2-Trifluormethylbenzyl |
| 336 | H | 3-Trifluormethylbenzyl |
| 337 | H | 4-Trifluormethylbenzyl |
| 338 | H | 2-Methoxybenzyl |
| 339 | H | 4-Methoxybenzyl |
| 340 | H | 4-tert.-Butoxybenzyl |
| 341 | H | 4-Phenoxybenzyl |
| 342 | H | 1-Phenethyl |
| 343 | H | 2-Phenethyl |
| 344 | H | 1-Phenylpropyl |
| 345 | H | 2-Phenylpropyl |
| 346 | H | 3-Phenylpropyl |
| 347 | H | 2-Methyl-2-phenylpropyl |
| 348 | H | 2-Methyl-3-phenylpropyl |
| 349 | H | 4-Phenylbutyl |
| 350 | H | 2-Phenyl-1-ethenyl |
| 351 | H | 1-Phenyl-1-ethenyl |
| 352 | H | 1-Phenyl-1-propenyl |
| 353 | H | 1-Phenyl-1-propen-2-yl |
| 356 | H | 2-Pyridyl |
| 357 | H | 3-Pyridyl |
| 358 | H | 4-Pyridyl |
| 359 | H | 2,6-Pyrimidinyl |
| 360 | H | 1,5-Pyrimidinyl |
| 361 | H | 2-Thienyl |
| 362 | H | 3-Thienyl |
| 363 | H | 2-Furyl |
| 364 | H | 3-Furyl |
| 365 | H | 1-Pyrrolyl |
| 366 | H | 1-Imidazolyl |
| 367 | H | 1,2,4-Triazolyl |
| 368 | H | 1,3,4-Triazolyl |
| 369 | H | 4-Thiazolyl |
| 370 | H | 2-Benzothiazolyl |
| 377 | H | 2-Pyridylmethyl |
| 378 | H | 3-Pyridylmethyl |
| 384 | H | 2'-Furyl-2-ethenyl |
| 385 | H | 2'-Thienyl-2-ethenyl |
| 386 | H | 3'-Pyridyl-2-ethenyl |
| 411 | H | Cyclopropyl |
| 412 | H | Cyclobutyl |
| 413 | H | Cyclopentyl |
| 414 | H | Cyclohexyl |
| 423 | H | Ethenyl |
| 424 | H | 1-Propenyl |
| 425 | H | 2-Methyl-1-propenyl |
| 426 | H | 4-Methylpent-3-en-1-yl |
| 427 | H | 2-Propenyl |
| 428 | H | 2-Butenyl |
| 429 | H | 1-Methyl-2-propenyl |
| 430 | H | 3-Methyl-2-butenyl |

bedeuten und Verbindungen der Formel Ib 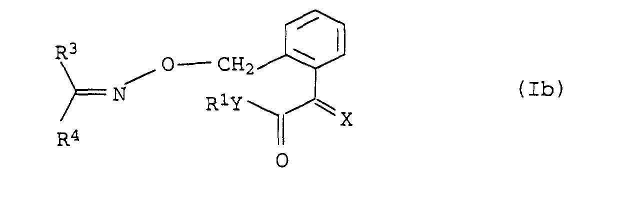 in denen

| Nr. | X | Y | R¹ | R³ | R⁴ |
|---|---|---|---|---|---|
| 5 | CHS-CH₃ | O | CH₃ | Cyclopropyl | 4-tert.-Butylphenyl |
| 25 | CHS-CH₃ | O | CH₃ | CH₃ | Benzyl |
| 26 | CHS-CH₃ | O | CH₃ | CH₃ | tert.-Butyl |
| 27 | CHS-CH₃ | O | CH₃ | CH₃ | iso-Propyl |
| 28 | CHS-CH₃ | O | CH₃ | CH₃ | 2-Methylbutyl |
| 29 | CHS-CH₃ | O | CH₃ | CH₃ | iso-Butyl |
| 30 | CHS-CH₃ | O | CH₃ | CH₃ | Phenyl |
| 36 | CHS-CH₃ | O | CH₃ | iso-Butyl | iso-Butyl |
| 38 | CHS-CH₃ | O | CH₃ | CH₂CH₂CH₂CH₂ | |
| 39 | CHS-CH₃ | O | CH₃ | CH₂CH₂CH₂CH₂CH₂ | |
| 196 | N-OCH₃ | O | C₂H₅ | CH₃ | Phenyl |
| 198 | N-OCH₃ | O | n-C₃H₅ | CH₃ | Phenyl |
| 205 | CHS-CH₃ | O | CH₃ | H | Phenyl |

bedeuten, ausgeschlossen sind,
b)
- X: CH-C₁-C₄-Alkoxy,
- Y: Sauerstoff oder Schwefel
- R¹, R²: unabhängig voneinander Wasserstoff oder C₁-C₄-Alkyl,
- Z¹, Z²: unabhängig voneinander Wasserstoff, Halogen, Methyl, Methoxy oder Cyano,
- R⁴: durch C₁-C₁₀-Alkoximino-C₁-C₂-alkyl substituiertes Phenyl und
- R³: Wasserstoff, Cyano, ggf. verzweigtes C₁-C₁₀-Alkyl, C₁-C₄-Halogenalkyl, C₃-C₆-Cycloalkyl, C₃-C₆-Halogencycloalkyl, C₃-C₆-Cycloalkyl-C₁-C₄-alkyl, C₁-C₄-Alkoxy-C₁-C₄-alkyl, C₁-C₄-Alkylthio-C₁-C₄-alkyl, Arylthio-C₁-C₄-alkyl, C₂-C₆-Alkenyl, C₂-C₅-Halogenalkenyl, C₃-C₆-Cycloalkenyl, C₃-C₆-Halogencycloalkenyl, C₂-C₆-Alkinyl, C₁-C₆-Alkoxy, C₁-C₆-Halogenalkoxy, C₁-C₄-Alkylthio, Benzylthio, C₁-C₄-Alkylcarbonyl, C₁-C₄-Alkoxycarbonyl, ggf. subst. Phenylcarbonyl, ggf. subst. Benzylcarbonyl, ggf. subst. Phenoxycarbonyl, ggf. subst. Benzyloxycarbonyl, ggf. subst. Aryl, ggf. subst. Aryloxy, ggf. subst. Arylthio, ggf. subst.
Aryl-C₁-C₄-alkyl, ggf. subst. Aryl-C₂-C₄alkenyl, ggf. subst. Aryloxy-C₁-C₄-alkyl, ggf. subst. Arylthio-C₁-C₄-alkyl, ggf. subst. Hetaryl, ggf. subst. Hetaryloxy, ggf. subst. Hetarylthio, ggf. subst. Hetaryl-C₁-C₄-alkyl, ggf. subst. Hetaryl-C₂-C₄-alkenyl, ggf. subst. Hetaryl-C₁-C₄-alkoxy, ggf. subst. Heterocyclyl, ggf. subst. Heterocyclyloxy,
N(R⁶)₂, wobei die Bedeutungen von R⁶ gleich oder verschieden sind oder
CON(R⁷)₂, wobei die Bedeutungen von R⁷ gleich oder verschieden sind,
- R⁶: Wasserstoff, C₁-C₆-Alkyl oder ggf. subst. Phenyl,
- R⁷: Wasserstoff oder C₁-C₄-Alkyl,
- ggf.: subst. neben Wasserstoff die Reste Halogen, Cyano, Nitro, C₁-C₄-Alkyl, C₁-C₄-Alkoxy, C₁-C₄-Halogenalkyl, C₁-C₄-Halogenalkoxy, C₁-C₁₀-Alkoximino-C₁-C₂-alkyl, Aryl, Arloxy, Benzyloxy, Hetaryl, Hetaryloxy, C₃-C₆-Cycloalkyl, Heterocyclyl oder Heterocyclyloxy,
eine ausgezeichnete fungizide, insektizide, nematizide und akarizide Wirkung haben, die besser ist, als die der bekannten Phenylessigsäurederivate.

Die fungizide Wirkung wird bevorzugt.
Die in der allgemeinen Formel I aufgeführten Reste können beispielsweise folgende Bedeutung haben:
X
   kann C₁-C₄-Alkyliden (z.B. Ethyliden, n- oder iso-Propyliden, n-, iso-, sec.- oder tert.-Butyliden), C₁-C₄-Alkoxymethyliden (z.B. Methoxy-, Ethoxy-, n- oder iso-Propoxy, n-, iso-, sec.- oder tert.-Butoxymethyliden), C₁-C₄-Alkylthiomethyliden (z.B. Methyl-, Ethyl-, n- oder iso-Propyl, n-, iso-, sec.- oder tert.-Butylthiomethyliden), C₁-C₄-Alkoxyimino (z.B. Methoxy-. Ethoxy-, n-oder iso-Propoxy-, n-, iso-, sec.- oder tert.-Butoxyimino),
Y
   kann O, S, NR⁵,
R¹, R², R⁵
   können H oder C₁-C₄-Alkyl (z.B. Methyl, Ethyl, n- oder iso-Propyl, n-, iso-, sec.- oder tert.-Butyl)
Z¹, Z²
   können H, Halogen (z.B. Fluor, Chlor, Brom, Jod), Methyl, Methoxy, Cyano
R³, R⁴
   können gleich oder verschieden sein und wasserstoff, Cyano, ggf. verzweigtes C₁-C₁₀-Alkyl (z.B. Methyl, Ethyl, n-oder iso-Propyl, n-, iso-, sec.- oder tert.-Butyl, n-, iso-, sec.-, tert.- oder neo-Pentyl, n-Hexyl, n-Decyl)
   C₁-C₄-Halogenalkyl (z.B. Trifluormethyl, 2-Fluorethyl, 2,2,2-Trifluorethyl, Pentafluorethyl, Fluordichlormethyl, Difluorchlormethyl, Chlormethyl, Dichlormethyl, Trichlormethyl, 2-Chlorethyl, 2,2,2-Trichlorethyl, Pentachlorethyl),
   C₃-C₆-Cycloalkyl (z.B. Cyclopropyl, Cyclobutyl, Cyclopentyl, Cyclohexyl),
   C₃-C₆-Halogencycloalkyl (z.B. 2,2-Difluorcyclopropyl, 2,2-Dichlorcyclopropyl, 2,2-Dibromcyclopropyl, 2,2-Dichlor-3-methylcyclopropyl, Tetrafluorcyclobutyl),
   C₃-C₆-Cycloalkyl-C₁-C₄-alkyl (z.B. 1-Methylcyclopropyl, 2,2-Dimethylcyclopropyl, 1-Methylcyclohexyl),
   C₁-C₄-Alkoxy-C₁-C₄-alkyl (z.B. Methoxymethyl, Ethoxymethyl, n- oder iso-Propoxylmethyl, n-, iso-, sec.- oder tert.-Butoxymethyl, 2-Methoxyprop-2-yl, 2-Ethoxyprop-2-yl, 2-n- oder iso-Propoxyprop-2-yl, 2-n-, iso-, sec.- oder tert.-Butoxy-prop-2-yl),
   C₁-C₄-Alkylthio-C₁-C₄-alkyl (z.B. Methylthiomethyl, Ethylthiomethyl, n-, oder iso-Propylthiomethyl, n-, iso-, sec.oder tert.-Butylthiomethyl, 2-Methylthioprop-2-yl, 2-Ethylthioprop-2-yl, 2- n- oder iso-Propylthio-prop-2-yl, 2-n-, iso-, sec.- oder tert.-Butylthio-prop-2-yl),
   Aryl(Phenyl)thio-C₁-C₄-alkyl (z.B. Phenylthiomethyl, 2-Chlorphenylthiomethyl)
   C₂-C₆-Alkenyl (z.B. Vinyl, 1-Propenyl, 2-Propenyl, 2-Butenyl, 3-Butenyl, 1-Methyl-2-propenyl, 3-Methyl-2-butenyl, 2-Methyl-2-penten-5-yl),
   C₂-C₅-Halogenalkenyl (z.B. 2,2-Difluorvinyl, 2,2-Dichlorvinyl, 3,3,3-Trifluorpropenyl, 3,3,3-Trichlorpropenyl, 3-Chlor-2-propenyl),
   C₃-C₆-Cycloalkenyl (z.B. Cydopent-1-enyl, Cyclopentadienyl, Cyclohex-1-enyl),
   C₃-C₆-Halogencycloalkenyl (z.B. Pentafluorcyclopentadienyl, Pentachlorcyclopentadienyl),
   C₂-C₄-Alkinyl (z.B. Ethinyl, 1-Propinyl, 1-Propargyl), C₁-C₄-Alkoxy (z.B. Methoxy, Ethoxy, n- oder iso-Propoxy, n-, iso-, sec.- oder tert.-Butoxy),
   C₁-C₄-Alkylthio (z.B. Methylthio, Ethylthio, n- oder iso-Propylthio, n-, iso-, sec.- oder tert.-Butylthio), Benzylthio,
   C₁-C₄-Halogenalkoxy (z.B. Trifluormethoxy, Pentafluorethoxy, 1,1,2,2-Tetrafluorethoxy),
   NR⁶₂ (z.B. Amino, Methylamino, Dimethylamino, Ethylamino, Diethylamino, Di-n-propylamino, Di-iso-propylamino, Di-n-butylamino, Di-iso-butylamino),
   C₁-C₄-Alkylcarbonyl (z.B. Acetyl, Propionyl, Butyryl, iso-Butyryl, Pivaloyl),
   ggf. subst. Phenylcarbonyl (z.B. Benzoyl, 4-Chlorbenzoyl),
   ggf. subst. Benzylcarbonyl (z.B. Benzylcarbonyl),
   C₁-C₄-Alkoxycarbonyl (z.b. Methoxycarbonyl, Ethoxycarbonyl, n- oder iso-Propoxycarbonyl, n-, iso-, sec.- oder tert.-Butoxycarbonyl),
   ggf. subst. Phenoxycarbonyl (z.B. Phenoxycarbonyl, 4-Chlorphenoxycarbonyl),
   ggf. subst. Benzyloxycarbonyl (z.B. Benzyloxycarbonyl),
   -CO-N(R⁷) ₂ (z.B. Aminocarbonyl, Dimethylaminocarbonyl, Diethylaminocarbonyl, Di-iso-propylaminocarbonyl, Phenylaminocarbonyl, N-Methyl-N-Phenylaminocarbonyl),
   ggf. subst. Aryl (z.B. Phenyl, Naphthyl, Anthryl),
   ggf. subst. Aryloxy (z.B. Phenoxy, Naphthoxy, Anthroxy),
   ggf. subst. Arylthio (z.B. Phenylthio),
   ggf. subst. Aryl-C₁-C₄-alkyl (z.B. Benzyl, 1-Phenethyl, 2-Phenethyl, 1-Phenylpropyl, 2-Phenylpropyl, 3-Phenylpropyl, 2-Methyl-3-phenylpropyl, 2-Methyl-2-phenylpropyl, 4-Phenylbutyl),
   ggf. subst. Aryl-C₂-C₄-alkenyl (z.B. Phenyl-1-ethenyl, 2-Phenyl-1-propenyl, 2,2-Diphenylethenyl, 1-Phenyl-1-propen-2-yl, 1-Phenyl-1-ethenyl),
   ggf. subst. Aryloxy-C₁-C₄-alkyl (z.B. Phenoxymethyl),
   ggf. subst. Arylthio-C₁-C₄-alkyl (z.B. Phenylthiomethyl),
   ggf. subst. Hetaryl (z.B. Pyridyl, 2-Pyridyl, 3-Pyridyl, 4-Pyridyl, Pyrimidinyl, 4-Pyrimidinyl, 2-Pyrimidinyl, Thienyl, 2-Thienyl, 3-Thienyl, Furyl, 2-Furyl, 3-Furyl, 1-Pyrrolyl, 1-Imidazolyl, 1,2,4-Triazolyl, 1,3,4-Triazolyl, 4-Thiazolyl, 2-Benzothiazolyl),
   ggf. subst. Hetaryloxy (z.B. 2-Pyridyloxy, 2-Pyrimidinyloxyl),
   ggf. subst. Hetarylthio (z.B. 2-Pyridylthio, 2-Pyrimidinylthio, 2-Benzothiazolylthio),
   ggf. subst. Hetaryl-C₁-C₄-alkyl (z.B. 2-Pyridylmethyl, 3-Pyridylmethyl),
   ggf. subst. Hetaryl-C₁-C₄-alkoxy (z.B. Furfurylmethoxy, Thienylmethoxy, 3-Isoxazolylmethoxy, 2-Oxazolylmethoxy, 2-Pyridylmethoxy),
   ggf. subst. Hetaryl-C₂-C₄-alkenyl (z.B. 2'-Furyl-2-ethenyl, 2'-Thienyl-2-ethenyl, 3'-Pyridyl-2-ethenyl),
   ggf. subst. Heterocyclyl (z.B. Oxiranyl, 1-Aziridinyl, 1-Azetidinyl, 1-Pyrrolidinyl, 2-Tetrahydrofuryl, 2-Tetrahydropyranyl, 3-Tetrahydropyranyl, 1-Piperidinyl, 1-Morpholinyl, 1-Piperazinyl, 1,3-Dioxanyl, 3-Tetrahydrothiopyranyl),
   ggf. subst. Heterocycloxy (z.B. 2-Dihydropyranyloxy, 2-Tetrahydropyranyloxy).

Die mit "ggf. substituiert" bezeichneten Reste im Vorstehenden sind neben Wasserstoff z.B. Fluor, Chlor, Brom, Jod, Cyano, Nitro, Methyl, Ethyl, iso-Propyl, tert.-Butyl, Methoxy, Ethoxy, iso-Propoxy, tert.-Butoxy, Trifluormethyl, Chlormethyl, Dichlormethyl, Trichlormethyl, Trifluormethoxy, 1,1,2,2-Tetrafluorethoxy, Methoxyiminomethyl, Ethoxyiminomethyl, n-Propoxyiminomethyl, n-Butoxyiminomethyl, n-Pentoxyiminomethyl, n-Hexoxyiminomethyl, Allyloxyiminomethyl, Benzyloxyiminomethyl, iso-Propoxyiminomethyl, iso-Butoxyiminomethyl, tert.-Butoxyiminomethyl, Methylimino-1-ethyl, Ethoxyimino-1-ethyl, n-Propoxyimino-1-ethyl, n-Butoxyimino-1-ethyl, n-Pentoxyimino-1-ethyl, n-Hexoxyimino-1-ethyl, Allyloxyimino-1-ethyl, Benzyloxyimono-1-ethyl, Phenyl, Phenoxy, Benzyloxy, Imidazol-1-yl, Piperazin-1-yl, 4-Morpholinyl, Piperidin-1-yl, Pyridyl-2-oxy, Cyclopropyl, Cyclohexyl, Oxiranyl, 1,3-Dioxan-2-yl, 1,3-Dioxolan-2-yl, Tetrahydropyran-2-yloxy.

Ebenso kann einer der Reste R³ oder R⁴ Halogen (z.B. Fluor, Chlor, Brom, Jod) sein.

Von den Verbindungen mit R³, R⁴ Wasserstoff werden die Verbindungen bevorzugt, in denen entweder nur R³ oder nur R⁴ Wasserstoff ist, insbesondere die Verbindungen, in denen R³ Wasserstoff ist.

Bevorzugt werden außerdem Verbindungen der Formel I, in der
X
   CHCH₃, CHC₂H₅, CHOCH₃, CHOC₂H₅, CHSCH₃, CHSC₂H₅
Y
   O
R¹
   C₁-C₄-Alkyl,
R²
   H, Methyl
R³, R⁴
   gleich oder verschieden sind und Wasserstoff Cyano, ggf. verzweigtes C₁-C₁₀-Alkyl, C₁-C₄-Halogenalkyl, C₃-C₆-Cycloalkyl, C₃-C₆-Halogencycloalxyl,
   C₃-C₆-Cycloalkyl-C₁-C₄-alkyl,
   C₁-C₄-Alkoxy-C₁-C₄-alkyl,
   C₁-C₄-Alkylthio-C₁-C₄-alkyl, Arylthio-C₁-C₄-alkyl,
   C₂-C₆-Alkenyl, C₂-C₆-Alkinyl, C₂-C₄-Halogenalkenyl,
   C₁-C₆-Alkoxy, C₁-C₆-Alkylthio, C₁-C₄-Alkylcarbonyl,
   C₁-C₄-Alkoxycarbonyl,
   ggf. subst. Aryl, ggf. subst. Aryloxy, ggf. subst. Arylthio, ggf. subst. Aryl-C₁-C₄-alkyl, ggf. subst. Aryl-C₂-C₄-alkenyl, ggf. subst. Aryloxy-C₁-C₄-alkyl, ggf. subst. Hetaryl, ggf. subst. Hetaryloxy, ggf. subst. Hetarylthio, ggf. subst. Heterocyclyl bedeuten,
   N(R⁶)₂, wobei R⁶ gleich oder verschieden sind und H, C₁-C₆-Alkyl, ggf. subst. Phenyl,
   -CO-N(R⁷)₂, wobei R⁷ gleich oder verschieden sind und H, C₁-C₄-Alkyl bedeuten;
   wobei "ggf. subst." die in Anspruch 1 angegebenen Reste bedeutet,
   R³ und R⁴ zusammen einen carbo- oder heterocyclischen Ring bilden können, der durch die unter "ggf. subst." genannten Reste substituiert sein kann,
   R³ oder R⁴ Halogen sein können und
   Z¹, Z² Wasserstoff, Fluor, Chlor, Brom, Jod, Methyl, Cyano oder Methoxy bedeuten.

Die neuen Verbindungen der allgemeinen Formel I können bei der Herstellung aufgrund der C=C- bzw. C=N-Doppelbindungen als E/Z-Isomerengemische anfallen. Diese können in der üblichen Weise, z.B. durch Kristallisation oder Chromatographie in die einzelnen Komponenten aufgetrennt werden. Sowohl die einzelnen isomeren Verbindungen als auch ihre Gemische werden von der Erfindung umfaßt und sind als Schädlingsbekämpfungsmittel brauchbar.

Die Herstellung der Verbindungen der allgemeinen Formel I gemäß Anspruch 1 erfolgt beispielsweise wie in Schema 1 beschrieben (Z¹, Z² gleich H).

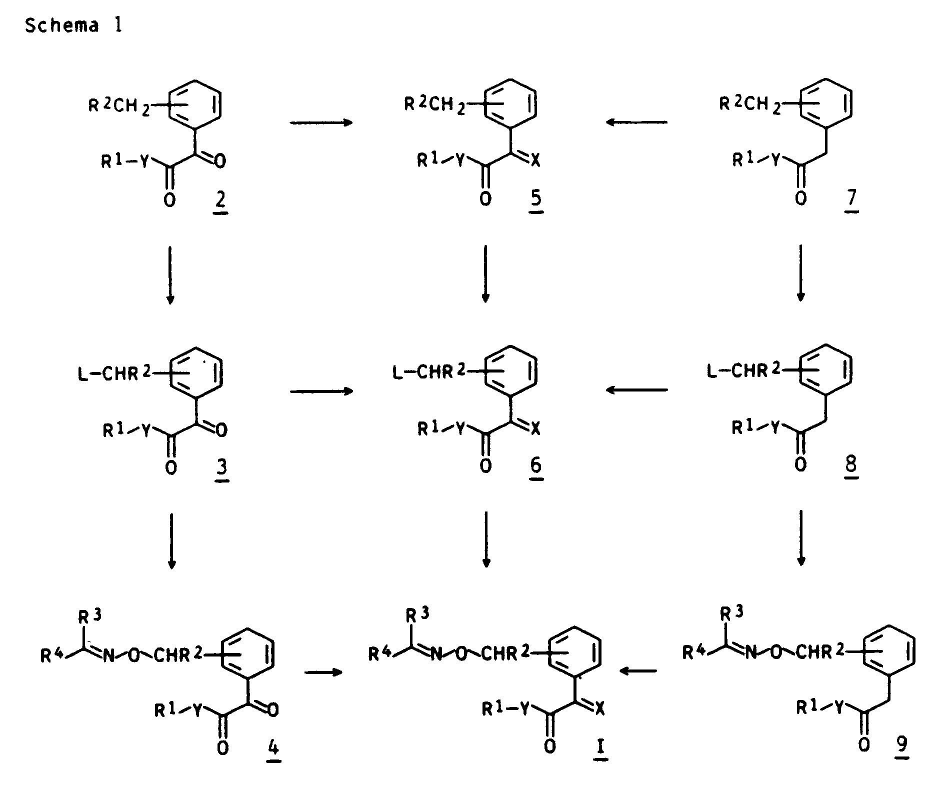

Die Verbindungen der allgemeinen Formel I, in denen X=CH-Alkyl, CH-Alkoxy ist, lassen sich beispielsweise aus den Ketoestern 4 durch Wittig- oder Wittig-Horner-Reaktion herstellen (vgl. EP 348 766, DE 3 705 389, EP 178 826). Ebenso erhält man die analogen Verbindungen 5 aus den Ketoestern 2.

Alternativ kann auch so vorgegangen werden, daß man Verbindungen der Formel 7, bzw. 9 mit geeigneten Reagentien kondensiert, z.B. für X=CH-Alkyl a) mit Aldehyden (vgl. D.M.Brown J. Chem. Soc. 1948, 2147) oder b) zuerst mit N,N-Dimethylformamiddimethylacetal, gefolgt von der Reaktion mit einem Grignardreagenz (analog zu C. Jutz Chem. Ber. 91, 1867 (1958)), für X=CH-O-Alkyl mit Ameisensäureester gefolgt von einer Alkylierung (s. EP 178826). Weitere Herstellvorschriften für die Verbindungen der Formel 5 und I mit X=CH-O-Alkyl sind beschrieben in EP 178 826.

Eine weitere Möglichkeit zur Herstellung der Verbindungen der Formel I mit X=CH-Alkyl und YR¹=OAlkyl ist die Umsetzung von Ketenacetalen mit Phenylchlorcarbenen (s. N. Slougui, G. Rousseau, Synth. Commun. 12 (5) 401-7 (1982)).

Für Verbindungen der allgemeinen Formel I in der X=CH-S-Alkyl ist, kann die Herstellung nach den Methoden aus EP 244077 oder 310954 erfolgen.

Die Zwischenprodukte der Formeln 3, 6 und 8 lassen sich aus den Verbindungen 2, 5 und 7 herstellen, indem man diese nach bekannten Methoden halogeniert, z.B. mit Chlor, Brom, N-Bromsuccinimid in einem inerten Lösungsmittel (z.B. CCl₄, Cyclohexan) unter Belichtung mit z.B. einer Hg-Dampflampe oder Radikalstartern, wie z.B. Dibenzoylperoxid, oder indem man über geeignete Zwischenverbindungen (L=Halogen, OH) die Reste L wie z.B. Mesylat, Tosylat, Acetat oder Triflat einführt.

Die Oximether der Formel I mit X = N-OAlkyl lassen sich aus 4 a) durch Umsetzung mit O-Alkylhydroxylaminhydrochlorid oder b) mit Hydroxylaminhydrochlorid und nachfolgender Alkylierung mit einem Alkylierungsmittel (wie z.B. Alkyliodid, Dialkylsulfat etc.) herstellen (vgl. DE 3 623 921).

Ebenso kann analog zur Methode im EP 254 426 ein Phenylessigester der Formel 9 mit einer Base (wie z.B. NaOMe, NaH, K-tert. Butylat, etc.) in einem Lösungsmittel (wie z.B. Diethylether, Toluol, tert.-Butanol etc.) in sein Anion überführt und mit einem geeigneten Nitrosierungsmittel (wie z.B. Methylnitrit, Amylnitrit, tert.-Butylnitrit etc.) oximiert werden. Das resultierende Oximat wird mit einem Alkylierungsmittel (wie z.B. Alkyliodid, Dialkylsulfat) alkyliert.

Dieselben Verfahren sind entsprechend auch auf die Verbindungen der Formel 2 und 7 übertragbar, wobei die resultierenden Oximether 5 wie bekannt EP 254 426) über die Intermediate 6 (L=z.B. Halogen) in die Zielverbindungen I überführt werden können.

Üblicherweise ist bei den vorbeschriebenen Herstellungsverfahren der Rest Y-R¹ = Alkoxy.

Die Verbindungen mit YR¹=OH (11) lassen sich nach literaturbekannten Methoden (Organikum 16. Auflage, S. 415, 622) aus den Verbindungen der allgemeinen Formel I, in der YR¹=OAlkyl (10) ist, herstellen (s. Schema 2):

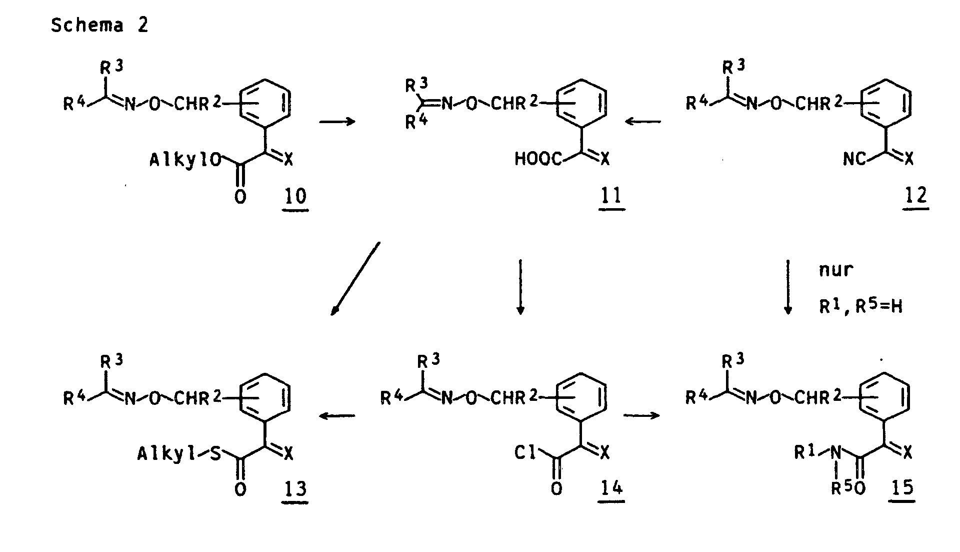

Alternativ dazu können die Nitrile 12 in bekannter Weise (vgl. Organikum 16. Auflage, S. 424f (1985)) in die Carbonsäuren 11 umgewandelt werden.

Aus den so erhalten Carbonsäuren 11 lassen sich in an sich bekannter Weise die Säurechloride 14 herstellen (vgl. Organikum 16. Auflage, S. 423f B. (1985)). Die Umwandlung von 14 in die Amide 15 erfolgt analog zu Organikum 16. Auflage S. 412 (1985).

Die Thiolester 13 erhält man aus den Säurechloriden 14 (analog zu Houben-Weyl Bd. 8 S. 464ff (1952)).

Alternativ können die Thiolester 13 auch aus den Säuren 11 hergestellt werden (analog zu Houben-Weyl Bd E5 S. 855f (1985)).

Die Herstellung der Amide 15 mit R¹, R⁵=H kann auch nach literaturbekannten Verfahren aus den Nitrilen 12 erfolgen (vgl. dazu Synthesis 1980, 243).

Die Herstellung der Verbindungen der allgemeinen Formeln 2 und 7 mit ortho-Methylsubstitution am Aromaten (R²=H) ist bekannt.

(YR¹=OAlkyl; s. EP 178826, EP 260 832).

Die zur Herstellung der Verbindungen der allgemeinen Formel I benötigten Oxime sind entweder bekannt oder können nach einem der in Schema 3 gezeigten Verfahren hergestellt werden.

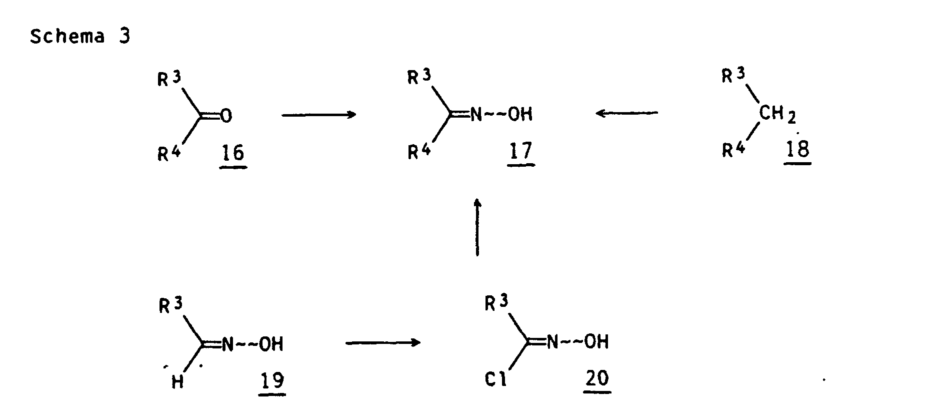

Methoden zur Umsetzung von 16 oder 18 zu 17 findet man in Houben-Weyl, Bd. 10/4 (1968).

Desweiteren können Aldoxime 19 nach bekannten Methoden chloriert und mit z.B. Cyanid zu den Derivaten 17 (R⁴=CN) umgesetzt werden (vgl. M.R. Zimmermann J. f. prakt. Chemie 66, 359 (1902)).

Die speziellen Derivate mit R³=CN, R⁴=Alkoxyalkyl werden nach EP 74047, mit R³=CN, R⁴=Alkylthioalkyl nach EP 150 822 und mit R³=CN, R⁴=Alkyl nach DE 2 304 848 hergestellt.

Für sterisch gehinderte Ketone kommt die Methode mit Me₃SiO-NH-SiMe₃ (R.U. Hoffmann, G.A. Buntain, Synthesis 1987, 831) zur Anwendung.

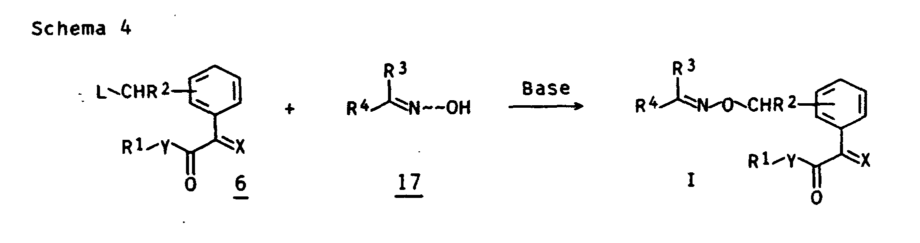

Die Darstellung der neuen Verbindungen der allgemeinen Formel I gemäß Anspruch 1 erfolgt beispielsweise so, daß man ein Oxim der Formel 17 mit einer substituierten Benzylverbindung 6 in der L eine Abgangsgruppe (z.B. Chlorid, Bromid, p-Toluolsulfonat, Methansulfonat, Triflat, Acetat) bedeutet, umsetzt. R¹-R⁴, X und Y haben die oben genannten Bedeutungen.

Die beschriebenen Umsetzungen können z.B. in einem inerten Lösungsmittel (z.B. Aceton, Acetonitril, Dimethylsulfoxid, Dimethylformamid, N-Methylpyrrolidon oder Pyridin) unter Verwendung einer Base (z.B. Natriumcarbonat, Kaliumcarbonat, Natriumhydroxid, Kaliumhydroxid, Natriumhydrid, Natriummethylat) durchgeführt werden.

Die Umsetzungen können auch im Zweiphasensystem (z.B. Dichlormethan, Wasser) unter Zuhilfenahme eines geeigneten Phasentransferkatalysators (z.B. Cetyltrimethylammoniumchlorid, Benzyltrimethylammoniumchlorid) durchgeführt werden.

Einen weiteren Weg zur Herstellung der Verbindungen I (YR¹ = OAlkyl, X = CH-OAlkyl oder N-OAlkyl, L=Cl, Br, Tosylat, Mesylat) illustriert Schema 5:

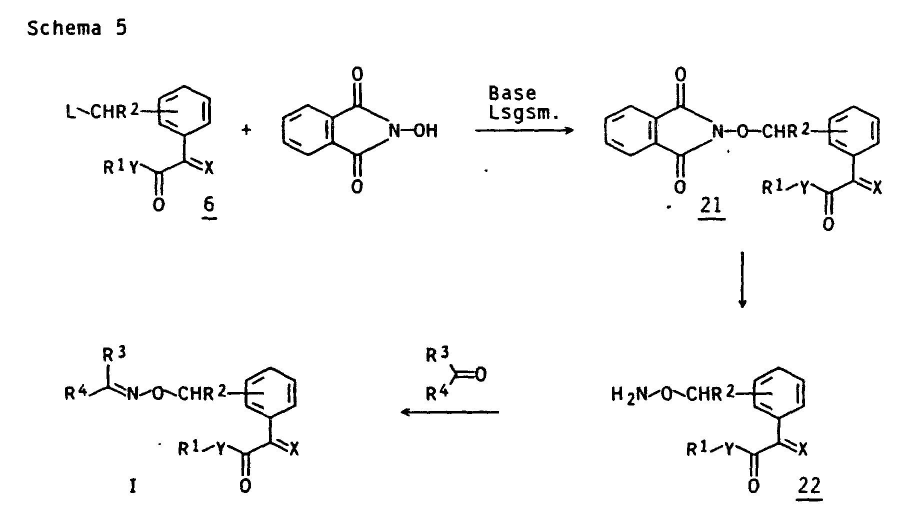

Diese Synthesesequenz erfolgt bis zur Verbindung 22 analog zu den in EP 244 786 beschriebenen Methoden.

So kann man N-Hydroxyphthalimid mit Halogeniden oder Sulfonsäureestern 6 in Gegenwart eines säurebindenden Mittels (z.B. Triethylamin, Kaliumcarbonat, etc.) in einem geeigneten Lösungsmittel (z.B. N-Methylpyrrolidon, DMF etc.) in die Imidether 21 überführen.

Die Spaltung zum O-substituierten Hydroxylamin 22 kann mit Mineralsäure (z.B. HCl ; s. Houben-Weyl Bd. 10/1 S. 1181ff.) oder im Basischen (z.B. mit Hydrazin, Ethanolamin) erfolgen.

Die Umsetzung des Amins 22 zu I erfolgt in an sich bekannter Weise (vgl. D. Otzanak J.C.S. Chem. Commun. 1986,903).

Die in den Tabelle I bis VI genannten Verbindungen können in Analogie zu den nachfolgenden Beispielen dargestellt werden (die Vorschriften 1-7 und die Beispiel 1-7 sind nicht erfindungsgemäß)

### Vorschrift 1: 3,4-Dichloracetophenonoxim

12,0 g (0,17 mol) Hydroxylammoniumhydrochlorid und 18,9 g (0,1 mol) 3,4-Dichloracetophenon werden in eine Mischung aus 20 ml H₂O, 100 ml Methanol und 8 g (0,1 mol) Pyridin gegeben. Nach 1 h Kochen am Rückfluß säuert man mit 2 N HCl an und extrahiert mit 3 x 100 ml tert.-Butylmethylether. Die org. Phase wird mit Wasser gewaschen, mit Na₂SO₄ getrocknet und eingeengt. Es fallen 19,6 g (96 % d. Th.) Kristalle mit einem Schmelzpunkt von 92°C an, die laut ¹H-NMR zu 90 % aus trans-Oxim bestehen.

### Vorschrift 2: 2-Oximino-2-tetrahydropyran-2'-yl-acetonitril

265,4 g (2,05 mol) Tetrahydrofuran-2-carbaldehydoxim werden in 2 I Diethylether bei -55 bis -60°C vorgelegt. Anschließend werden 153,5 g (2,15 mol) Chlor eingeleitet und die Temperatur auf -20°C erhöht; 1 h nachrühren, dann am Rotationsverdampfer bei 10°C einengen, den Rückstand in 1,5 I Diethylether aufnehmen und über Nacht unter Lichtausschluß bei Eiskühlung rühren. Danach wird abfiltriert und unter Eiskühlung zu 147,3 g (2,27 mol) Kaliumcyanid in 1 I Methanol bei 10-15°C getropft (exotherm). Nach 5 h Rühren bei Raumtemperatur (20°C) wird der Niederschlag abgesaugt und zweimal mit Diethylether gewaschen. Die org. Phasen werden zwischen tert.-Butylmethylether und Wasser verteilt und der Rückstand der Etherphasen aus Dichtormethan/n-Hexan bei 0°C kristallisiert.

Nach Absaugen und Trocknen verbleiben 208 g (66 % d. Th.) einer spektroskopisch reinen Substanz mit dem Schmelzpunkt Fp.: 105-106°C.

### Vorschrift 3: 3-Methoxy-3-methyl-2-oximino-butyronitril

53,6 g (0,46 mol) 2-Methoxy-2-methyl-propionaldehydoxim werden in Ether (ca. 1 M) bei -5°C bis -10°C vorgelegt. Nach Eingasen von 35,8 g (0,5 mol) Chlor wird 1 h bei dieser Temperatur gerührt, dann bei 10°C eingeengt und der Rückstand in Diethylether aufgenommen. 24,7 (0,5 mol) Natriumcyanid werden bei 10°C im 375 ml Methanol/H₂O 20:1 vorgelegt und die obige etherische Lösung zügig zugetropft. Nach 4 h bei Raumtemperatur wird abgesaugt und mit 2 x 100 ml MeOH gewaschen. Die vereinigten Lösungen engt man ein und unterwirft sie einer Verteilung zwischen Methyl-tert.-butylether und Wasser. Trocknen der organischen Phase (Na₂SO₄), einengen und kristallisieren (Dichlormethan/n-Hexan) führt zu 41,1 g (63 % d. Th.) eines weißen Pulvers mit dem Schmelzpunkt 102-104°C.

### Vorschrift 4:

### 3-Methoxy-2-[2'-(phthalimidooxy)methyl]phenyl-acrylsäure-methylester

10 g (35 mmol) 3-Methoxy-2-(2'-brommethyl)phenyl-acrylsäuremethylester, 5,7 g (35 mmol) Hydroxyphthalimid, 3,9 g (38,6 mmol) Triethylamin und 50 ml N-Methylpyrrolidon werden zusammengegeben und 2 h bei 60°C gerührt. Dann gießt man auf Eiswasser, saugt den Rückstand ab, wäscht ihn mit Wasser und iso-Propanot und trocknet i. Vak.. Es verbleiben 9,0 g (70 % d.Th.) kristallines Produkt mit dem Schmelzpunkt 156-158°C.

¹H-NMR(CDCl₃) : δ = 3.60(s,3H); 3.75(s,3H); 5.12(s,2H); 7.13(dbr,1H); 7.35(m,2H); 7.62(s,1H); 7.7-7.9(m,5H).

### Vorschrift 5:

### 3-Methoxy-2-(2'-aminooxymethyl)phenyl-acrylsäuremethylester

10,0 g (27 mmol) des Produkts aus Vorschrift 4 wird in 150 ml Methanol gelöst und mit 1,4 g Hydrazinhydrat (27 mmol) 2 h bei Raumtemperatur gerührt. Der Niederschlag wird abgesaugt, die Mutterlauge eingeengt, mit Diethylether verrührt, der Niederschlag wiederum abgesaugt und die Mutterlauge eingeengt. Es resultieren 6,0 g (92 % d. Th.) eines gelben öls (Reinheit laut ¹H-NMR ca. 90 %).

¹H-NMR(CDCl₃): δ = 3.71(s,3H); 3.80(s,3H); 4.60(s,2H); 5.35(sbr,2H); 7.0-7.50(m,4H); 7.58(s,1H).

### Vorschrift 6:

### 2-Methobmino-2-(2'-phthalimidooxymethyl)phenylessigsäure-methylester

2,0 g (7 mmol), 2-Methoxyimino-2-(2'-brommethyl)phenylessigsäuremethylester, 1,1 g (7 mmol) Hydroxyphthalimid, 0,8 g (7,7 mmol) Triethylamin werden in 10 ml N-Methylpyrrolidon gelöst und 2 h bei 70°C gerührt. Zur Aufarbeitung wird mit Eiswasser versetzt, die Kristalle abgesaugt, mit Wasser und Methyl-tert.-butylether nachgewaschen und getrocknet.

Es vertreiben 1,5 g (58 % d. Th.) Kristalle mit dem Schmelzpunkt 152-155°C.

¹H-NMR(CDCl₃): δ = 3.83(s,3H); 3.98(s,3H); 5.07(s,2H); 7.15(dbr,1H); 7.45(mc,2H); 7.60-7.85(m,5H)

### Vorschrift 7:

### 2-Methoximino-2-(2'-aminooxymethyl)phenylessigsäure-methylester

15,0 g (41 mmol) Produkt aus Vorschrift 6 werden mit 2,1 g (42 mmol) Hydrazinhydrat in 150 ml Methanol 2 h bei Raumtemperatur gerührt. Der Rückstand wird abgesaugt, die Mutterlauge eingeengt, mit Diethylether verrieben, wiederum der Rückstand abgesaugt, die Mutterlauge eingeengt. Es verbleiben 7,8 g (80 % d. Th.) eines säureempfindlichen öls.

¹H-NMR(CDCl₃): δ = 3.90(s,3H); 4.03(s,3H); 4.59(s,2H); 5.35(sbr,2H); 7.15(dbr,1H); 7.40(sbr,3H)

### Beispiel 1:

### 3-Methoxy-2-[2'[1"-(3''',5'''dichlorphenyl)-1"-methyl]-iminooxymethyl]-phenyl-acrylsäuremethylester (Nr. 582, Tab. I)

0,6 g (25 mmol) Natriumhydridpulver werden in 100 ml Acetonitril vorgelegt, 5,1 g (25 mmol) 3,5-Dichloracetophenoxim zugegeben und 1 h am Rückfluß gekocht. Dann werden 9,3 g (33 mmol) 2-(2'-Brommethyl)phenyl-3-methoxy-acrylsäuremethylester in 50 ml Acetonitril zugetropft und weitere 4 h am Rückfluß gekocht. Nach Einengen i.Vak. wird zwischen Methyl-tert.-butylether und gesätt. Ammoniumchloridlösung verteilt, die org. Phase mit Wasser gewaschen und der durch Einengen gewonnene Rückstand aus Methylbutylether/n-Hexan kristallisiert. Man erhält 4,6 g (45 % d. Th.) einer Substanz mit dem Schmelzpunkt Fp: 87-88°C.

¹H-NMR(CDCl₃): δ = 2.19(s.3H); 3.70(s,3H); 3.81(s,3H); 5.15(s,2H); 7.08(m,1H); 7.17(m,3H); 7.50(m,3H); 7.58(s,1H)

### Beispiel 2:

### 3-Methoxy-2-[2'-(1"-cyano-(-1"-methoxy-1"-methylethyl)-iminooxymethyl]-phenyl-acrylsäuremethylester (Nr. 36, Tab. I)

3,3 g (23 mmol) 3-Methoxy-3-methyl-2-oximino-butyronitril, 6,6 g (23 mmol) 2-(2'-Brommethylphenyl)-3-methoxyacrylsäuremethylester und 3,2 g (23 mmol) Kaliumcarbonat werden in 60 ml N,N-Dimethylformamid 15 h bei Raumtemp. gerührt. Dann engt man ein, nimmt in Essigester auf, wäscht mit 3 x 50 ml Wasser, trocknet die org. Phase über Natriumsulfat, engt ein und chromatographiert an Kieselgel (Toluol/Essigester 40:1).

Ausbeute: 7,0 g (88 % d. Th.) eines Öls.

IR (Film): 1285, 1258, 1189, 1180, 1131, 1111, 1069, 1008 cm⁻¹.

### Beispiel 3:

### 2-Methoximino-2-[2'-(1"-(3''',5'''-dichiorphenyl)-1"-methyl)iminooxymethyl]-phenylessigsäuremethylester (Nr. 582, Tab. II)

0,6 g (25 mmol) NaH werden in 50 ml Acetonitril vorgelegt und bei Raumtemp. 5,1 g (25 mmol) 3,5-Dichloracetophenonoxim zugegeben. Nach 1 h bei Rückfluß werden 9,4 g (33 mmol) 2-Methobmino-2-(2'-brommethyl)phenylessigsäuremethylester in 50 ml Acetonitril zugetropft und weitere 4 h am Rückfluß erhitzt. Nach Einengen, verteilen zwischen Wasser/Methyl-tert.-butylether, waschen der org. Phase mit Wasser, Trocknen der org. Phase mit Natriumsulfat und Einengen wird der Rückstand einer Säulenchromatographie an Kieselgel (Methyl-tert.-butylether/Hexan) unterworfen. Es resultieren 5,4 g (53 % d. Th.) Kristalle mit den Schmelzpunkt 95-97°C.

¹H-NMR(CDCl₃) : δ = 2br7(s,3H); 3.82(s,3H); 4.02(s,3H); 5.15(s,2H); 7.20(dbr,1H); 7.25-7.55(m,6H)

### Beispiel 4:

### 3-Methoxy-2-[2'-(1"-(4'''-bromphenyl)1"-methyl)iminooxymethyl]phenylacrytsäuremethylester (Nr. 593, Tab. I)

2,37 g (10 mmol) 3-Methoxy-2-(2'aminooxymethyl)phenylacrylsäuremethylester, 1,99 g (10 mmol) 4-Bromacetophenon, 2 ml Wasser, 0,8 g Pyridin und 10 ml Methanol werden zusammengegeben und 24 h bei Raumtemp. nachgerührt. Nach Einengen, verteilen zwischen Wasser/Methyl-tert.-butylether, waschen der org. Phase mit 2 N HCl und neutralwaschen mit NaHCO₃ wird eingeengt und an SiO₂ chromatographiert (MtBE/Hexan). Es resultieren 2,1 g (51 % d. Th.) Kristalle, Fp: 105-107°C.

¹H-NMR(CDCl₃): δ = 2.22(s,3H); 3.70(s,3H); 3.80(s,3H); 5.15(s,2H); 7.16(dbr,1H); 7.33(m,2H); 7.45(m,5H); 7.58(s,1H)

### Beispiel 5:

### 2-Methoximino-2-[2'-(1"-(4'''-nitrophenyl)1 "-methyl)iminooxymethyl]phenylessigsäuremethylester (Nr. 614, Tab. II)

2,38 g (10 mmol) 2-Methoximino-2-(2'-aminooxymethyl)phenylessigsäuremethylester, 1,65 g (10 mmol) 4-Nitroacetophenon, 2 ml Wasser, 0,8 g Pyridin und 10 ml Methanol werden wie in Beispiel 4 zur Reaktion gebracht. Man erhält 2,1 g (55 % d. Th.) Kristalle mit dem Festpunkt 87-89°C.

1H-NMR(CDCl3): δ = 2.22(s,3H); 3.81(s,3H); 4.02(s,3H); 5.18(s,2H); 7.20(dd,1H); 7.4(m,3H); 7.75(dd,2H); 8.18(dd,2H).

### Beispiel 6:

### 3-Methoxy-2-[2'-(1"-(4'''-chlorphenyl) iminooxymethyl]phenylacrylsäuremethylester (Nr. 95 Tab. V)

a) 5,5 g (15 mmol) 3-Methoxy-2-[2'-(phtalimidooxy)methyl]phenylacrylsäuremethylester (aus Vorschrift 4) werden in 100 ml Methanol vorgelegt und bei Raumtemperatur 0,75 g (15 mmol) Hydrazinhydrat zugegeben. Nach 15 min bei dieser Temperatur engt man ein, verreibt den Rückstand mit Methyl-tert.-butylether und saugt das Spaltprodukt ab. Die Mutterlauge wird eingeengt, mit 50 ml Methanol aufgenommen und tropfenweise mit 2,1 g (15 mmol) 4-Chlorbenzaldehyd in 50 ml Methanol plus 1 Tropfen Pyridin versetzt.
   Nach Rühren über Nacht bei 23°C konnte man das Produkt absaugen, mit Methanol waschen und trocknen.
   Man erhielt 2,9 g (54 % d.Th.) Kristalle mit dem Fp: 87-89°C.
   ¹H-NMR (CDCl₃): δ = 3.66 (s;3H), 3.80 (s;3H), 5.13 (s;2H), 7.17 (m;1H), 7.35 (m;4H), 7.50 (me;3H), 7.60 (s,1H), 8.06 (s;1H)
b) 1,3 g (55 mmol) NaH-Pulver werden unter Stickstoffatmosphäre langsam mit 50 ml Methanol versetzt (Wasserstoffentwicklung!). Dann gibt man bei Raumtemperatur 7,8 g (50 mmol) 4-Chlorbenzaldehydoxim in 50 ml Methanol zu. Nach halbstündigem Rühren werden 14,3 g (50 mmol) 3-Methoxy-2-(2'-Brommethyl)phenyl-acrylsäuremethylester in 50 ml Methanol zugetropft und 48 h weiter bei Raumtemp. gerührt. Der weiße Rückstand wird abgesaugt, mit Methanol gewaschen und getrocknet. Man erhält 1,9 g (11 % d.Th.) Produkt mit dem Schmelzpunkt: 82-84°C.

### Beispiel 7

### 2-Methoximino-2-(2'-phthalimidooxymethyl)phenylessigsäuremethylester (Nr. 1, Tab. VIII)

2,0 g (7 mmol) 2-Methoxyimino-2-(2'-brommethyl)phenylessigsäuremethylester, 1,1 g (7 mmol) Hydroxyphthalimid, 0,8 g (7,7 mmol) Triethylamin werden in 10 ml N-Methylpyrrolidon gelöst und 2 h bei 70°C gerührt. Zur Aufarbeitung wird mit Eiswasser versetzt, die Kristalle abgesaugt, mit Wasser und Methyl-tert.-Butylether nachgewaschen und getrocknet.

Es verbleiben 1,5 g (58 % d. Th.) Kristalle mit dem Schmelzpunkt 152 - 155°C.

¹H-NMR(CDCl₃): δ = 3.83 (s, 3H); 3.98 (s,3H); 5.07 (s,2H); 7.15 (dbr, 1H); 7.45 (mc, 2H); 7.60-7.85 (m, 5H).

In entsprechender Weise lassen sich die in den Tabellen I-VI aufgeführten Verbindungen herstellen.

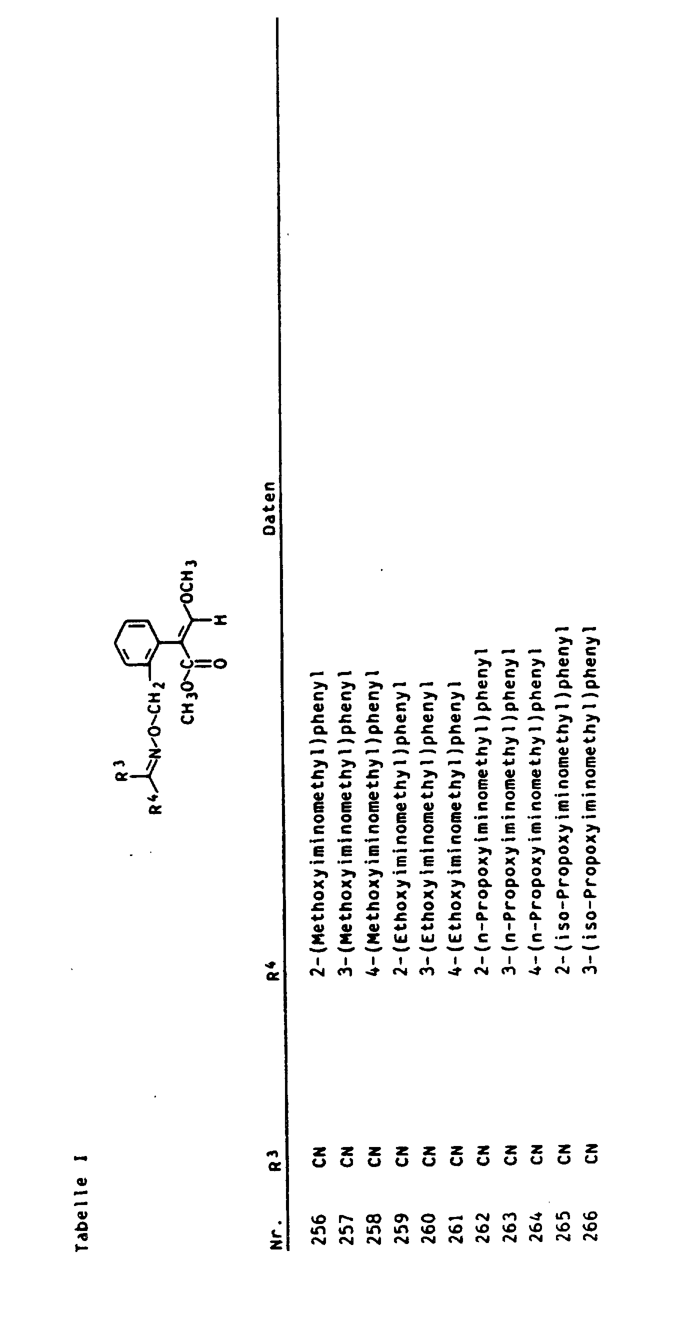

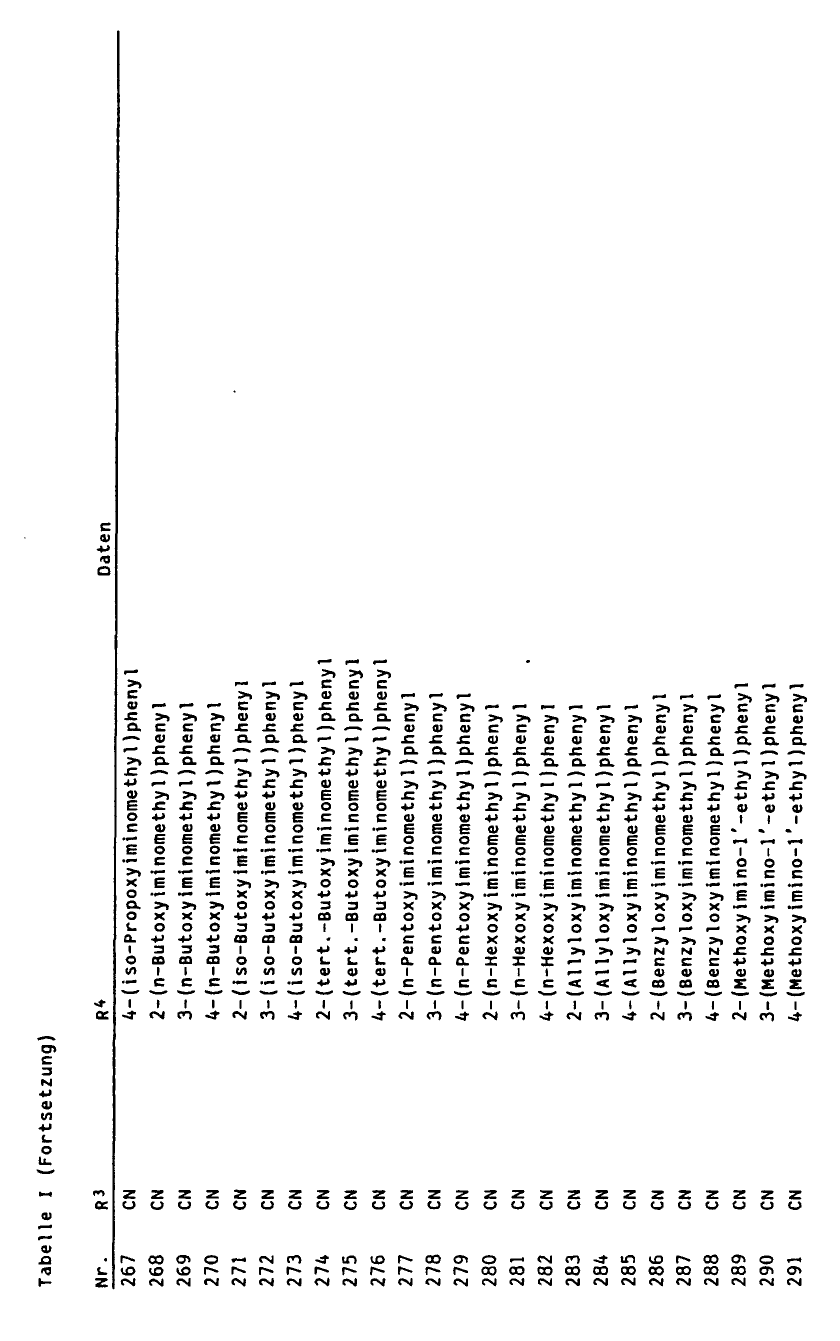

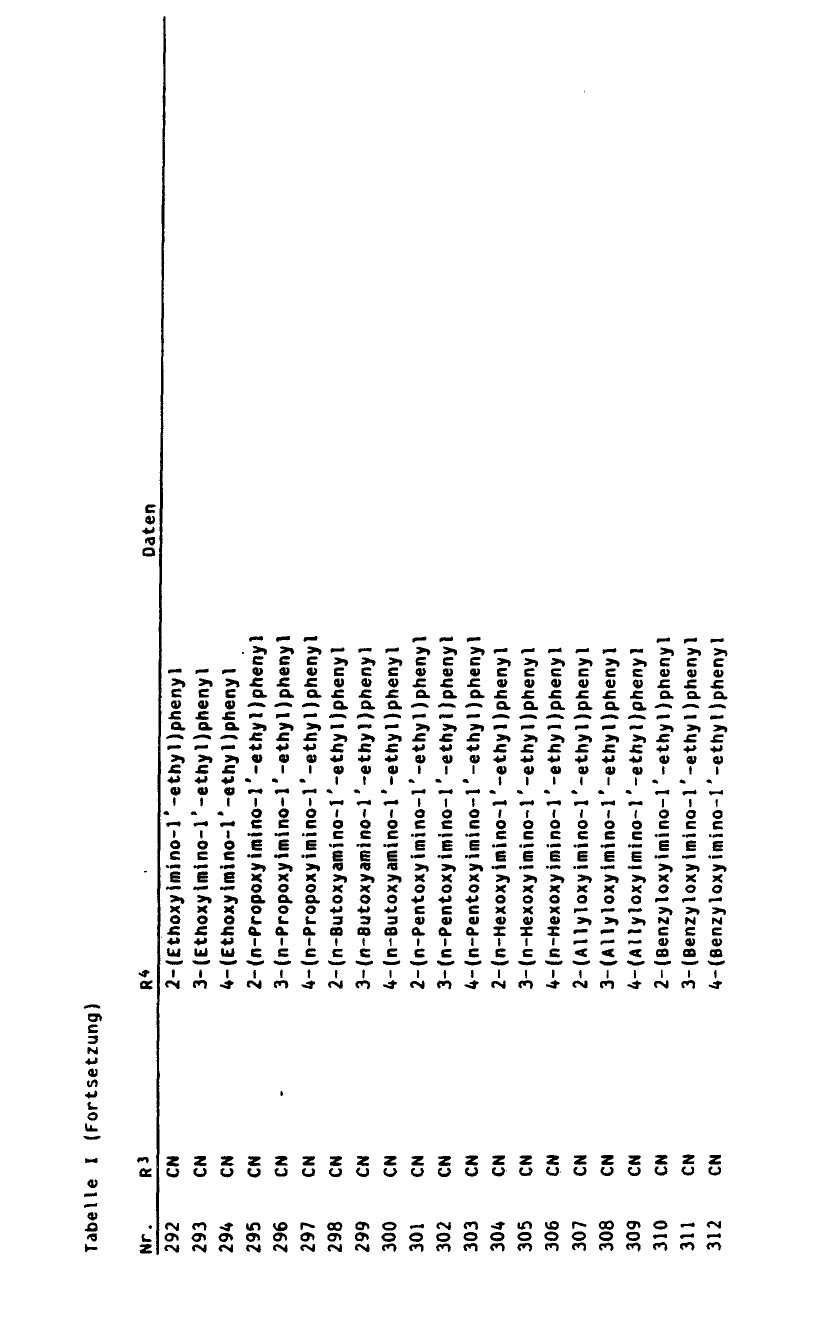

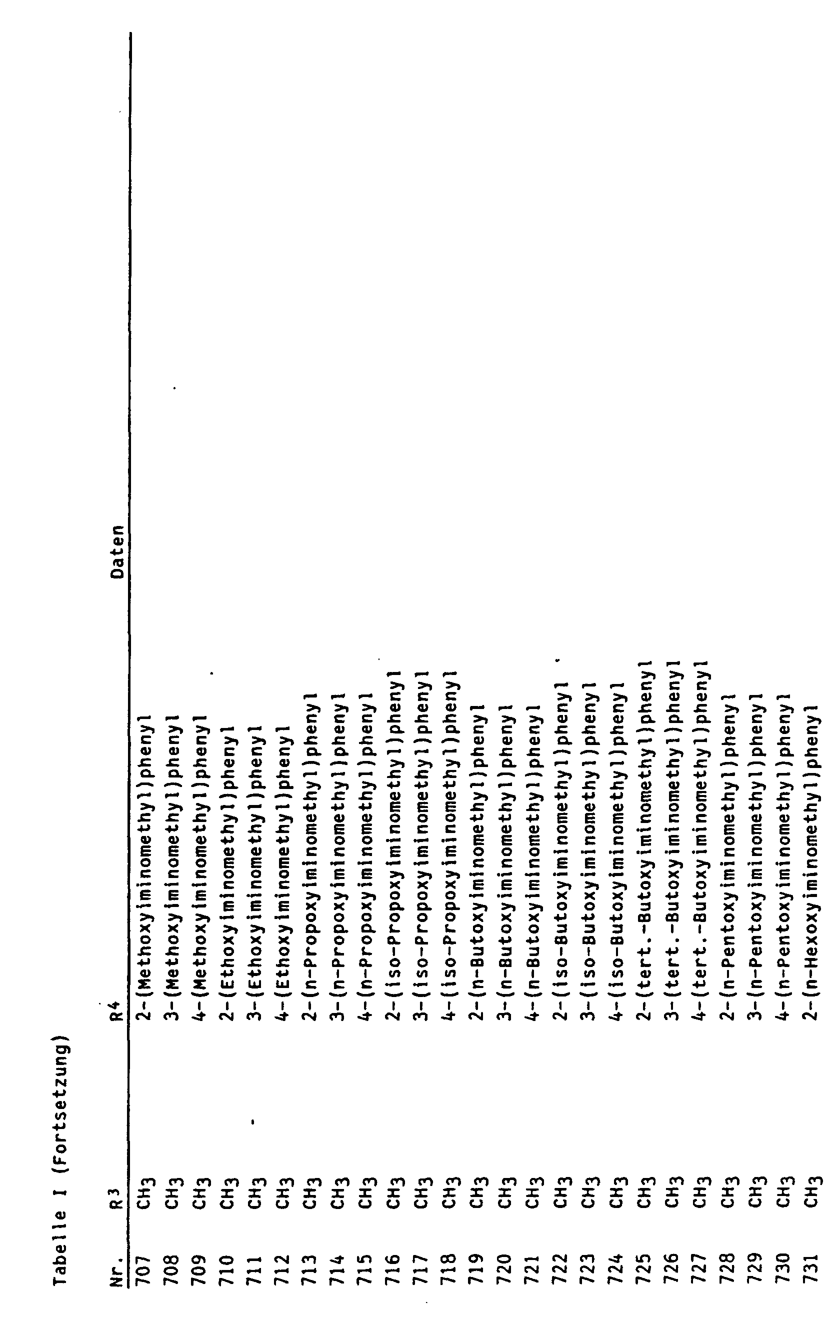

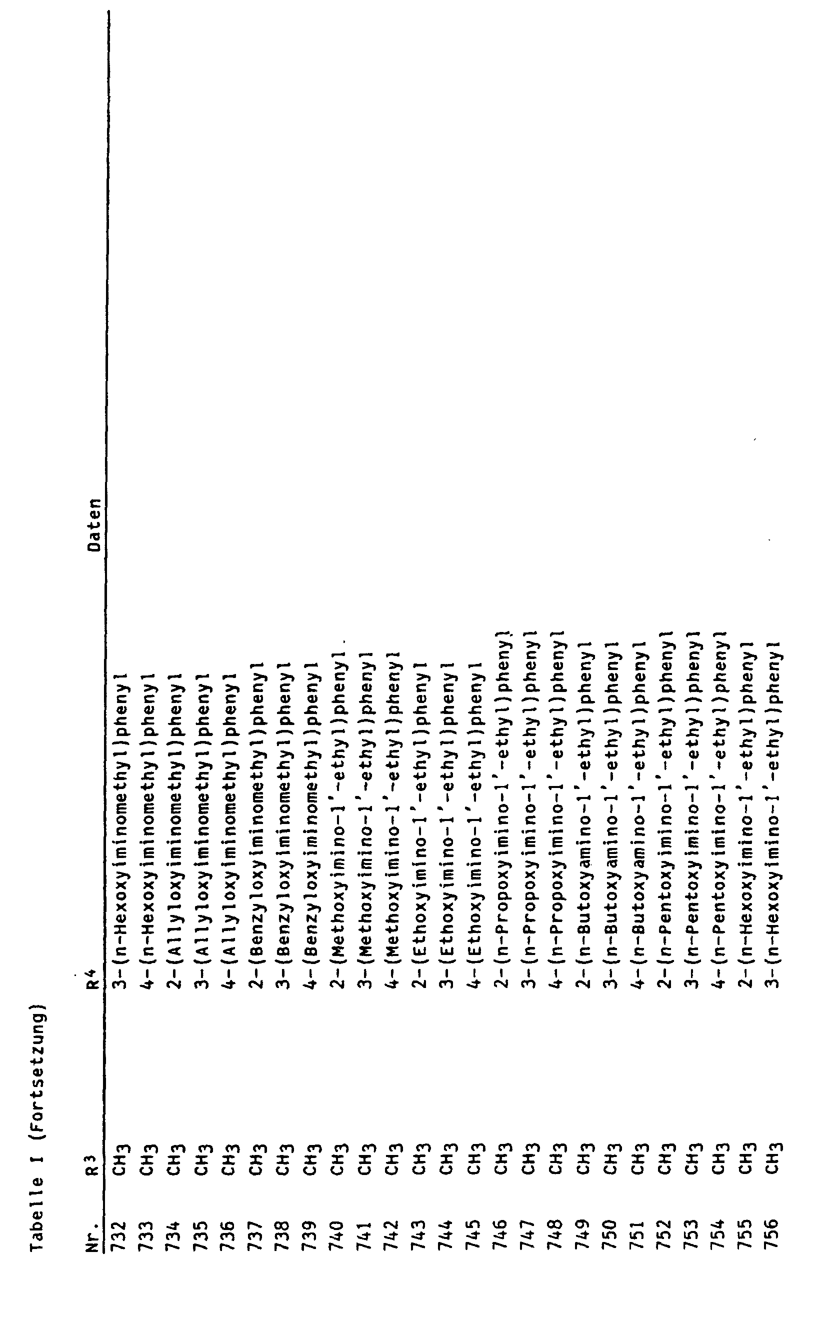

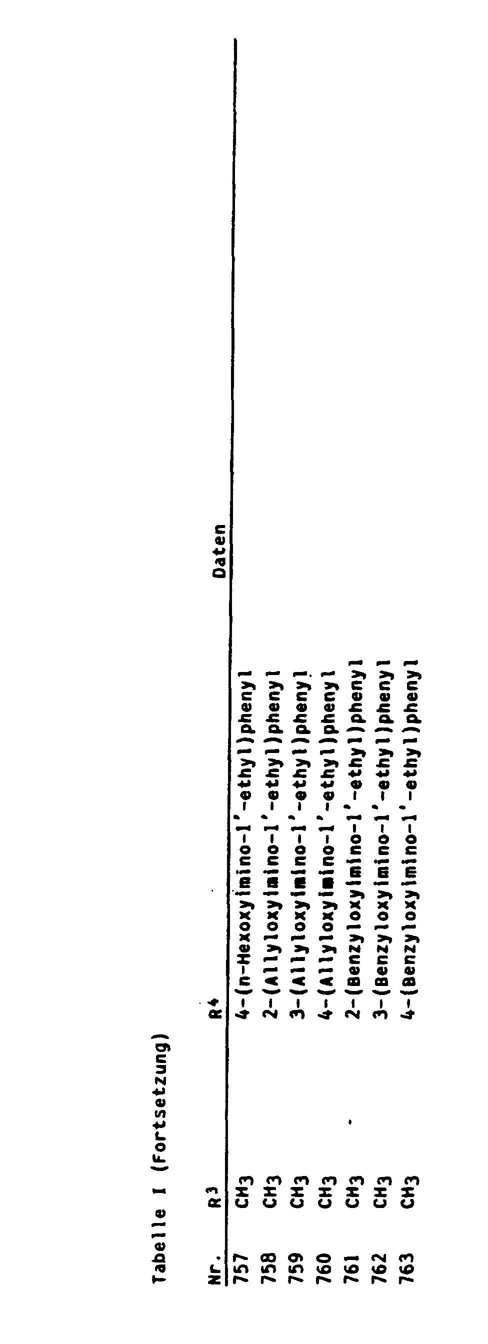

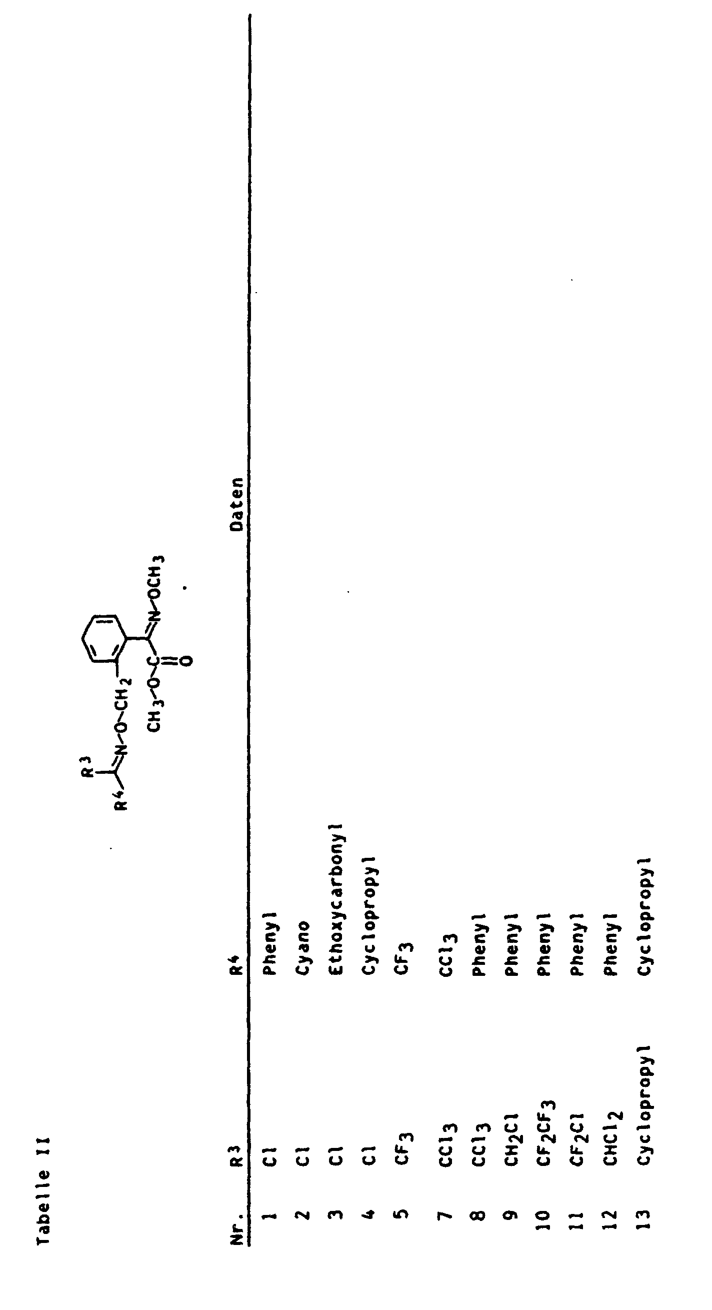

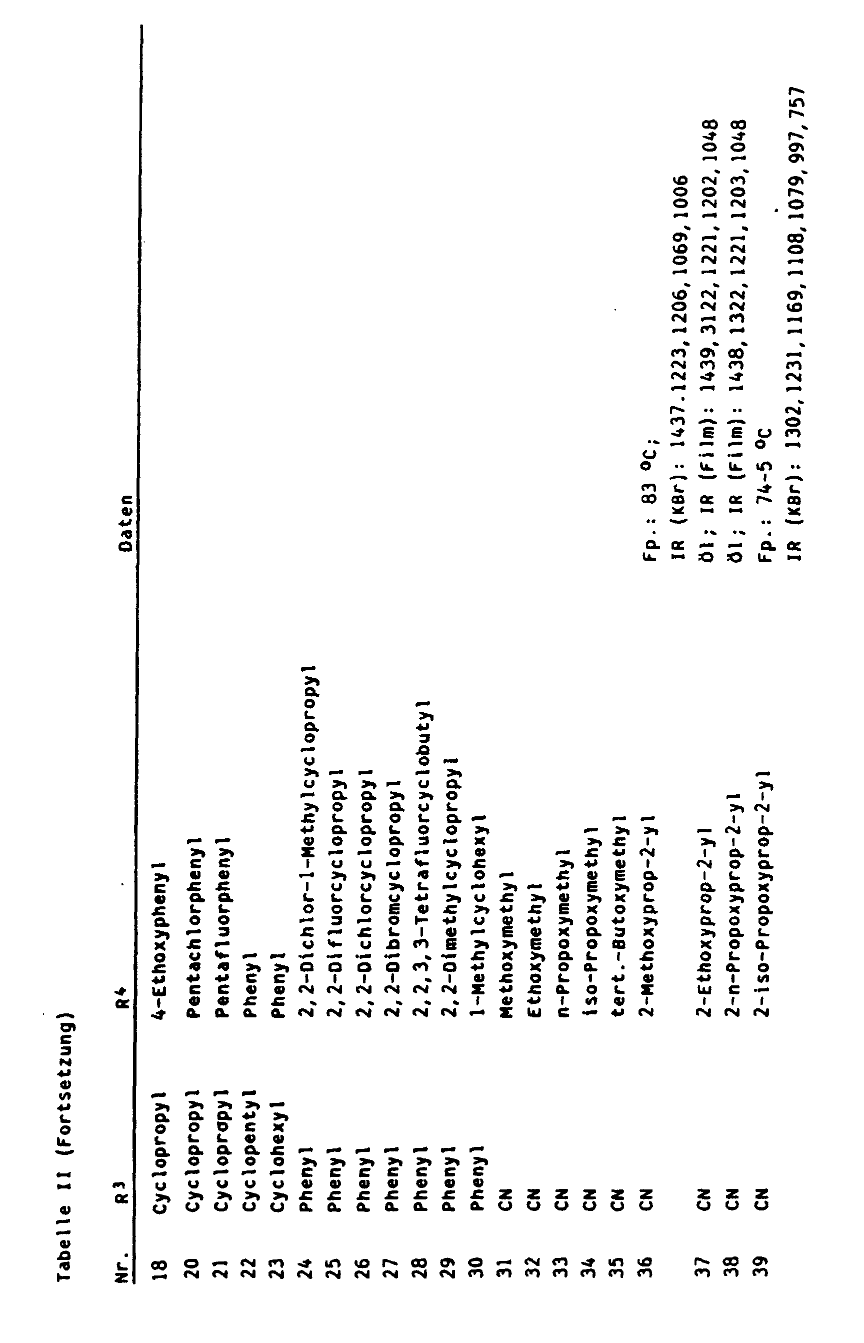

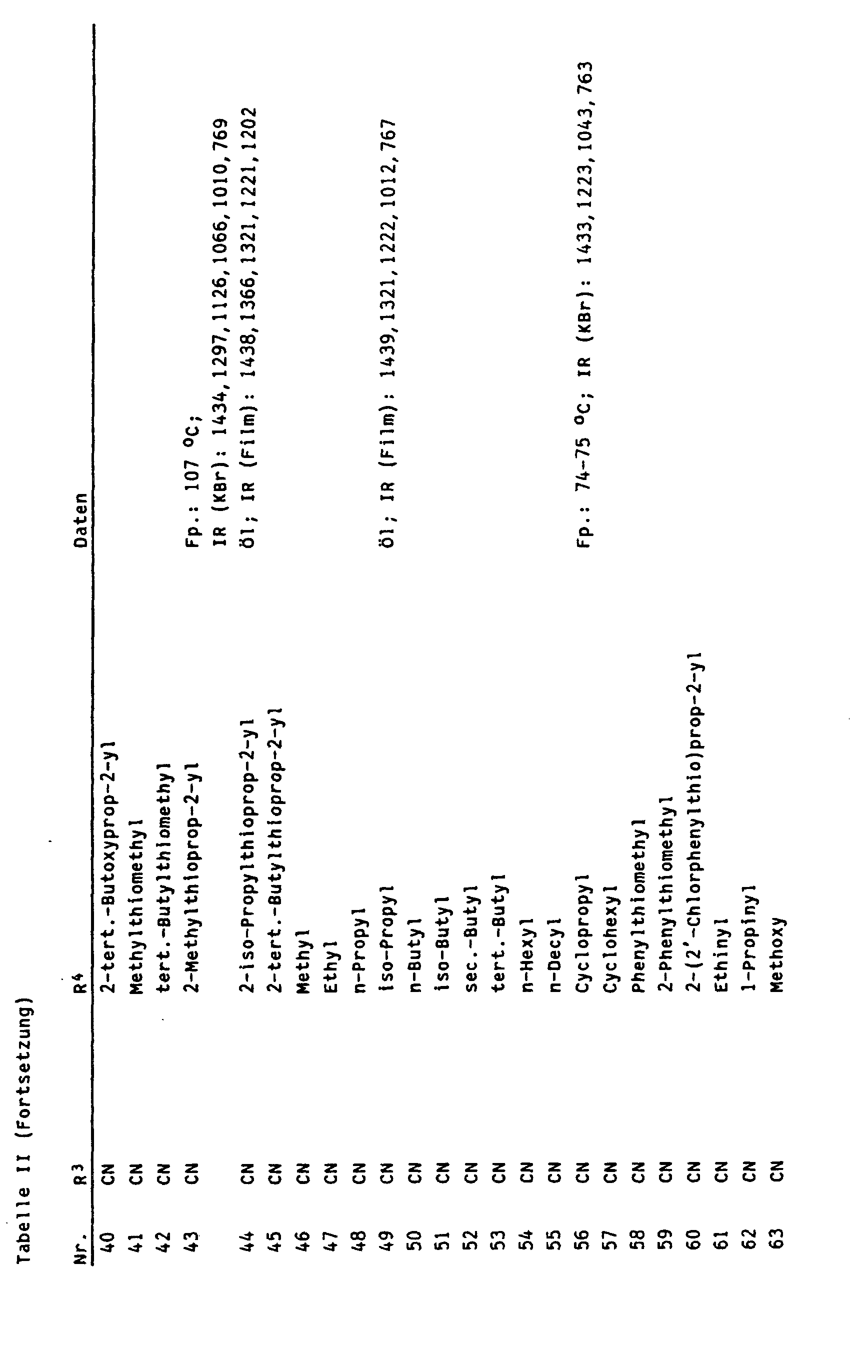

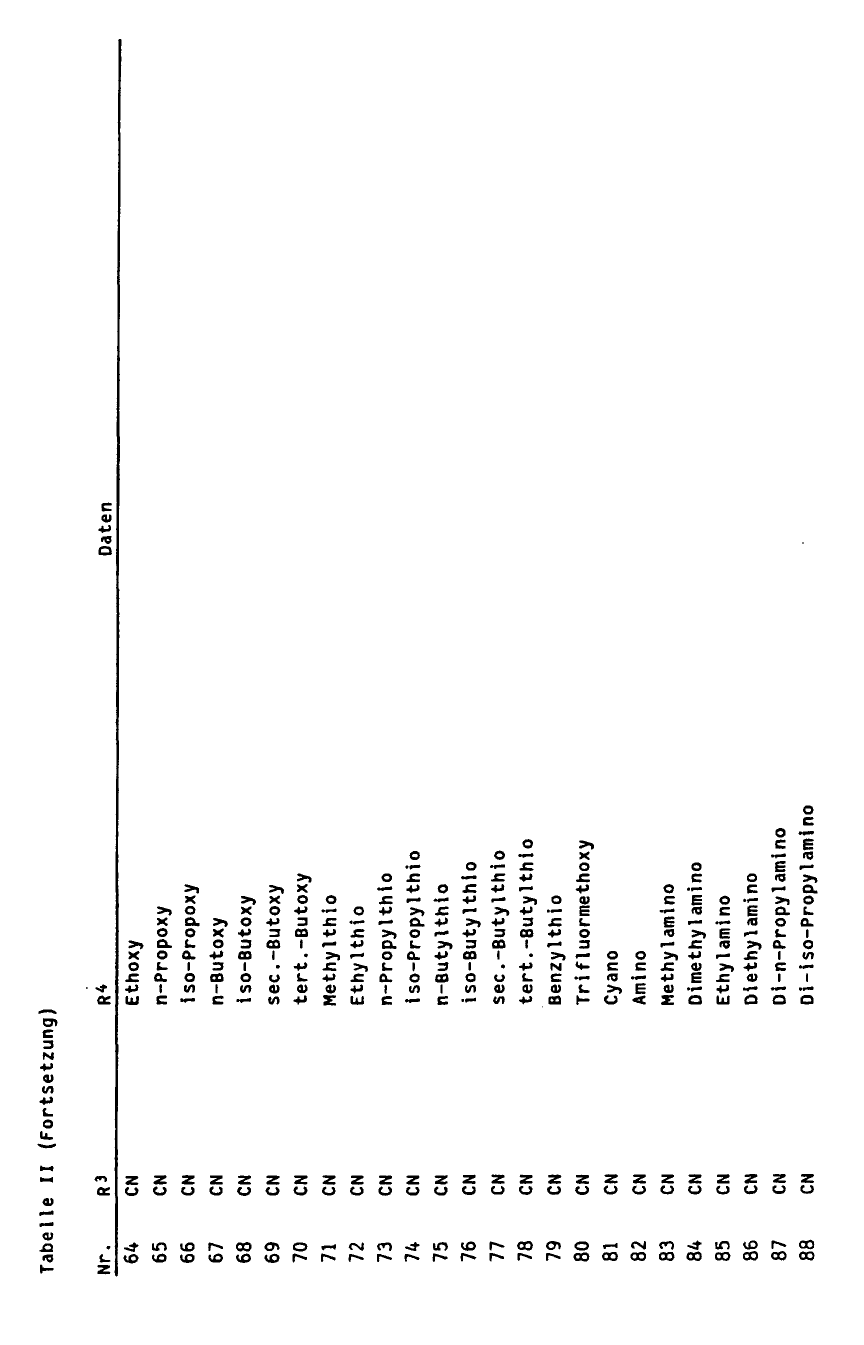

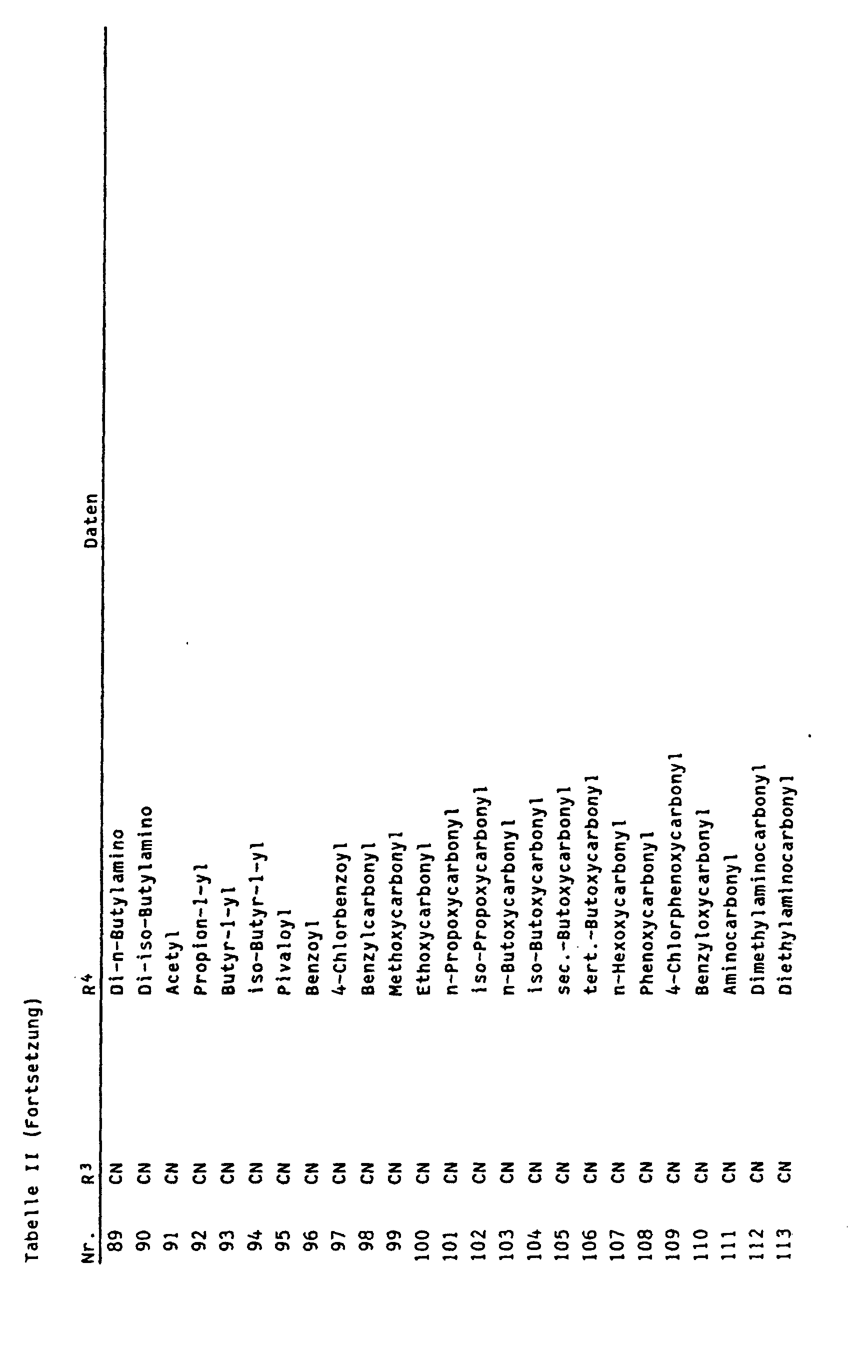

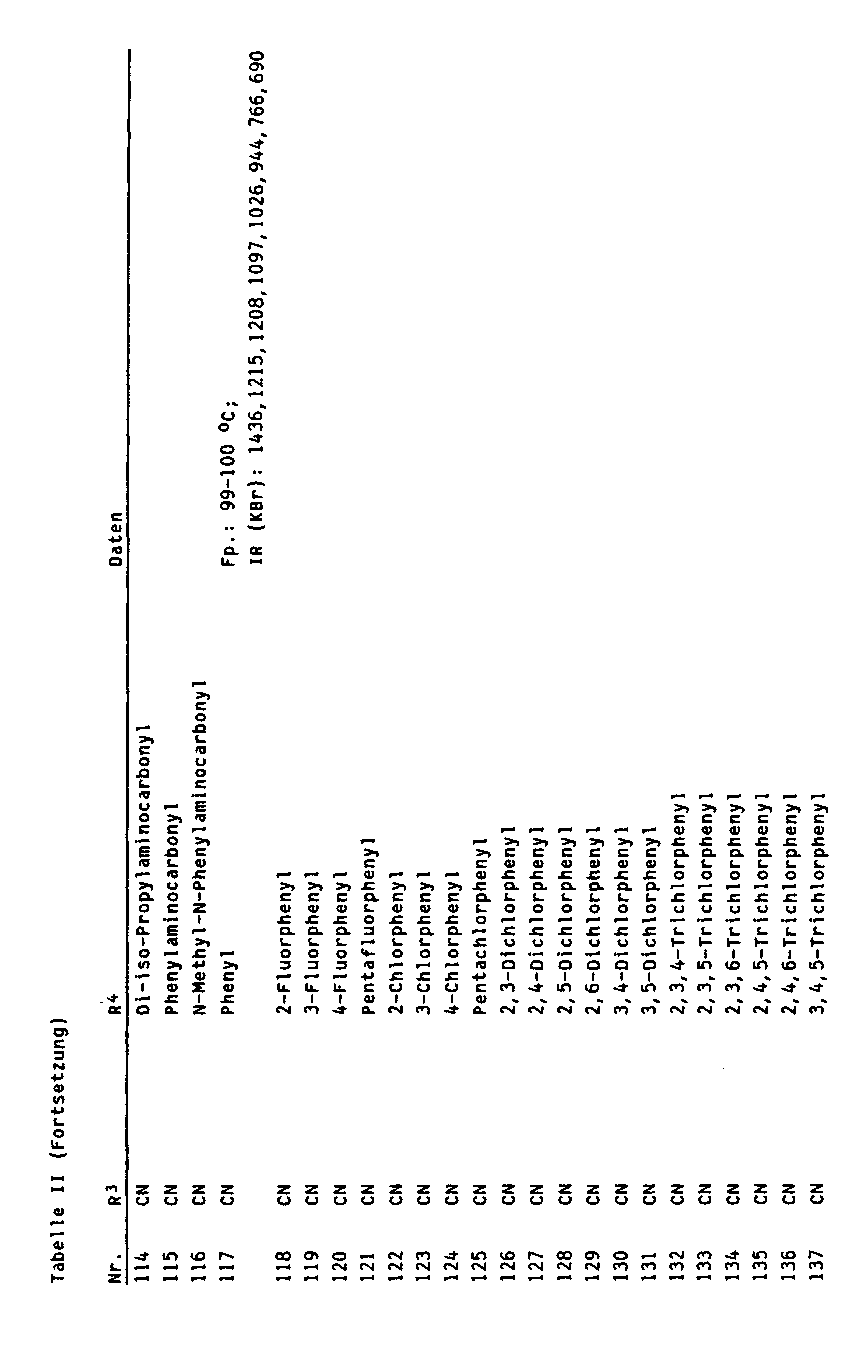

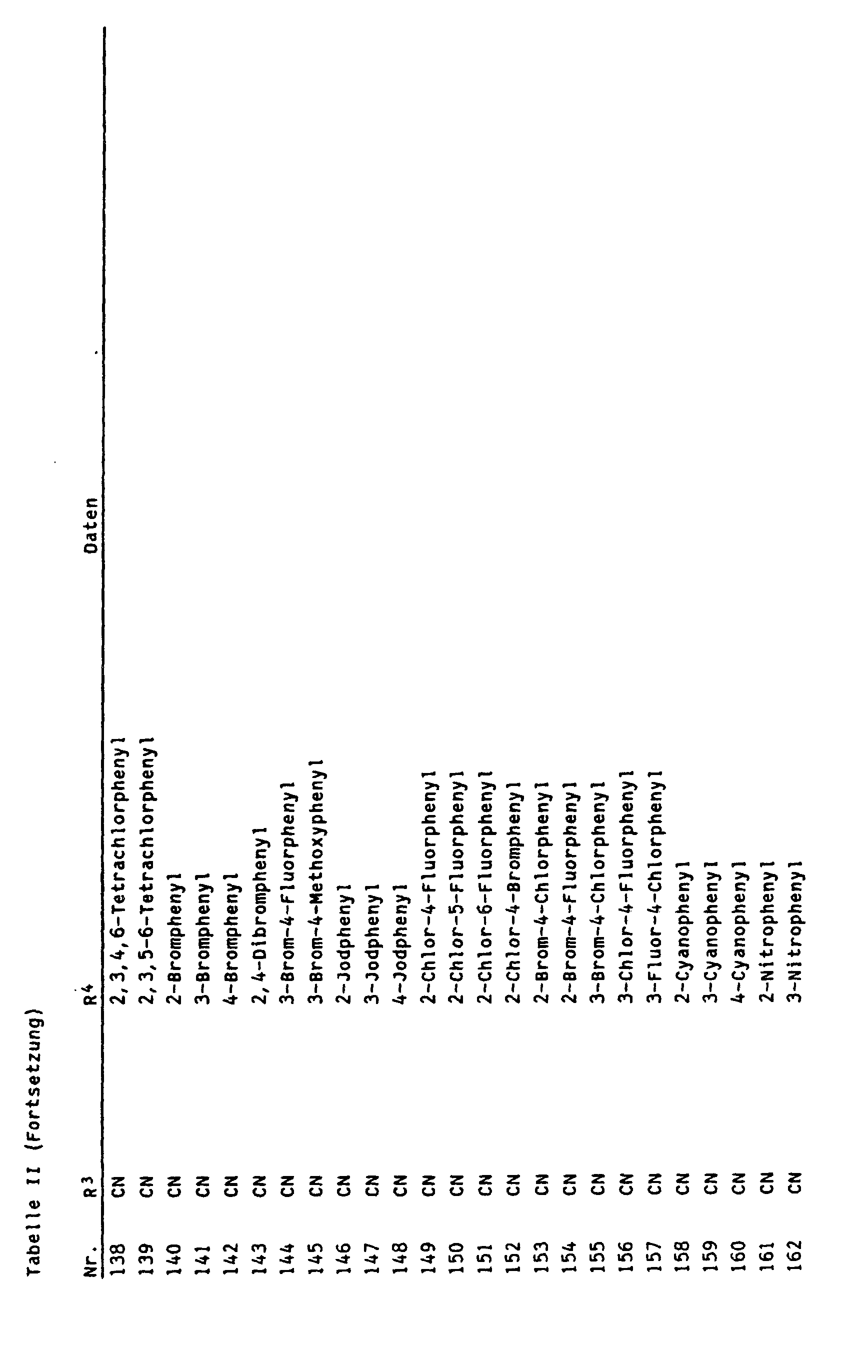

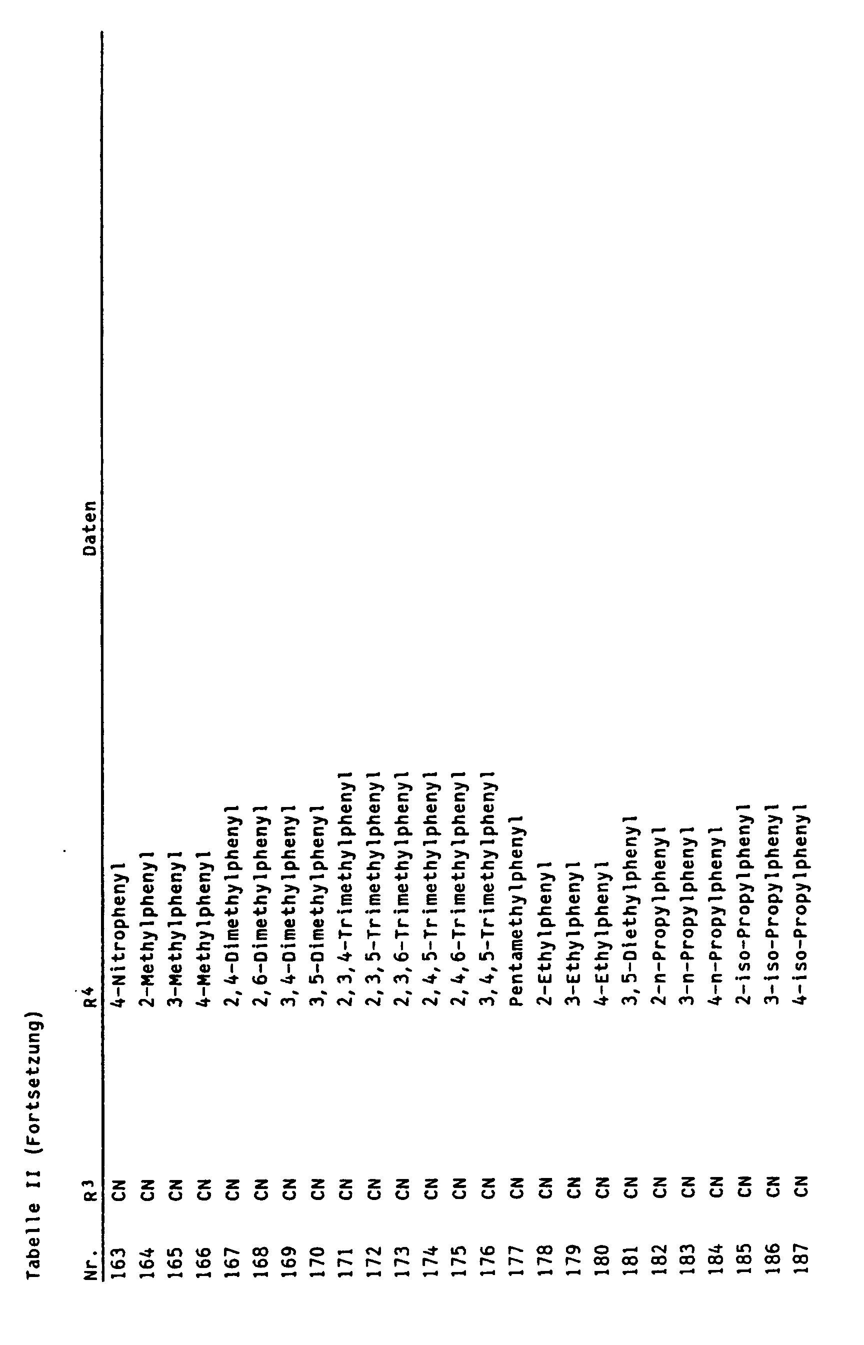

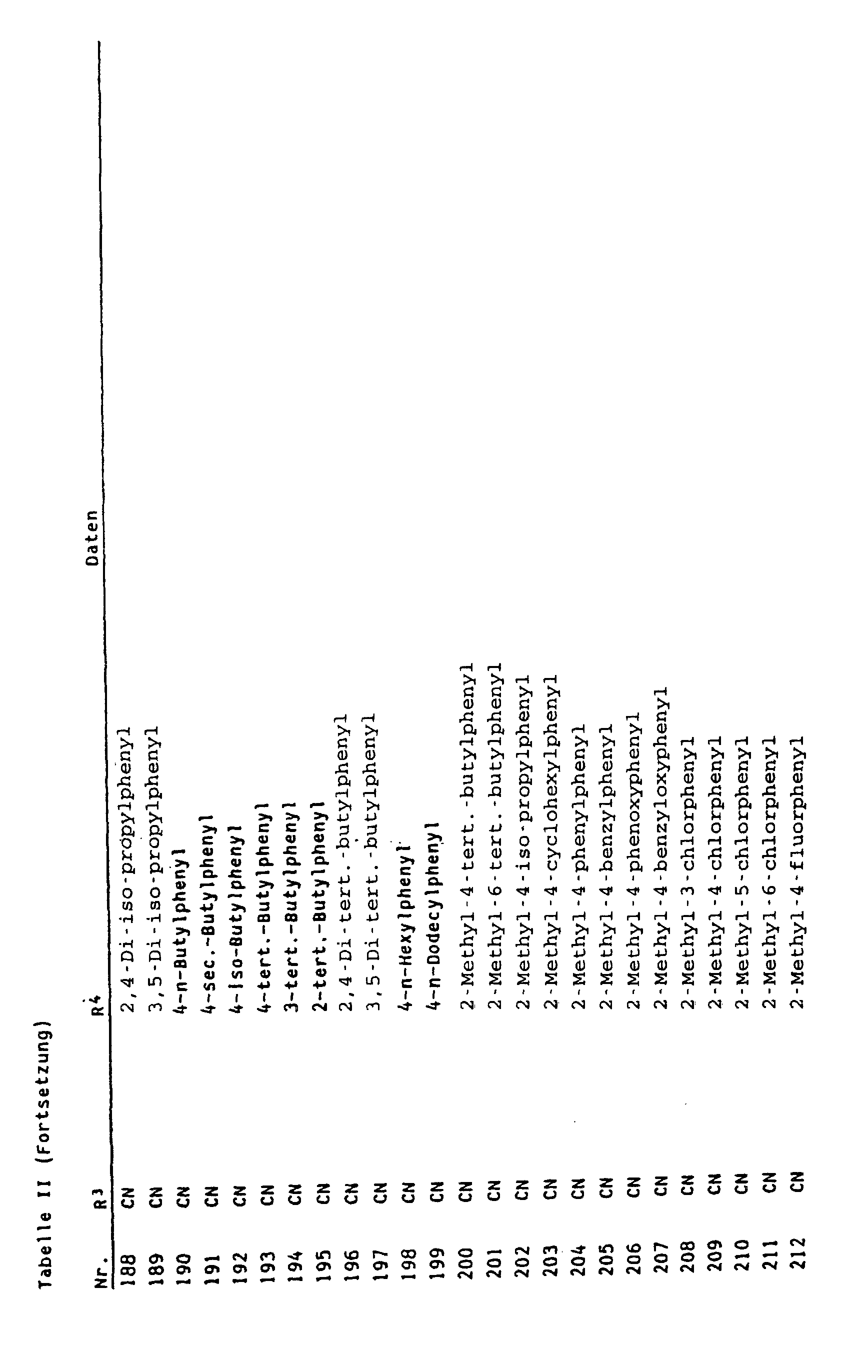

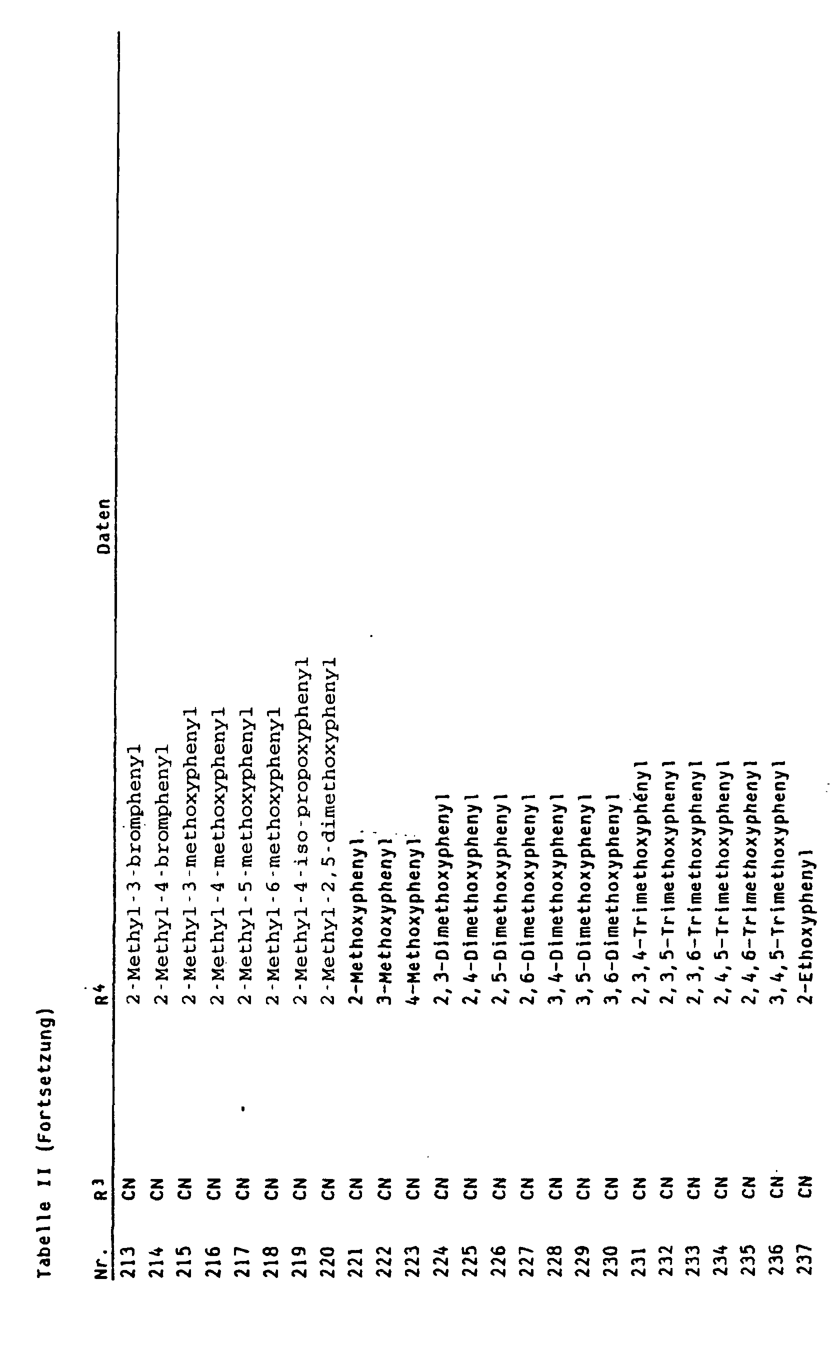

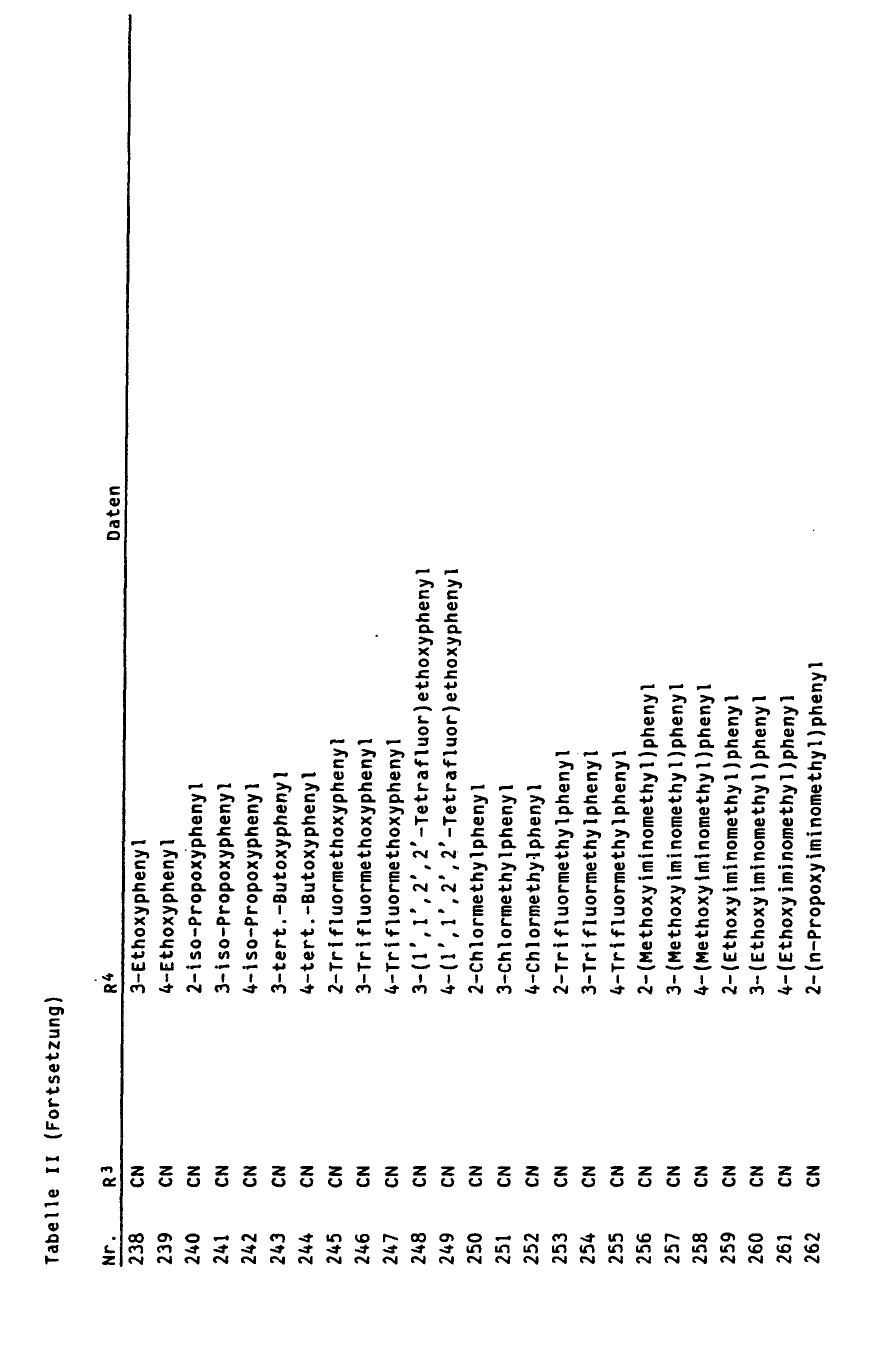

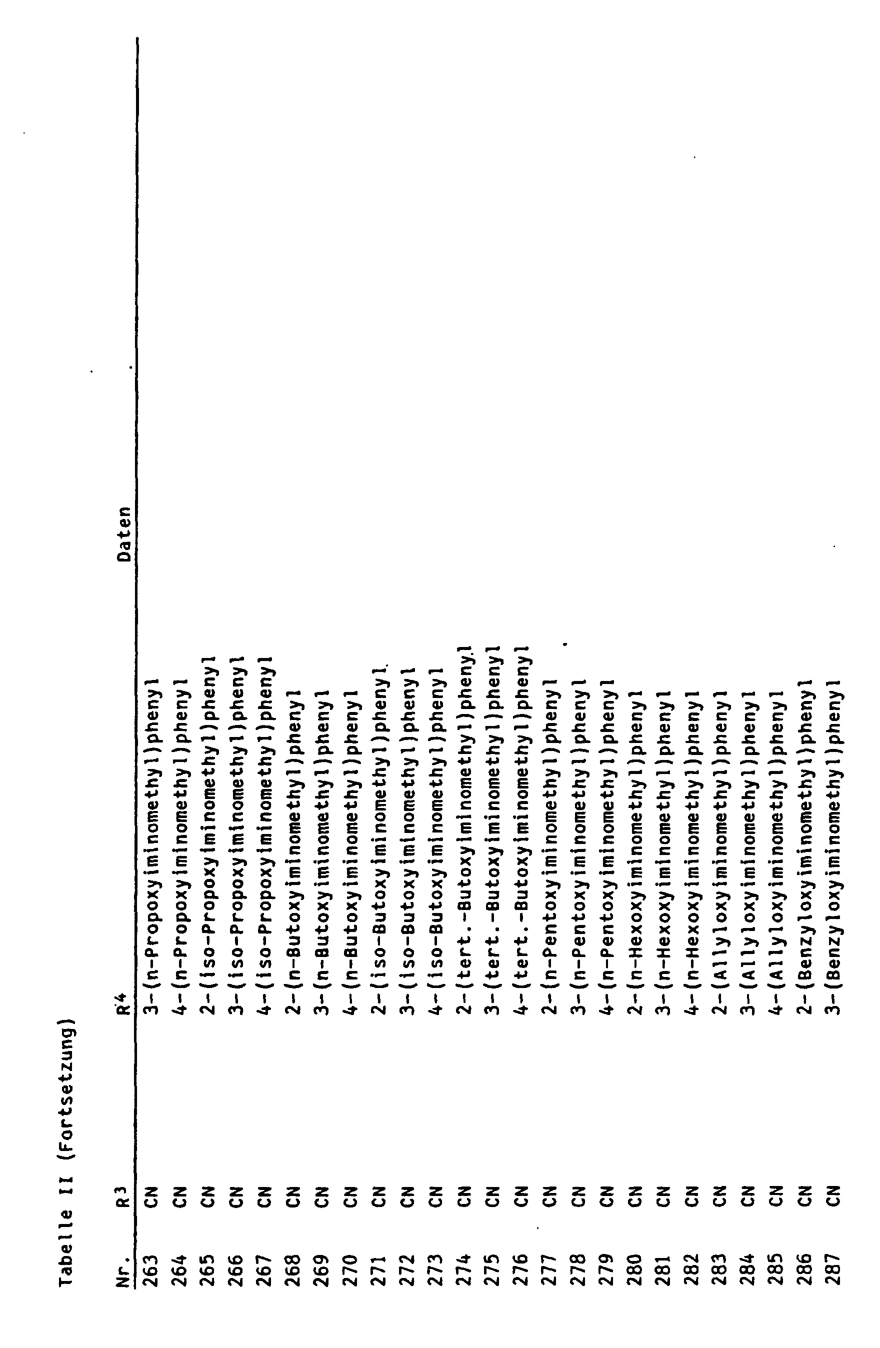

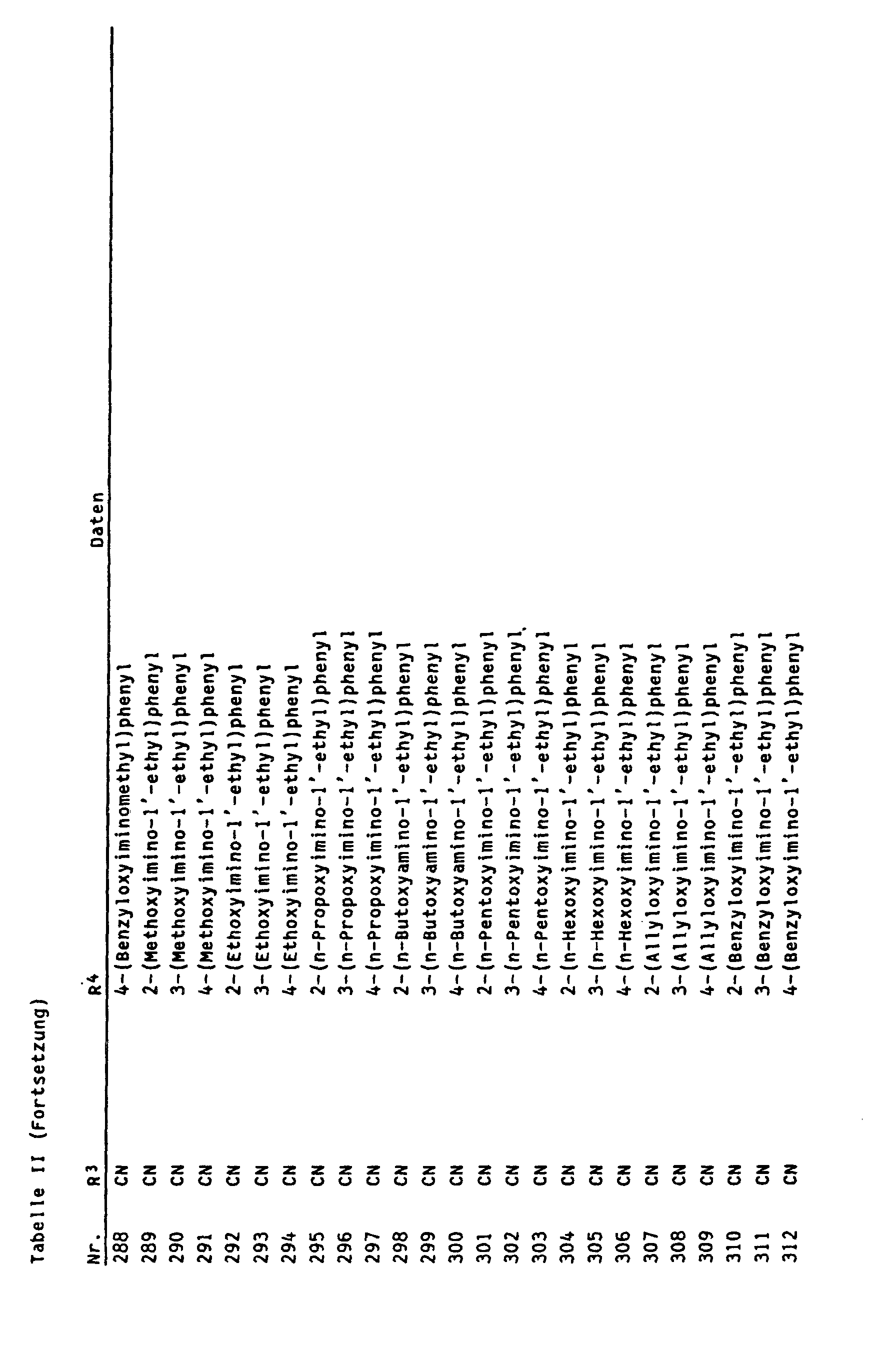

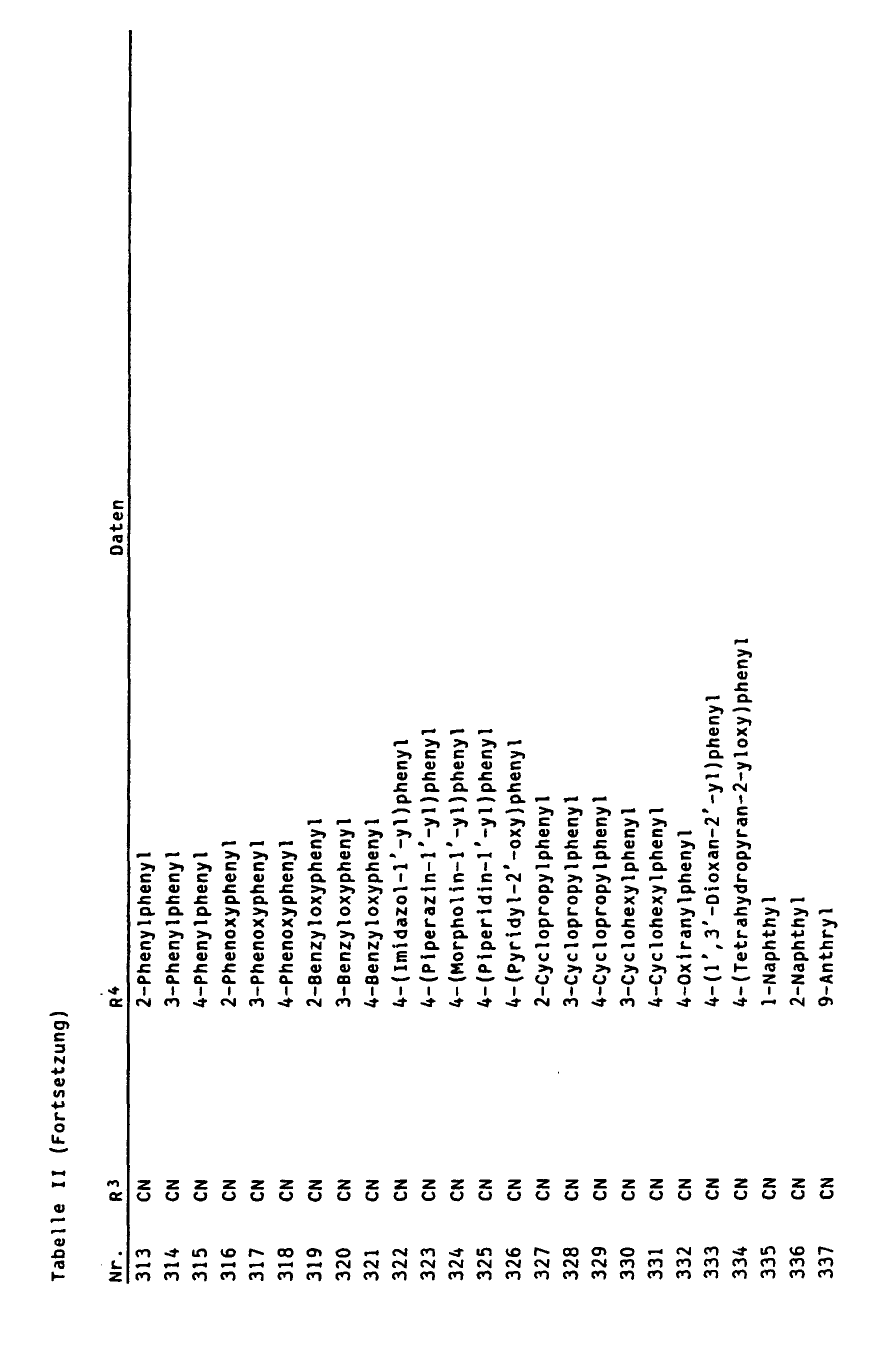

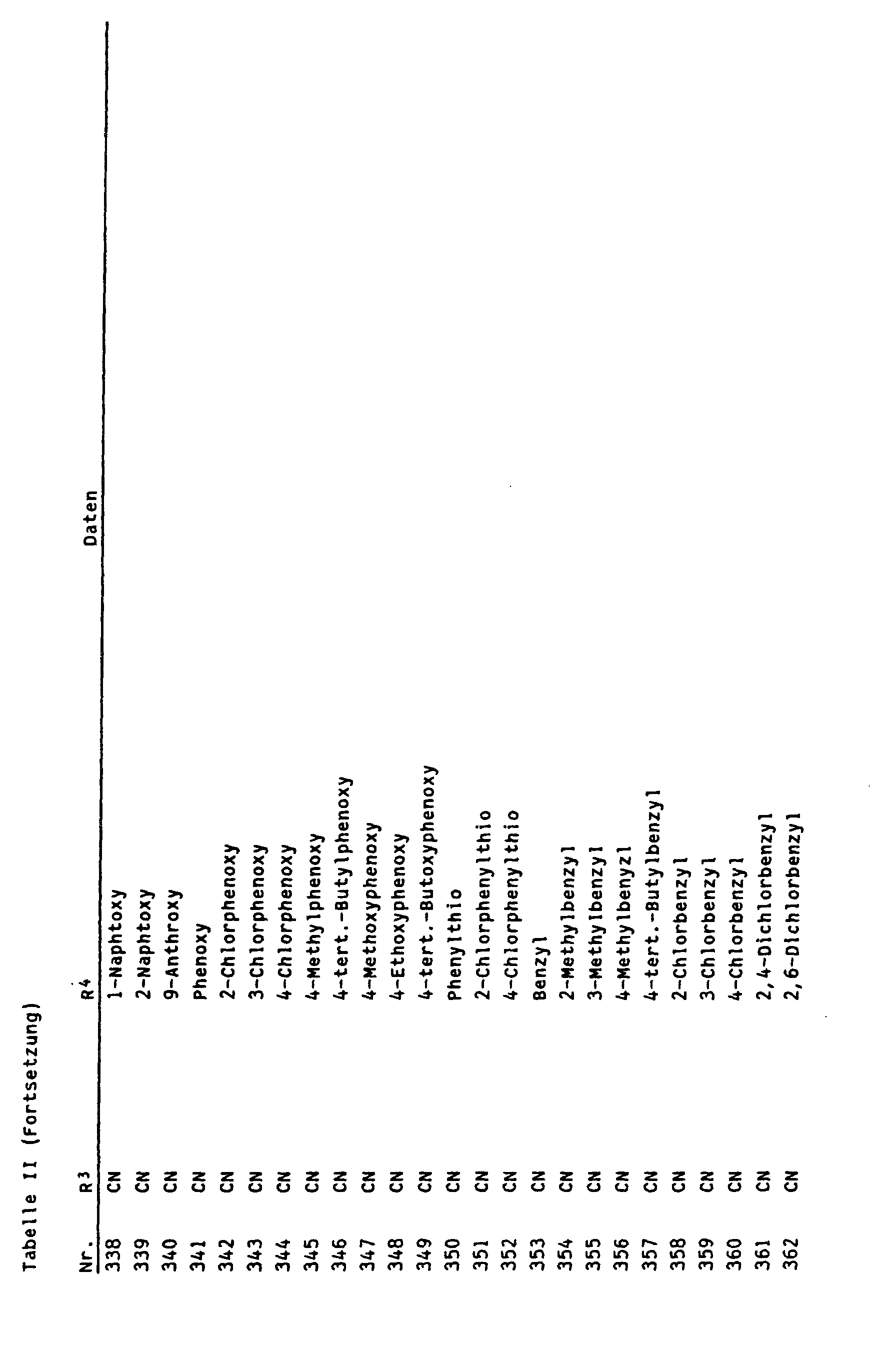

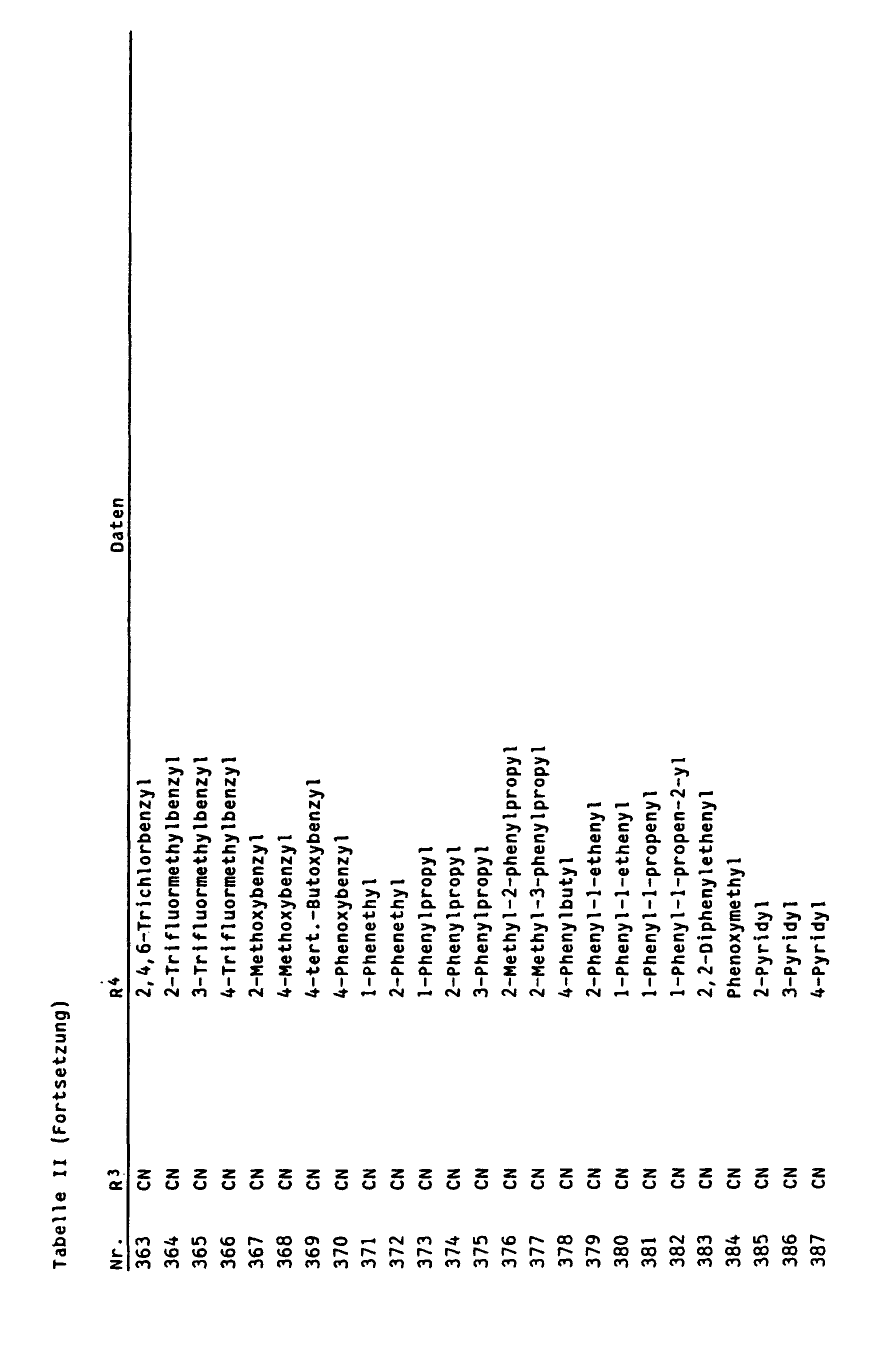

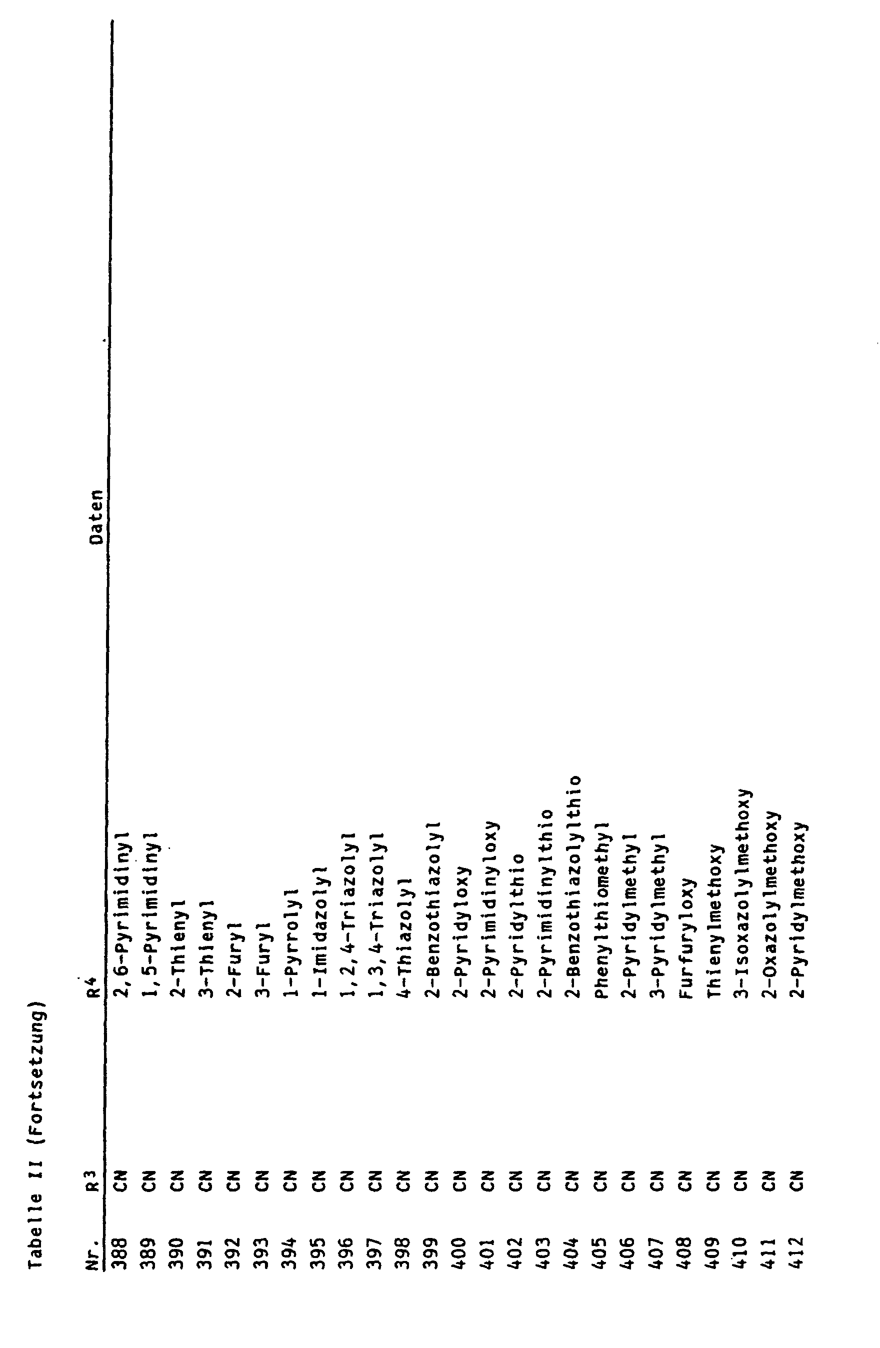

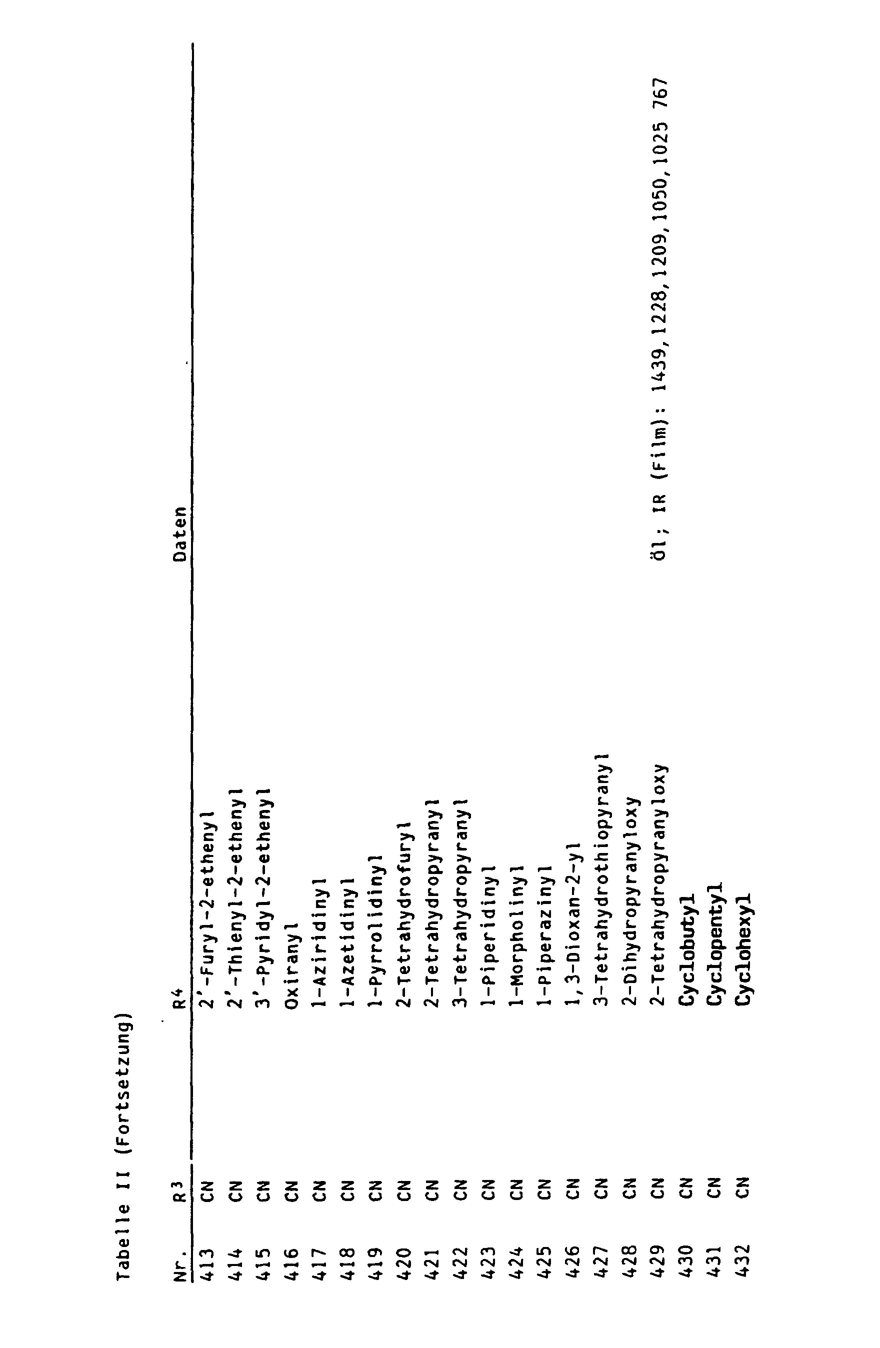

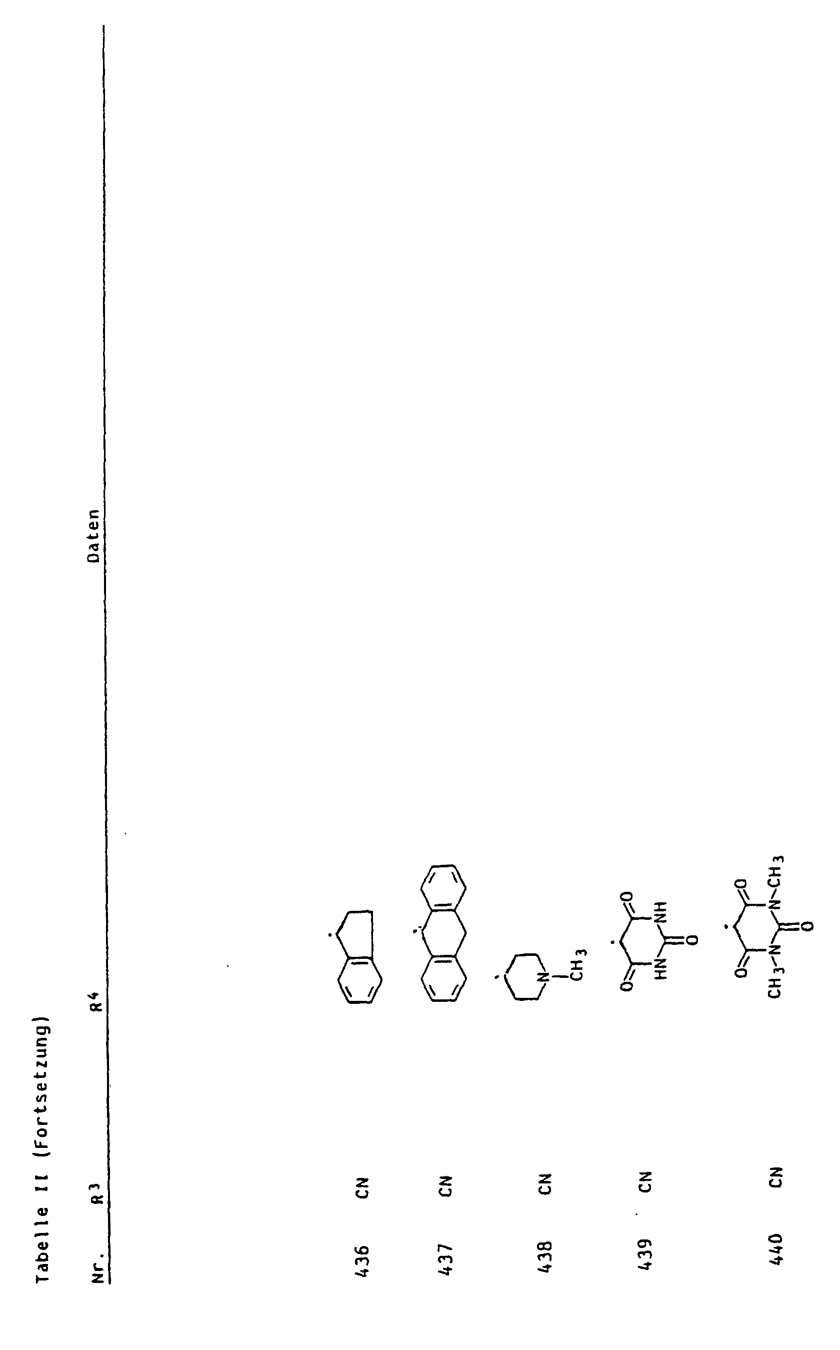

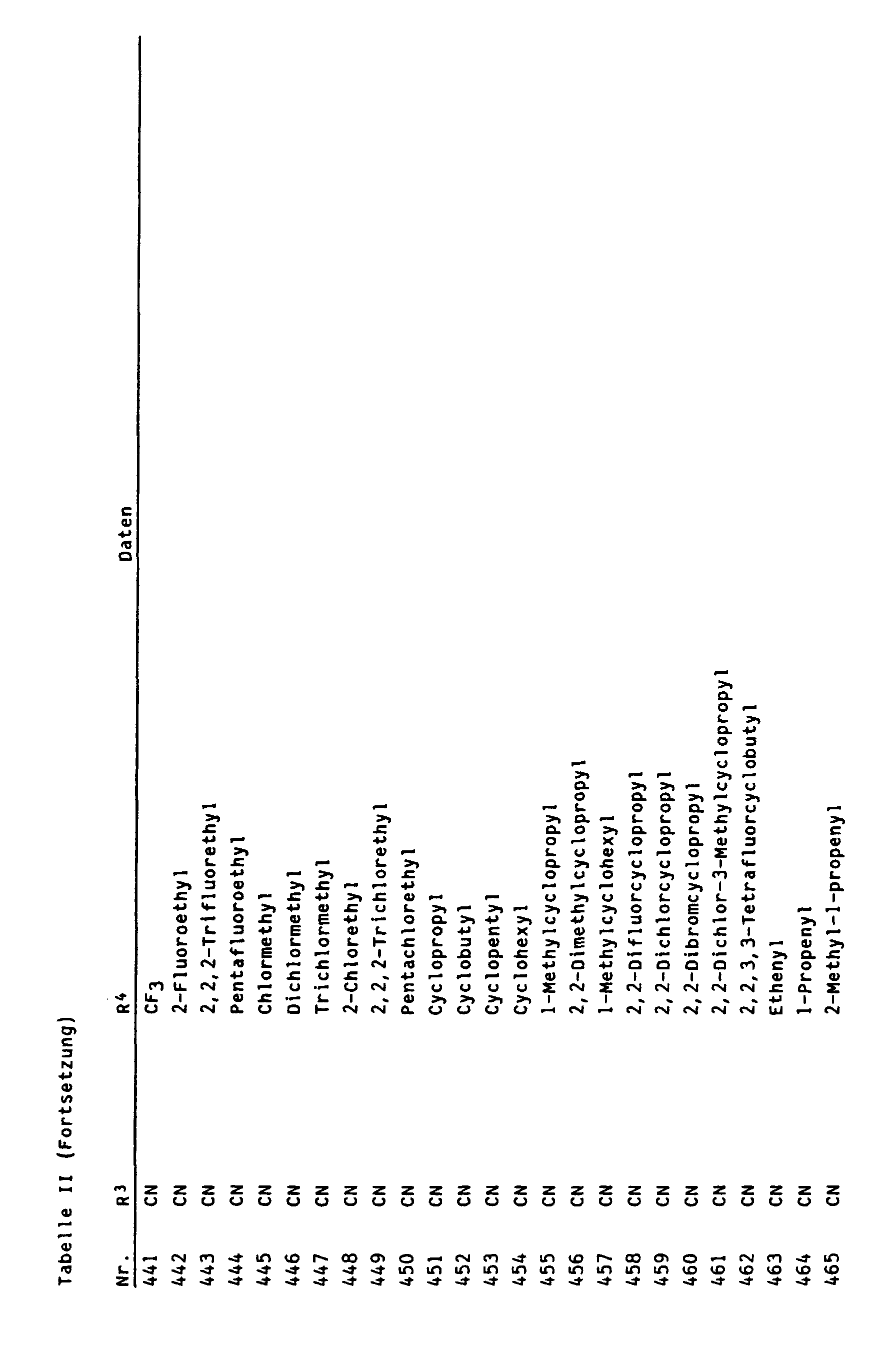

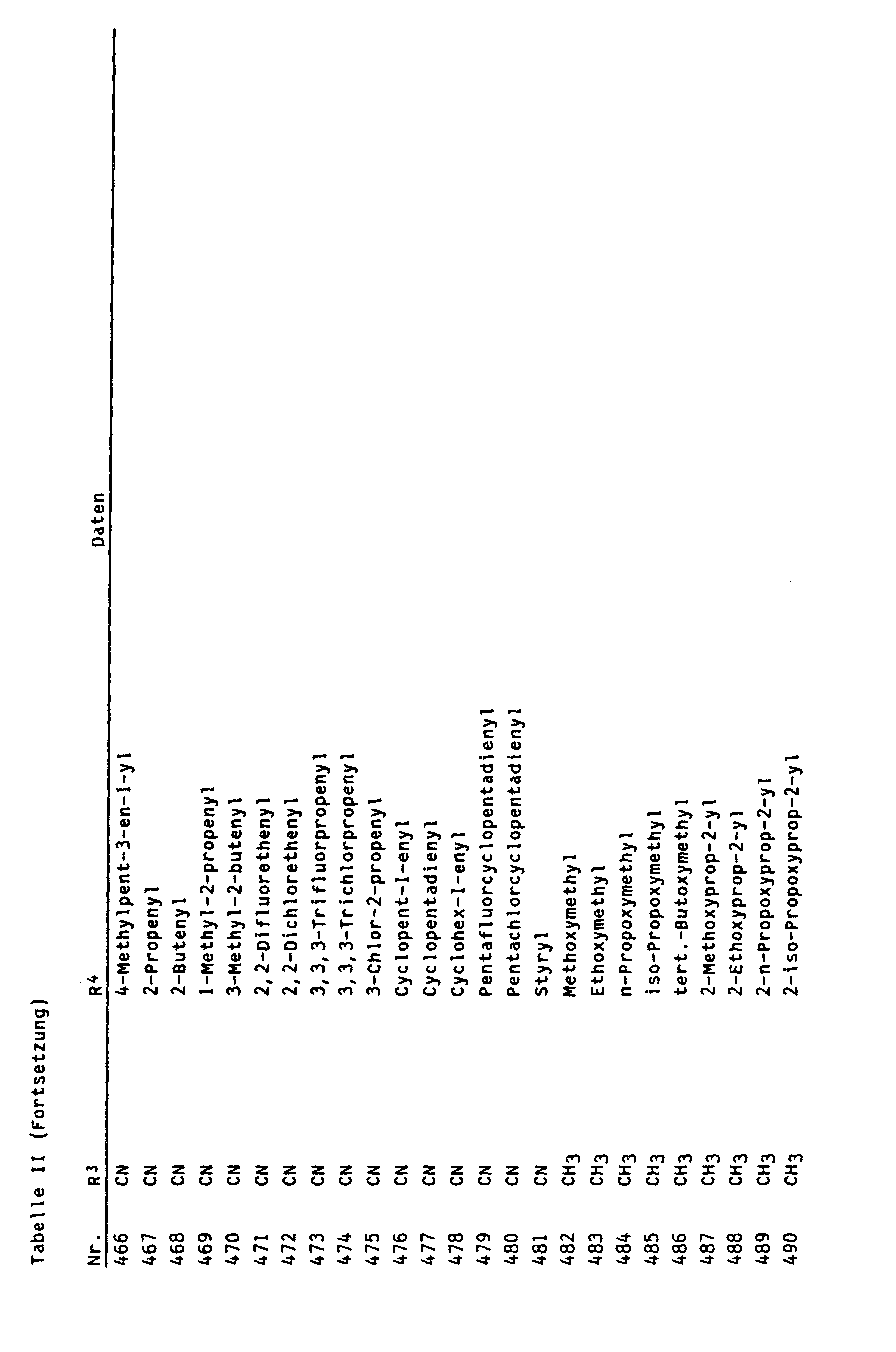

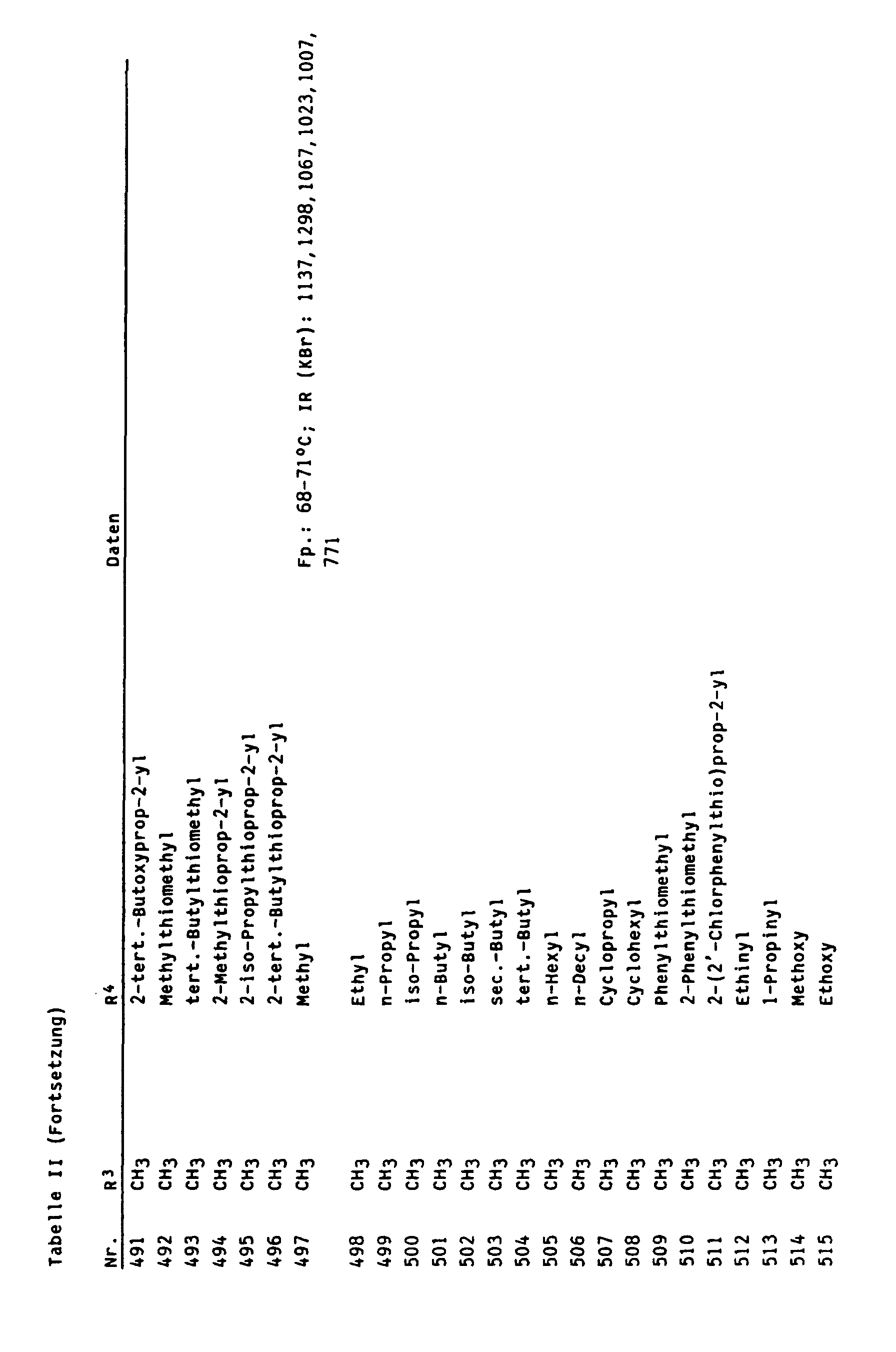

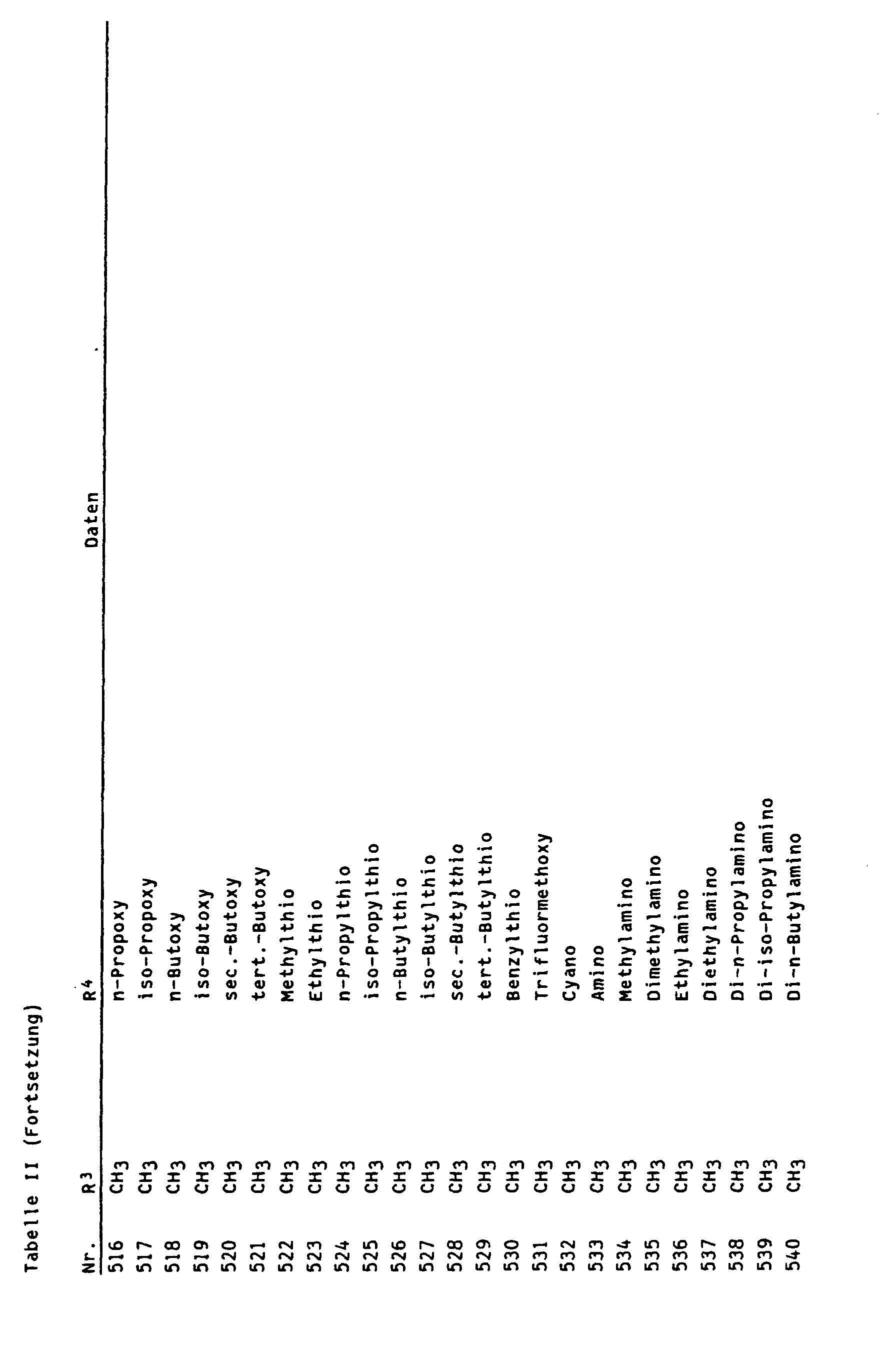

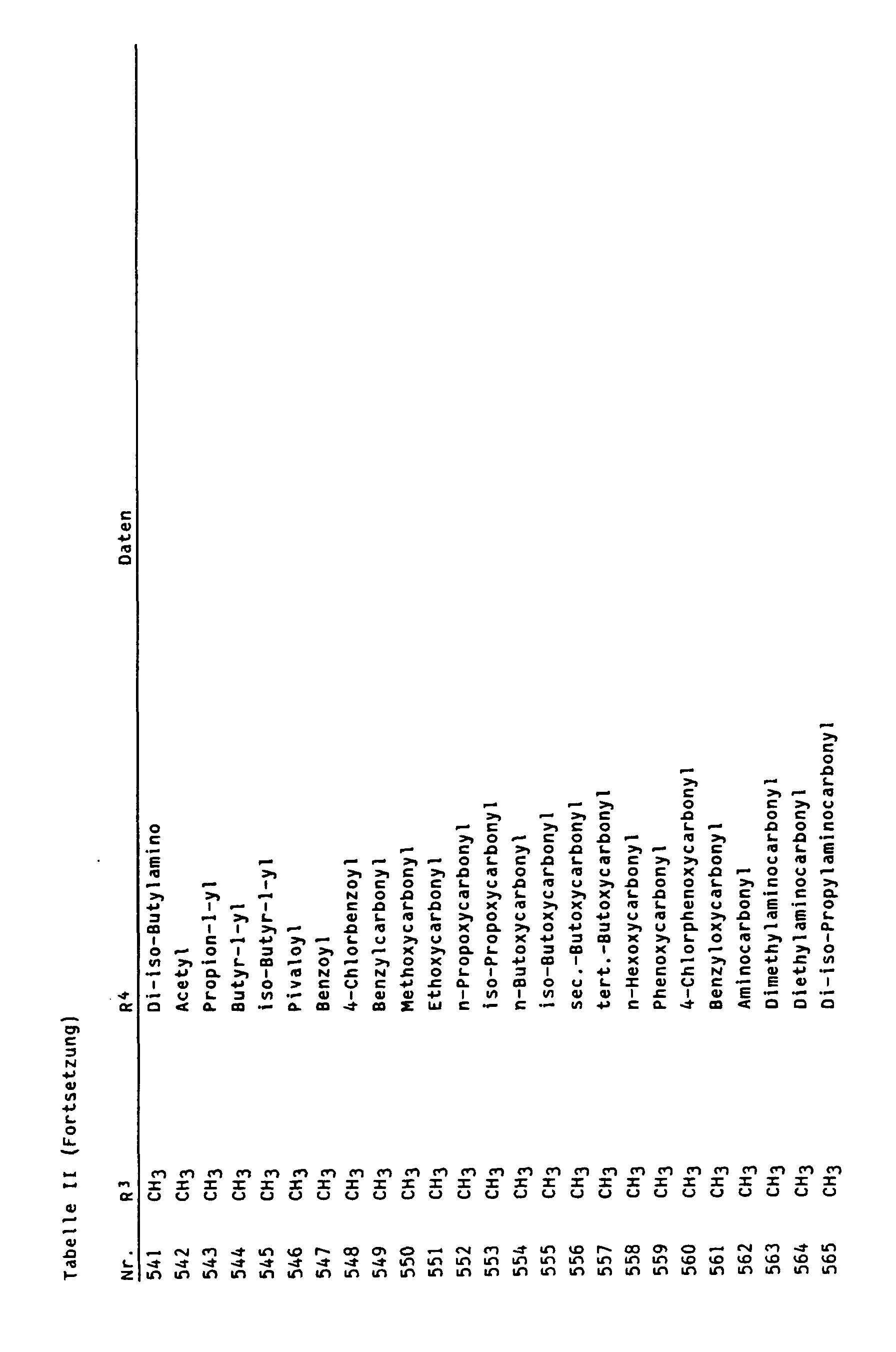

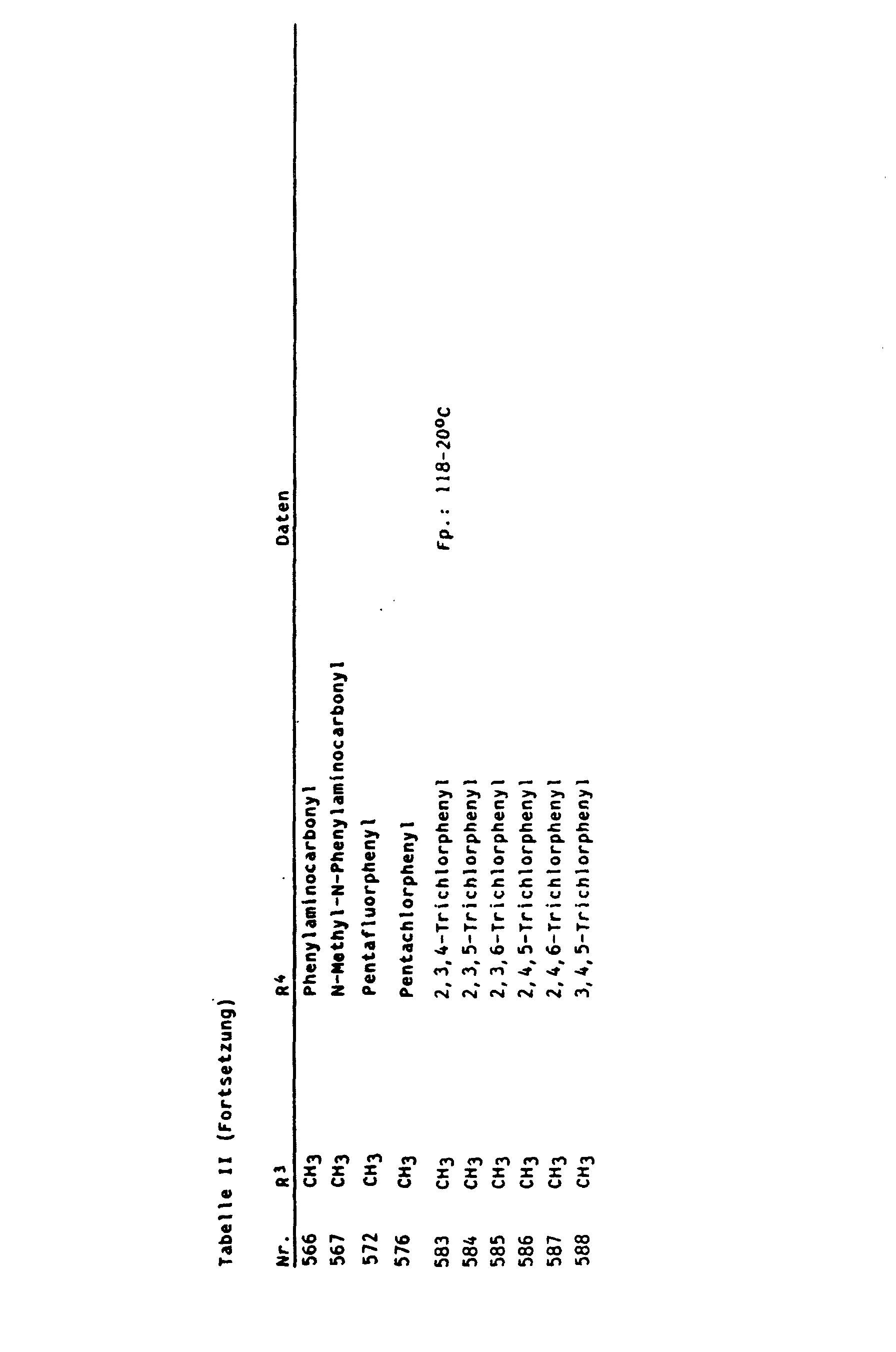

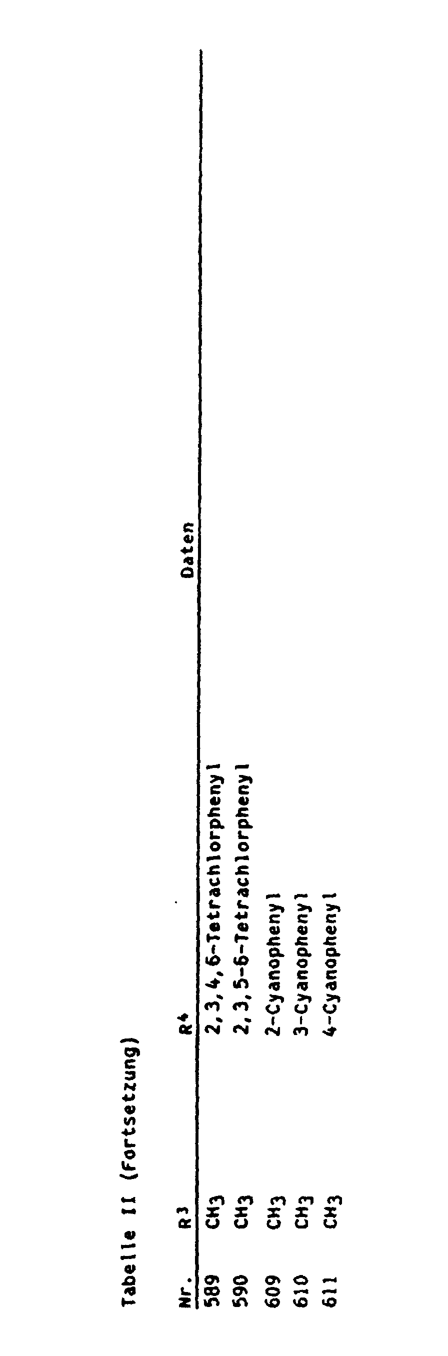

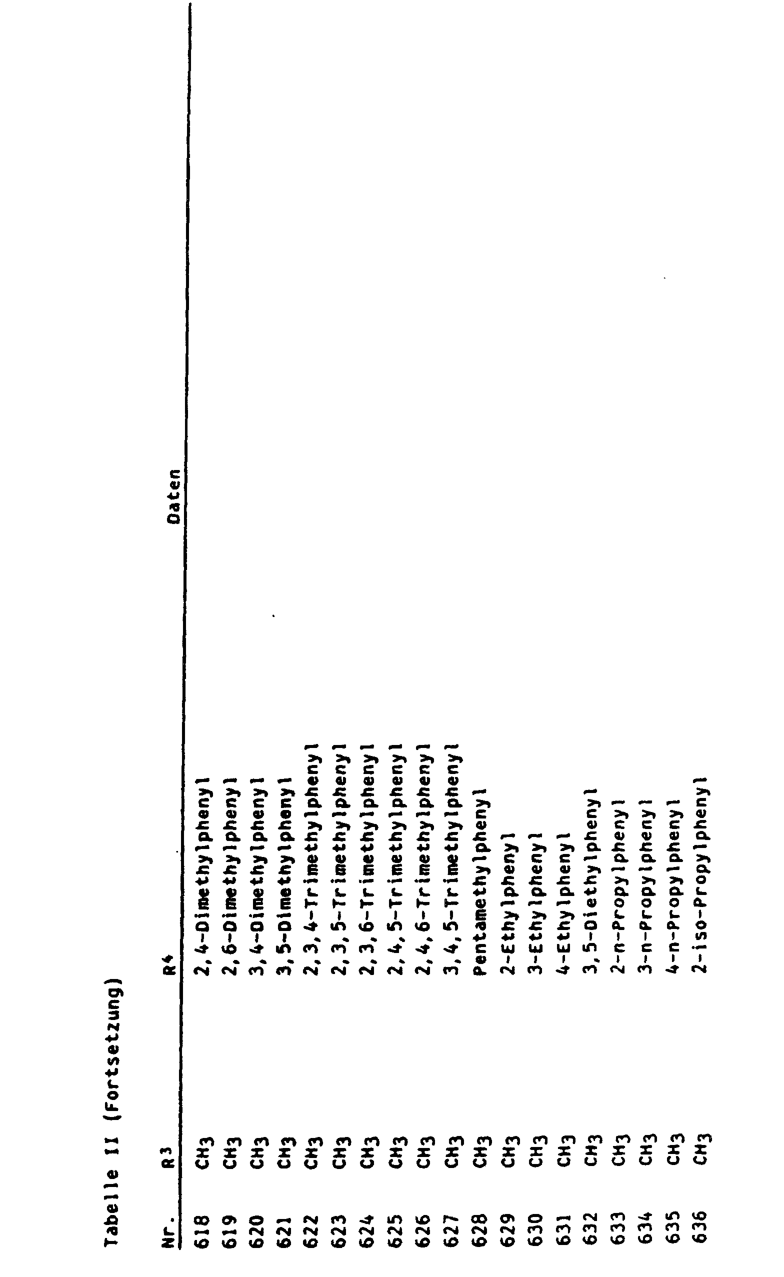

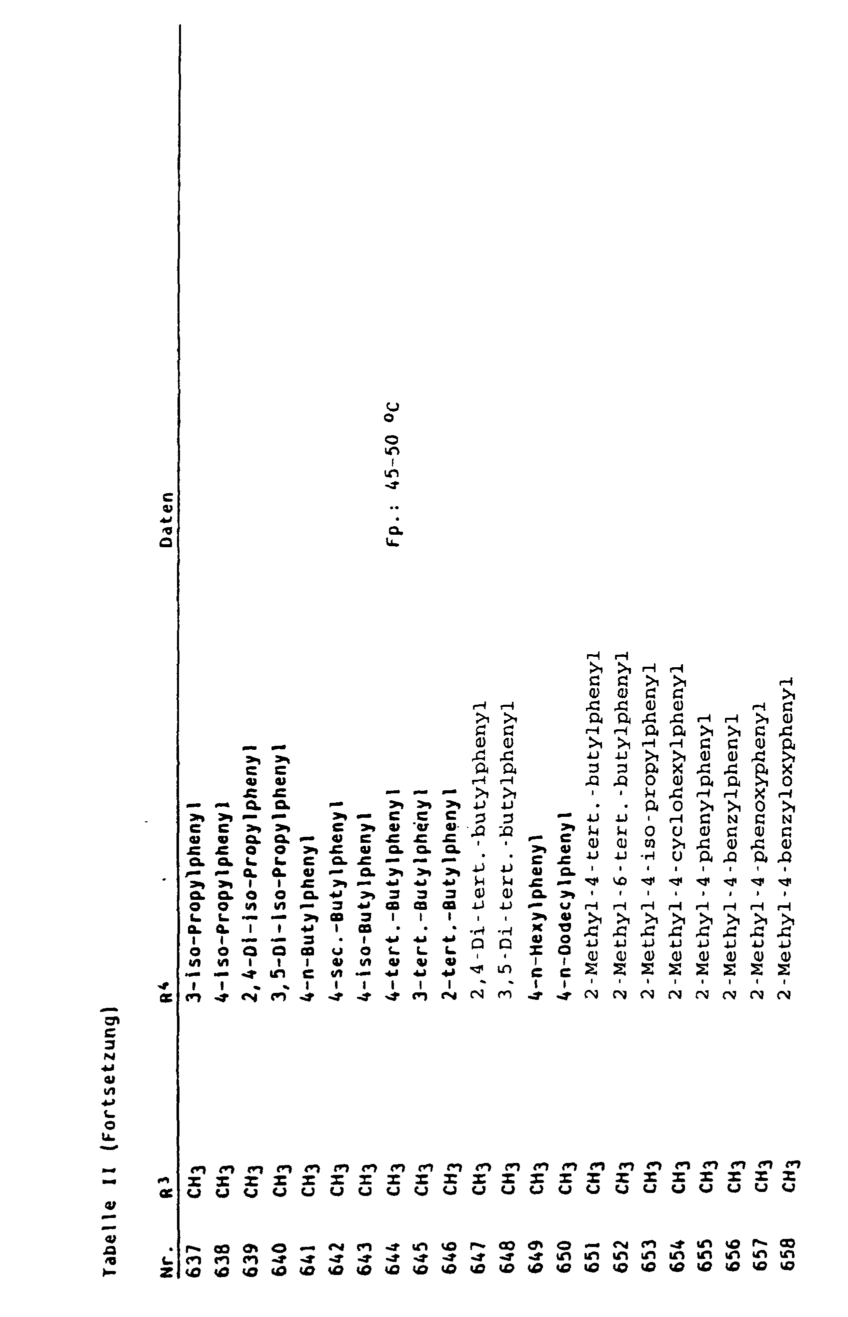

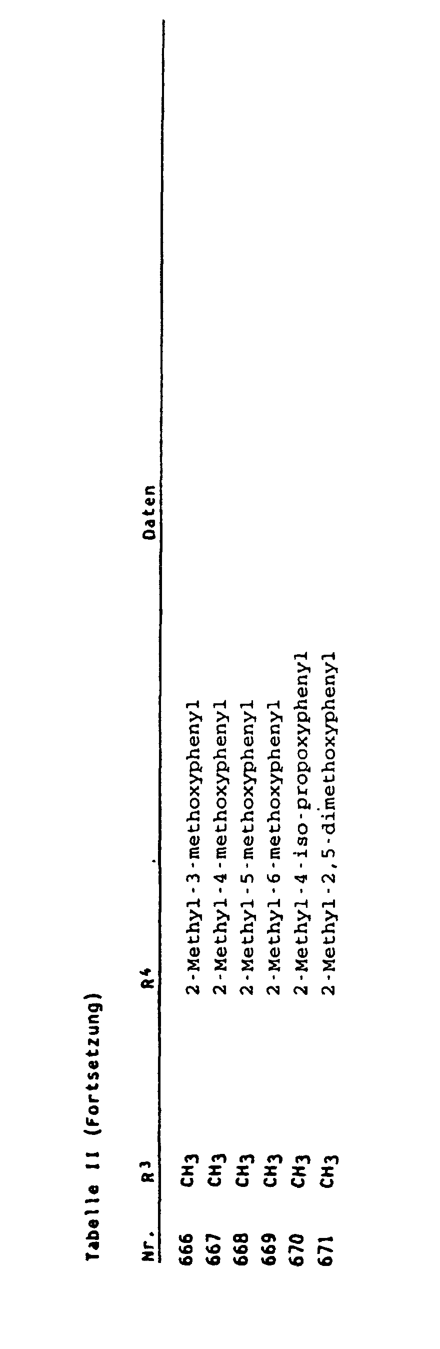

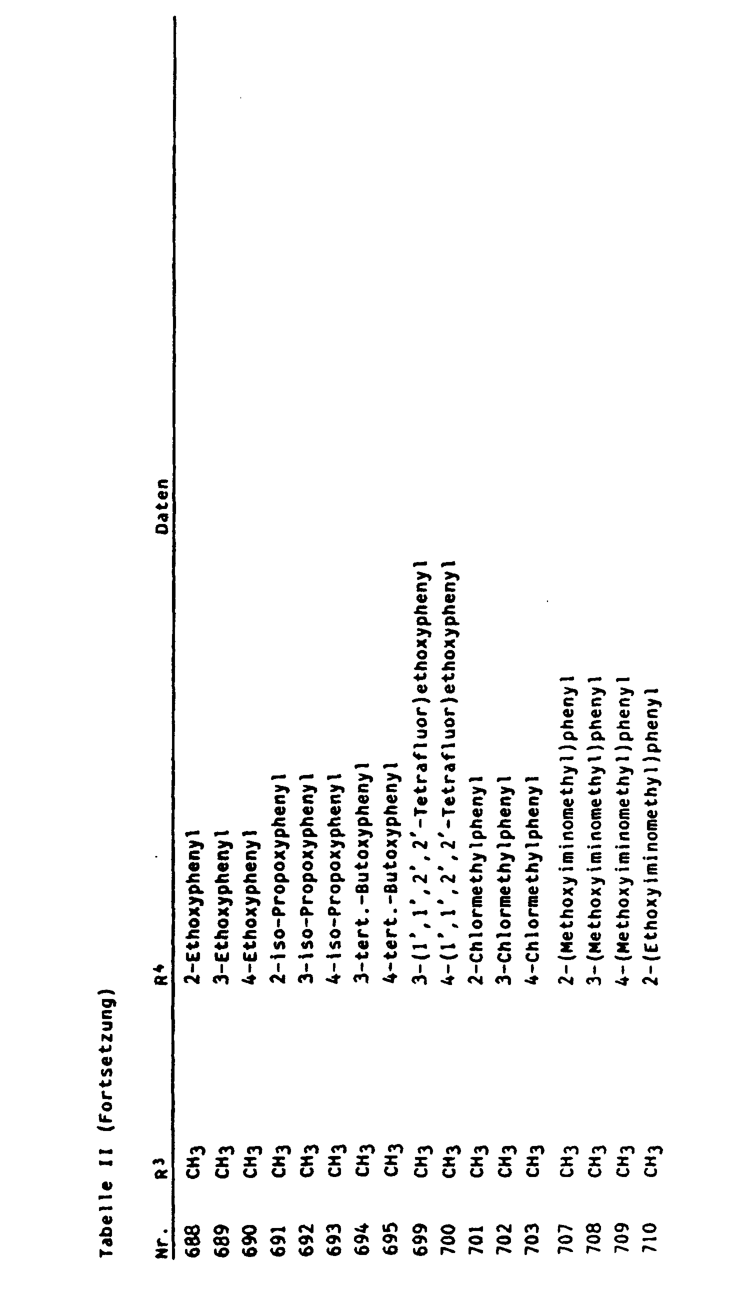

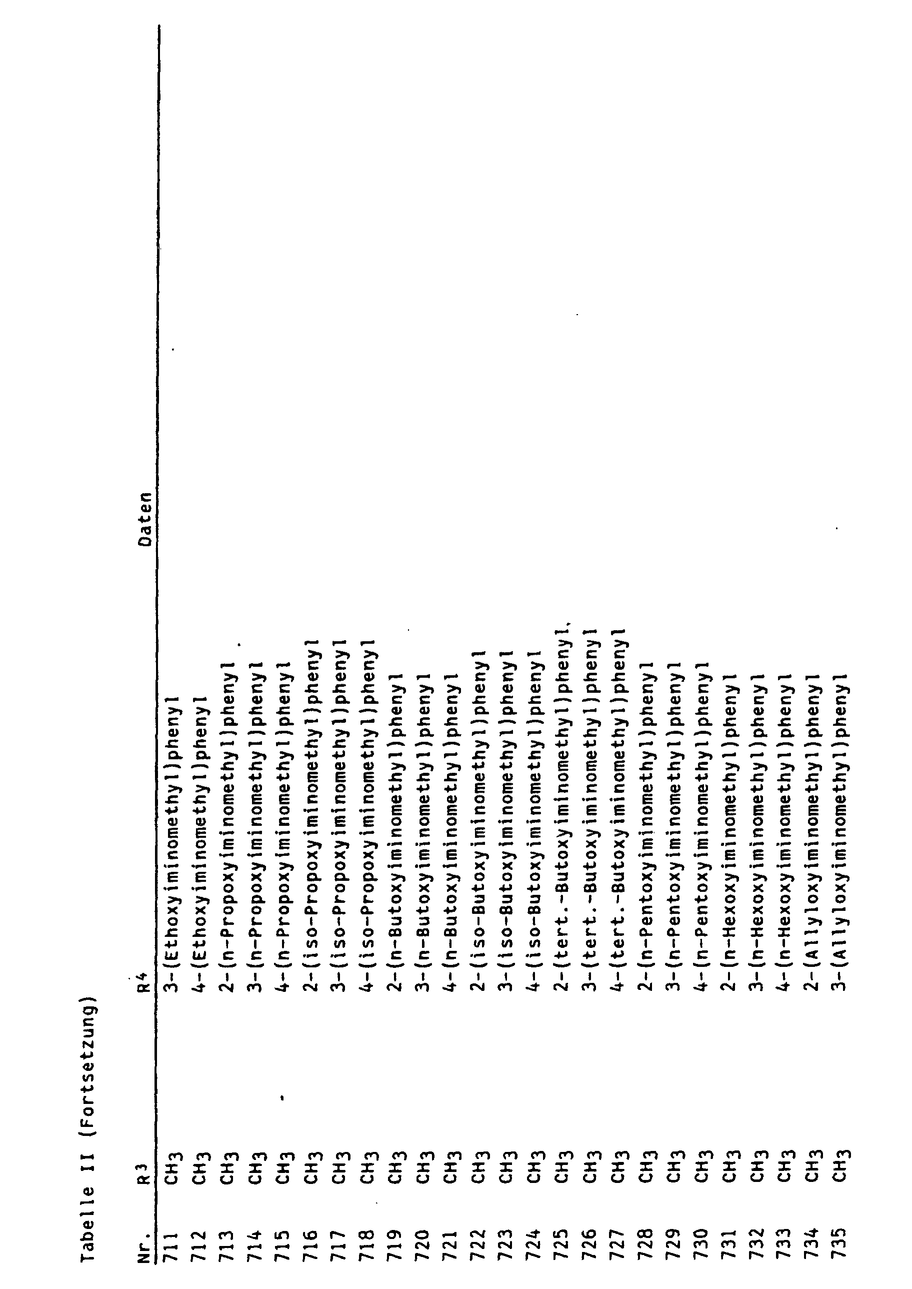

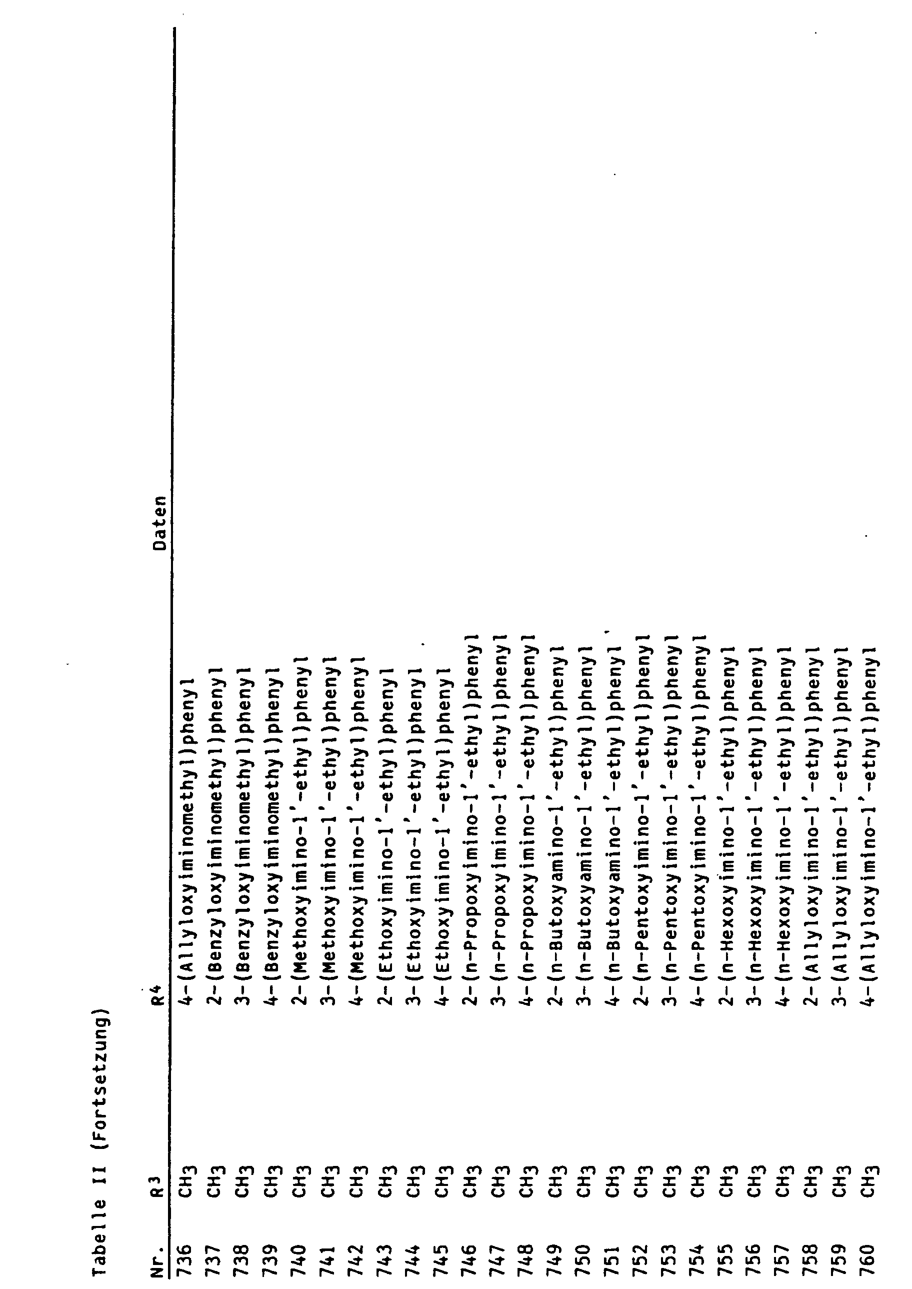

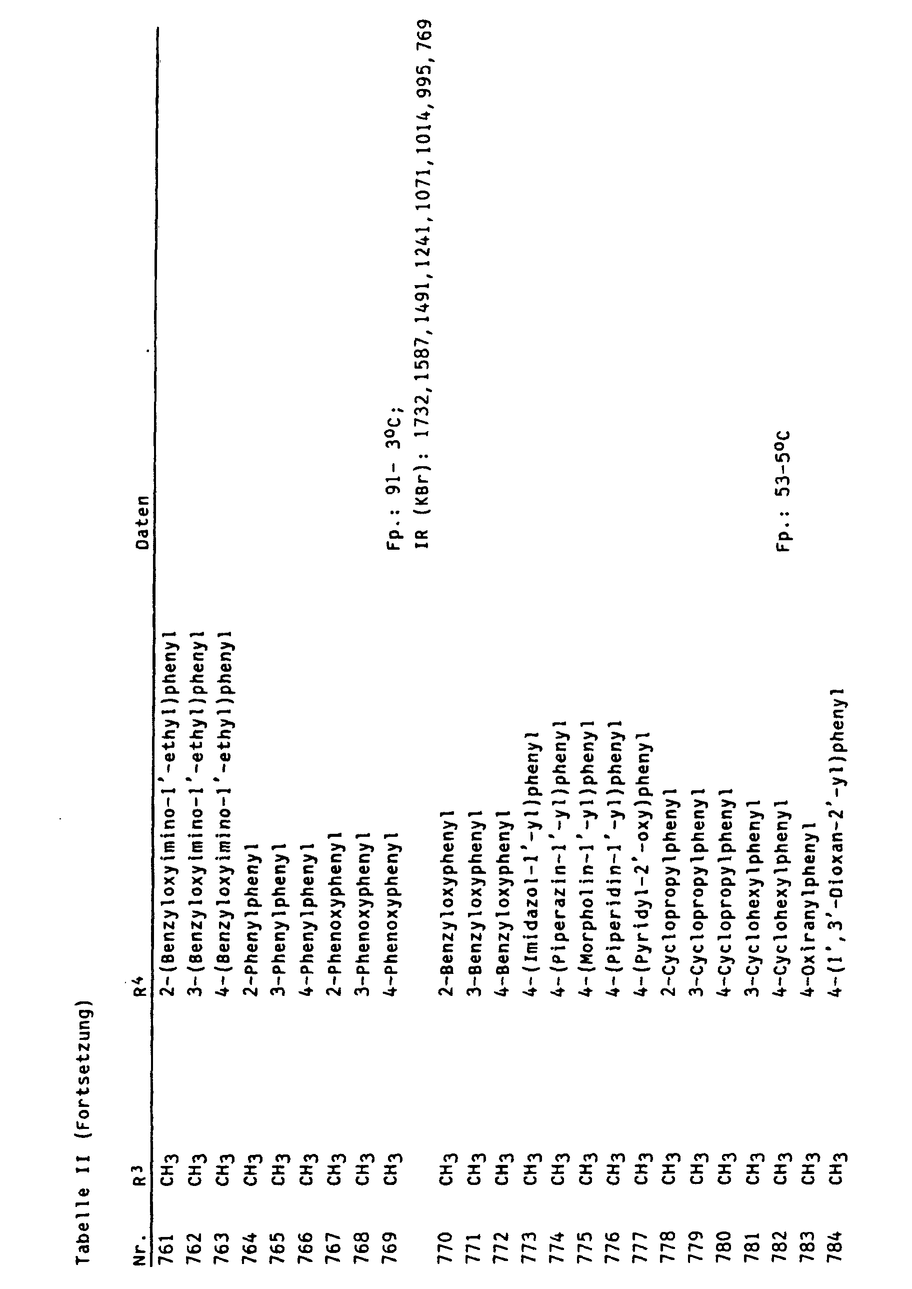

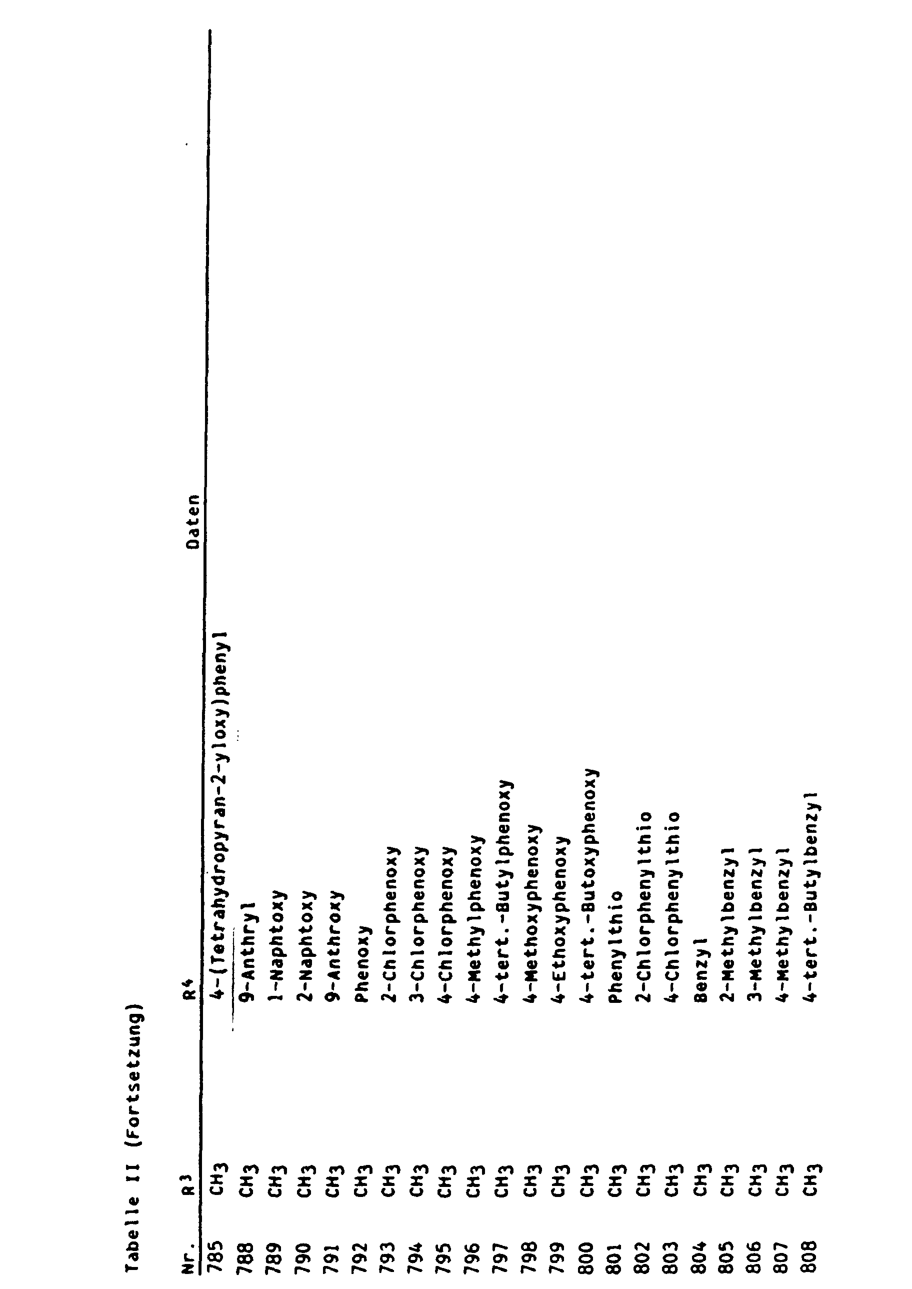

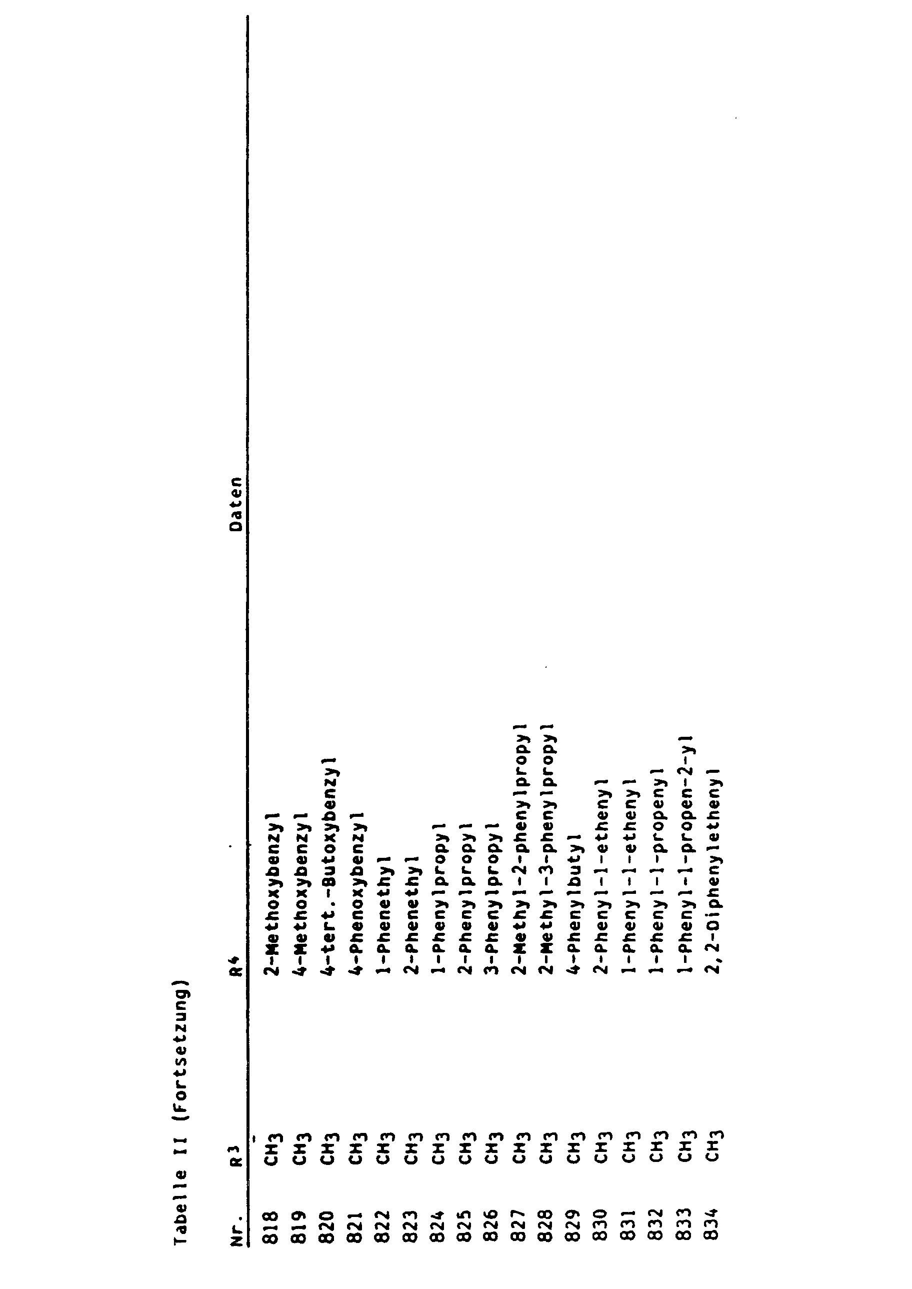

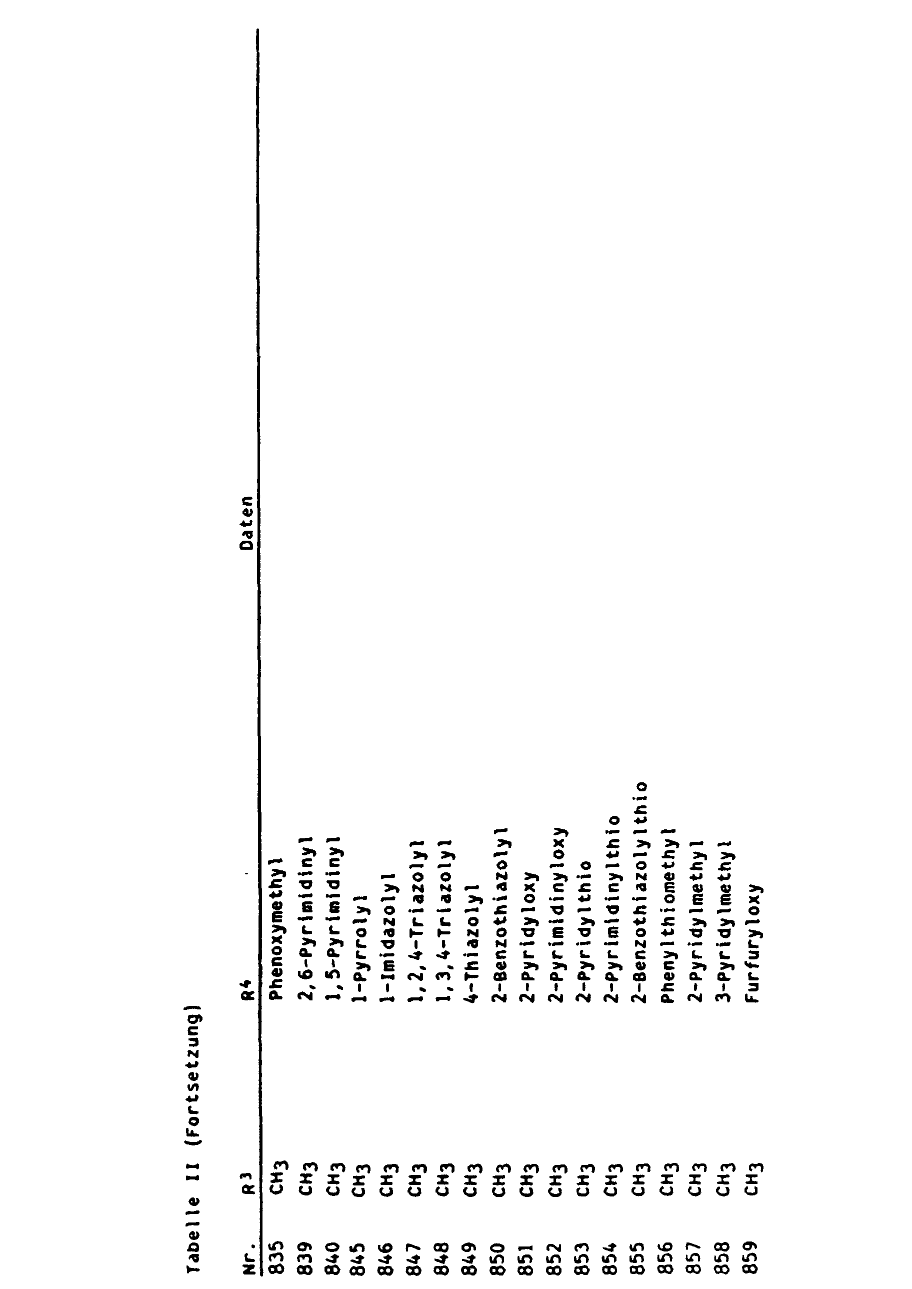

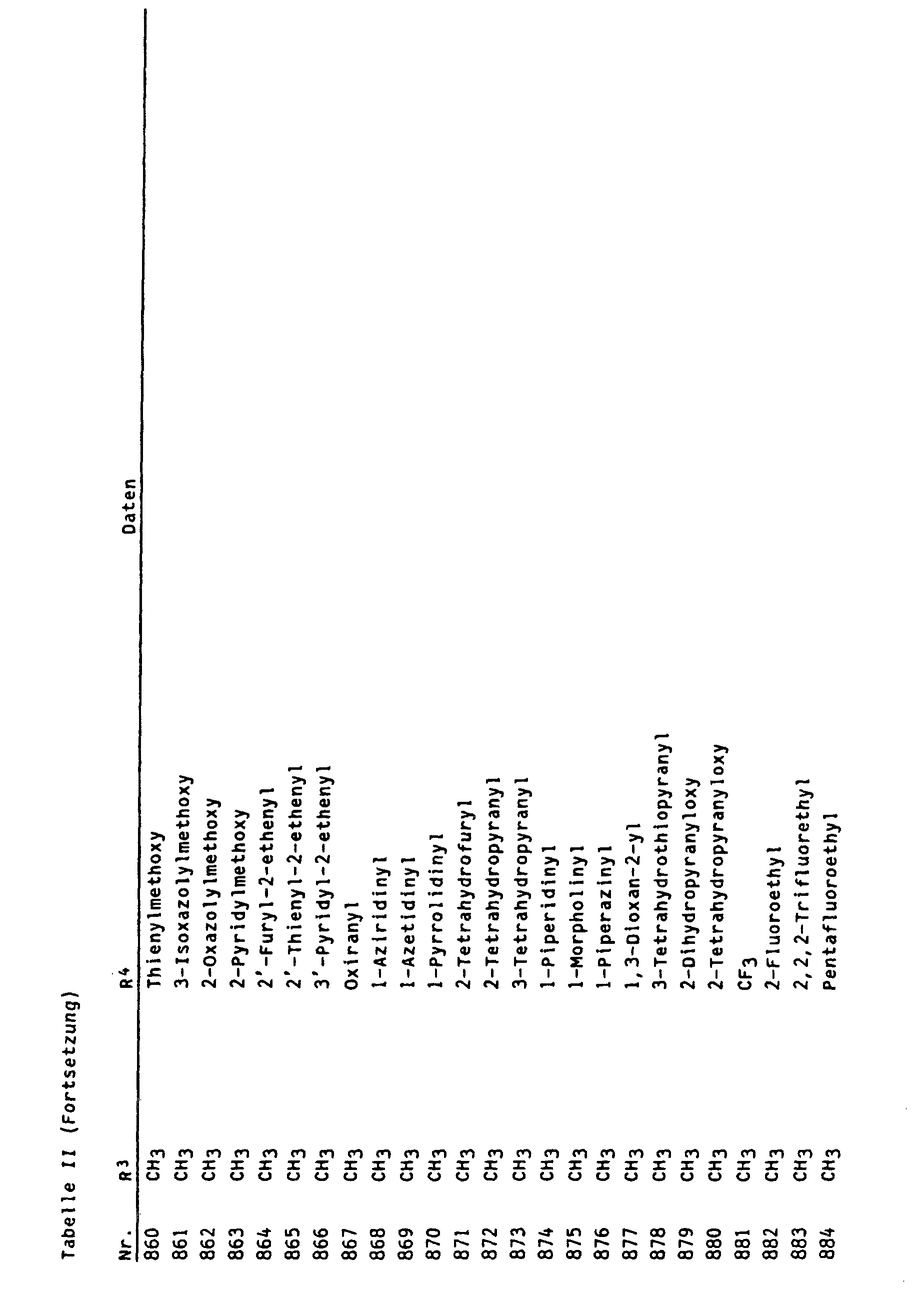

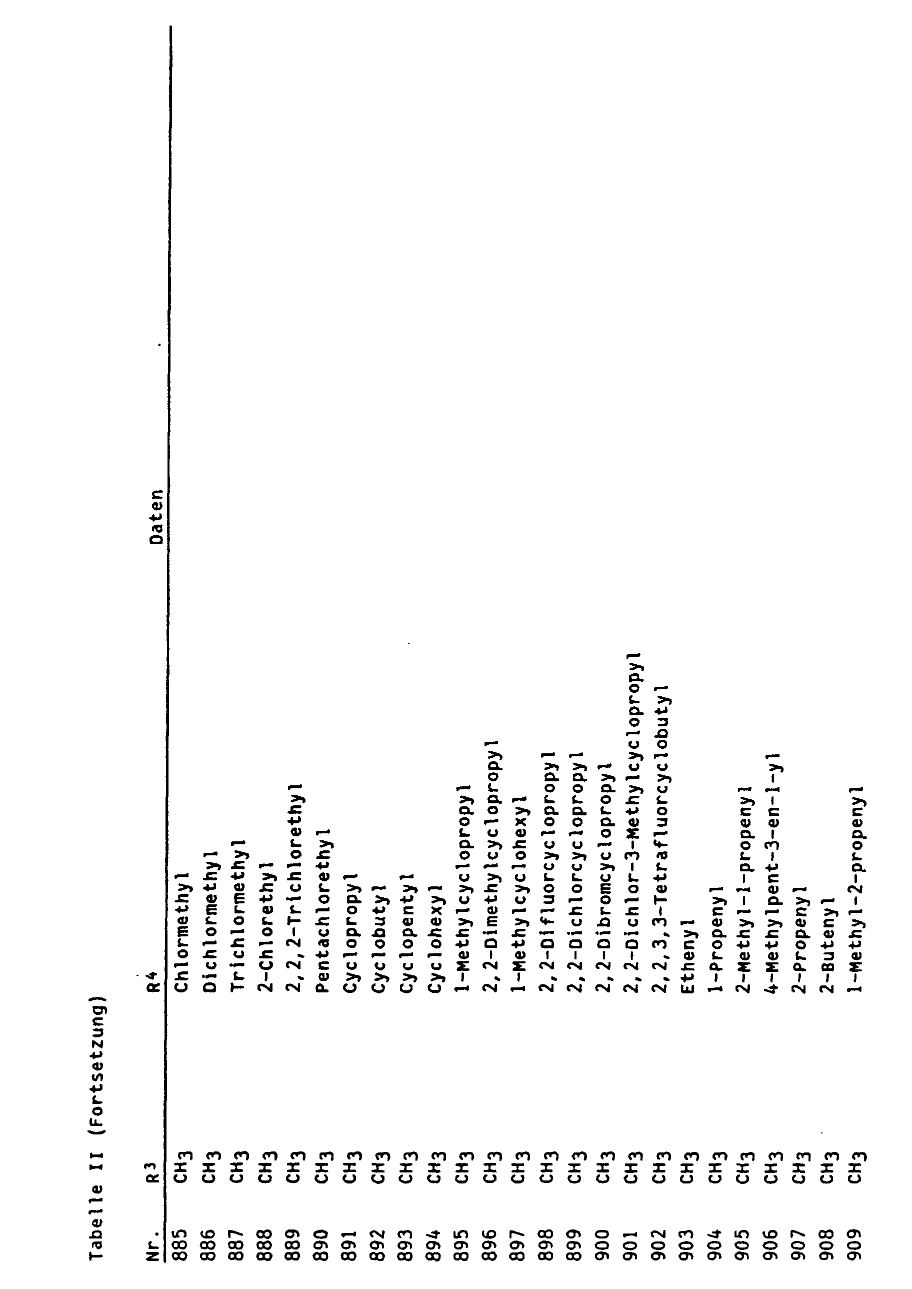

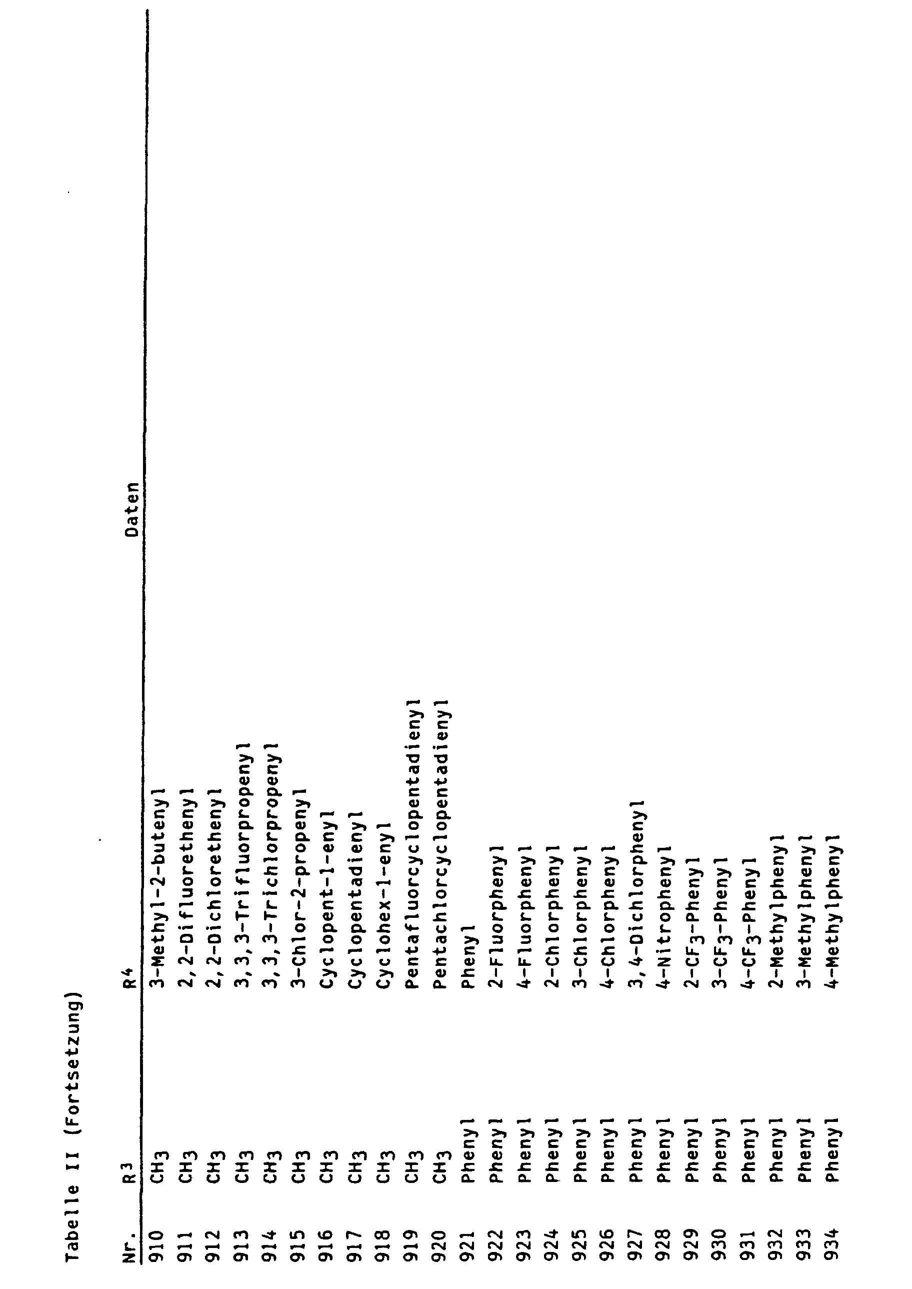

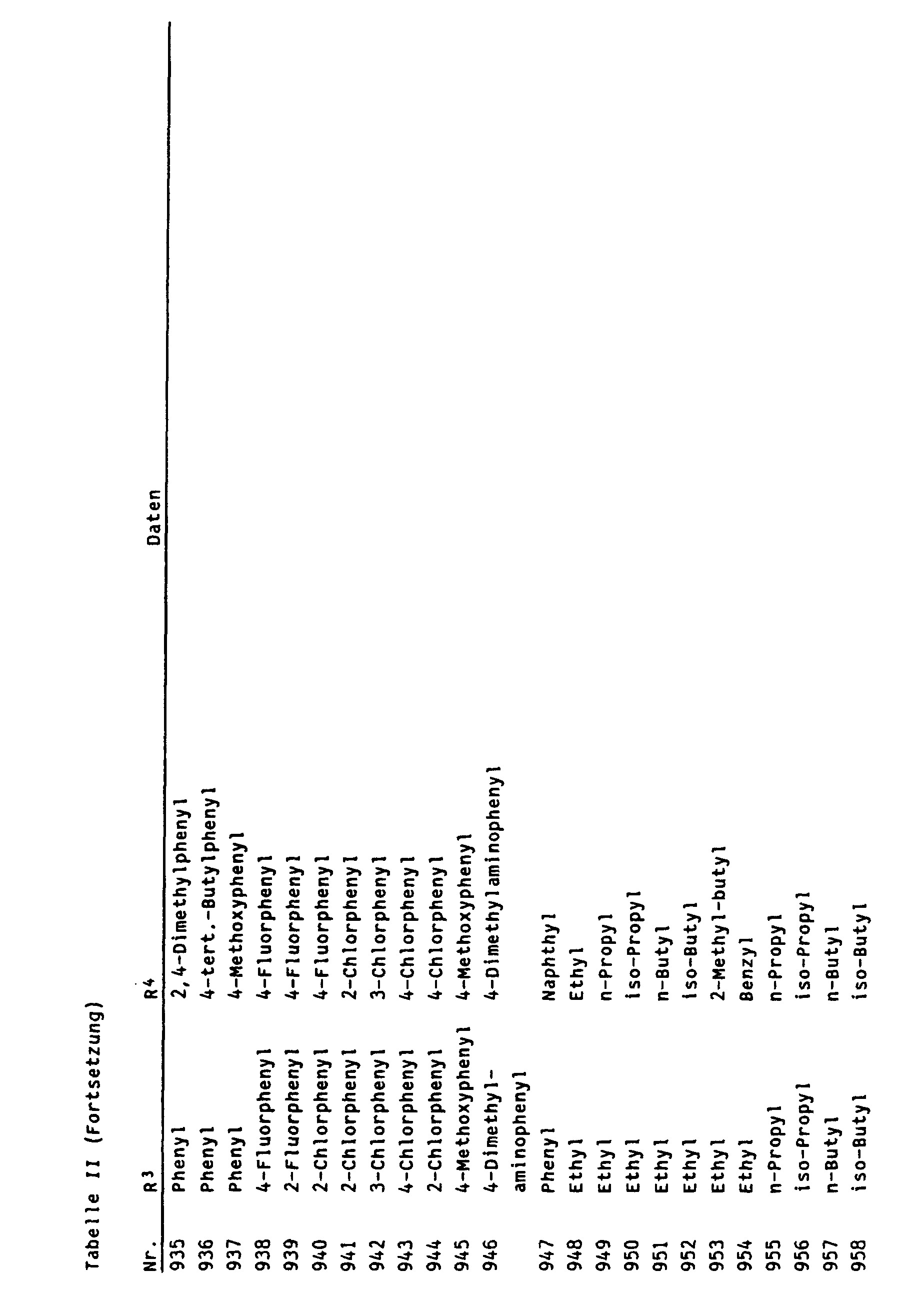

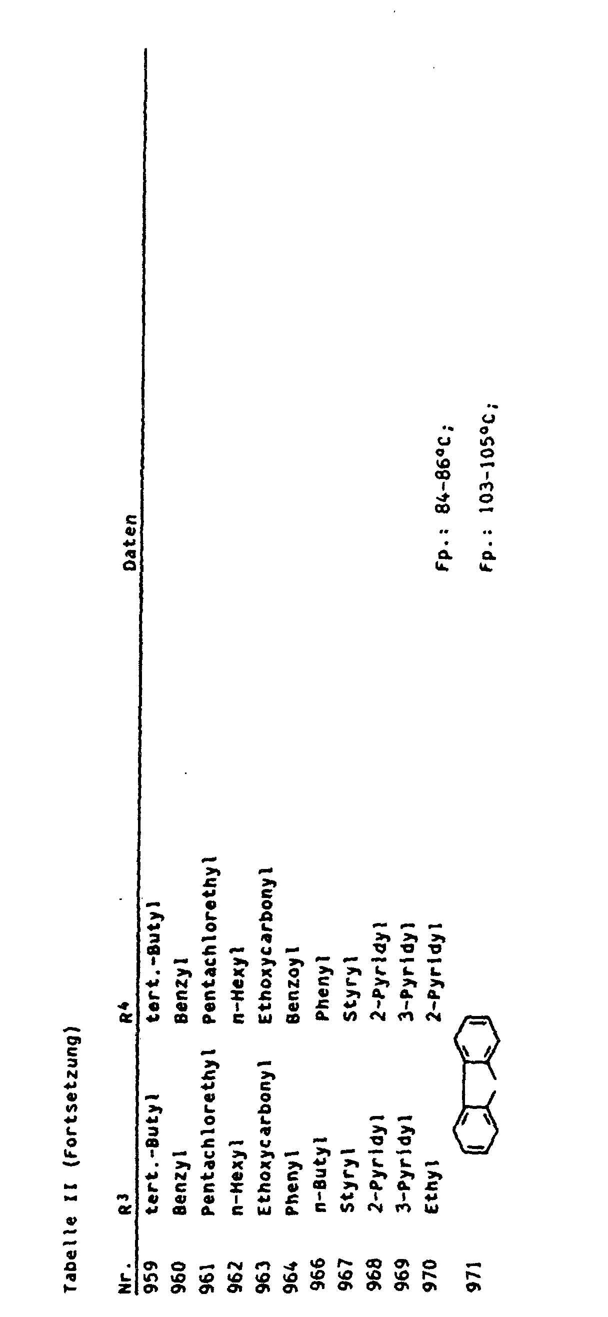

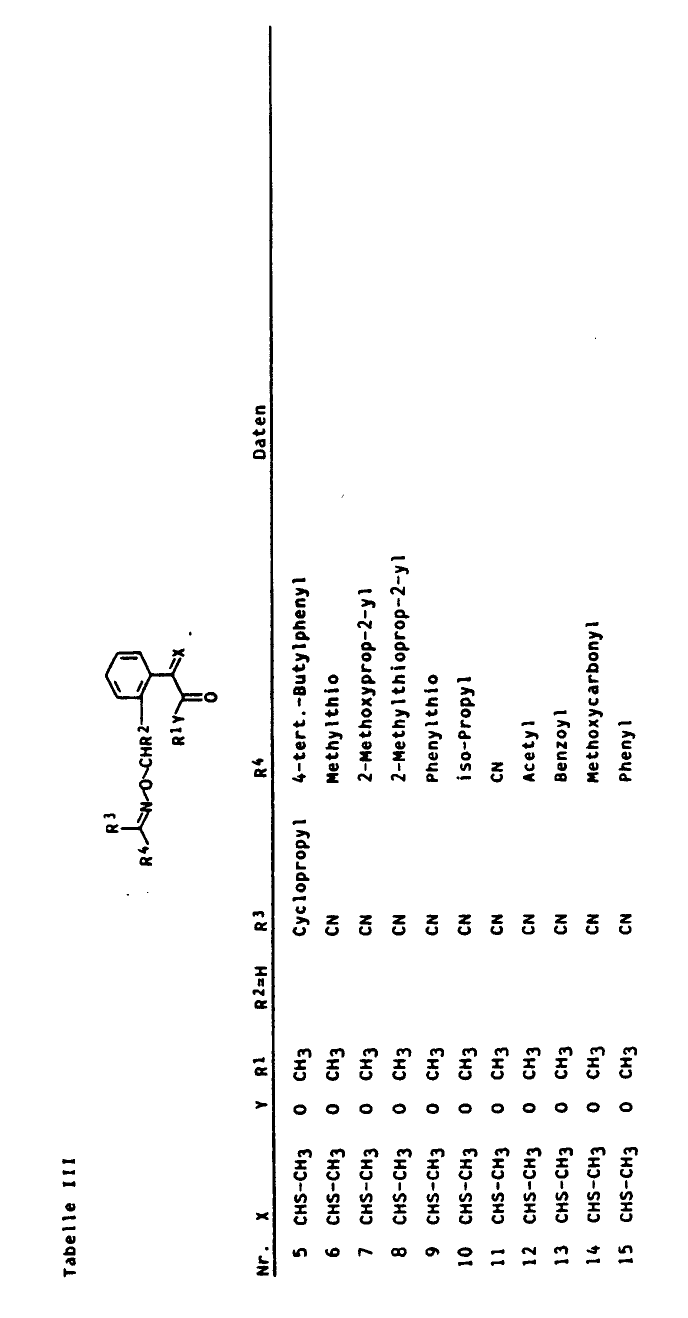

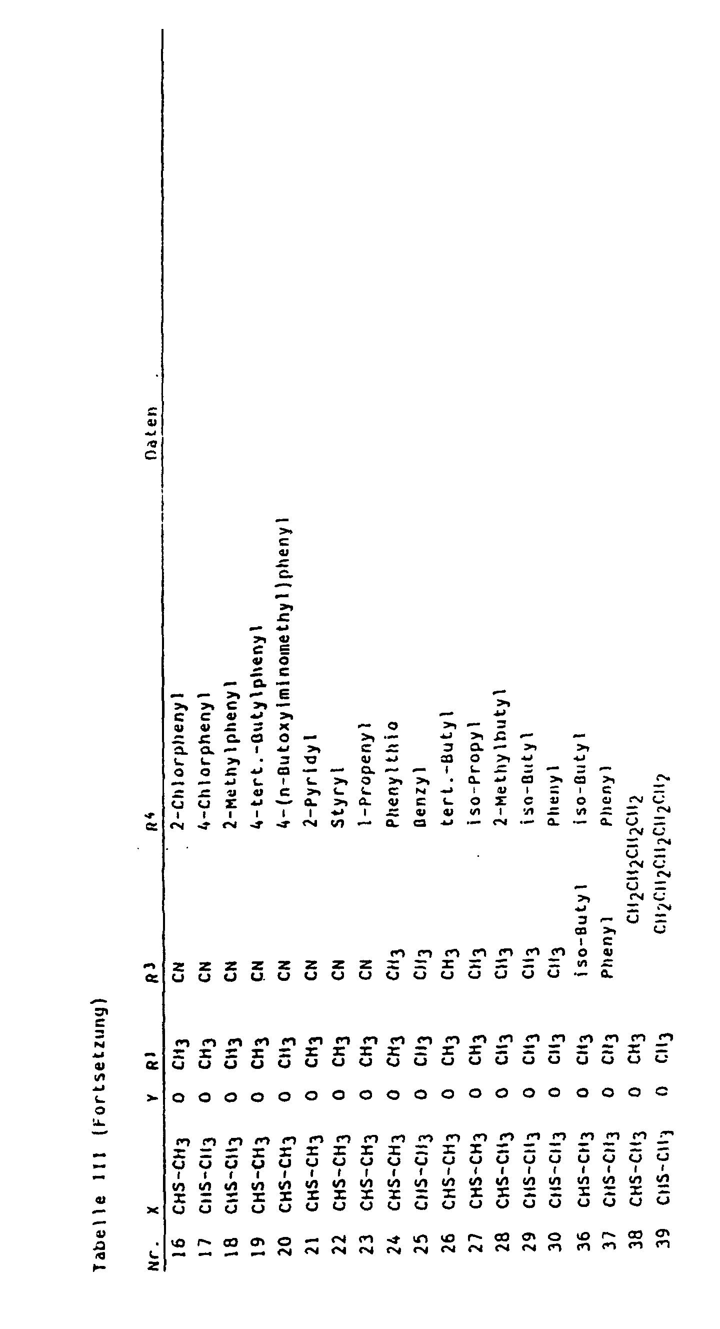

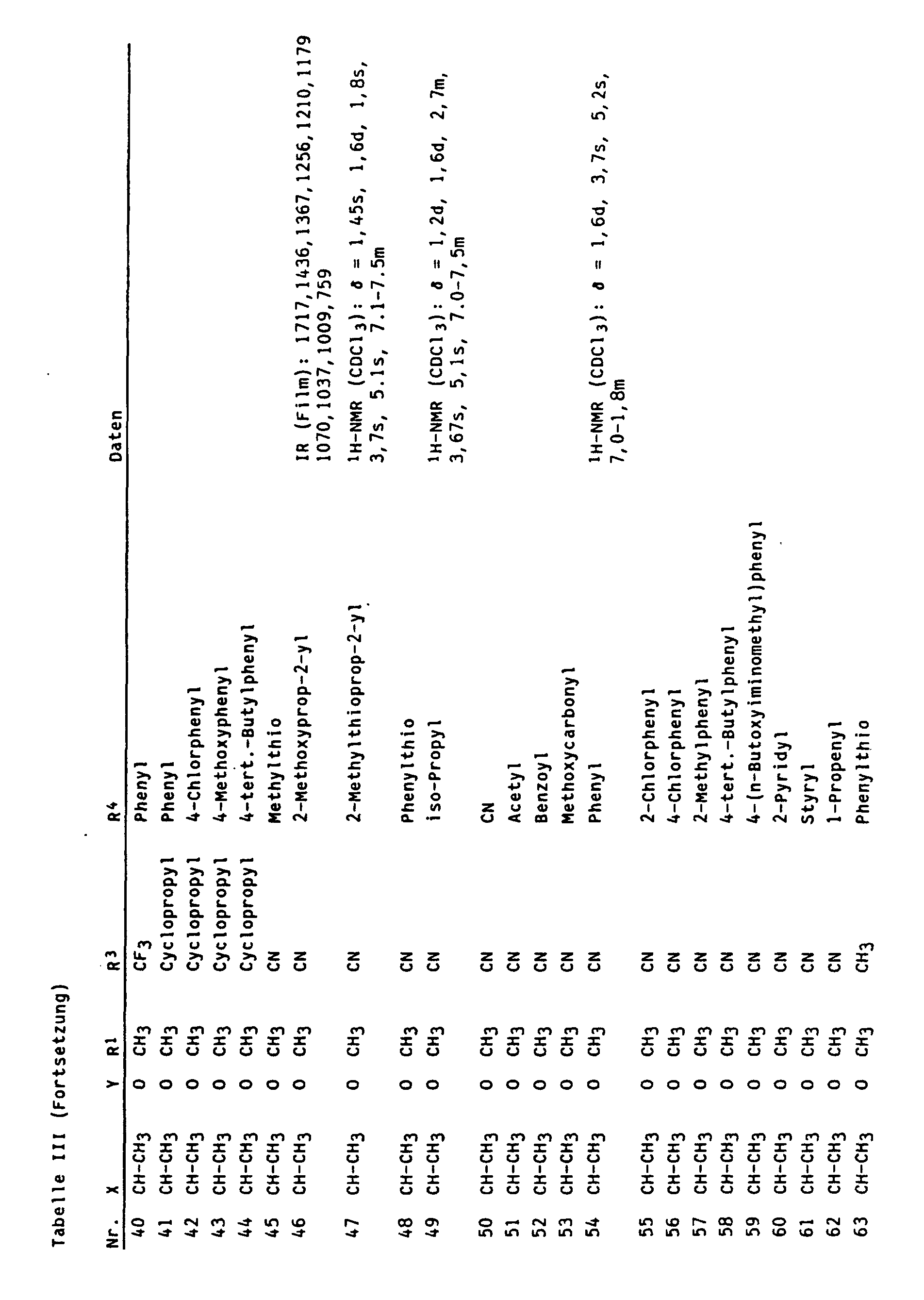

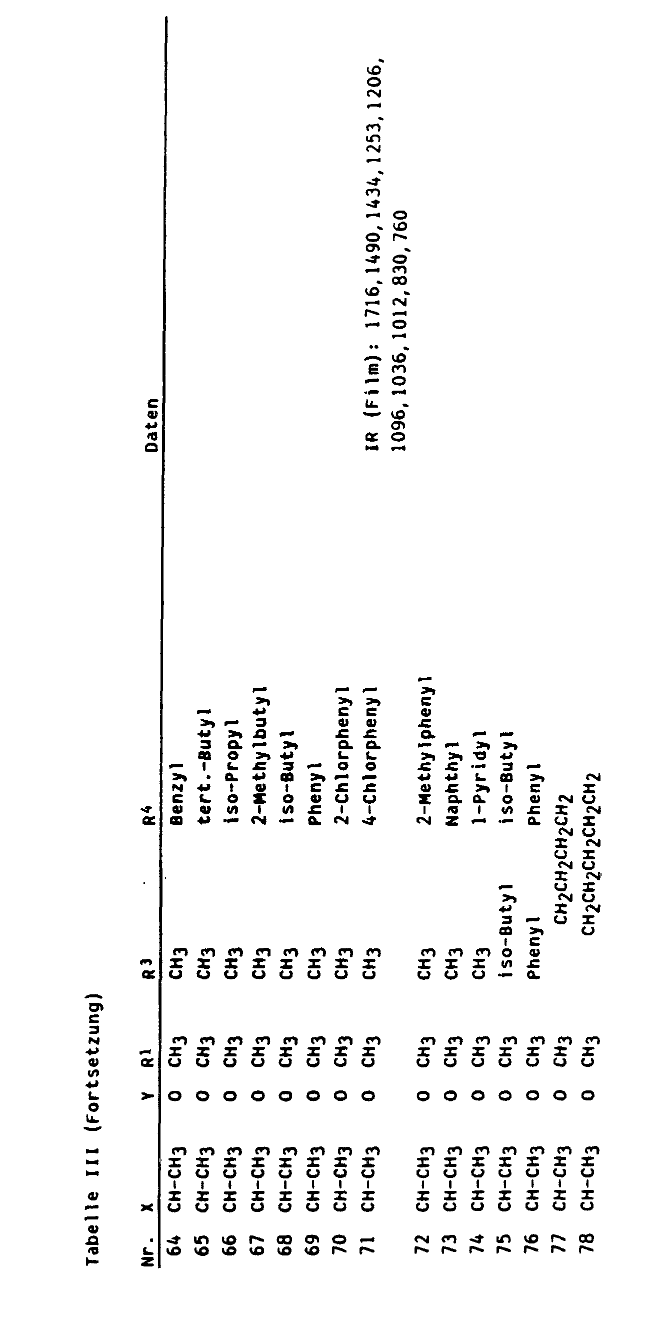

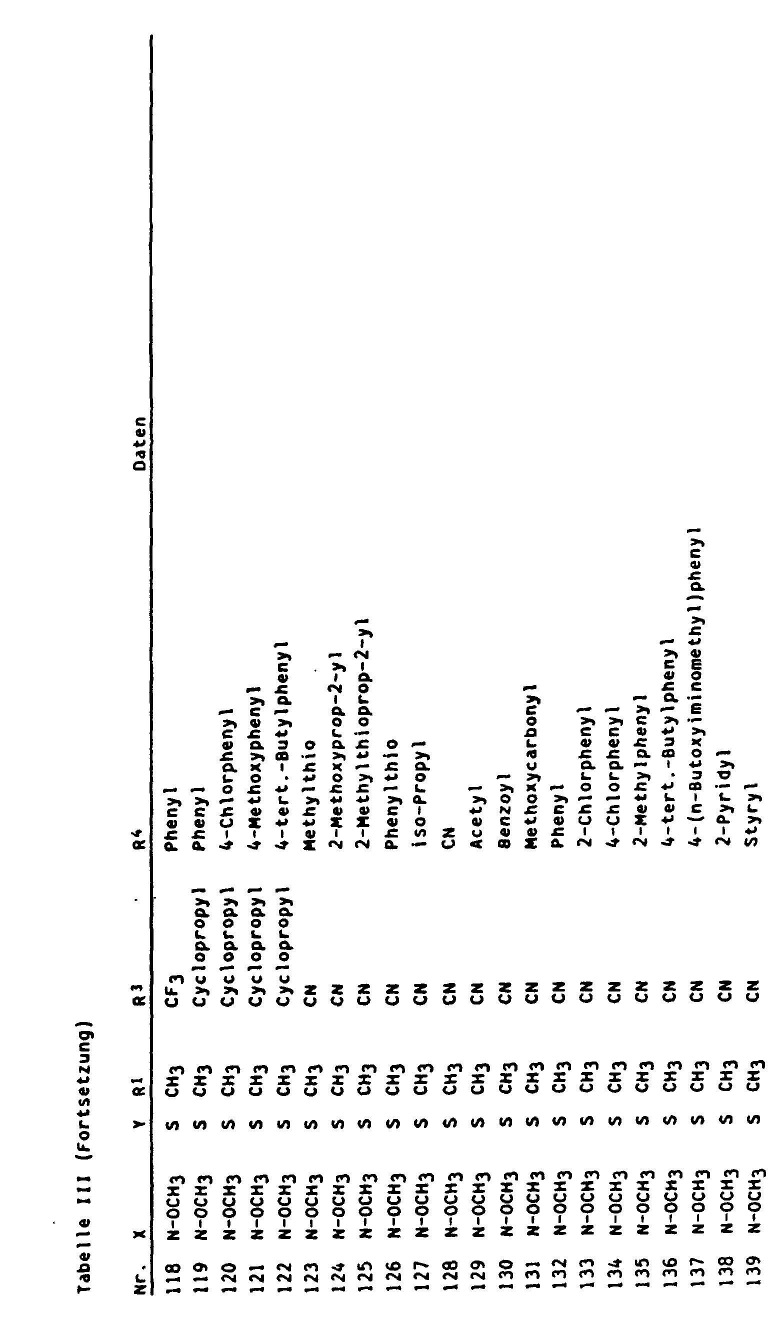

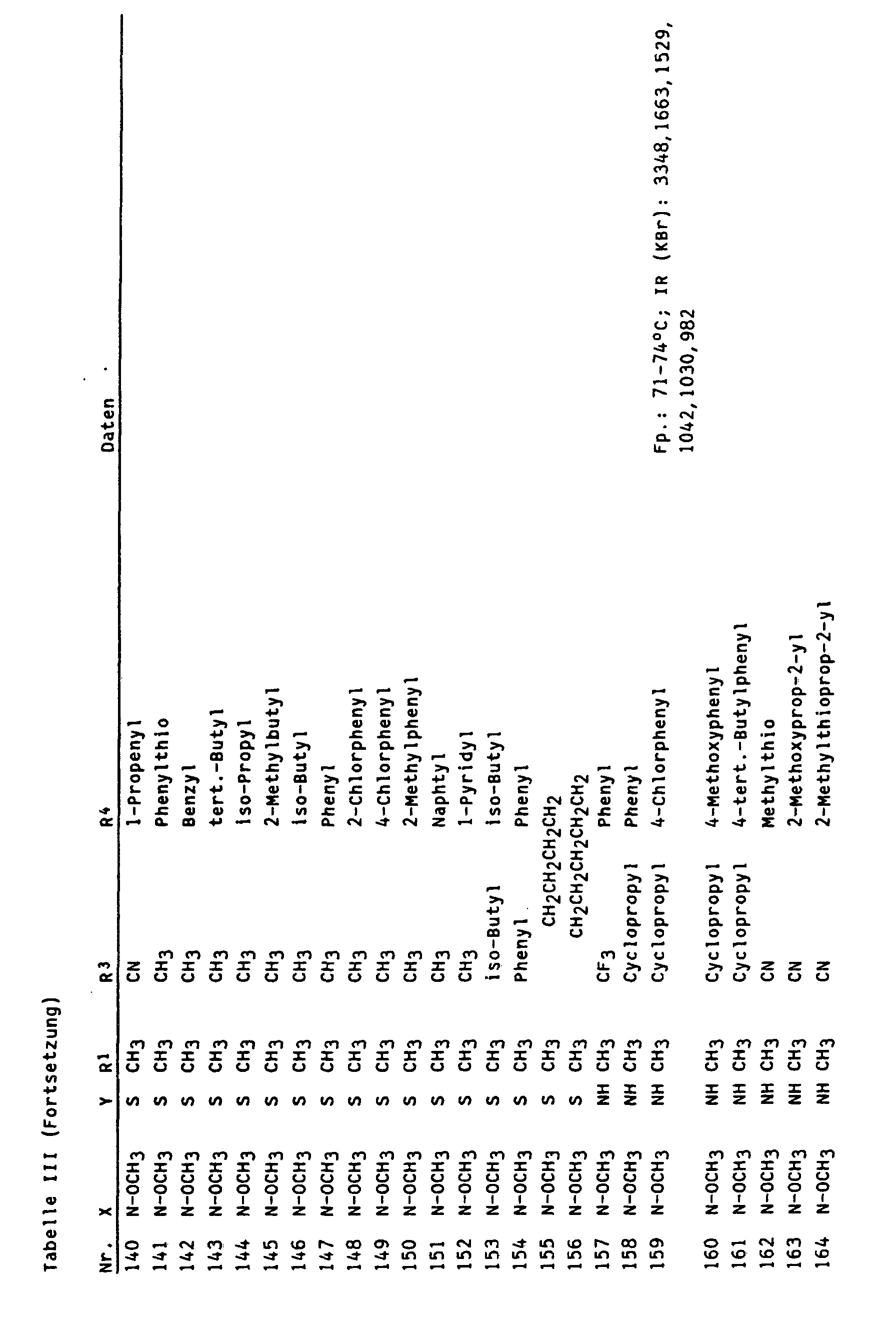

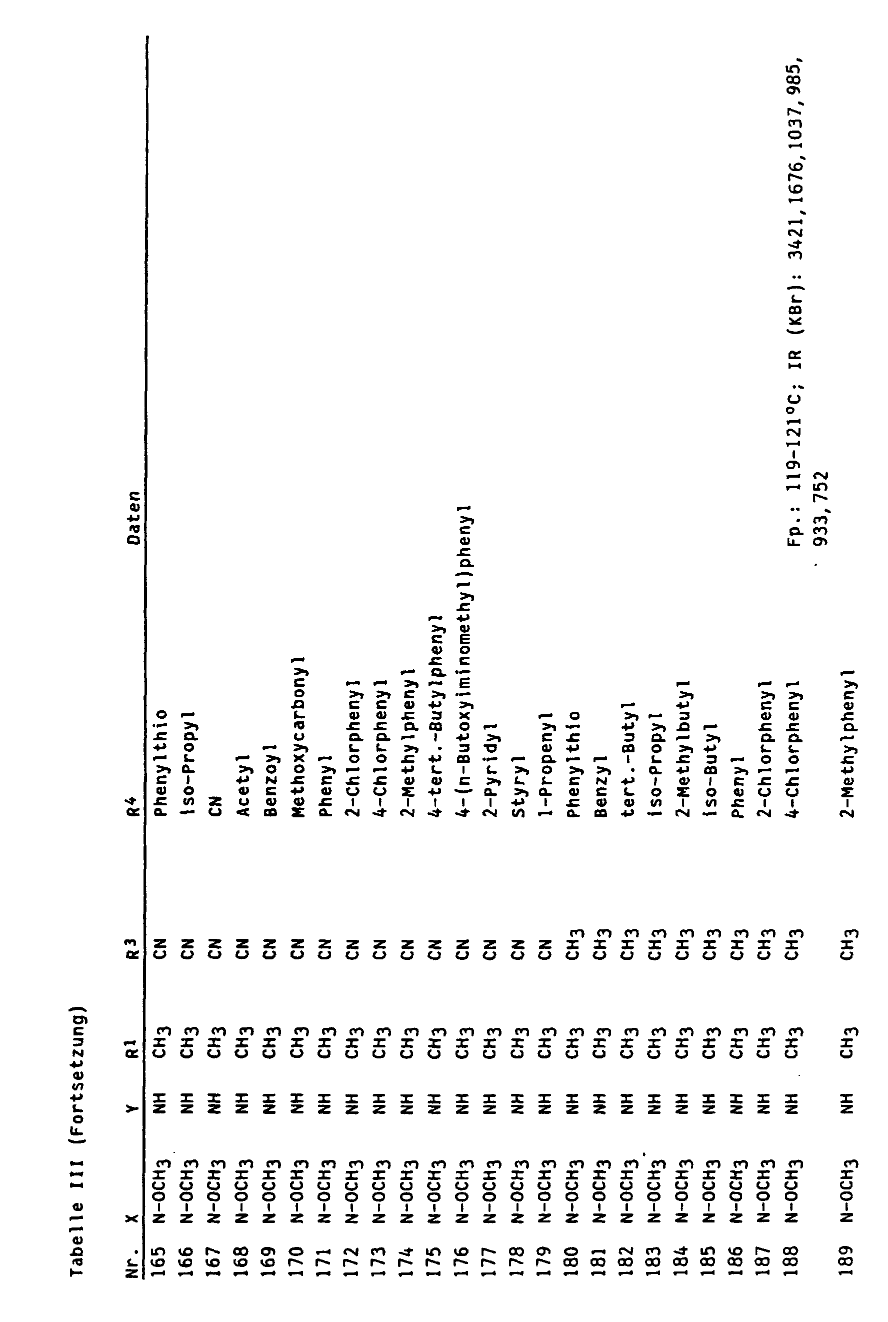

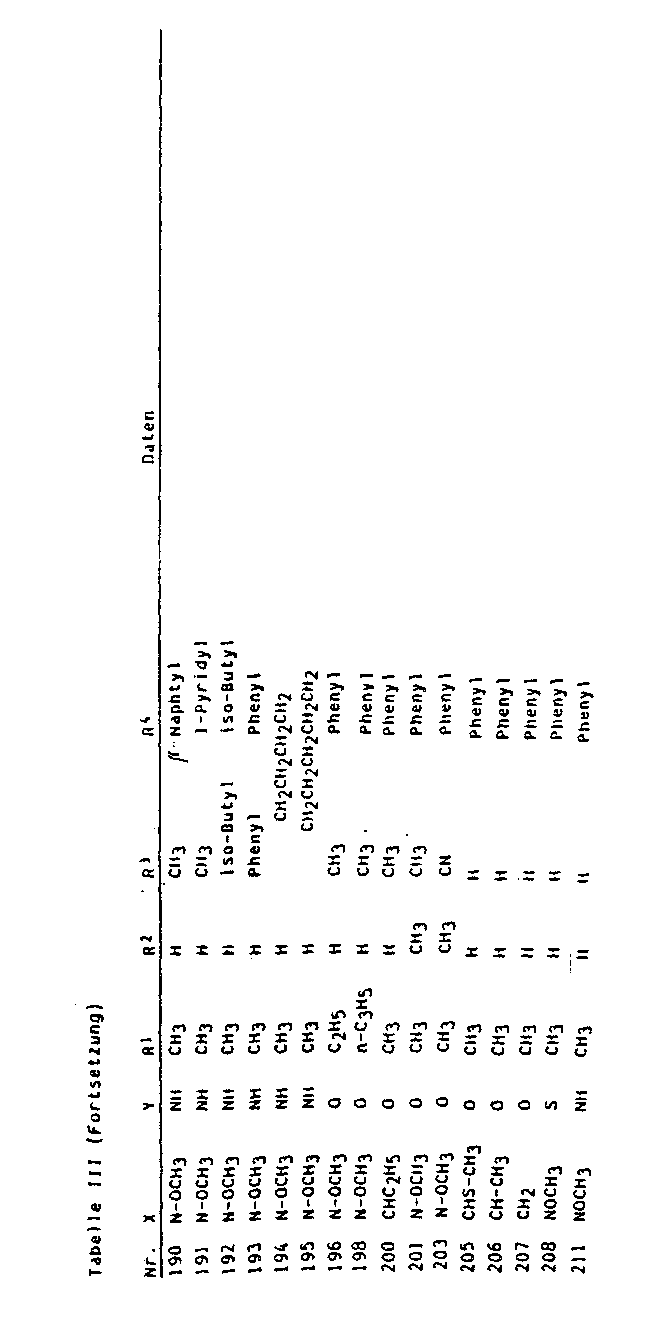

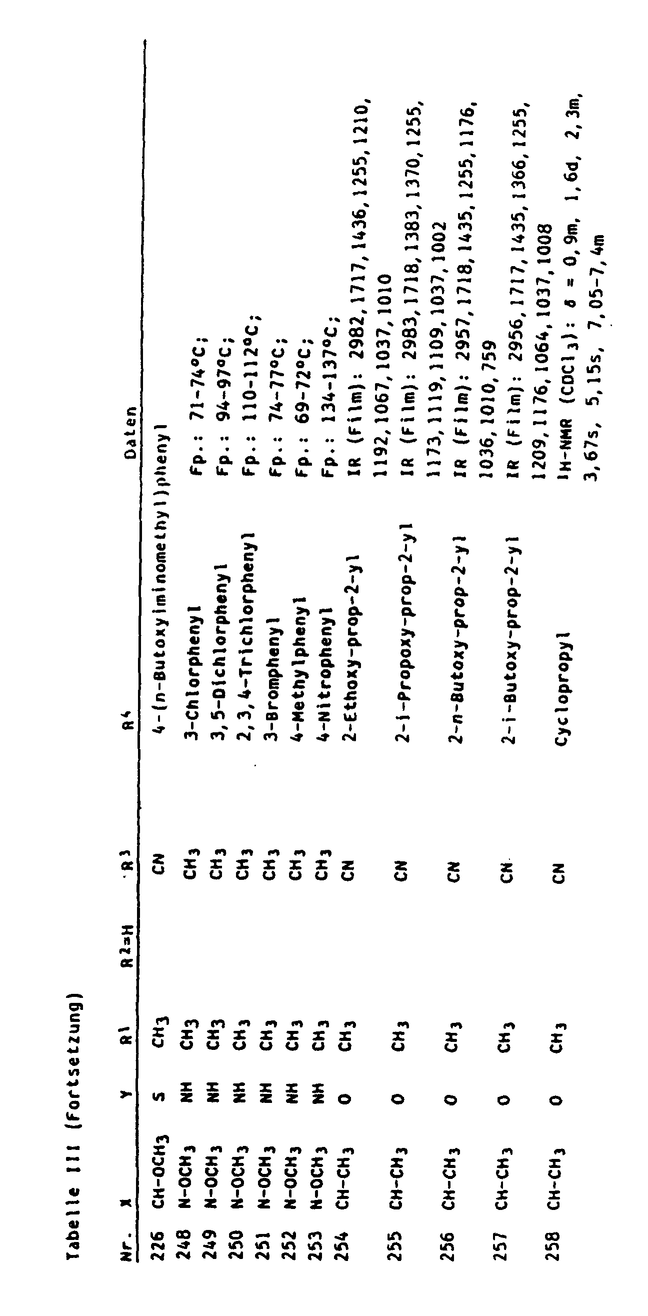

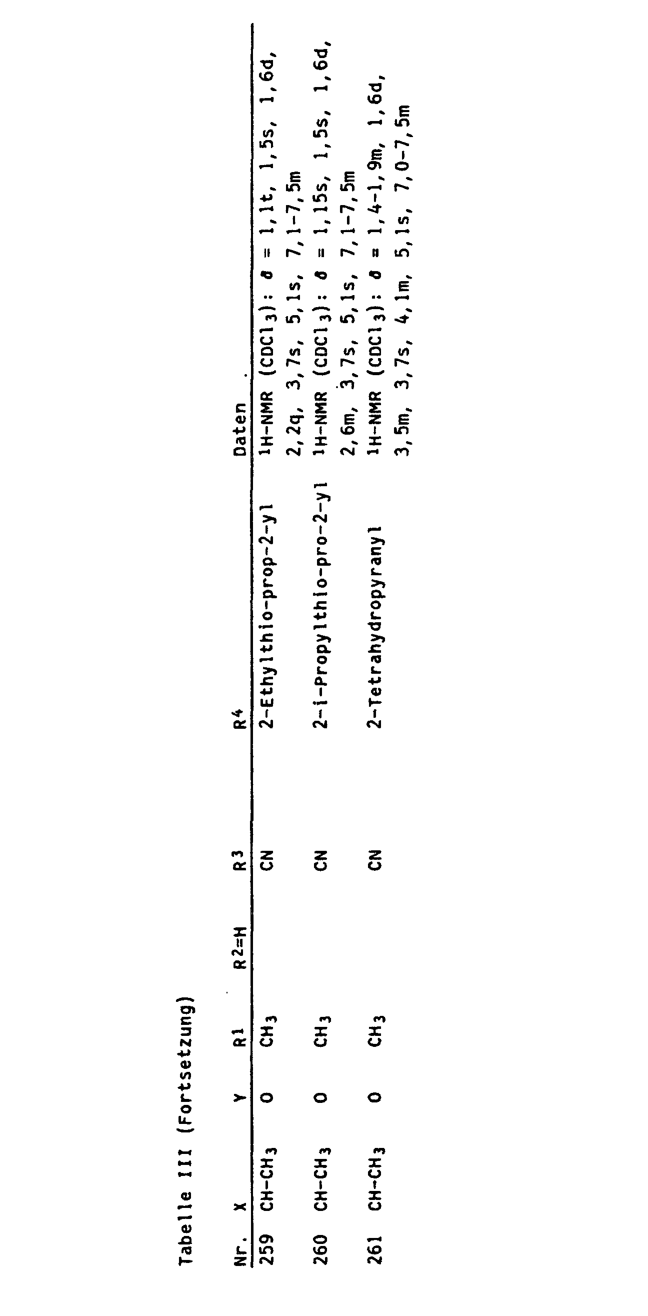

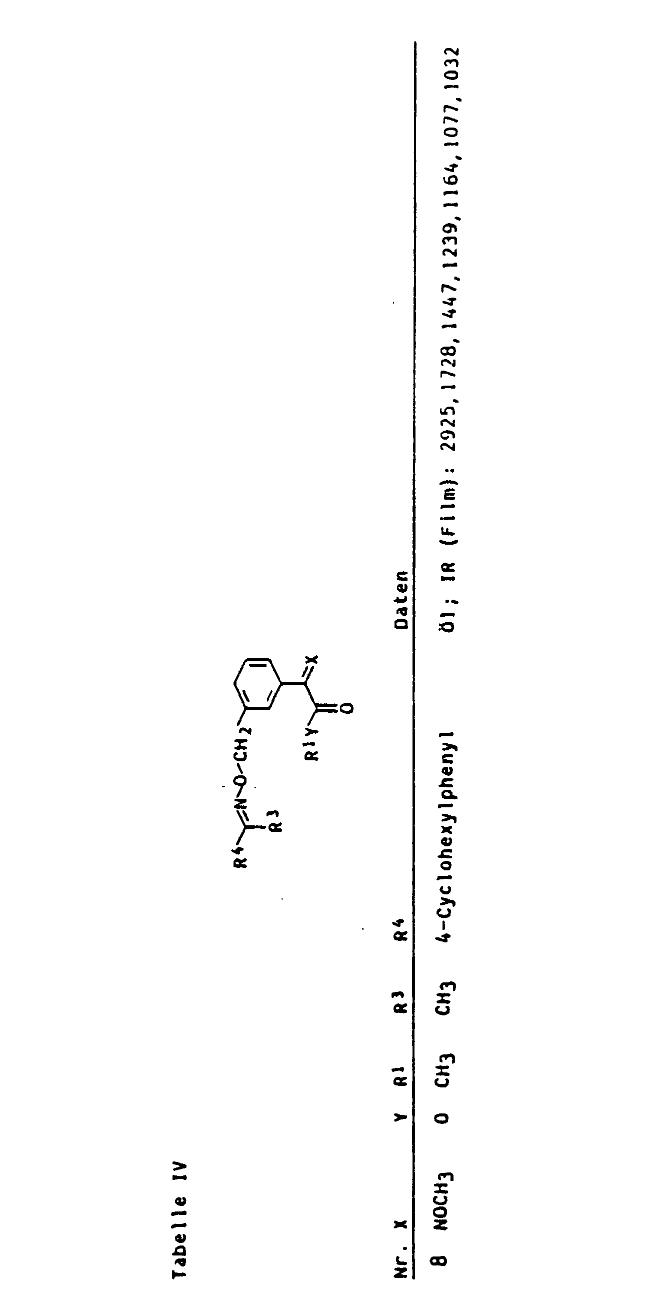

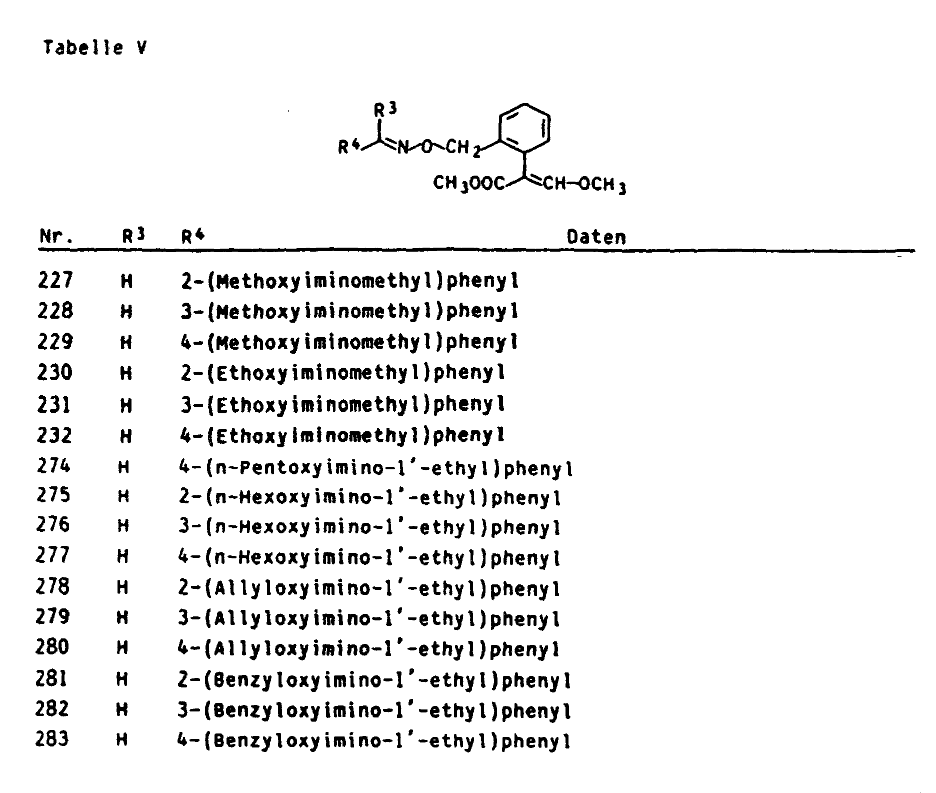

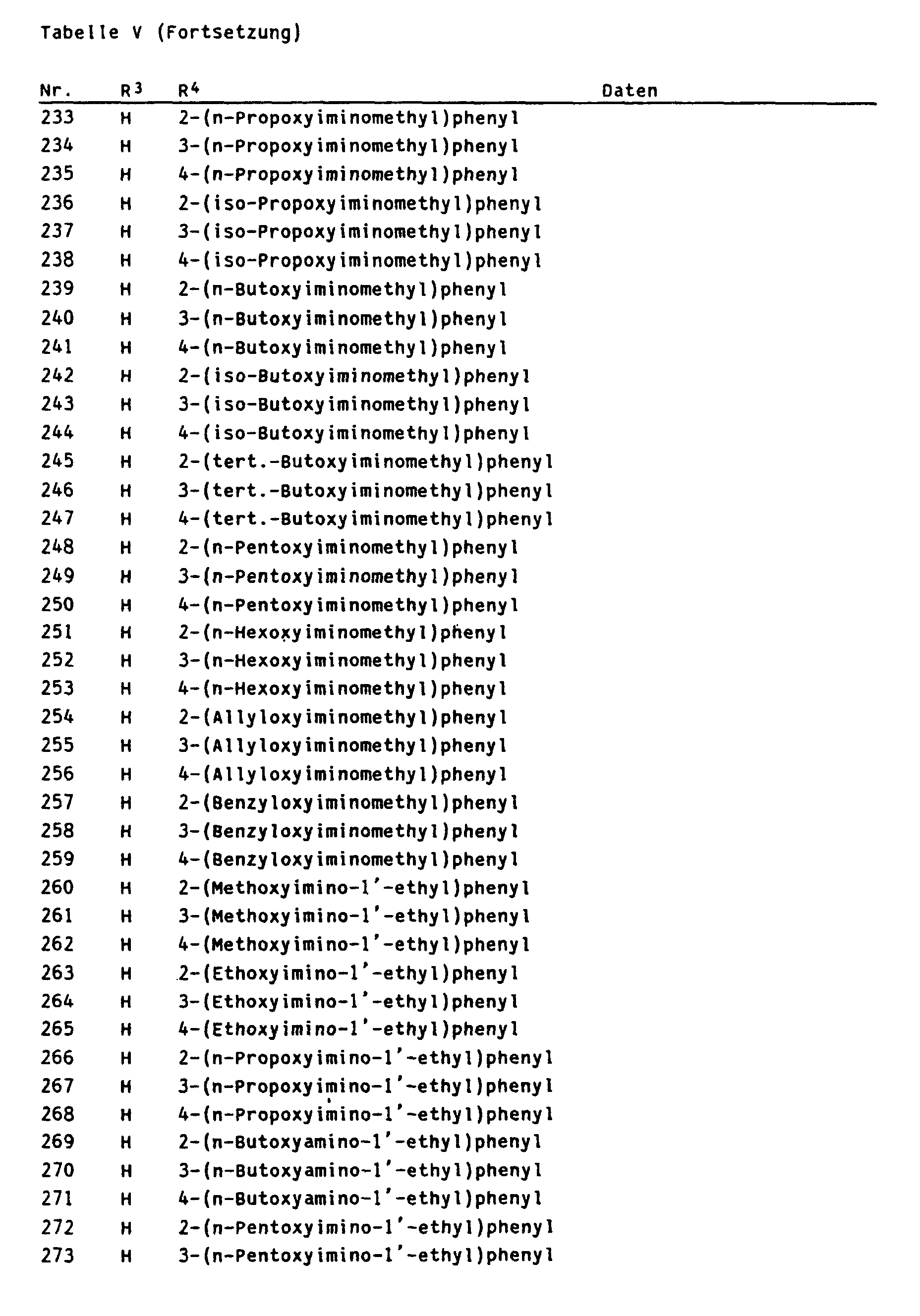

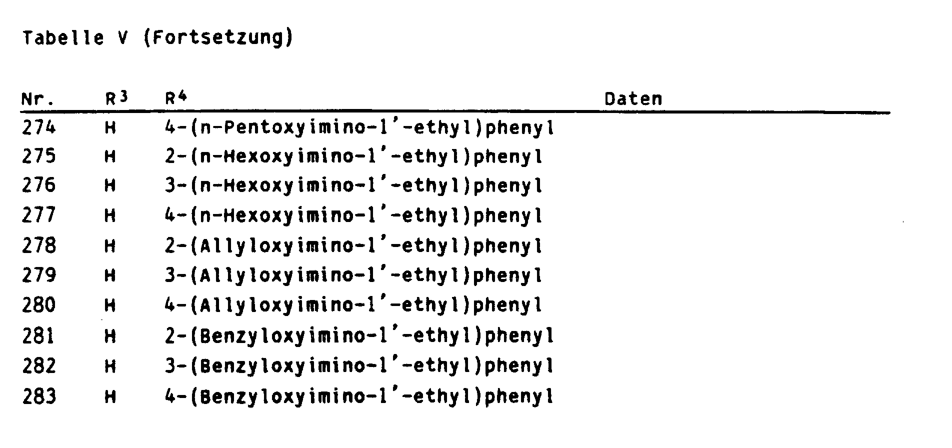

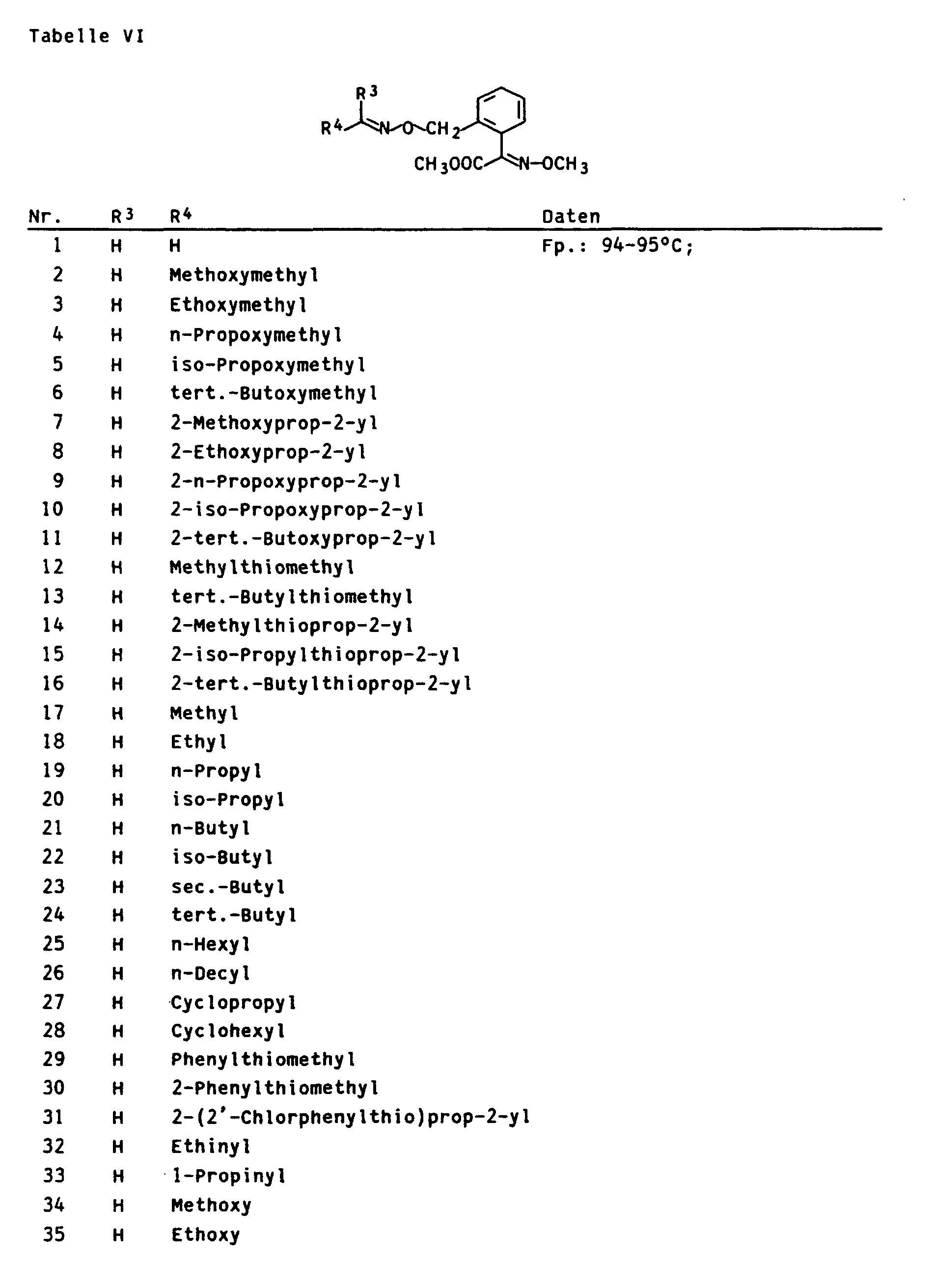

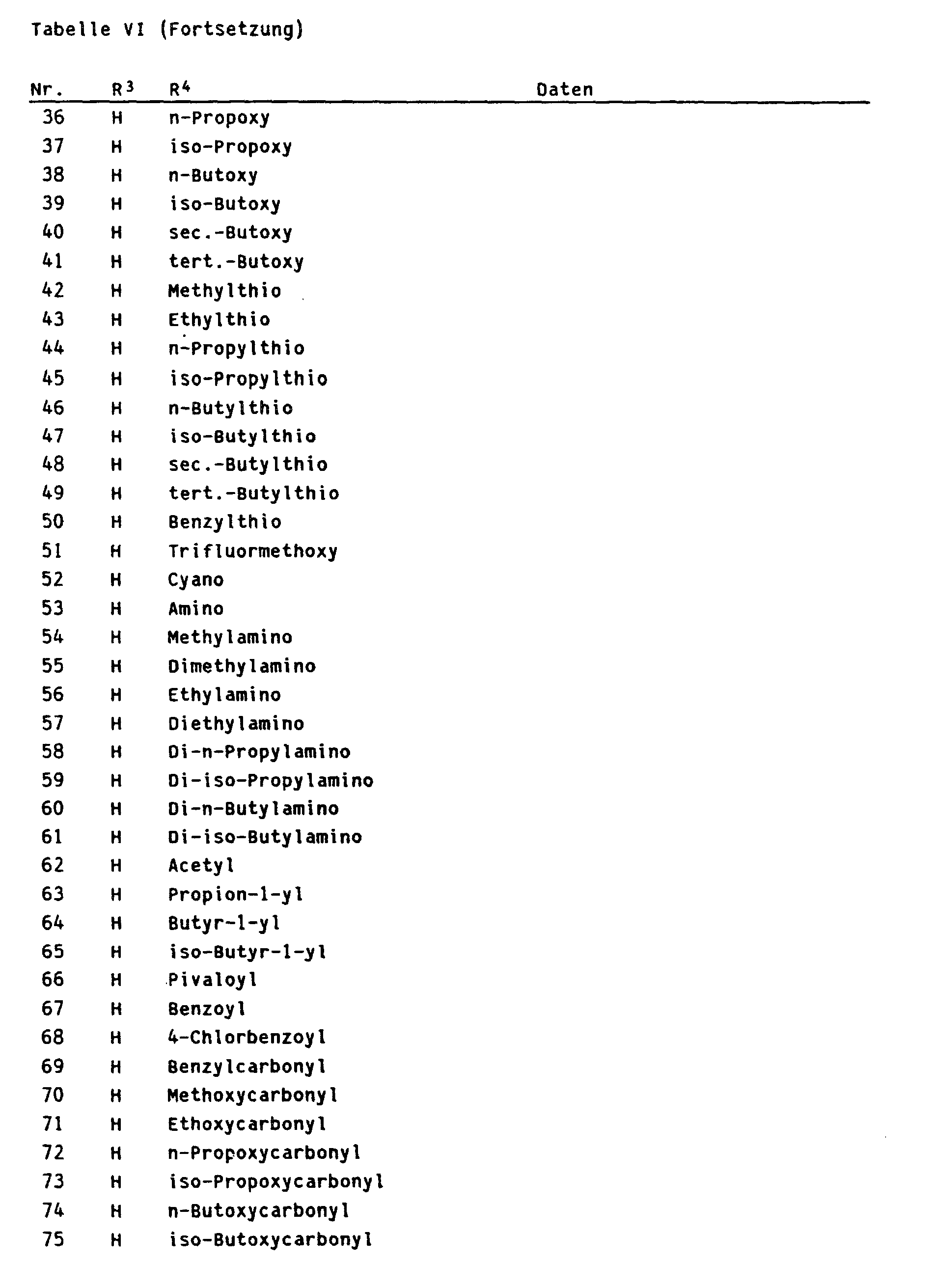

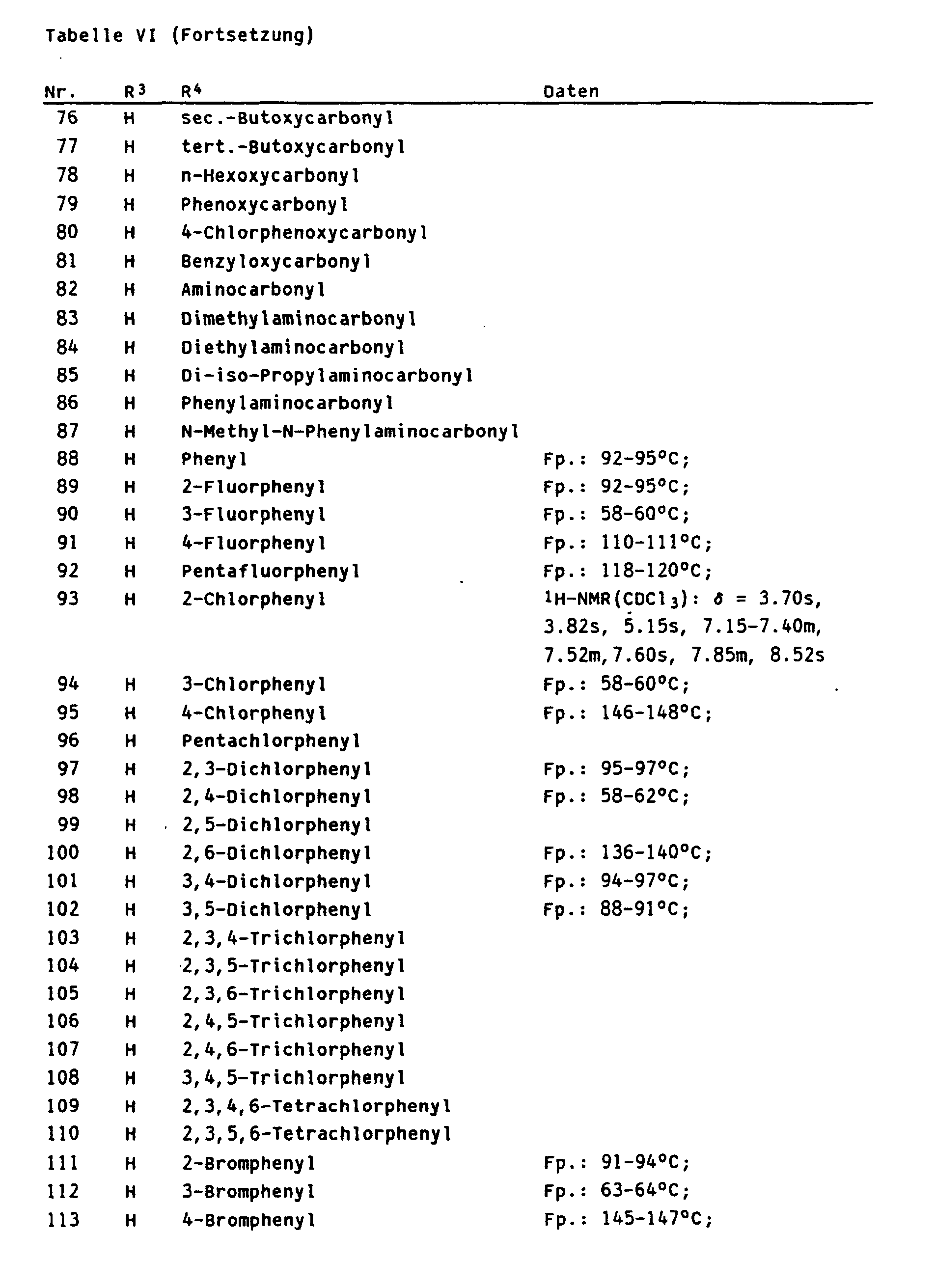

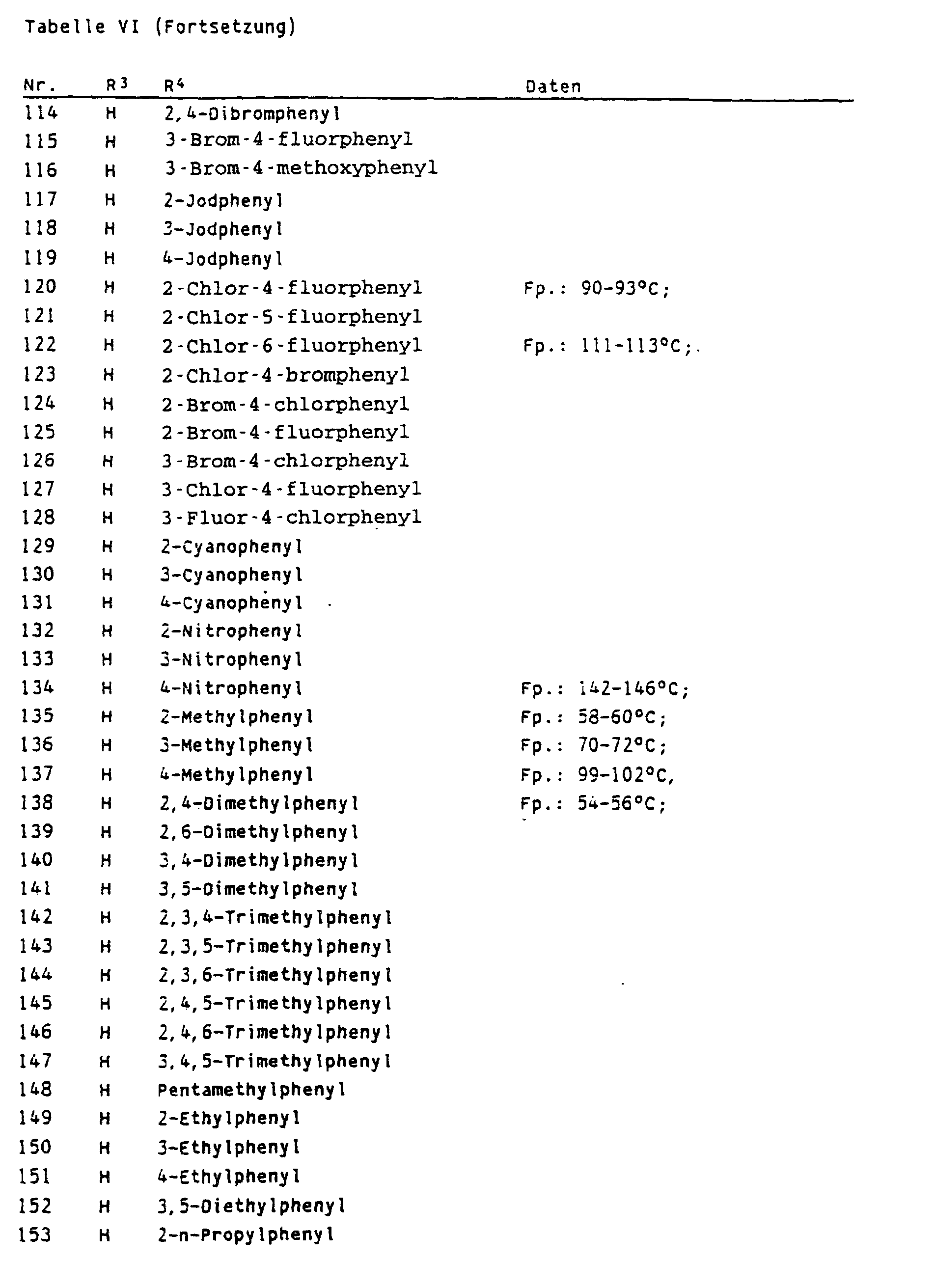

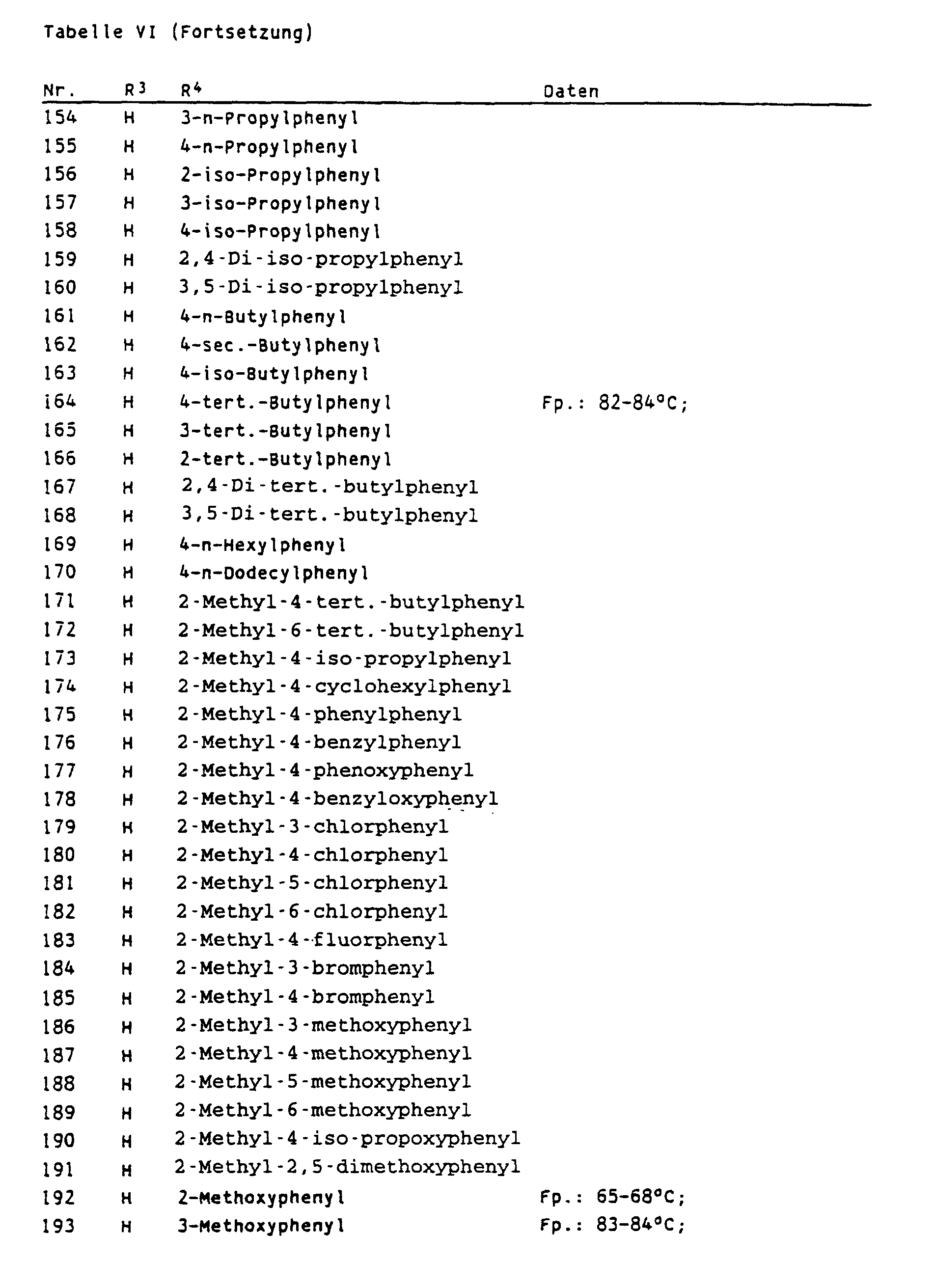

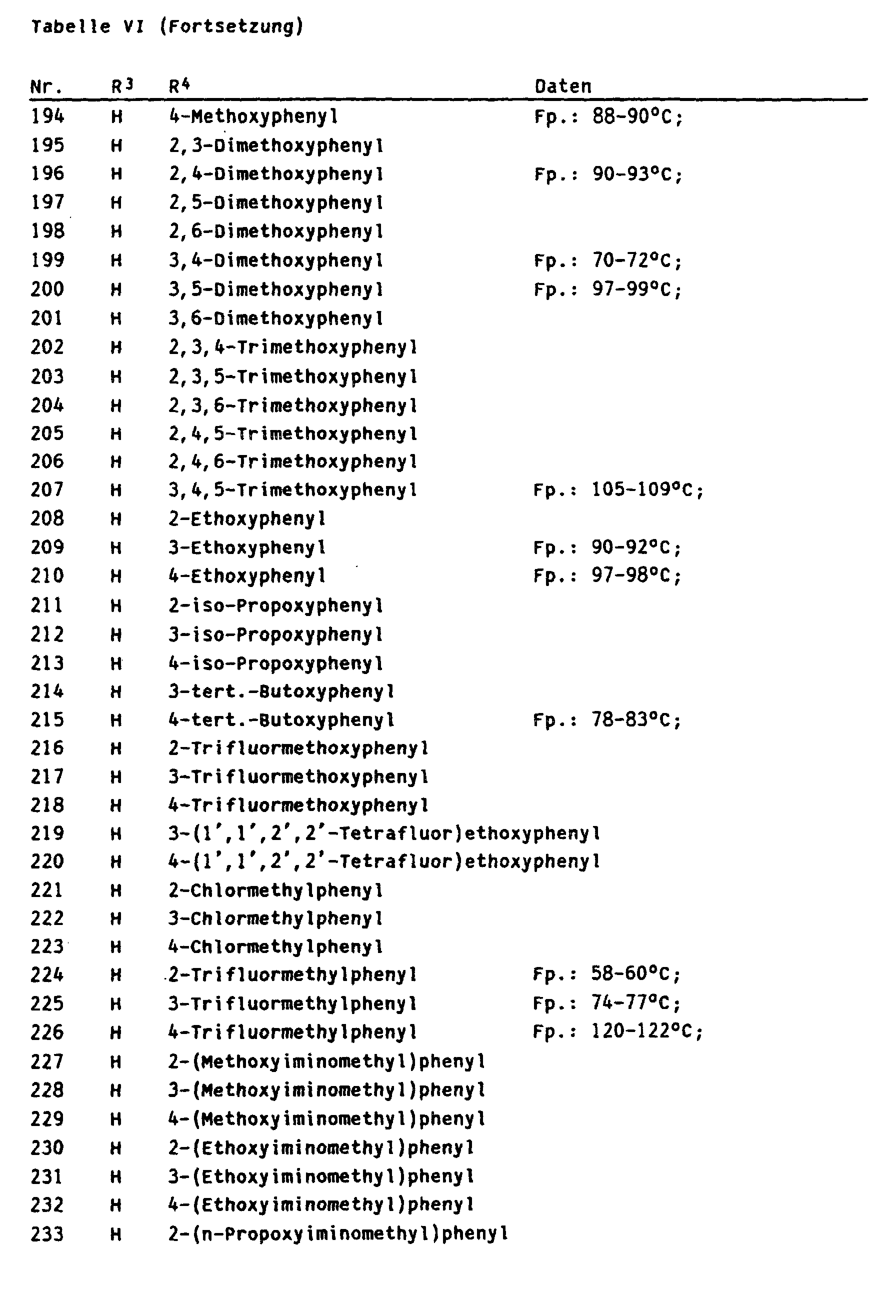

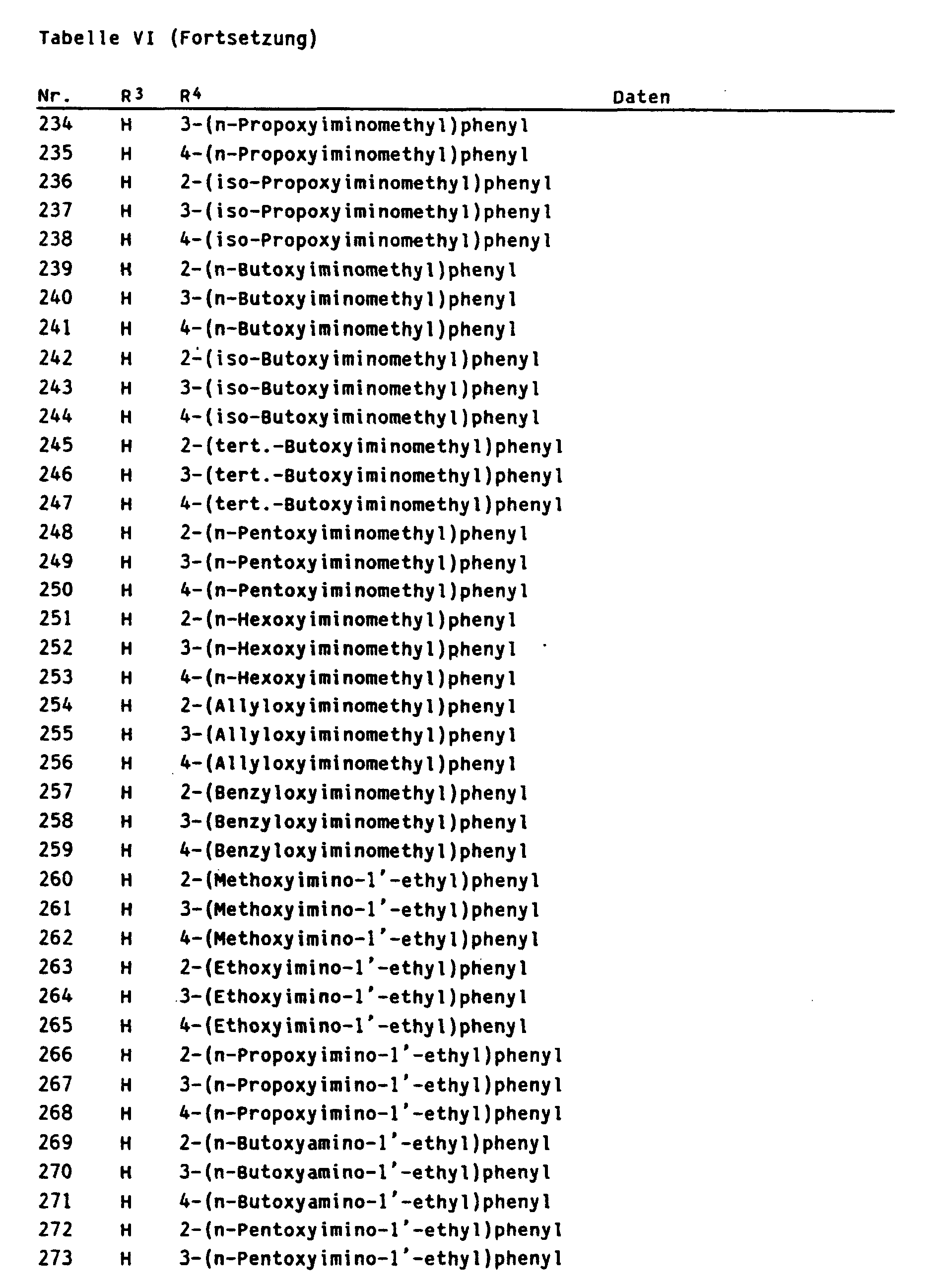

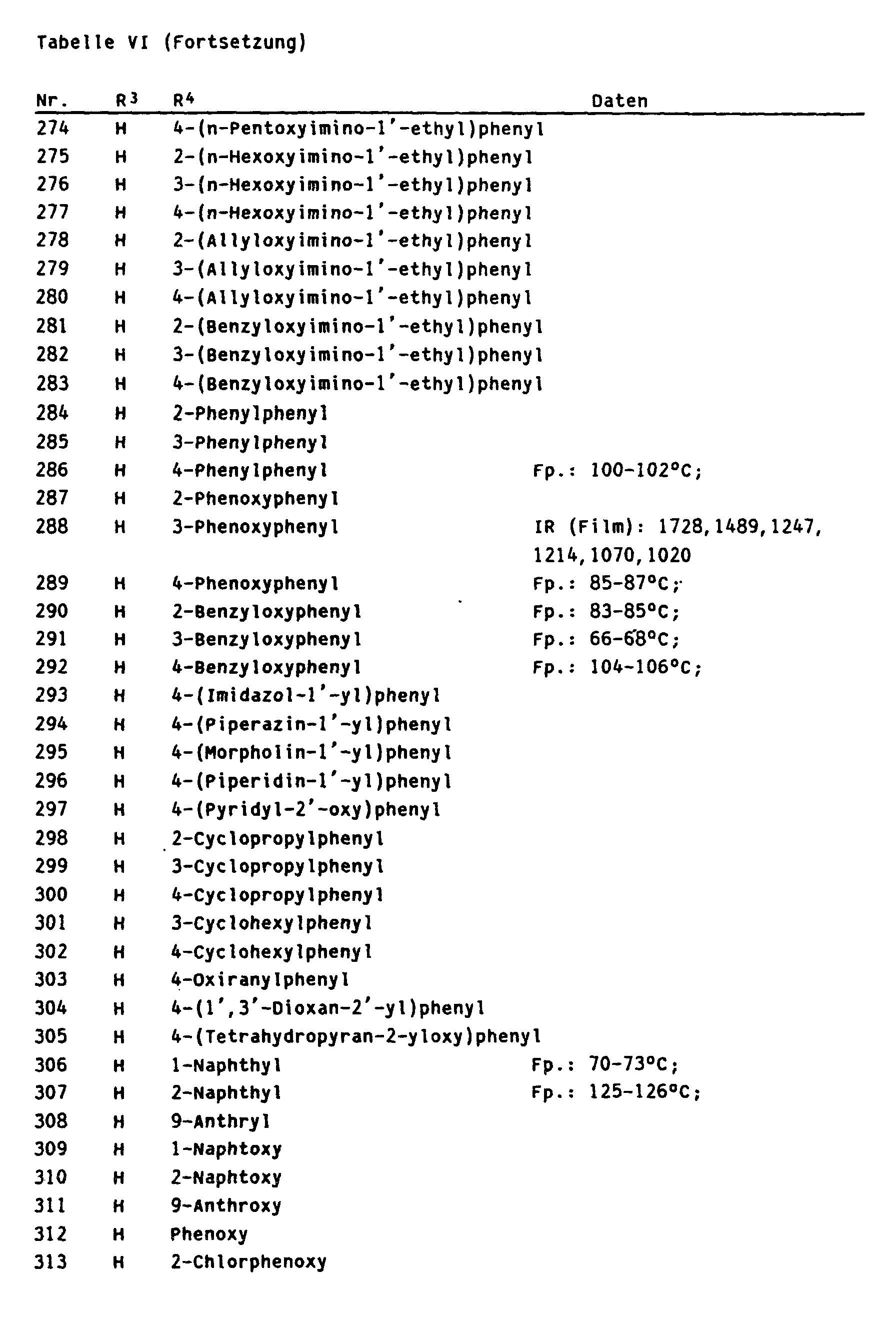

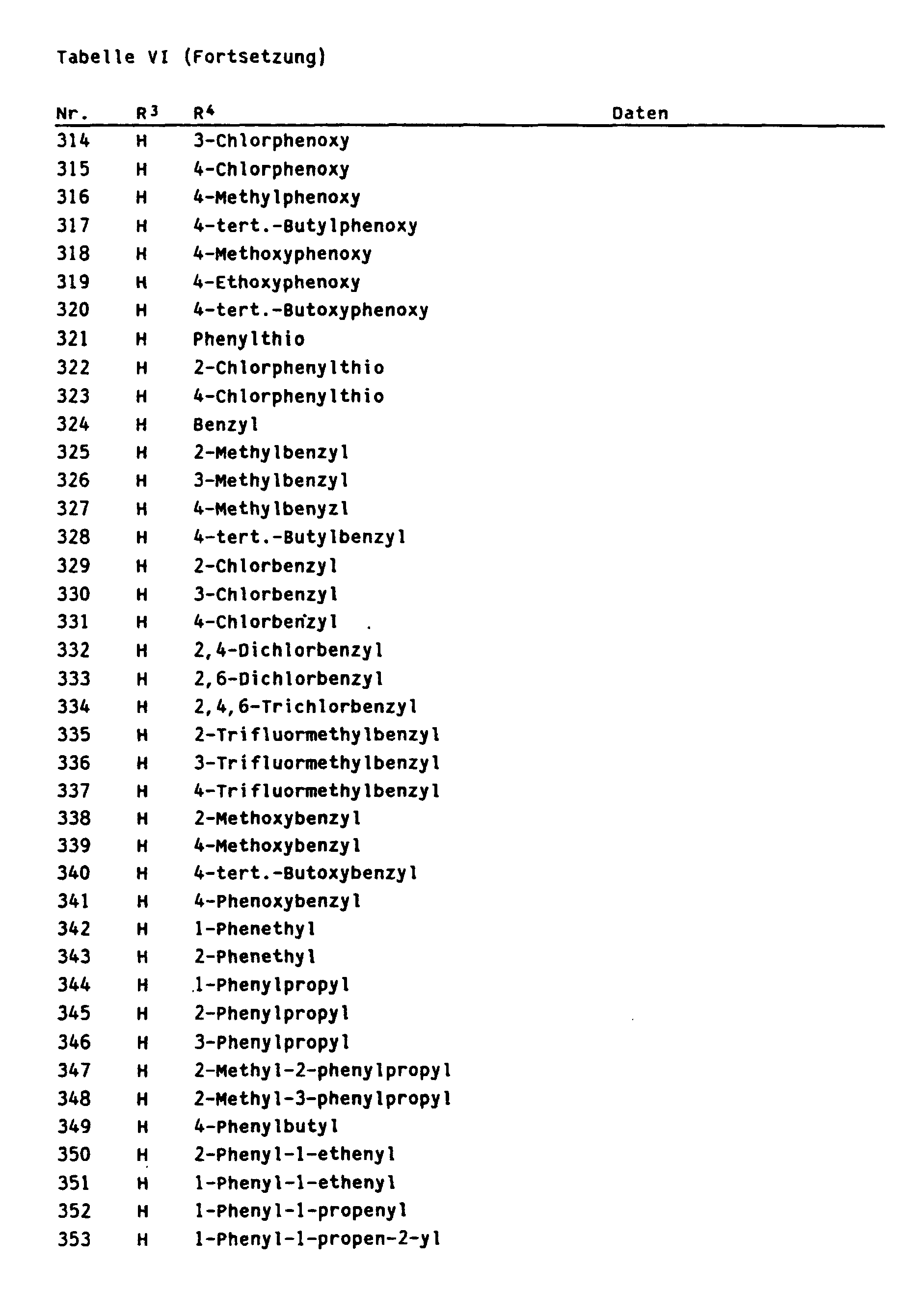

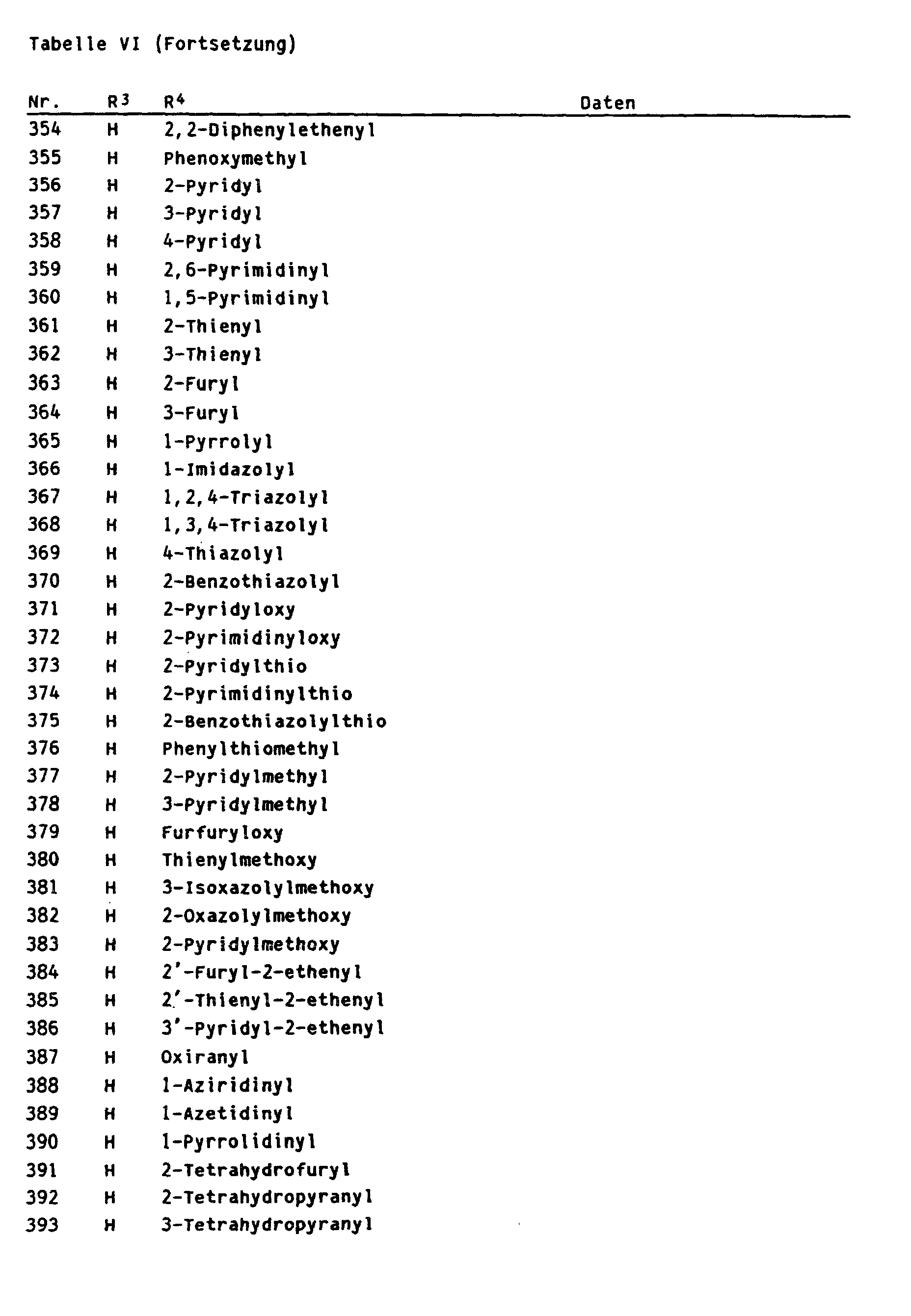

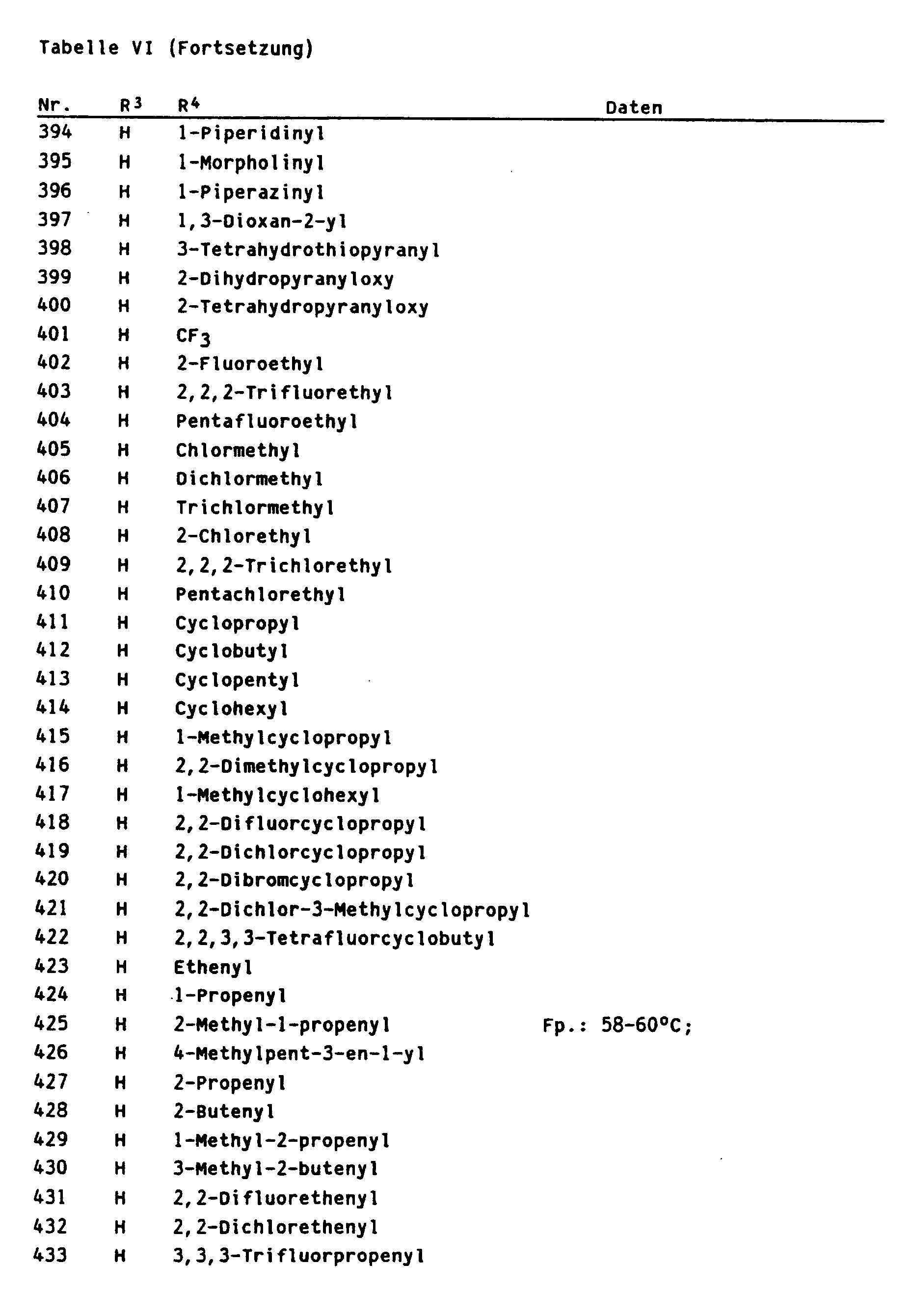

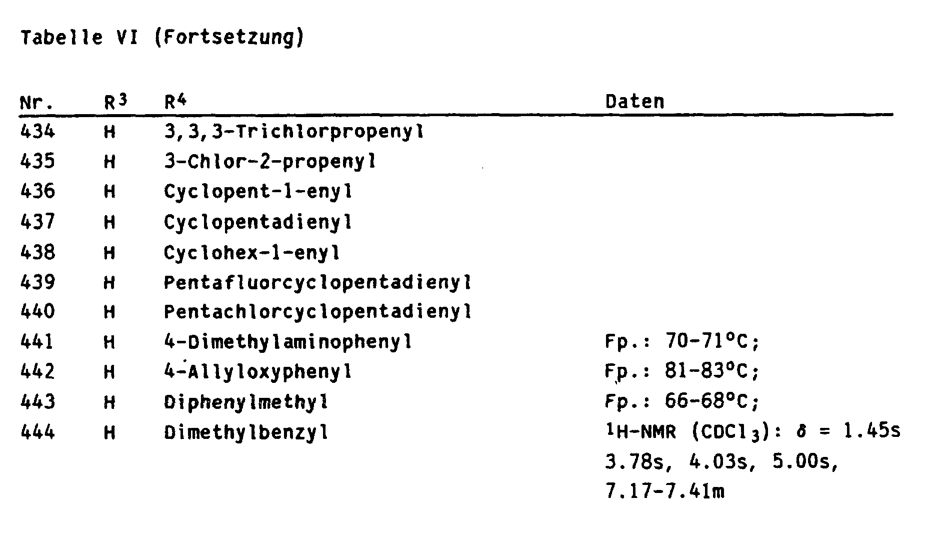

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind diefungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden die Pilze oder die vor Pilzbefall zu schützenden Pflanzen, Saatgüter, Materialien oder der Erdboden mit einer fungizid wirksamen Menge des Wirkstoffs behandelt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii. Bei der Saatgutbehandlung werden im allgemeinen Wirkstoffmengen von 0,001 bis 50 g, vorzugsweise 0,01 bis 10 g, je Kilogramm Saatgut benötigt.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

### Anwendungsbeispiele

Als Vergleichswirkstoff wurde 2-(Phenoxymethyl)-phenylglyoxylsäuremethylester-O-methyloxim (A) - bekannt aus EP 253 213 - benutzt.

### Anwendungsbeispiel 1

### Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22°C und 65 bis 70 % relativer Luftfeuchte aufgestellt. Nach 8 Tagen wurde das Ausmaß der Rostpilzentwicklung auf den Blättern ermittelt.

Das Ergebnis zeigt, daß der Wirkstoff aus Tabelle II Nr. 644 bei der Anwendung als 0,025 %-ige (Gew.-%) Spritzbrühe eine bessere fungizide Wirkung zeigt (95%) als der bekannte Vergleichswirkstoff A (50%).

### Anwendungsbeispiel 2

### Wirksamkeit gegen Pyricularia oryzae (protektiv)

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inokuliert. Anschließend wurden die Versuchspflanzen in Klimakammern bei 22 bis 24°C und 95 bis 99 % relativer Luftfeuchtigkeit aufgestellt. Nach 6 Tagen wurde das Ausmaß des Krankheitsbefalls ermittelt.

Das Ergebnis zeigt, daß die Wirkstoffe aus Tabelle II Nr. 644, 769, 782, 583 bei der Anwendung als 0,05 %-ige (Gew.-%) Spritzbrühe eine sehr gute fungizide Wirkung zeigten (100%).

Die neuen Verbindungen sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritars, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Franldiniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Zur Klasse der Nematoden zählen beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Hetrodera glycinae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Paratylenchus curvitatus, Partylenchus goodeyi.

Für die Anwendung zur Schädlingsbekämpfung können die Wirkstoffkonzentrationen in den anwendungsfertigen Zubereitungen in größeren Bereichen variiert werden.

Im allgemeinen liegen sie zwischen 0,0001 und 10 %, vorzugsweise zwischen 0,001 und 0,1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff für die Schädlingsbekämpfung beträgt unter Freilandbedingungen 0,01 bis 10, vorzugsweise 0,1 bis 1,0 kg/ha.

### Anwendungsbeispiel 3

### Tetranychus telarius, Rote Spinne, Kontaktwirkung, Spritzversuch

Getopfte Buschbohnen, die das zweite Folgeblattpaar zeigen, werden in der Spritzkabine mit der wäßrigen Wirkstoffaufbereitung tropfnaß gespritzt. Die Pflanzen kommen dazu auf den Drehteller und werden von allen Seiten mit insgesamt 50 ml Spritzbrühe besprüht. Die Pflanzen müssen einen starken Milbenbefall und reichliche Eiablage aufweisen. Die Wirkung wird nach 5 Tagen mittels Binokular bonitiert. Dabei wird darauf geachtet, ob alle Stadien gleichmäßig erfaßt sind. Die Pflanzen stehen während dieser Zeit unter normalen Gewächshausbedingungen.

| Verbindung Nr. aus Tabelle I | Aufwandmenge in ppm | Mortalität in % |
|---|---|---|
| 429 | 1000 | 100 |

| Verbindung Nr. aus Tabelle II | Aufwandmenge in ppm | Mortalität in % |
|---|---|---|
| 37 | 1000 | 100 |
| 39 | 1000 | 100 |
| 45 | 1000 | 100 |

### Anwendungsbeispiel 4

### Musca domestica, Stubenfliege, Dauerkontakt

Beide Teile einer Petrischale von 10 cm Durchmesser werden mit insgesamt 1 ml der acetonischen Lösung der Wirkstoffe ausgekleidet. Nach Verdunsten des Lösungsmittels bringt man je 10 Fliegen und Wasserwatte in die Schalen, schließt sie und zählt nach 24 Stunden die Tiere in Rückenlage.

Tritt bei 0,01 mg/Petrischale eine Wirkung von 80-100 % Mortalität auf, so wird der Test mit fallenden Konzentrationen fortgesetzt.

### Anwendungsbeispiel 5

### Plutella maculipennis, Kohlschabe, Fraßverhindernde Wirkung

Junge Kohlrabiblätter werden 3 sec. in die wäßrige Aufbereitung der Prüfsubstanz getaucht und auf einen mit 0,5 ml Wasser angefeuchteten Rundfilter (Φ9 cm) in eine Petrischale gelegt (Φ10 cm). Darauf wird das Blatt mit 10 Raupen im 4. Larvenstadium besetzt und die Petrischale geschlossen. Nach 48 Stunden beurteilt man die Fraßverhinderung in %.

### Anwendungsbeispiel 6

### Wirksamkeit gegen Rebenperonospora

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Befalls der Blätter.

Das Ergebnis zeigt, daß die Wirkstoffe aus Tabelle II Nr. 644, 117, 429, 56, 43, 36, 49, bei der Anwendung als 0,025 %ige (Gew.-%) Spritzbrühe eine bessere fungizide Wirkung zeigen (95 %) als der bekannte Vergleichswirkstoff A (50 %).

## Patentansprüche

1. O-Benzyl-Oximether der allgemeinen Formel I 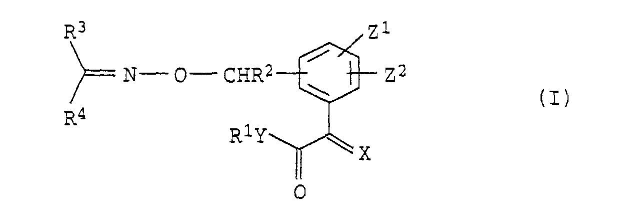 in der die Substituenten die folgende Bedeutung haben:
a)
X CH-C₁-C₄-Alkyl, CH-C₁-C₄-Alkylthio oder N-C₁-C₄-Alkoxy,
Y Sauerstoff, Schwefel oder NR⁵,
R¹, R², R⁵ unabhängig voneinander Wasserstoff oder C₁-C₄-Alkyl,
Z¹, Z² unabhängig voneinander
Wasserstoff, Halogen, Methyl, Methoxy oder Cyano,
R³, R⁴ unabhängig voneinander Wasserstoff, Cyano, ggf. verzweigte C₁-C₁₀-Alkyl, C₁-C₄-Halogenalkyl, C₃-C₆-Cycloalkyl, C₃-C₆-Halogencycloalkyl, C₃-C₆-Cycloalkyl-C₁-C₄-alkyl, C₁-C₄-Alkoxy-C₁-C₄-alkyl, C₁-C₄-Alkylthio-C₁-C₄-alkyl, Arylthio-C₁-C₄-alkyl, C₂-C₆-Alkenyl, C₂-C₅-Halogenalkenyl, C₃-C₆-Cycloalkenyl, C₃-C₆-Halogencycloalkenyl, C₂-C₆-Alkinyl, C₁-C₆-Alkoxy, C₁-C₆-Halogenalkoxy, C₁-C₄-Alkylthio, Benzylthio, C₁-C₄-Alkylcarbonyl, C₁-C₄-Alkoxycarbonyl, ggf. subst. Phenylcarbonyl, ggf. subst. Benzylcarbonyl, ggf. subst. Phenoxycarbonyl, ggf. subst. Benzyloxycarbonyl, ggf. subst. Aryl, ggf. subst. Aryloxy, ggf. subst. Arylthio, ggf. subst. Aryl-C₁-C₄-alkyl, ggf. subst. Aryl-C₂-C₄-alkenyl, ggf. subst. Aryloxy-C₁-C₄-alkyl, ggf. subst. Arylthio-C₁-C₄-alkyl, ggf. subst. Hetaryl, ggf. subst. Hetaryloxy, ggf. subst. Hetarylthio, ggf. subst. Hetaryl-C₁-C₄-alkyl, ggf. subst. Hetaryl-C₂-C₄-alkenyl, ggf. subst. Hetaryl-C₁-C₄-alkoxy, ggf. subst. Heterocyclyl, ggf. subst. Heterocyclyloxy,
N(R⁶)₂, wobei die Bedeutungen von R⁶ gleich oder verschieden sind,
CON(R⁷)₂, wobei die Bedeutungen von R⁷ gleich oder verschieden sind,
R³ und R⁴zusammen ein carbocyclischer oder heterocyclischer Ring, der durch die unter "ggf. subst." genannten Reste substituiert sein kann, oder
einer der Reste R³ oder R⁴ Halogen,
R⁶ Wasserstoff, C₁-C₆-Alkyl oder ggf. subst. Phenyl,
R⁷ Wasserstoff oder C₁-C₄-Alkyl,
ggf. subst. neben Wasserstoff die Reste Halogen, Cyano, Nitro, C₁-C₄-Alkyl, C₁-C₄-Alkoxy, C₁-C₄-Halogenalkyl, C₁-C₄-Halogenalkoxy, C₁-C₁₀-Alkoximino-C₁-C₂-alkyl, Aryl, Aryloxy, Benzyloxy, Hetaryl, Hetaryloxy, C₃-C₆-Cycloalkyl, Heterocyclyl oder Heterocyclyloxy,
außer den Verbindungen, in denen
X CH-C₁-C₂-Alkylthio oder N-C₁-C₂-Alkoxy,
Y Sauerstoff,
Z¹, Z² und R2 Wasserstoff,
R¹ C₁-C₄-Alkyl,
R³ Wasserstoff, C₁-C₄-Alkyl, C₁-C₄-Halogenalkyl oder C₃-C₆-Cycloalkyl,
R⁴ C₁-C₆-Alkyl, ggf. subst. Aryl-C₁-C₄-alkyl, ggf. subst. Hetaryl-C₁-C₄-alkyl, C₂-C₆-Alkenyl, ggf. subst. Aryl-C₂-C₄-alkenyl, ggf. subst. Hetaryl-C₂-C₄-alkenyl, C₃-C₆-Cycloalkyl, ggf. subst. Aryl,
ggf. subst. Hetaryl, C₁-C₃-Alkylcarbonyl, ggf. subst. Phenylcarbonyl oder
R³ und R⁴ zusammen mit dem C-Atom, an das sie gebunden sind, einen gegebenenfalls substutierten, gegebenenfalls ein Sauerstoff- oder Schwefelatom enthaltenden vierbis siebengliedrigen gesättigten Ring bilden, der zudem einen gegebenenfalls substituierten ankondensierten Benzolring aufweisen kann,
wobei von den ausgenommenen Verbindungen, die folgenden Einzelverbindungen der Formel Ia 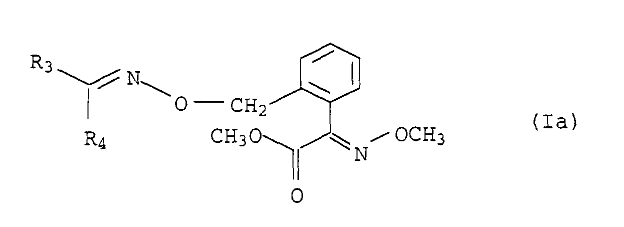
| | R³ | R⁴ |
|---|---|---|
| 8 | CCl₃ | Phenyl |
| 9 | CH₂Cl | Phenyl |
| 10 | CF₂CF₃ | Phenyl |
| 11 | CF₂Cl | Phenyl |
| 12 | CHCl₂ | Phenyl |
| 13 | Cyclopropyl | Cyclopropyl |
| 18 | Cyclopropyl | 4-Ethoxyphenyl |
| 20 | Cyclopropyl | Pentachlorphenyl |
| 21 | Cyclopropyl | Pentafluorphenyl |
| 22 | Cyclopentyl | Phenyl |
| 23 | Cyclohexyl | Phenyl |
| 497 | CH₃ | Methyl |
| 498 | CH₃ | Ethyl |
| 499 | CH₃ | n-Propyl |
| 500 | CH₃ | iso-Propyl |
| 501 | CH₃ | n-Butyl |
| 502 | CH₃ | iso-Butyl |
| 503 | CH₃ | sec.-Butyl |
| 504 | CH₃ | tert.-Butyl |
| 505 | CH₃ | n-Hexyl |
| 507 | CH₃ | Cyclopropyl |
| 508 | CH₃ | Cyclohexyl |
| 542 | CH₃ | Acetyl |
| 543 | CH₃ | Propion-1-yl |
| 544 | CH₃ | Butyr-1-yl |
| 545 | CH₃ | iso-Butyr-1-yl |
| 546 | CH₃ | Pivaloyl |
| 572 | CH₃ | Pentafluorphenyl |
| 576 | CH₃ | Pentachlorphenyl |
| 583 | CH₃ | 2,3,4-Trichlorphenyl |
| 584 | CH₃ | 2,3,5-Trichlorphenyl |
| 585 | CH₃ | 2,3,6-Trichlorphenyl |
| 586 | CH₃ | 2,4,5-Trichlorphenyl |
| 587 | CH₃ | 2,4,6-Trichlorphenyl |
| 588 | CH₃ | 3,4,5-Trichlorphenyl |
| 589 | CH₃ | 2,3,4,6-Tetrachlorphenyl |
| 590 | CH₃ | 2,3,5,6-Tetrachlorphenyl |
| 609 | CH₃ | 2-Cyanophenyl |
| 610 | CH₃ | 3-Cyanophenyl |
| 611 | CH₃ | 4-Cyanophenyl |
| 618 | CH₃ | 2,4-Dimethylphenyl |
| 619 | CH₃ | 2,6-Dimethylphenyl |
| 620 | CH₃ | 3,4-Dimethylphenyl |
| 621 | CH₃ | 3,5-Dimethylphenyl |
| 622 | CH₃ | 2,3,4-Trimethylphenyl |
| 623 | CH₃ | 2,3,5-Trimethylphenyl |
| 624 | CH₃ | 2,3,6-Trimethylphenyl |
| 625 | CH₃ | 2,4,5-Trimethylphenyl |
| 626 | CH₃ | 2,4,6-Trimethylphenyl |
| 627 | CH₃ | 3,4,5-Trimethylphenyl |
| 628 | CH₃ | Pentamethylphenyl |
| 629 | CH₃ | 2-Ethylphenyl |
| 630 | CH₃ | 3-Ethylphenyl |
| 631 | CH₃ | 4-Ethylphenyl |
| 632 | CH₃ | 3,5-Diethylphenyl |
| 633 | CH₃ | 2-n-Propylphenyl |
| 634 | CH₃ | 3-n-Propylphenyl |
| 635 | CH₃ | 4-n-Propylphenyl |
| 636 | CH₃ | 2-iso-Propylphenyl |
| 637 | CH₃ | 3-iso-Propylphenyl |
| 638 | CH₃ | 4-iso-Propylphenyl |
| 639 | CH₃ | 2,4-Di-iso-Propylphenyl |
| 640 | CH₃ | 3,5-Di-iso-Propylphenyl |
| 641 | CH₃ | 4-n-Butylphenyl |
| 642 | CH₃ | 4-sec.-Butylphenyl |
| 643 | CH₃ | 4-iso-Butylphenyl |
| 644 | CH₃ | 4-tert.-Butylphenyl |
| 645 | CH₃ | 3-tert.-Butylphenyl |
| 646 | CH₃ | 2-tert.-Butylphenyl |
| 647 | CH₃ | 2,4-Di-tert.-butylphenyl |
| 648 | CH₃ | 3,5-Di-tert.-butylphenyl |
| 650 | CH₃ | 4-n-Dodecylphenyl |
| 651 | CH₃ | 2-Methyl-4-tert.-butylphenyl |
| 652 | CH₃ | 2-Methyl-6-tert.-butylphenyl |
| 653 | CH₃ | 2-Methyl-4-iso-propylphenyl |
| 654 | CH₃ | 2-Methyl-4-cyclohexylphenyl |
| 655 | CH₃ | 2-Methyl-4-phenylphenyl |
| 656 | CH₃ | 2-Methyl-4-benzylphenyl |
| 657 | CH₃ | 2-Methyl-4-phenoxyphenyl |
| 658 | CH₃ | 2-Methyl-4-benzyloxyphenyl |
| 666 | CH₃ | 2-Methyl-3-methoxyphenyl |
| 667 | CH₃ | 2-Methyl-4-methoxyphenyl |
| 668 | CH₃ | 2-Methyl-5-methoxyphenyl |
| 669 | CH₃ | 2-Methyl-6-methoxyphenyl |
| 670 | CH₃ | 2-Methyl-4-iso-propoxyphenyl |
| 671 | CH₃ | 2-Methyl-2,5-dimethoxyphenyl |
| 688 | CH₃ | 2-Ethoxyphenyl |
| 689 | CH₃ | 3-Ethoxyphenyl |
| 690 | CH₃ | 4-Ethoxyphenyl |
| 691 | CH₃ | 2-iso-Propoxyphenyl |
| 692 | CH₃ | 3-iso-Propoxyphenyl |
| 693 | CH₃ | 4-iso-Propoxyphenyl |
| 694 | CH₃ | 3-tert.-Butoxyphenyl |
| 695 | CH₃ | 4-tert.-Butoxyphenyl |
| 699 | CH₃ | 3-(1',1',2',2'-Tetrafluor)ethoxyphenyl |
| 700 | CH₃ | 4-(1',1',2',2'-Tetrafluor)ethoxyphenyl |
| 701 | CH₃ | 2-Chlormethylphenyl |
| 702 | CH₃ | 3-Chlormethylphenyl |
| 703 | CH₃ | 4-Chlormethylphenyl |
| 707 | CH₃ | 2-(Methoxyiminomethyl)phenyl |
| 708 | CH₃ | 3-(Methoxyiminomethyl)phenyl |
| 709 | CH₃ | 4-(Methoxyiminomethyl)phenyl |
| 710 | CH₃ | 2-(Ethoxyiminomethyl)phenyl |
| 711 | CH₃ | 3-(Ethoxyiminomethyl)phenyl |
| 712 | CH₃ | 4-(Ethoxyiminomethyl)phenyl |
| 713 | CH₃ | 2-(n-Propoxyiminomethyl)phenyl |
| 714 | CH₃ | 3-(n-Propoxyiminomethyl)phenyl |
| 715 | CH₃ | 4-(n-Propoxyiminomethyl)phenyl |
| 716 | CH₃ | 2-(iso-Propoxyiminomethyl)phenyl |
| 717 | CH₃ | 3-(iso-Propoxyiminomethyl)phenyl |
| 718 | CH₃ | 4-(iso-Propoxyiminomethyl)phenyl |
| 719 | CH₃ | 2-(n-Butoxyiminomethyl)phenyl |
| 720 | CH₃ | 3-(n-Butoxyiminomethyl)phenyl |
| 721 | CH₃ | 4-(n-Butoxyiminomethyl)phenyl |
| 722 | CH₃ | 2-(iso-Butoxyiminomethyl)phenyl |
| 723 | CH₃ | 3-(iso-Butoxyiminomethyl)phenyl |
| 724 | CH₃ | 4-(iso-Butoxyiminomethyl)phenyl |
| 725 | CH₃ | 2-(tert.-Butoxyiminomethyl)phenyl |
| 726 | CH₃ | 3-(tert.-Butoxyiminomethyl)phenyl |
| 727 | CH₃ | 4-(tert.-Butoxyiminomethyl)phenyl |
| 728 | CH₃ | 2-(n-Pentoxyiminomethyl)phenyl |
| 729 | CH₃ | 3-(n-Pentoxyiminomethyl)phenyl |
| 730 | CH₃ | 4-(n-Pentoxyiminomethyl)phenyl |
| 731 | CH₃ | 2-(n-Hexoxyiminomethyl)phenyl |
| 732 | CH₃ | 3-(n-Hexoxyiminomethyl)phenyl |
| 733 | CH₃ | 4-(n-Hexoxyiminomethyl)phenyl |
| 740 | CH₃ | 2-(Methoxyimino-1'-ethyl)phenyl |
| 741 | CH₃ | 3-(Methoxyimino-1'-ethyl)phenyl |
| 742 | CH₃ | 4-(Methoxyimino-1'-ethyl)phenyl |
| 743 | CH₃ | 2-(Ethoxyimino-1'-ethyl)phenyl |
| 744 | CH₃ | 3-(Ethoxyimino-1'-ethyl)phenyl |
| 745 | CH₃ | 4-(Ethoxyimino-1'-ethyl)phenyl |
| 746 | CH₃ | 2-(n-Propoxyimino-1'-ethyl)phenyl |
| 747 | CH₃ | 3-(n-Propoxyimino-1'-ethyl)phenyl |
| 748 | CH₃ | 4-(n-Propoxyimino-1'-ethyl)phenyl |
| 749 | CH₃ | 2-(n-Butoxyamino-1'-ethyl)phenyl |
| 750 | CH₃ | 3-(n-Butoxyamino-1'-ethyl)phenyl |
| 751 | CH₃ | 4-(n-Butoxyamino-1'-ethyl)phenyl |
| 752 | CH₃ | 2-(n-Pentoxyimino-1'-ethyl)phenyl |
| 753 | CH₃ | 3-(n-Pentoxyimino-1'-ethyl)phenyl |
| 754 | CH₃ | 4-(n-Pentoxyimino-1'-ethyl)phenyl |
| 755 | CH₃ | 2-(n-Hexoxyimino-1'-ethyl)phenyl |
| 756 | CH₃ | 3-(n-Hexoxyimino-1'-ethyl)phenyl |
| 757 | CH₃ | 4-(n-Hexoxyimino-1'-ethyl)phenyl |
| 764 | CH₃ | 2-Phenylphenyl |
| 765 | CH₃ | 3-Phenylphenyl |
| 766 | CH₃ | 4-Phenylphenyl |
| 767 | CH₃ | 2-Phenoxyphenyl |
| 768 | CH₃ | 3-Phenoxyphenyl |
| 769 | CH₃ | 4-Phenoxyphenyl |
| 770 | CH₃ | 2-Benzyloxyphenyl |
| 771 | CH₃ | 3-Benzyloxyphenyl |
| 772 | CH₃ | 4-Benzyloxyohenyl |
| 773 | CH₃ | 4-(Imidazol-1'-yl)phenyl |
| 774 | CH₃ | 4-(Piperazin-1'-yl)phenyl |
| 777 | CH₃ | 4-(Pyridyl-2'-oxy)phenyl |
| 778 | CH₃ | 2-Cyclopropylphenyl |
| 779 | CH₃ | 3-Cyclopropylphenyl |
| 780 | CH₃ | 4-Cyclopropylphenyl |
| 781 | CH₃ | 3-Cyclohexylphenyl |
| 782 | CH₃ | 4-Cyclohexylphenyl |
| 783 | CH₃ | 4-Oxiranylphenyl |
| 784 | CH₃ | 4-(1',3'-Dioxan-2'-yl)phenyl |
| 785 | CH₃ | 4-(Tetrahydropyran-2-yloxy)phenyl |
| 788 | CH₃ | 9-Anthryl |
| 804 | CH₃ | Benzyl |
| 805 | CH₃ | 2-Methylbenzyl |
| 806 | CH₃ | 3-Methylbenzyl |
| 807 | CH₃ | 4-Methylbenzyl |
| 808 | CH₃ | 4-tert.-Butylbenzyl |
| 818 | CH₃ | 2-Methoxybenzyl |
| 819 | CH₃ | 4-Methoxybenzyl |
| 820 | CH₃ | 4-tert.-Butoxybenzyl |
| 821 | CH₃ | 4-Phenoxybenzyl |
| 822 | CH₃ | 1-Phenethyl |
| 823 | CH₃ | 2-Phenethyl |
| 824 | CH₃ | 1-Phenylpropyl |
| 825 | CH₃ | 2-Phenylpropyl |
| 826 | CH₃ | 3-Phenylpropyl |
| 827 | CH₃ | 2-Methyl-2-phenylpropyl |
| 828 | CH₃ | 2-Methyl-3-phenylpropyl |
| 829 | CH₃ | 4-Phenylbutyl |
| 830 | CH₃ | 2-Phenyl-1-ethenyl |
| 831 | CH₃ | 1-Phenyl-1-ethenyl |
| 832 | CH₃ | 1-Phenyl-1-propenyl |
| 833 | CH₃ | 1-Phenyl-1-propen-2-yl |
| 839 | CH₃ . | 2,6-Pyrimidinyl |
| 840 | CH₃ | 1,5-Pyrimidinyl |
| 845 | CH₃ | 1-Pyrrolyl |
| 846 | CH₃ | 1-Imidazolyl |
| 847 | CH₃ | 1,2,4-Triazolyl |
| 848 | CH₃ | 1,3,4-Triazolyl |
| 849 | CH₃ | 4-Thiazolyl |
| 857 | CH₃ | 2-Pyridylmethyl |
| 858 | CH₃ | 3-Pyridylmethyl |
| 864 | CH₃ | 2'-Furyl-2-ethenyl |
| 865 | CH₃ | 2'-Thienyl-2-ethenyl |
| 866 | CH₃ | 3'-Pyridyl-2-ethenyl |
| 891 | CH₃ | Cyclopropyl |
| 892 | CH₃ | Cyclobutyl |
| 893 | CH₃ | Cyclopentyl |
| 894 | CH₃ | Cyclohexyl |
| 903 | CH₃ | Ethenyl |
| 904 | CH₃ | 1-Propenyl |
| 905 | CH₃ | 2-Methyl-1-propenyl |
| 906 | CH₃ | 4-Methylpent-3-en-1-yl |
| 907 | CH₃ | 2-Propenyl |
| 908 | CH₃ | 2-Butenyl |
| 909 | CH₃ | 1-Methyl-2-propenyl |
| 910 | CH₃ | 3-Methyl-2-butenyl |
| 948 | Ethyl | Ethyl |
| 949 | Ethyl | n-Propyl |
| 950 | Ethyl | iso-Propyl |
| 951 | Ethyl | n-Butyl |
| 952 | Ethyl | iso-Butyl |
| 953 | Ethyl | 2-Methyl-butyl |
| 954 | Ethyl | Benzyl |
| 955 | n-Propyl | n-Propyl |
| 956 | iso-Propyl | iso-Propyl |
| 957 | n-Butyl | n-Butyl |
| 958 | iso-Butyl | iso-Butyl |
| 959 | tert.-Butyl | tert.-Butyl |
| 966 | n-Butyl | Phenyl |
| | R³ | R⁴ |
|---|---|---|
| 17 | H | Methyl |
| 18 | H | Ethyl |
| 19 | H | n-Propyl |
| 20 | H | iso-Propyl |
| 21 | H | n-Butyl |
| 22 | H | iso-Butyl |
| 23 | H | sec.-Butyl |
| 24 | H | tert.-Butyl |
| 25 | H | n-Hexyl |
| 27 | H | Cyclopropyl |
| 28 | H | Cyclohexyl |
| 62 | H | Acetyl |
| 63 | H | Proption-1-yl |
| 64 | H | Butyr-1-yl |
| 65 | H | iso-Butyr-1-yl |
| 66 | H | Pivaloyl |
| 88 | H | Phenyl |
| 89 | H | 2-Fluorphenyl |
| 90 | H | 3-Fluorphenyl |
| 91 | H | 4-Fluorphenyl |
| 92 | H | Pentafluorphenyl |
| 93 | H | 2-Chlorphenyl |
| 94 | H | 3-Chlorphenyl |
| 95 | H | 4-Chlorphenyl |
| 97 | H | 2,3-Dichlorphenyl |
| 98 | H | 2,4-Dichlorphenyl |
| 99 | H | 2,5-Dichlorphenyl |
| 100 | H | 2,6-Dichlorphenyl |
| 101 | H | 3,4-Dichlorphenyl |
| 102 | H | 3,5-Dichlorphenyl |
| 103 | H | 2,3,4-Trichlorphenyl |
| 104 | H | 2,3,5-Trichlorphenyl |
| 105 | H | 2,3,6-Trichlorphenyl |
| 106 | H | 2,4,5-Trichlorphenyl |
| 107 | H | 2,4,6-Trichlorphenyl |
| 108 | H | 3,4,5-Trichlorphenyl |
| 111 | H | 2-Bromphenyl |
| 112 | H | 3-Bromphenyl |
| 113 | H | 4-Bromphenyl |
| 114 | H | 2,4-Dibromphenyl |
| 115 | H | 3-Brom-4-fluorphenyl |
| 116 | H | 3-Brom-4-methoxyphenyl |
| 117 | H | 2-Jodphenyl |
| 118 | H | 3-Jodphenyl |
| 119 | H | 4-Jodphenyl |
| 120 | H | 2-Chlor-4-fluorphenyl |
| 121 | H | 2-Chlor-5-fluorphenyl |
| 122 | H | 2-Chlor-6-fluorphenyl |
| 123 | H | 2-Chlor-4-bromphenyl |
| 124 | H | 2-Brom-4-chlorphenyl |
| 125 | H | 2-Brom-4-fluorphenyl |
| 126 | H | 3-Brom-4-chlorphenyl |
| 127 | H | 3-Chlor-4-fluorphenyl |
| 128 | H | 3-Fluor-4-chlorphenyl |
| 129 | H | 2-Cyanophenyl |
| 130 | H | 3-Cyanophenyl |
| 131 | H | 4-Cyanophenyl |
| 132 | H | 2-Nitrophenyl |
| 133 | H | 3-Nitrophenyl |
| 134 | H | 4-Nitrophenyl |
| 135 | H | 2-Methylphenyl |
| 136 | H | 3-Methylphenyl |
| 137 | H | 4-Methylphenyl |
| 138 | H | 2,4-Dimethylphenyl |
| 139 | H | 2,6-Dimethylphenyl |
| 140 | H | 3,4-Dimethylphenyl |
| 141 | H | 3,5-Dimethylphenyl |
| 142 | H | 2,3,4-Trimethylphenyl |
| 143 | H | 2,3,5-Trimethylphenyl |
| 144 | H | 2,3,6-Trimethylphenyl |
| 145 | H | 2,4,5-Trimethylphenyl |
| 146 | H | 2,4,6-Trimethylphenyl |
| 147 | H | 3,4,5-Trimethylphenyl |
| 148 | H | Pentamethylphenyl |
| 149 | H | 2-Ethylphenyl |
| 150 | H | 3-Ethylphenyl |
| 151 | H | 4-Ethylphenyl |
| 152 | H | 3,5-Diethylphenyl |
| 153 | H | 2-n-Propylphenyl |
| 154 | H | 3-n-Propylphenyl |
| 155 | H | 4-n-Propylphenyl |
| 156 | H | 2-iso-Propylphenyl |
| 157 | H | 3-iso-Propylphenyl |
| 158 | H | 4-iso-Propylphenyl |
| 159 | H | 2,4-Di-iso-propylphenyl |
| 160 | H | 3,5-Di-iso-propylphenyl |
| 161 | H | 4-n-Butylphenyl |
| 162 | H | 4-sec.-Butylphenyl |
| 163 | H | 4-iso-Butylphenyl |
| 164 | H | 4-tert.-Butylphenyl |
| 165 | H | 3-tert.-Butylphenyl |
| 166 | H | 2-tert.-Butylphenyl |
| 167 | H | 2,4-Di-tert.-butylphenyl |
| 168 | H | 3,5-Di-tert.-butylphenyl |
| 171 | H | 2-Methyl-4-tert.-butylphenyl |
| 172 | H | 2-Methyl-6-tert.-butylphenyl |
| 173 | H | 2-Methyl-4-iso-propylphenyl |
| 174 | H | 2-Methyl-4-cyclohexylphenyl |
| 175 | H | 2-Methyl-4-phenylphenyl |
| 177 | H | 2-Methyl-4-phenoxyphenyl |
| 178 | H | 2-Methyl-4-benzyloxyphenyl |
| 179 | H | 2-Methyl-3-chlorphenyl |
| 180 | H | 2-Methyl-4-chlorphenyl |
| 181 | H | 2-Methyl-5-chlorphenyl |
| 182 | H | 2-Methyl-6-chlorphenyl |
| 183 | H | 2-Methyl-4-fluorphenyl |
| 184 | H | 2-Methyl-3-bromphenyl |
| 185 | H | 2-Methyl-4-bromphenyl |
| 186 | H | 2-Methyl-3-methoxyphenyl |
| 187 | H | 2-Methyl-4-methoxyphenyl |
| 188 | H | 2-Methyl-5-methoxyphenyl |
| 189 | H | 2-Methyl-6-methoxyphenyl |
| 190 | H | 2-Methyl-4-iso-propoxyphenyl |
| 191 | H | 2-Methyl-2,5-dimethoxyphenyl |
| 192 | H | 2-Methoxyphenyl |
| 193 | H | 3-Methoxyphenyl |
| 194 | H | 4-Methoxyphenyl |
| 195 | H | 2,3-Dimethoxyphenyl |
| 196 | H | 2,4-Dimethoxyphenyl |
| 197 | H | 2,5-Dimethoxyphenyl |
| 198 | H | 2,6-Dimethoxyphenyl |
| 199 | H | 3,4-Dimethoxyphenyl |
| 200 | H | 3,5-Dimethoxyphenyl |
| 201 | H | 3,6-Dimethoxyphenyl |
| 202 | H | 2,3,4-Trimethoxyphenyl |
| 203 | H | 2,3,5-Trimethoxyphenyl |
| 204 | H | 2,3,6-Trimethoxyphenyl |
| 205 | H | 2,4,5-Trimethoxyphenyl |
| 206 | H | 2,4,6-Trimethoxyphenyl |
| 207 | H | 3,4,5-Trimethoxyphenyl |
| 208 | H | 2-Ethoxyphenyl |
| 209 | H | 3-Ethoxyphenyl |
| 210 | H | 4-Ethoxyphenyl |
| 211 | H | 2-iso-Propoxyphenyl |
| 212 | H | 3-iso-Propoxyphenyl |
| 213 | H | 4-iso-Propoxyphenyl |
| 214 | H | 3-tert.-Butoxyphenyl |
| 215 | H | 4-tert.-Butoxyphenyl |
| 216 | H | 2-Trifluormethoxyphenyl |
| 217 | H | 3-Trifluormethoxyphenyl |
| 218 | H | 4-Trifluormethoxyphenyl |
| 219 | H | 3-(1',1',2',2'-Tetrafluor)ethoxyphenyl |
| 220 | H | 4-(1',1',2',2'-Tetrafluor)ethoxyphenyl |
| 221 | H | 2-Chlormethylphenyl |
| 222 | H | 3-Chlormethylphenyl |
| 223 | H | 4-Chlormethylphenyl |
| 224 | H | 2-Trifluormethylphenyl |
| 225 | H | 3-Trifluormethylphenyl |
| 226 | H | 4-Trifluormethylphenyl |
| 227 | H | 2-(Methoxyiminomethyl)phenyl |
| 228 | H | 3-(Methoxyiminomethyl)phenyl |
| 229 | H | 4-(Methoxyiminomethyl)phenyl |
| 230 | H | 2-(Ethoxyiminomethyl)phenyl |
| 231 | H | 3-(Ethoxyiminomethyl)phenyl |
| 232 | H | 4-(Ethoxyiminomethyl)phenyl |
| 233 | H | 2-(n-Propoxyiminomethyl)phenyl |
| 234 | H | 3-(n-Propoxyiminomethyl)phenyl |
| 235 | H | 4-(n-Propoxyiminomethyl)phenyl |
| 236 | H | 2-(iso-Propoxyiminomethyl)phenyl |
| 237 | H | 3-(iso-Propoxyiminomethyl)phenyl |
| 238 | H | 4-(iso-Propoxyiminomethyl)phenyl |
| 239 | H | 2-(n-Butoxyiminomethyl)phenyl |
| 240 | H | 3-(n-Butoxyiminomethyl)phenyl |
| 241 | H | 4-(n-Butoxyiminomethyl)phenyl |
| 242 | H | 2-(iso-Butoxyiminomethyl)phenyl |
| 243 | H | 3-(iso-Butoxyiminomethyl)phenyl |
| 244 | H | 4-(iso-Butoxyiminomethyl)phenyl |
| 245 | H | 2-(tert.-Butoxyiminomethyl)phenyl |
| 246 | H | 3-(tert.-Butoxyiminomethyl)phenyl |
| 247 | H | 4-(tert.-Butoxyiminomethyl)phenyl |
| 248 | H | 2-(n-Pentoxyimonomethyl)phenyl |
| 249 | H | 3-(n-Pentoxyimonomethyl)phenyl |
| 250 | H | 4-(n-Pentoxyimonomethyl)phenyl |
| 251 | H | 2-(n-Hexoxyimonomethyl)phenyl |
| 252 | H | 3-(n-Hexoxyimonomethyl)phenyl |
| 253 | H | 4-(n-Hexoxyimonomethyl)phenyl |
| 260 | H | 2-(Methoxyimino-1'-ethyl)phenyl |
| 261 | H | 3-(Methoxyimino-1'-ethyl)phenyl |
| 262 | H | 4-(Methoxyimino-1'-ethyl)phenyl |
| 263 | H | 2-(Ethoxyimino-1'-ethyl)phenyl |
| 264 | H | 3-(Ethoxyimino-1'-ethyl)phenyl |
| 265 | H | 4-(Ethoxyimino-1'-ethyl)phenyl |
| 266 | H | 2-(n-Propoxyimino-1'-ethyl)phenyl |
| 267 | H | 3-(n-Propoxyimino-1'-ethyl)phenyl |
| 268 | H | 4-(n-Propoxyimino-1'-ethyl)phenyl |
| 269 | H | 2-(n-Butoxyamino-1'-ethyl)phenyl |
| 270 | H | 3-(n-Butoxyamino-1'-ethyl)phenyl |
| 271 | H | 4-(n-Butoxyamino-1'-ethyl)phenyl |
| 272 | H | 2-(n-Pentoxyimino-1'-ethyl)phenyl |
| 273 | H | 3-(n-Pentoxyimino-1'-ethyl)phenyl |
| 274 | H | 4-(n-Pentoxyimino-1'-ethyl)phenyl |
| 275 | H | 2-(n-Hexoxyimino-1'-ethyl)phenyl |
| 276 | H | 3-(n-Hexoxyimino-1'-ethyl)phenyl |
| 277 | H | 4-(n-Hexoxyimino-1'-ethyl)phenyl |
| 284 | H | 2-Phenylphenyl |
| 285 | H | 3-Phenylphenyl |
| 286 | H | 4-Phenylphenyl |
| 287 | H | 2-Phenoxyphenyl |
| 288 | H | 3-Phenoxyphenyl |
| 289 | H | 4-Phenoxyphenyl |
| 293 | H | 4-(Imidazol-1'-yl)phenyl |
| 294 | H | 4-(Piperazin-1'-yl)phenyl |
| 295 | H | 4-(Morpholin-1'-yl)phenyl |
| 296 | H | 4-(Piperidin-1'-yl)phenyl |
| 297 | H | 4-(Pyridyl-2'-oxy)phenyl |
| 298 | H | 2-Cyclopropylphenyl |
| 299 | H | 3-Cyclopropylphenyl |
| 300 | H | 4-Cyclopropylphenyl |
| 301 | H | 3-Cyclohexylphenyl |
| 302 | H | 4-Cyclohexylphenyl |
| 303 | H | 4-Oxiranylphenyl |
| 304 | H | 4-(1',3'-Dioxan-2'-yl)phenyl |
| 305 | H | 4-(Tetrahydropyran-2yloxy)phenyl |
| 306 | H | 1-Naphthyl |
| 307 | H | 2-Naphthyl |
| 308 | H | 9-Anthryl |
| 324 | H | Benzyl |
| 325 | H | 2-Methylbenzyl |
| 326 | H | 3-Methylbenzyl |
| 327 | H | 4-Methylbenzyl |
| 328 | H | 4-tert.-Butylbenzyl |
| 329 | H | 2-Chlorbenzyl |
| 330 | H | 3-Chlorbenzyl |
| 331 | H | 4-Chlorbenzyl |
| 332 | H | 2,4-Dichlorbenzyl |
| 333 | H | 2,6-Dichlorbenzyl |
| 334 | H | 2,4,6-Trichlorbenzyl |
| 335 | H | 2-Trifluormethylbenzyl |
| 336 | H | 3-Trifluormethylbenzyl |
| 337 | H | 4-Trifluormethylbenzyl |
| 338 | H | 2-Methoxybenzyl |
| 339 | H | 4-Methoxybenzyl |
| 340 | H | 4-tert.-Butoxybenzyl |
| 341 | H | 4-Phenoxybenzyl |
| 342 | H | 1-Phenethyl |
| 343 | H | 2-Phenethyl |
| 344 | H | 1-Phenylpropyl |
| 345 | H | 2-Phenylpropyl |
| 346 | H | 3-Phenylpropyl |
| 347 | H | 2-Methyl-2-phenylpropyl |
| 348 | H | 2-Methyl-3-phenylpropyl |
| 349 | H | 4-Phenylbutyl |
| 350 | H | 2-Phenyl-1-ethenyl |
| 351 | H | 1-Phenyl-1-ethenyl |
| 352 | H | 1-Phenyl-1-propenyl |
| 353 | H | 1-Phenyl-1-propen-2-yl |
| 356 | H | 2-Pyridyl |
| 357 | H | 3-Pyridyl |
| 358 | H | 4-Pyridyl |
| 359 | H | 2,6-Pyrimidinyl |
| 360 | H | 1,5-Pyrimidinyl |
| 361 | H | 2-Thienyl |
| 362 | H | 3-Thienyl |
| 363 | H | 2-Furyl |
| 364 | H | 3-Furyl |
| 365 | H | 1-Pyrrolyl |
| 366 | H | 1-Imidazolyl |
| 367 | H | 1,2,4-Triazolyl |
| 368 | H | 1,3,4-Triazolyl |
| 369 | H | 4-Thiazolyl |
| 370 | H | 2-Benzothiazolyl |
| 377 | H | 2-Pyridylmethyl |
| 378 | H | 3-Pyridylmethyl |
| 384 | H | 2'-Furyl-2-ethenyl |
| 385 | H | 2'-Thienyl-2-ethenyl |
| 386 | H | 3'-Pyridyl-2-ethenyl |
| 411 | H | Cyclopropyl |
| 412 | H | Cyclobutyl |
| 413 | H | Cyclopentyl |
| 414 | H | Cyclohexyl |
| 423 | H | Ethenyl |
| 424 | H | 1-Propenyl |
| 425 | H | 2-Methyl-1-propenyl |
| 426 | H | 4-Methylpent-3-en-1-yl |
| 427 | H | 2-Propenyl |
| 428 | H | 2-Butenyl |
| 429 | H | 1-Methyl-2-propenyl |
| 430 | H | 3-Methyl-2-butenyl |
bedeuten und Verbindungen der Formel Ib 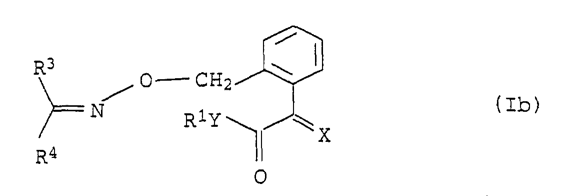 in denen
| Nr. | X | Y | R¹ | R³ | R⁴ |
|---|---|---|---|---|---|
| 5 | CHS-CH₃ | O | CH₃ | Cyclopropyl | 4-tert.-Butylphenyl |
| 25 | CHS-CH₃ | O | CH₃ | CH₃ | Benzyl |
| 26 | CHS-CH₃ | O | CH₃ | CH₃ | tert.-Butyl |
| 27 | CHS-CH₃ | O | CH₃ | CH₃ | iso-Propyl |
| 28 | CHS-CH₃ | 0 | CH₃ | CH₃ | 2-Methylbutyl |
| 29 | CHS-CH₃ | O | CH₃ | CH₃ | iso-Butyl |
| 30 | CHS - CH₃ | O | CH₃ | CH₃ | Phenyl |
| 36 | CHS-CH₃ | O | CH₃ | iso-Butyl | iso-Butyl |
| 38 | CHS-CH₃ | 0 | CH₃ | CH₂CH₂CH₂CH₂ | |
| 39 | CHS-CH₃ | O | CH₃ | CH₂CH₂CH₂CH₂CH₂ | |
| 196 | N-OCH₃ | O | C₂H₅ | CH₃ | Phenyl |
| 198 | N-OCH₃ | O | n-C₃H₅ | CH₃ | Phenyl |
| 205 | CHS-CH₃ | O | CH₃ | H | Phenyl |
bedeuten, ausgeschlossen sind.
b)
X CH-C₁-C₄-Alkoxy,
Y Sauerstoff oder Schwefel
R¹, R² unabhängig voneinander Wasserstoff oder C₁-C₄-Alkyl,
Z¹, Z² unabhängig voneinander Wasserstoff, Halogen, Methyl, Methoxy oder Cyano,
R⁴ durch C₁-C₁₀-Alkoximino-C₁-C₂-alkyl substituiertes Phenyl und
R³ Wasserstoff, Cyano, ggf. verzweigtes C₁-C₁₀-Alkyl, C₁-C₄-Halogenalkyl, C₃-C₆-Cycloalkyl, C₃-C₆-Halogencycloalkyl, C₃-C₆-Cycloalkyl-C₁-C₄-alkyl, C₁-C₄-Alkoxy-C₁-C₄-alkyl, C₁-C₄-Alkylthio-C₁-C₄-alkyl, Arylthio-C₁-C₄-alkyl, C₂-C₆-Alkenyl, C₂-C₅-Halogenalkenyl, C₃-C₆-Cycloalkenyl, C₃-C₆-Halogencycloalkenyl, C₂-C₆-Alkinyl, C₁-C₆-Alkoxy, C₁-C₆-Halogenalkoxy, C₁-C₄-Alkylthio, Benzylthio, C₁-C₄-Alkylcarbonyl, C₁-C₄-Alkoxycarbonyl, ggf. subst. Phenylcarbonyl, ggf. subst. Benzylcarbonyl, ggf. subst. Phenoxycarbonyl, ggf. subst. Benzyloxycarbonyl, ggf. subst. Aryl, ggf. subst. Aryloxy, ggf. subst. Arylthio, ggf. subst. Aryl-C₁-C₄-alkyl, ggf. subst. Aryl-C₂-C₄alkenyl, ggf. subst. Aryloxy-C₁-C₄-alkyl, ggf. subst. Arylthio-C₁-C₄-alkyl, ggf, subst. Hetaryl, ggf. subst. Hetaryloxy, ggf. subst. Hetarylthio, ggf. subst. Hetaryl-C₁-C₄-alkyl, ggf. subst. Hetaryl-C₂-C₄-alkenyl, ggf. subst. Hetaryl-C₁-C₄-alkoxy, ggf. subst. Heterocyclyl, ggf. subst. Heterocyclyloxy,
N(R⁶)₂, wobei die Bedeutungen von R⁶ gleich oder verschieden sind oder
CON(R⁷)₂, wobei die Bedeutungen von R⁷ gleich oder verschieden sind,
R⁶ Wasserstoff, C₁-C₆-Alkyl oder ggf. subst. Phenyl,
R⁷ Wasserstoff oder C₁-C₄-Alkyl,
ggf. subst. neben Wasserstoff die Reste Halogen, Cyano, Nitro, C₁-C₄-Alkyl, C₁-C₄-Alkoxy, C₁-C₄-Halogenalkyl, C₁-C₄-Halogenalkoxy, C₁-C₁₀-Alkoximino-C₁-C₂-alkyl, Aryl, Arloxy, Benzyloxy, Hetaryl, Hetaryloxy, C₃-C₆-Cycloalkyl, Heterocyclyl oder Heterocyclyloxy.

2. O-Benzyl-Oximether der Formel II 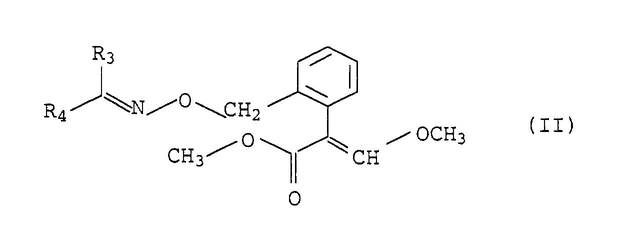 mit den in Anspruch 1 gegebenen Definitionen der Reste R³ bis R⁷.

3. O-Benzyl-Oximether der Formel III 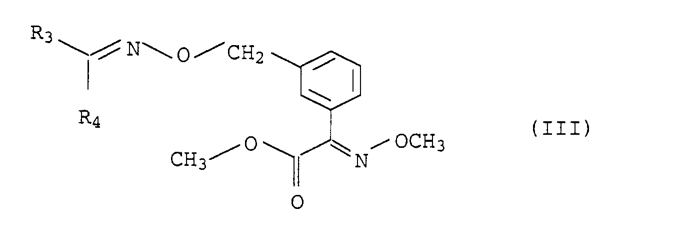 mit den in Anspruch 1 gegebenen Definitionen der Reste R³ bis R⁷.

4. Verfahren zur Herstellung von O-Benzyl-Oximethern der Formel I gemäß Anspruch 1, **dadurch gekennzeichnet, daß** man
a) ein Benzylderivat der Formel 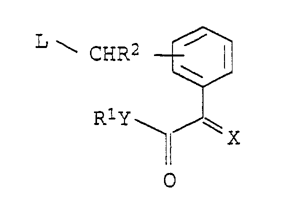 in der R¹, R², X und Y die im Anspruch 1 genannten Bedeutungen haben und L eine Abgangsgruppe bedeutet, mit N-Hydroxyphthalimid der Formel 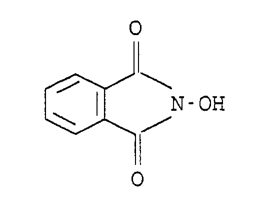 umsetzt und gegebenenfalls
b) die so erhaltene Verbindung der Formel 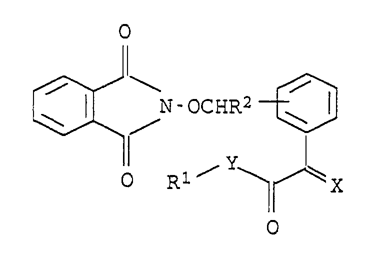 mit Mineralsäure oder mit Basen in das 0-substituierte Hydroxylamin der Formel 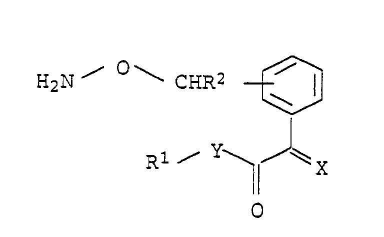 überführt und dieses mit einem Aldehyd oder Keton der Formel 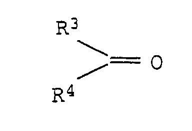 in der R³ und R⁴ die im Anspruch 1 genannten Bedeutungen haben, umsetzt oder
c) das Benzylderivat der Formel 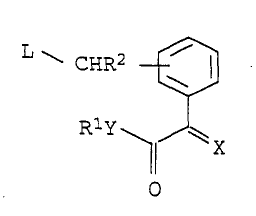 mit einem Oxim der Formel 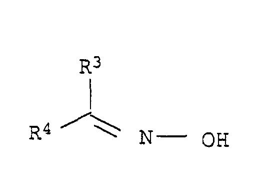 in er R³ und R⁴ die im Anspruch 1 genannten Bedeutungen haben, umsetzt.

5. Verbindungen der Formel I gemäß Anspruch 1, in der die Substituenten die folgende Bedeutung haben:
x NOCH₃ oder NOCH₂CH₃,
Y Sauerstoff,
R¹ C₁-C₄-Alkyl,
R² Wasserstoff oder C₁-C₂-Alkyl,
Z¹, Z² Wasserstoff,
R³, R⁴ unabhängig voneinander Wasserstoff, Cyano, ggf. verzweigtes C₁-C₁₀-Alkyl, C₁-C₄-Halogenalkyl, C₃-C₆-Cycloalkyl, C₃-C₆-Halogencycloalkyl, C₃-C₆-Cycloalkyl-C₁-C₄-alkyl, C₁-C₄-Alkoxy-C₁-C₄-alkyl, C₁-C₄-Alkylthio-C₁-C₄-alkyl, Arylthio-C₁-C₄-alkyl, C₂-C₆-Alkenyl, C₂-C₄-Halogenalkenyl, C₂-C₆-Alkinyl, C₁-C₆-Alkoxy, C₁-C₄-Alkylthio, C₁-C₄-Alkylcarbonyl, C₁-C₄-Alkoxycarbonyl, ggf. subst. Aryl, ggf. Aryloxy, ggf. subst. Arylthio, ggf. subst. Aryl-C₁-C₄-alkyl, ggf. subst. Aryl-C₂-C₄-alkenyl, ggf. subst. Hetaryl, ggf. subst. Hetaryloxy, ggf. subst. Hetarylthio, ggf. subst. Heterocyclyl,
N(R⁶)₂, wobei die Bedeutungen von R⁶ gleich oder verschieden sind,
CON(R⁷)₂, wobei die Bedeutungen von R⁷ gleich oder verschieden sind,
R³ und R⁴ zusammen
ein carbocyclischer oder heterocyclischer Ring, der durch die unter "ggf. subst." genannten Reste substituiert sein kann, oder
einer der Reste R³ oder R⁴ Halogen,
R⁶ Wasserstoff, C₁-C₆-Alkyl oder ggf. subst. Phenyl,
R⁷ Wasserstoff oder C₁-C₄-Alkyl,
ggf. subst. neben Wasserstoff die Reste Halogen, Cyano, Nitro, C₁-C₄-Alkyl, C₁-C₄-Alkoxy, C₁-C₄-Halogenalkyl, C₁-C₄-Halogenalkoxy, C₁-C₁₀-Alkoximino-C₁-C₂-alkyl, Aryl, Aryloxy, Benzyloxy, Hetaryl, Hetaryloxy, C₃-C₆-Cycloalkyl, Heterocyclyl oder Heterocyclyloxy, außer den in Anspruch 1 ausgenommenen Verbindung.

6. Fungizid, enthaltend einen inerten Trägerstoff und eine fungizid wirksame Menge eines Benzyl-Oximethers der Formel I gemäß Anspruch 1.

7. Verfahren zur Bekämpfung von Pilzen, **dadurch gekennzeichnet, daß** man die Pilze oder die von Pilzbefall bedrohten Materialien, Pflanzen, Saatgut oder den Erdboden mit einer fungizid wirksamen Menge einer Verbindung der Formel I gemäß Anspruch I behandelt.

8. Verbindungen der Formeln

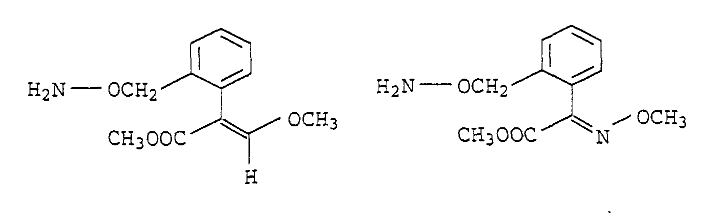

9. Verbindungen der Formel I gemäß Anspruch I, in der R¹ Methyl, R² Wasserstoff, R³ Cyano, R⁴ Cyclopropyl, X NOCH₃, Y Sauerstoff und Z¹ und Z² Wasserstoff bedeuten.

10. Verbindungen der Formel I gemäß Anspruch 1, in der R¹ Methyl, R² Wasserstoff, R³ Wasserstoff oder Methyl, R⁴ durch C₁-C₄-Alkoximino-C₁-C₂-alkyl substituiertes Phenyl, X CH-C₁-C₄-Alkoxy und Y Sauerstoff bedeuten.

## Claims

1. An O-benzyloxime ether of the general formula I 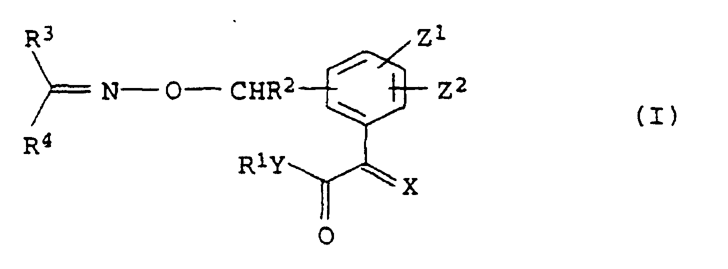 where
a)
X is CH-C₁-C₄-alkyl, CH-C₁-C₄-alkylthio or N-C₁-C₄-alkoxy,
Y is oxygen, sulfur or NR⁵,
R¹, R² and R⁵, independently of one another, are each hydrogen or C₁-C₄-alkyl,
Z¹ and Z² independently of one another, are each hydrogen, halogen, methyl, methoxy or cyano,
R³ and R⁴ independently of one another, are each hydrogen, cyano, straight-chain or branched C₁-C₁₀-alkyl, C₁-C₄-haloalkyl, C₃-C₆-cycloalkyl, C₃-C₆-halocycloalkyl, C₃-C₆-cycloalkyl-C₁-C₄-alkyl, C₁-C₄-alkoxy-C₁-C₄-alkyl, C₁-C₄-alkylthio-C₁-C₄-alkyl, arylthio-C₁-C₄-alkyl, C₂-C₆-alkenyl, C₂-C₅-haloalkenyl, C₃-C₆-cycloalkenyl, C₃-C₆-halocycloalkenyl, C₂-C₆-alkynyl, C₁-C₆-alkoxy, C₁-C₆-haloalkoxy, C₁-C₄-alkylthio, benzylthio, C₁-C₄-alkylcarbonyl, C₁-C₄-alkoxycarbonyl, unsubstituted or substituted phenylcarbonyl, unsubstituted or substituted benzylcarbonyl, unsubstituted or substituted phenoxycarbonyl, unsubstituted or substituted benzyloxycarbonyl, unsubstituted or substituted aryl, unsubstituted or substituted aryloxy, unsubstituted or substituted arylthio, unsubstituted or substituted aryl-C₁-C₄-alkyl, unsubstituted or substituted aryl-C₂-C₄-alkenyl, unsubstituted or substituted aryloxy-C₁-C₄-alkyl, unsubstituted or substituted arylthio-C₁-C₄-alkyl, unsubstituted or substituted hetaryl, unsubstituted or substituted hetaryloxy, unsubstituted or substituted hetarylthio, unsubstituted or substituted hetaryl-C₁-C₄-alkyl, unsubstituted or substituted hetaryl-C₂-C₄-alkenyl, unsubstituted or substituted hetaryl-C₁-C₄-alkoxy, unsubstituted or substituted heterocyclyl, unsubstituted or substituted heterocyclyloxy,
N(R⁶)₂, where the meanings of R⁶ are identical or different,
CON(R⁷)₂, where the meanings of R⁷ are identical or different,
R³ and R⁴ together form a carbocyclic or heterocyclic ring which may be substituted by the radicals stated under substituents, or one of the radicals R³ or R⁴ is halogen,
R⁶ is hydrogen, C₁-C₆-alkyl or unsubstituted or substituted phenyl,
R⁷ is hydrogen or C₁-C₄-alkyl,
substituents in addition to hydrogen, being halogen, cyano, nitro, C₁-C₄-alkyl, C₁-C₄-alkoxy, C₁-C₄-haloalkyl, C₁-C₄-haloalkoxy, C₁-C₁₀-alkoximino-C₁- or -C₂-alkyl, aryl, aryloxy, benzyloxy, hetaryl, hetaryloxy, C₃-C₆-cycloalkyl, heterocyclyl or heterocyclyloxy,
with the exception of the compounds in which
X is CH-C₁-C₂-alkylthio or N-C₁-C₂-alkoxy,
Y is oxygen,
Z¹, Z² and R² are each hydrogen,
R¹ is C₁-C₄-alkyl,
R³ is hydrogen, C₁-C₄-alkyl, C₁-C₄-haloalkyl or C₃-C₆-cycloalkyl,
R⁴ is C₁-C₆-alkyl, unsubstituted or substituted aryl-C₁-C₄-alkyl, unsubstituted or substituted hetaryl-C₁-C₄-alkyl, C₂-C₆-alkenyl, unsubstituted or substituted aryl-C₁-C₄-alkenyl, unsubstituted or substituted hetaryl-C₂-C₄-alkenyl, C₃-C₆-cycloalkyl, unsubstituted or substituted aryl,
unsubstituted or substituted hetaryl, C₁-C₃-alkylcarbonyl, unsubstituted or substituted phenylcarbonyl or
R³and R⁴, together with the carbon atom to which they are bonded, form an unsubstituted or substituted four- to seven-membered saturated ring which may contain an oxygen or sulfur atom and may also have an unsubstituted or substituted fused-on benzene ring,
of the disclaimed compounds, the following individual compounds of the formula Ia 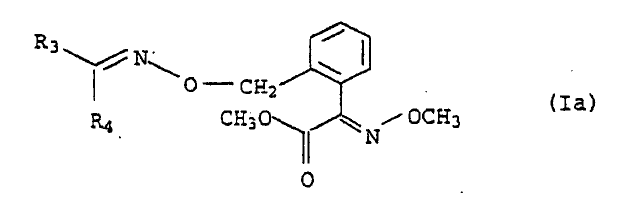 in which
| | R³ | R⁴ |
|---|---|---|
| 8 | CCl₃ | Phenyl |
| 9 | CH₂Cl | Phenyl |
| 10 | CF₂CF₃ | Phenyl |
| 11 | CF₂Cl | Phenyl |
| 12 | CHCl₂ | Phenyl |
| 13 | Cyclopropyl | Cyclopropyl |
| 18 | Cyclopropyl | 4-Ethoxyphenyl |
| 20 | Cyclopropyl | Pentachlorophenyl |
| 21 | Cyclopropyl | Pentafluorophenyl |
| 22 | Cyclopentyl | Phenyl |
| 23 | Cyclohexyl | Phenyl |
| 497 | CH₃ | Methyl |
| 498 | CH₃ | Ethyl |
| 499 | CH₃ | n-Propyl |
| 500 | CH₃ | Isopropyl |
| 501 | CH₃ | n-Butyl |
| 502 | CH₃ | Isobutyl |
| 503 | CH₃ | sec-Butyl |
| 504 | CH₃ | tert-Butyl |
| 505 | CH₃ | n-Hexyl |
| 507 | CH₃ | Cyclopropyl |
| 508 | CH₃ | Cyclohexyl |
| 542 | CH₃ | Acetyl |
| 543 | CH₃ | Propion-1-yl |
| 544 | CH₃ | Butyr-1-yl |
| 545 | CH₃ | Isobutyr-1-yl |
| 546 | CH₃ | Pivaloyl |
| 572 | CH₃ | Pentafluorophenyl |
| 576 | CH₃ | Pentachlorophenyl |
| 583 | CH₃ | 2,3,4-Trichlorophenyl |
| 584 | CH₃ | 2,3,5-Trichlorophenyl |
| 585 | CH₃ | 2,3,6-Trichlorophenyl |
| 586 | CH₃ | 2,4,5-Trichlorophenyl |
| 587 | CH₃ | 2,4,6-Trichlorophenyl |
| 588 | CH₃ | 3,4,5-Trichlorophenyl |
| 589 | CH₃ | 2,3,4,6-Tetrachlorophenyl |
| 590 | CH₃ | 2,3,5,6-Tetrachlorophenyl |
| 609 | CH₃ | 2-Cyanophenyl |
| 610 | CH₃ | 3-Cyanophenyl |
| 611 | CH₃ | 4-Cyanophenyl |
| 618 | CH₃ | 2,4-Dimethylphenyl |
| 619 | CH₃ | 2,6-Dimethylphenyl |
| 620 | CH₃ | 3,4-Dimethylphenyl |
| 621 | CH₃ | 3,5-Dimethylphenyl |
| 622 | CH₃ | 2,3,4-Trimethylphenyl |
| 623 | CH₃ | 2,3,5-Trimethylphenyl |
| 624 | CH₃ | 2,3,6-Trimethylphenyl |
| 625 | CH₃ | 2,4,5-Trimethylphenyl |
| 626 | CH₃ | 2,4,6-Trimethylphenyl |
| 627 | CH₃ | 3,4,5-Trimethylphenyl |
| 628 | CH₃ | Pentamethylphenyl |
| 629 | CH₃ | 2-Ethylphenyl |
| 630 | CH₃ | 3-Ethylphenyl |
| 631 | CH₃ | 4-Ethylphenyl |
| 632 | CH₃ | 3,5-Diethylphenyl |
| 633 | CH₃ | 2-n-Propylphenyl |
| 634 | CH₃ | 3-n-Propylphenyl |
| 635 | CH₃ | 4-n-Propylphenyl |
| 636 | CH₃ | 2-Isopropylphenyl |
| 637 | CH₃ | 3-Isopropylphenyl |
| 638 | CH₃ | 4-Isopropylphenyl |
| 639 | CH₃ | 2,4-Diisopropylphenyl |
| 640 | CH₃ | 3,5-Diisopropylphenyl |
| 641 | CH₃ | 4-n-Butylphenyl |
| 642 | CH₃ | 4-sec-Butylphenyl |
| 643 | CH₃ | 4-Isobutylphenyl |
| 644 | CH₃ | 4-tert-Butylphenyl |
| 645 | CH₃ | 3-tert-Butylphenyl |
| 646 | CH₃ | 2-tert-Butylphenyl |
| 647 | CH₃ | 2,4-Di-tert-butylphenyl |
| 648 | CH₃ | 3,5-Di-tert-butylphenyl |
| 650 | CH₃ | 4-n-Dodecylphenyl |
| 651 | CH₃ | 2-Methyl-4-tert-butylphenyl |
| 652 | CH₃ | 2-Methyl-6-tert-butylphenyl |
| 653 | CH₃ | 2-Methyl-4-isopropylphenyl |
| 654 | CH₃ | 2-Methyl-4-cyclohexylphenyl |
| 655 | CH₃ | 2-Methyl-4-phenylphenyl |
| 656 | CH₃ | 2-Methyl-4-benzylphenyl |
| 657 | CH₃ | 2-Methyl-4-phenoxyphenyl |
| 658 | CH₃ | 2-Methyl-4-benzyloxyphenyl |
| 666 | CH₃ | 2-Methyl-3-methoxyphenyl |
| 667 | CH₃ | 2-Methyl-4-methoxyphenyl |
| 668 | CH₃ | 2-Methyl-5-methoxyphenyl |
| 669 | CH₃ | 2-Methyl-6-methoxyphenyl |
| 670 | CH₃ | 2-Methyl-4-isopropoxyphenyl |
| 671 | CH₃ | 2-Methyl-2,5-dimethoxyphenyl |
| 688 | CH₃ | 2-Ethoxyphenyl |
| 689 | CH₃ | 3-Ethoxyphenyl |
| 690 | CH₃ | 4-Ethoxyphenyl |
| 691 | CH₃ | 2-Isopropoxyphenyl |
| 692 | CH₃ | 3-Isopropoxyphenyl |
| 693 | CH₃ | 4-Isopropoxyphenyl |
| 694 | CH₃ | 3-tert-Butoxyphenyl |
| 695 | CH₃ | 4-tert-Butoxyphenyl |
| 699 | CH₃ | 3-(1',1',2',2'-Tetrafluoro)ethoxyphenyl |
| 700 | CH₃ | 4-(1',1',2',2'-Tetrafluoro)ethoxyphenyl |
| 701 | CH₃ | 2-Chloromethylphenyl |
| 702 | CH₃ | 3-Chloromethylphenyl |
| 703 | CH₃ | 4-Chloromethylphenyl |
| 707 | CH₃ | 2-(Methoxyiminomethyl)phenyl |
| 708 | CH₃ | 3-(Methoxyiminomethyl)phenyl |
| 709 | CH₃ | 4-(Methoxyiminomethyl)phenyl |
| 710 | CH₃ | 2-(Ethoxyiminomethyl)phenyl |
| 711 | CH₃ | 3-(Ethoxyiminomethyl)phenyl |
| 712 | CH₃ | 4-(Ethoxyiminomethyl)phenyl |
| 713 | CH₃ | 2-(n-Propoxyiminomethyl)phenyl |
| 714 | CH₃ | 3-(n-Propoxyiminomethyl)phenyl |
| 715 | CH₃ | 4-(n-Propoxyiminomethyl)phenyl |
| 716 | CH₃ | 2-(Isopropoxyiminomethyl)phenyl |
| 717 | CH₃ | 3-(Isopropoxyiminomethyl)phenyl |
| 718 | CH₃ | 4-(Isopropoxyiminomethyl)phenyl |
| 719 | CH₃ | 2-(n-Butoxyiminomethyl)phenyl |
| 720 | CH₃ | 3-(n-Butoxyiminomethyl)phenyl |
| 721 | CH₃ | 4-(n-Butoxyiminomethyl)phenyl |
| 722 | CH₃ | 2-(Isobutoxyiminomethyl)phenyl |
| 723 | CH₃ | 3-(Isobutoxyiminomethyl)phenyl |
| 724 | CH₃ | 4-(Isobutoxyiminomethyl)phenyl |
| 725 | CH₃ | 2-(tert-Butoxyiminomethyl)phenyl |
| 726 | CH₃ | 3-(tert-Butoxyiminomethyl)phenyl |
| 727 | CH₃ | 4-(tert-Butoxyiminomethyl)phenyl |
| 728 | CH₃ | 2-(n-Pentoxyiminomethyl)phenyl |
| 729 | CH₃ | 3-(n-Pentoxyiminomethyl)phenyl |
| 730 | CH₃ | 4-(n-Pentoxyiminomethyl)phenyl |
| 731 | CH₃ | 2-(n-Hexoxyiminomethyl)phenyl |
| 732 | CH₃ | 3-(n-Hexoxyiminomethyl)phenyl |
| 733 | CH₃ | 4-(n-Hexoxyiminomethyl)phenyl |
| 740 | CH₃ | 2-(Methoxyimino-1'-ethyl)phenyl |
| 741 | CH₃ | 3-(Methoxyimino-1'-ethyl)phenyl |
| 742 | CH₃ | 4-(Methoxyimino-1'-ethyl)phenyl |
| 743 | CH₃ | 2-(Ethoxyimino-1'-ethyl)phenyl |
| 744 | CH₃ | 3-(Ethoxyimino-1'-ethyl)phenyl |
| 745 | CH₃ | 4-(Ethoxyimino-1'-ethyl)phenyl |
| 746 | CH₃ | 2-(n-Propoxyimino-1'-ethyl)phenyl |
| 747 | CH₃ | 3-(n-Propoxyimino-1'-ethyl)phenyl |
| 748 | CH₃ | 4-(n-Propoxyimino-1'-ethyl)phenyl |
| 749 | CH₃ | 2-(n-Butoxyamino-1'-ethyl)phenyl |
| 750 | CH₃ | 3-(n-Butoxyamino-1'-ethyl)phenyl |
| 751 | CH₃ | 4-(n-Butoxyamino-1'-ethyl)phenyl |
| 752 | CH₃ | 2-(n-Pentoxyimino-1'-ethyl)phenyl |
| 753 | CH₃ | 3-(n-Pentoxyimino-1'-ethyl)phenyl |
| 754 | CH₃ | 4-(n-Pentoxyimino-1'-ethyl)phenyl |
| 755 | CH₃ | 2-(n-Hexoxyimino-1'-ethyl)phenyl |
| 756 | CH₃ | 3-(n-Hexoxyimino-1'-ethyl)phenyl |
| 757 | CH₃ | 4-(n-Hexoxyimino-1'-ethyl)phenyl |
| 764 | CH₃ | 2-Phenylphenyl |
| 765 | CH₃ | 3-Phenylphenyl |
| 766 | CH₃ | 4-Phenylphenyl |
| 767 | CH₃ | 2-Phenoxyphenyl |
| 768 | CH₃ | 3-Phenoxyphenyl |
| 769 | CH₃ | 4-Phenoxyphenyl |
| 770 | CH₃ | 2-Benzyloxyphenyl |
| 771 | CH₃ | 3-Benzyloxyphenyl |
| 772 | CH₃ | 4-Benzyloxyphenyl |
| 773 | CH₃ | 4-(Imidazol-1'-yl)phenyl |
| 774 | CH₃ | 4-(Piperazin-1'-yl)phenyl |
| 777 | CH₃ | 4-(Pyridyl-2'-oxy)phenyl |
| 778 | CH₃ | 2-Cyclopropylphenyl |
| 779 | CH₃ | 3-Cyclopropylphenyl |
| 780 | CH₃ | 4-Cyclopropylphenyl |
| 781 | CH₃ | 3-Cyclohexylphenyl |
| 782 | CH₃ | 4-Cyclohexylphenyl |
| 783 | CH₃ | 4-Oxiranylphenyl |
| 784 | CH₃ | 4-(1',3'-Dioxan-2'-yl)phenyl |
| 785 | CH₃ | 4-(Tetrahydropyran-2-yloxy) phenyl |
| 788 | CH₃ | 9-Anthryl |
| 804 | CH₃ | Benzyl |
| 805 | CH₃ | 2-Methylbenzyl |
| 806 | CH₃ | 3-Methylbenzyl |
| 807 | CH₃ | 4-Methylbenzyl |
| 808 | CH₃ | 4-tert-Butylbenzyl |
| 818 | CH₃ | 2-Methoxybenzyl |
| 819 | CH₃ | 4-Methoxybenzyl |
| 820 | CH₃ | 4-tert-Butoxybenzyl |
| 821 | CH₃ | 4-Phenoxybenzyl |
| 822 | CH₃ | 1-Phenethyl |
| 823 | CH₃ | 2-Phenethyl |
| 824 | CH₃ | 1-Phenylpropyl |
| 825 | CH₃ | 2-Phenylpropyl |
| 826 | CH₃ | 3-Phenylpropyl |
| 827 | CH₃ | 2-Methyl-2-phenylpropyl |
| 828 | CH₃ | 2-Methyl-3-phenylpropyl |
| 829 | CH₃ | 4-Phenylbutyl |
| 830 | CH₃ | 2-Phenyl-1-ethenyl |
| 831 | CH₃ | 1-Phenyl-1-ethenyl |
| 832 | CH₃ | 1-Phenyl-1-propenyl |
| 833 | CH₃ | 1-Phenyl-1-propen-2-yl |
| 839 | CH₃ | 2,6-Pyrimidinyl |
| 840 | CH₃ | 1,5-Pyrimidinyl |
| 845 | CH₃ | 1-Pyrrolyl |
| 846 | CH₃ | 1-Imidazolyl |
| 847 | CH₃ | 1,2,4-Triazolyl |
| 848 | CH₃ | 1,3,4-Triazolyl |
| 849 | CH₃ | 4-Thiazolyl |
| 857 | CH₃ | 2-Pyridylmethyl |
| 858 | CH₃ | 3-Pyridylmethyl |
| 864 | CH₃ | 2'-Furyl-2-ethenyl |
| 865 | CH₃ | 2'-Thienyl-2-ethenyl |
| 866 | CH₃ | 3'-Pyridyl-2-ethenyl |
| 891 | CH₃ | Cyclopropyl |
| 892 | CH₃ | Cyclobutyl |
| 893 | CH₃ | Cyclopentyl |
| 894 | CH₃ | Cyclohexyl |
| 903 | CH₃ | Ethenyl |
| 904 | CH₃ | 1-Propenyl |
| 905 | CH₃ | 2-Methyl-1-propenyl |
| 906 | CH₃ | 4-Methylpent-3-en-1-yl |
| 907 | CH₃ | 2-Propenyl |
| 908 | CH₃ | 2-Butenyl |
| 909 | CH₃ | 1-Methyl-2-propenyl |
| 910 | CH₃ | 3-Methyl-2-butenyl |
| 948 | Ethyl | Ethyl |
| 949 | Ethyl | n-Propyl |
| 950 | Ethyl | Isopropyl |
| 951 | Ethyl | n-Butyl |
| 952 | Ethyl | Isobutyl |
| 953 | Ethyl | 2-Methylbutyl |
| 954 | Ethyl | Benzyl |
| 955 | n-Propyl | n-Propyl |
| 956 | Isopropyl | Isopropyl |
| 957 | n-Butyl | n-Butyl |
| 958 | Isobutyl | Isobutyl |
| 959 | tert-Butyl | tert-Butyl |
| 966 | n-Butyl | Phenyl |
| | R³ | R⁴ |
|---|---|---|
| 17 | H | Methyl |
| 18 | H | Ethyl |
| 19 | H | n-Propyl |
| 20 | H | Isopropyl |
| 21 | H | n-Butyl |
| 22 | H | Isobutyl |
| 23 | H | sec-Butyl |
| 24 | H | tert-Butyl |
| 25 | H | n-Hexyl |
| 27 | H | Cyclopropyl |
| 28 | H | Cyclohexyl |
| 62 | H | Acetyl |
| 63 | H | Propion-1-yl |
| 64 | H | Butyr-1-yl |
| 65 | H | Isobutyr-1-yl |
| 66 | H | Pivaloyl |
| 88 | H | Phenyl |
| 89 | H | 2-Fluorophenyl |
| 90 | H | 3-Fluorophenyl |
| 91 | H | 4-Fluorophenyl |
| 92 | H | Pentafluorophenyl |
| 93 | H | 2-Chlorophenyl |
| 94 | H | 3-Chlorophenyl |
| 95 | H | 4-Chlorophenyl |
| 97 | H | 2,3-Dichlorophenyl |
| 98 | H | 2,4-Dichlorophenyl |
| 99 | H | 2,5-Dichlorophenyl |
| 100 | H | 2,6-Dichlorophenyl |
| 101 | H | 3,4-Dichlorophenyl |
| 102 | H | 3,5-Dichlorophenyl |
| 103 | H | 2,3,4-Trichlorophenyl |
| 104 | H | 2,3,5-Trichloropheny |
| 105 | H | 2,3,6-Trichlorophenyl |
| 106 | H | 2,4,5-Trichlorophenyl |
| 107 | H | 2,4,6-Trichlorophenyl |
| 108 | H | 3,4,5-Trichlorophenyl |
| 111 | H | 2-Bromophenyl |
| 112 | H | 3-Bromophenyl |
| 113 | H | 4-Bromophenyl |
| 114 | H | 2,4-Dibromophenyl |
| 115 | H | 3-Bromo-4-fluorophenyl |
| 116 | H | 3-Bromo-4-methoxyphenyl |
| 117 | H | 2-Iodophenyl |
| 118 | H | 3-Iodophenyl |
| 119 | H | 4-Iodophenyl |
| 120 | H | 2-Chloro-4-fluorophenyl |
| 121 | H | 2-Chloro-5-fluorophenyl |
| 122 | H | 2-Chloro-6-fluorophenyl |
| 123 | H | 2-Chloro-4-bromophenyl |
| 124 | H | 2-Bromo-4-chlorophenyl |
| 125 | H | 2-Bromo-4-fluorophenyl |
| 126 | H | 3-Bromo-4-chlorophenyl |
| 127 | H | 3-Chloro-4-fluorophenyl |
| 128 | H | 3-Fluoro-4-chlorophenyl |
| 129 | H | 2-Cyanophenyl |
| 130 | H | 3-Cyanophenyl |
| 131 | H | 4-Cyanophenyl |
| 132 | H | 2-Nitrophenyl |
| 133 | H | 3-Nitrophenyl |
| 134 | H | 4-Nitrophenyl |
| 135 | H | 2-Methylphenyl |
| 136 | H | 3-Methylphenyl |
| 137 | H | 4-Methylphenyl |
| 138 | H | 2,4-Dimethylphenyl |
| 139 | H | 2,6-Dimethylphenyl |
| 140 | H | 3,4-Dimethylphenyl |
| 141 | H | 3,5-Dimethylphenyl |
| 142 | H | 2,3,4-Trimethylphenyl |
| 143 | H | 2,3,5-Trimethylphenyl |
| 144 | H | 2,3,6-Trimethylphenyl |
| 145 | H | 2,4,5-Trimethylphenyl |
| 146 | H | 2,4,6-Trimethylphenyl |
| 147 | H | 3,4,5-Trimethylphenyl |
| 148 | H | Pentamethylphenyl |
| 149 | H | 2-Ethylphenyl |
| 150 | H | 3-Ethylphenyl |
| 151 | H | 4-Ethylphenyl |
| 152 | H | 3,5-Diethylphenyl |
| 153 | H | 2-n-Propylphenyl |
| 154 | H | 3-n-Propylphenyl |
| 155 | H | 4-n-Propylphenyl |
| 156 | H | 2-Isopropylphenyl |
| 157 | H | 3-Isopropylphenyl |
| 158 | H | 4-Isopropylphenyl |
| 159 | H | 2,4-Diisopropylphenyl |
| 160 | H | 3,5-Diisopropylphenyl |
| 161 | H | 4-n-Butylphenyl |
| 162 | H | 4-sec-Butylphenyl |
| 163 | H | 4-Isobutylphenyl |
| 164 | H | 4-tert-Butylphenyl |
| 165 | H | 3-tert-Butylphenyl |
| 166 | H | 2-tert-Butylphenyl |
| 167 | H | 2,4-Di-tert-butylphenyl |
| 168 | H | 3,5-Di-tert-butylphenyl |
| 171 | H | 2-Methyl-4-tert-butylphenyl |
| 172 | H | 2-Methyl-6-tert-butylphenyl |
| 173 | H | 2-Methyl-4-isopropylphenyl |
| 174 | H | 2-Methyl-4-cyclohexylphenyl |
| 175 | H | 2-Methyl-4-phenylphenyl |
| 177 | H | 2-Methyl-4-phenoxyphenyl |
| 178 | H | 2-Methyl-4-benzyloxyphenyl |
| 179 | H | 2-Methyl-3-chlorophenyl |
| 180 | H | 2-Methyl-4-chlorophenyl |
| 181 | H | 2-Methyl-5-chlorophenyl |
| 182 | H | 2-Methyl-6-chlorophenyl |
| 183 | H | 2-Methyl-4-fluorophenyl |
| 184 | H | 2-Methyl-3-bromophenyl |
| 185 | H | 2-Methyl-4-bromophenyl |
| 186 | H | 2-Methyl-3-methoxyphenyl |
| 187 | H | 2-Methyl-4-methoxyphenyl |
| 188 | H | 2-Methyl-5-methoxyphenyl |
| 189 | H | 2-Methyl-6-methoxyphenyl |
| 190 | H | 2-Methyl-4-isopropoxyphenyl |
| 191 | H | 2-Methyl-2,5-dimethoxyphenyl |
| 192 | H | 2-Methoxyphenyl |
| 193 | H | 3-Methoxyphenyl |
| 194 | H | 4-Methoxyphenyl |
| 195 | H | 2,3-Dimethoxyphenyl |
| 196 | H | 2,4-Dimethoxyphenyl |
| 197 | H | 2,5-Dimethoxyphenyl |
| 198 | H | 2,6-Dimethoxyphenyl |
| 199 | H | 3,4-Dimethoxyphenyl |
| 200 | H | 3,5-Dimethoxyphenyl |
| 201 | H | 3,6-Dimethoxyphenyl |
| 202 | H | 2,3,4-Trimethoxyphenyl |
| 203 | H | 2,3,5-Trimethoxyphenyl |
| 204 | H | 2,3,6-Trimethoxyphenyl |
| 205 | H | 2,4,5-Trimethoxyphenyl |
| 206 | H | 2,4,6-Trimethoxyphenyl |
| 207 | H | 3,4,5-Trimethoxyphenyl |
| 208 | H | 2-Ethoxyphenyl |
| 209 | H | 3-Ethoxyphenyl |
| 210 | H | 4-Ethoxyphenyl |
| 211 | H | 2-Isopropoxyphenyl |
| 212 | H | 3-Isopropoxyphenyl |
| 213 | H | 4-Isopropoxyphenyl |
| 214 | H | 3-tert-Butoxyphenyl |
| 215 | H | 4-tert-Butoxyphenyl |
| 216 | H | 2-Trifluoromethoxyphenyl |
| 217 | H | 3-Trifluoromethoxyphenyl |
| 218 | H | 4-Trifluoromethoxyphenyl |
| 219 | H | 3-(1',1',2',2'-Tetrafluoro)ethoxyphenyl |
| 220 | H | 4-(1',1',2',2'-Tetrafluoro)ethoxyphenyl |
| 221 | H | 2-Chloromethylphenyl |
| 222 | H | 3-Chloromethylphenyl |
| 223 | H | 4-Chloromethylphenyl |
| 224 | H | 2-Trifluoromethylphenyl |
| 225 | H | 3-Trifluoromethylphenyl |
| 226 | H | 4-Trifluoromethylphenyl |
| 227 | H | 2-(Methoxyiminomethyl)phenyl |
| 228 | H | 3-(Methoxyiminomethyl)phenyl |
| 229 | H | 4-(Methoxyiminomethyl)phenyl |
| 230 | H | 2-(Ethoxyiminomethyl)phenyl |
| 231 | H | 3-(Ethoxyiminomethyl)phenyl |
| 232 | H | 4-(Ethoxyiminomethyl)phenyl |
| 233 | H | 2-(n-Propoxyiminomethyl)phenyl |
| 234 | H | 3-(n-Propoxyiminomethyl)phenyl |
| 235 | H | 4-(n-Propoxyiminomethyl)phenyl |
| 236 | H | 2-(Isopropoxyiminomethyl)phenyl |
| 237 | H | 3-(Isopropoxyiminomethyl)phenyl |
| 238 | H | 4-(Isopropoxyiminomethyl)phenyl |
| 239 | H | 2-(n-Butoxyiminomethyl)phenyl |
| 240 | H | 3-(n-Butoxyiminomethyl)phenyl |
| 241 | H | 4-(n-Butoxyiminomethyl)phenyl |
| 242 | H | 2-(Isobutoxyiminomethyl)phenyl |
| 243 | H | 3-(Isobutoxyiminomethyl)phenyl |
| 244 | H | 4-(Isobutoxyiminomethyl)phenyl |
| 245 | H | 2-(tert-Butoxyiminomethyl)phenyl |
| 246 | H | 3-(tert-Butoxyiminomethyl)phenyl |
| 247 | H | 4-(tert-Butoxyiminomethyl)phenyl |
| 248 | H | 2-(n-Pentoxyiminomethyl)phenyl |
| 249 | H | 3-(n-Pentoxyiminomethyl)phenyl |
| 250 | H | 4-(n-Pentoxyiminomethyl)phenyl |
| 251 | H | 2-(n-Hexoxyiminomethyl)phenyl |
| 252 | H | 3-(n-Hexoxyiminomethyl)phenyl |
| 253 | H | 4-(n-Hexoxyiminomethyl)phenyl |
| 260 | H | 2-(Methoxyimino-1'-ethyl)phenyl |
| 261 | H | 3-(Methoxyimino-1'-ethyl)phenyl |
| 262 | H | 4-(Methoxyimino-1'-ethyl)phenyl |
| 263 | H | 2-(Ethoxyimino-1'-ethyl)phenyl |
| 264 | H | 3-(Ethoxyimino-1'-ethyl)phenyl |
| 265 | H | 4-(Ethoxyimino-1'-ethyl)phenyl |
| 266 | H | 2-(n-Propoxyimino-1'-ethyl)phenyl |
| 267 | H | 3-(n-Propoxyimino-1'-ethyl)phenyl |
| 268 | H | 4-(n-Propoxyimino-1'-ethyl)phenyl |
| 269 | H | 2-(n-Butoxyamino-1'-ethyl)phenyl |
| 270 | H | 3-(n-Butoxyamino-1'-ethyl)phenyl |
| 271 | H | 4-(n-Butoxyamino-1'-ethyl)phenyl |
| 272 | H | 2-(n-Pentoxyimino-1'-ethyl)phenyl |
| 273 | H | 3-(n-Pentoxyimino-1'-ethyl)phenyl |
| 274 | H | 4-(n-Pentoxyimino-1'-ethyl)phenyl |
| 275 | H | 2-(n-Hexoxyimino-1'-ethyl)phenyl |
| 276 | H | 3-(n-Hexoxyimino-1'-ethyl)phenyl |
| 277 | H | 4-(n-Hexoxyimino-1'-ethyl)phenyl |
| 284 | H | 2-Phenylphenyl |
| 285 | H | 3-Phenylphenyl |
| 286 | H | 4-Phenylphenyl |
| 287 | H | 2-Phenoxyphenyl |
| 288 | H | 3-Phenoxyphenyl |
| 289 | H | 4-Phenoxyphenyl |
| 293 | H | 4-(Imidazol-1'-yl)phenyl |
| 294 | H | 4-(Piperazin-1'-yl)phenyl |
| 295 | H | 4-(Morpholin-1'-yl)phenyl |
| 296 | H | 4-(Piperidin-1'-yl)phenyl |
| 297 | H | 4-(Pyridyl-2'-oxy)phenyl |
| 298 | H | 2-Cyclopropylphenyl |
| 299 | H | 3-Cyclopropylphenyl |
| 300 | H | 4-Cyclopropylphenyl |
| 301 | H | 3-Cyclohexylphenyl |
| 302 | H | 4-Cyclohexylphenyl |
| 303 | H | 4-Oxiranylphenyl |
| 304 | H | 4-(1',3'-Dioxan-2'-yl)phenyl |
| 305 | H | 4-(Tetrahydropyran-2-yloxy)phenyl |
| 306 | H | 1-Naphthyl |
| 307 | H | 2-Naphthyl |
| 308 | H | 9-Anthryl |
| 324 | H | Benzyl |
| 325 | H | 2-Methylbenzyl |
| 326 | H | 3-Methylbenzyl |
| 327 | H | 4-Methylbenzyl |
| 328 | H | 4-tert-Butylbenzyl |
| 329 | H | 2-Chlorobenzyl |
| 330 | H | 3-Chlorobenzyl |
| 331 | H | 4-Chorobenzyl |
| 332 | H | 2,4-Dichlorobenzyl |
| 333 | H | 2,6-Dichlorobenzyl |
| 334 | H | 2,4,6-Trichlorobenzyl |
| 335 | H | 2-Trifluoromethylbenzyl |
| 336 | H | 3-Trifluoromethylbenzyl |
| 337 | H | 4-Trifluoromethylbenzyl |
| 338 | H | 2-Methoxybenzyl |
| 339 | H | 4-Methoxybenzyl |
| 340 | H | 4-tert-Butoxybenzyl |
| 341 | H | 4-Phenoxybenzyl |
| 342 | H | 1-Phenethyl |
| 343 | H | 2-Phenethyl |
| 344 | H | 1-Phenylpropyl |
| 345 | H | 2-Phenylpropyl |
| 346 | H | 3-Phenylpropyl |
| 347 | H | 2-Methyl-2-phenylpropyl |
| 348 | H | 2-Methyl-3-phenylpropyl |
| 349 | H | 4-Phenylbutyl |
| 350 | H | 2-Phenyl-1-ethenyl |
| 351 | H | 1-Phenyl-1-ethenyl |
| 352 | H | 1-Phenyl-1-propenyl |
| 353 | H | 1-Phenyl-1-propen-2-yl |
| 356 | H | 2-Pyridyl |
| 357 | H | 3-Pyridyl |
| 358 | H | 4-Pyridyl |
| 359 | H | 2,6-Pyrimidinyl |
| 360 | H | 1,5-Pyrimidinyl |
| 361 | H | 2-Thienyl |
| 362 | H | 3-Thienyl |
| 363 | H | 2-Furyl |
| 364 | H | 3-Furyl |
| 365 | H | 1-Pyrrolyl |
| 366 | H | 1-Imidazolyl |
| 367 | H | 1,2,4-Triazolyl |
| 368 | H | 1,3,4-Triazolyl |
| 369 | H | 4-Thiazolyl |
| 370 | H | 2-Benzothiazolyl |
| 377 | H | 2-Pyridylmethyl |
| 378 | H | 3-Pyridylmethyl |
| 384 | H | 2'-Furyl-2-ethenyl |
| 385 | H | 2'-Thienyl-2-ethenyl |
| 386 | H | 3'-Pyridyl-2-ethenyl |
| 411 | H | Cyclopropyl |
| 412 | H | Cyclobutyl |
| 413 | H | Cyclopentyl |
| 414 | H | Cyclohexyl |
| 423 | H | Ethenyl |
| 424 | H | 1-Propenyl |
| 425 | H | 2-Methyl-1-propenyl |
| 426 | H | 4-Methylpent-3-en-1-yl |
| 427 | H | 2-Propenyl |
| 428 | H | 2-Butenyl |
| 429 | H | 1-Methyl-2-propenyl |
| 430 | H | 3-Methyl-2-butenyl |
and compounds of the formula Ib 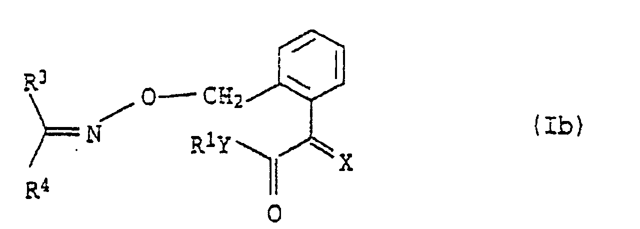 in which
| No. | X | Y | R¹ | R³ | R⁴ |
|---|---|---|---|---|---|
| 5 | CHS-CH₃ | O | CH₃ | Cyclopropyl | 4-tert-Butylphenyl |
| 25 | CHS-CH₃ | O | CH₃ | CH₃ | Benzyl |
| 26 | CHS-CH₃ | O | CH₃ | CH₃ | tert-Butyl |
| 27 | CHS-CH₃ | O | CH₃ | CH₃ | Isopropyl |
| 28 | CHS-CH₃ | O | CH₃ | CH₃ | 2-Methylbutyl |
| 29 | CHS-CH₃ | O | CH₃ | CH₃ | Isobutyl |
| 30 | CHS-CH₃ | O | CH₃ | CH₃ | Phenyl |
| 36 | CHS-CH₃ | O | CH₃ | Isobutyl | Isobutyl |
| 38 | CHS-CH₃ | O | CH₃ | CH₂CH₂CH₂CH₂ | |
| 39 | CHS-CH₃ | O | CH₃ | CH₂CH₂CH₂CH₂CH₂ | |
| 196 | N-OCH₃ | O | C₂H₅ | CH₃ | Phenyl |
| 198 | N-OCH₃ | O | n-C₃H₅ | CH₃ | Phenyl |
| 205 | CHS-CH₃ | O | CH₃ | H | Phenyl |
being excepted,
b)
X is CH-C₁-C₄-alkoxy,
Y is oxygen or sulfur,
R¹ and R², independently of one another, are each hydrogen or C₁-C₄-alkyl,
Z¹ and Z², independently of one another, are each hydrogen, halogen, methyl, methoxy or cyano,
R⁴ is C₁-C₁₀-alkoximino-C₁- or -C₂-alkyl-substituted phenyl and
R³ is hydrogen, cyano, straight-chain or branched C₁-C₁₀-alkyl, C₁-C₄-haloalkyl, C₃-C₆-cycloalkyl, C₃-C₆-halocycloalkyl, C₃-C₆-cycloalkyl-C₁-C₄-alkyl, C₁-C₄-alkoxy-C₁-C₄-alkyl, C₁-C₄-alkylthio-C₁-C₄-alkyl, arylthio-C₁-C₄-alkyl, C₂-C₆-alkenyl, C₂-C₅-haloalkenyl, C₃-C₆-cycloalkenyl, C₃-C₆-halocycloalkenyl, C₂-C₆-alkynyl, C₁-C₆-alkoxy, C₁-C₆-haloalkoxy, C₁-C₄-alkylthio, benzylthio, C₁-C₄-alkylcarbonyl, C₁-C₄-alkoxycarbonyl, unsubstituted or substituted phenylcarbonyl, unsubstituted or substituted benzylcarbonyl, unsubstituted or substituted phenoxycarbonyl, unsubstituted or substituted benzyloxycarbonyl, unsubstituted or substituted aryl, unsubstituted or substituted aryloxy, unsubstituted or substituted arylthio, unsubstituted or substituted aryl-C₁-C₄-alkyl, unsubstituted or substituted aryl-C₂-C₄-alkenyl, unsubstituted or substituted aryloxy-C₁-C₄-alkyl, unsubstituted or substituted arylthio-C₁-C₄-alkyl, unsubstituted or substituted hetaryl, unsubstituted or substituted hetaryloxy, unsubstituted or substituted hetarylthio, unsubstituted or substituted hetaryl-C₁-C₄-alkyl, unsubstituted or substituted hetaryl-C₂-C₄-alkenyl, unsubstituted or substituted hetaryl-C₁-C₄-alkoxy, unsubstituted or substituted heterocyclyl, unsubstituted or substituted heterocyclyloxy,
N(R⁶)₂, where the meanings of R⁶ are identical or different, or
CON(R⁷)₂, where the meanings of R⁷ are identical or different,
R⁶ is hydrogen, C₁-C₆-alkyl or unsubstituted or substituted phenyl,
R⁷ is hydrogen or C₁-C₄-alkyl,
substituents, in addition to hydrogen, being halogen, cyano, nitro, C₁-C₄-alkyl, C₁-C₄-alkoxy, C₁-C₄-haloalkyl, C₁-C₄-haloalkoxy, C₁-C₁₀-alkoximino-C₁- or -C₂-alkyl, aryl, aryloxy, benzyloxy, hetaryl, hetaryloxy, C₃-C₆-cycloalkyl, heterocyclyl or heterocyclyloxy.

2. An O-benzyloxime ether of the formula II 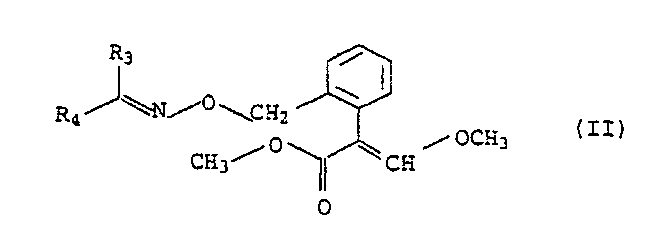 having the definitions of the radicals R³ to R⁷ stated in claim 1.

3. An O-benzyloxime ether of the formula III 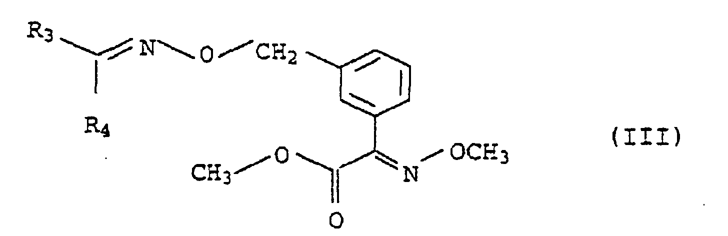 having the definitions of the radicals R³ to R⁷ stated in claim 1.

4. A process for the preparation of an O-benzyloxime ether of the formula I as claimed in claim 1, wherein
a) a benzyl derivative of the formula 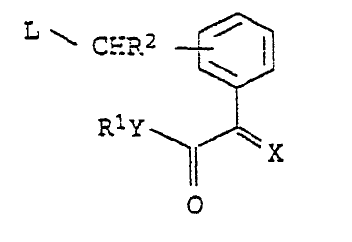 where R¹, R², X and Y have the meanings stated in claim 1 and L is a leaving group, is reacted with N-hydroxyphthalimide of the formula 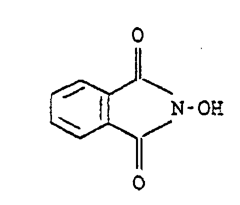 and, if required,
b) the resulting compound of the formula 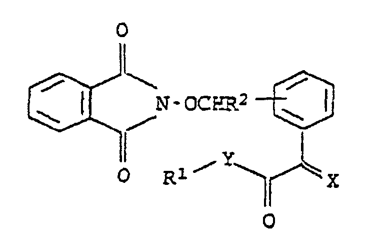 is converted with a mineral acid or with a base into the O-substituted hydroxylamine of the formula 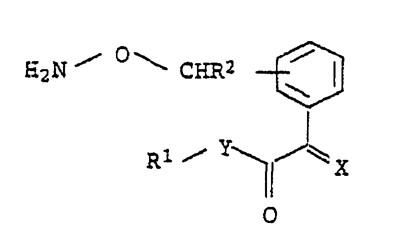 and this is reacted with an aldehyde or ketone of the formula 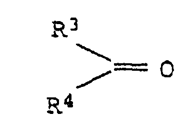 where R³ and R⁴ have the meanings stated in claim 1, or
c) the benzyl derivative of the formula 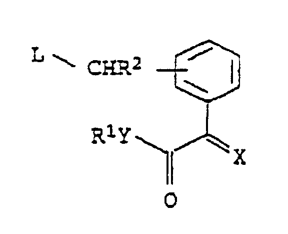 is reacted with an oxime of the formula 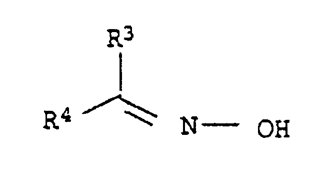
where R³ and R⁴ have the meanings stated in claim 1.

5. A compound of the formula I as claimed in claim 1, in which
x is NOCH₃ or NOCH₂CH₃,
Y is oxygen,
R¹ is C₁-C₄-alkyl,
R² is hydrogen or C₁- or C₂-alkyl,
Z¹ and Z² are each hydrogen,
R³ and R⁴, independently of one another, are each hydrogen, cyano, straight-chain or branched C₁-C₁₀-alkyl, C₁-C₄-haloalkyl, C₃-C₆-cycloalkyl, C₃-C₆-halocycloalkyl, C₃-C₆-cycloalkyl-C₁-C₄-alkyl, C₁-C₄-alkoxy-C₁-C₄-alkyl, C₁-C₄-alkylthio-C₁-C₄-alkyl, arylthio-C₁-C₄-alkyl, C₂-C₆-alkenyl, C₂-C₄-haloalkenyl, C₂-C₆-alkynyl, C₁-C₆-alkoxy, C₁-C₄-alkylthio, C₁-C₄-alkylcarbonyl, C₁-C₄-alkoxycarbonyl, unsubstituted or substituted aryl, unsubstituted or substituted aryloxy, unsubstituted or substituted arylthio, unsubstituted or substituted aryl-C₁-C₄-alkyl, unsubstituted or substituted aryl-C₂-C₄-alkenyl, unsubstituted or substituted hetaryl, unsubstituted or substituted hetaryloxy, unsubstituted or substituted hetarylthio, unsubstituted or substituted heterocyclyl,
N(R⁶)₂, where the meanings of R⁶ are identical or different,
CON(R⁷)₂, where the meanings of R⁷ are identical or different,
R³ and R⁴ together form a carbocyclic or heterocyclic ring which may be substituted by the radicals stated under substituents, or
one of the radicals R³ or R⁴ is halogen,
R⁶ is hydrogen, C₁-C₆-alkyl or unsubstituted or substituted phenyl,
R⁷ is hydrogen or C₁-C₄-alkyl,
substituents, in addition to hydrogen, being halogen, cyano, nitro, C₁-C₄-alkyl, C₁-C₄-alkoxy, C₁-C₄-haloalkyl, C₁-C₄-haloalkoxy, C₁-C₁₀-alkoximino-C₁-or -C₂-alkyl, aryl, aryloxy, benzyloxy, hetaryl, hetaryloxy, C₃-C₆-cycloalkyl, heterocyclyl or heterocyclyloxy,
with the exception of the compound excepted in claim 1.

6. A fungicide containing an inert carrier and a fungicidal amount of a benzyloxime ether of the formula I as claimed in claim 1.

7. A method of controlling fungi, which comprises treating the fungi or the materials, plants, seed or soil threatened by fungal attack with a fungicidal amount of a compound of the formula I as claimed in claim 1.

8. A compound of the formulae

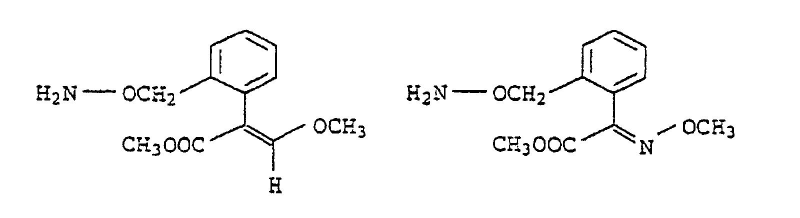

9. A compound of the formula I as claimed in claim 1, where R¹ is methyl, R² is hydrogen, R³ is cyano, R⁴ is cyclopropyl, X is NOCH₃, Y is oxygen and Z¹ and Z² are each hydrogen.

10. A compound of the formula I as claimed in claim 1, where R¹ is methyl, R² is hydrogen, R³ is hydrogen or methyl, R⁴ is C₁-C₄-alkoximino-C₁- or -C₂-alkyl-substituted phenyl, X is CH-C₁-C₄-alkoxy and Y is oxygen.

## Revendications

1. Ethers O-benzyliques d'oximes répondant à la formule générale I 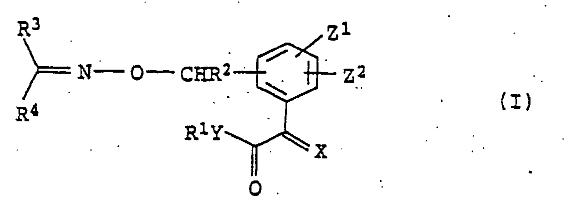 dans laquelle les substituants ont les significations suivantes:
a)
x CH-alkyle en C₁-C₄, CH-alkylthio en C₁-C₄ ou N-alcoxy en C₁-C₄,
Y l'oxygène, le soufre ou NR⁵,
R¹, R², R⁵ indépendamment les uns des autres, l'hydrogène ou un alkyle en C₁-C₄,
Z¹, Z² indépendamment l'un de l'autre,
l'hydrogène, un halogène, ou un groupe méthyle, méthoxy ou cyano,
R³, R⁴ indépendamment l'un de l'autre, hydrogène, cyano, alkyle en C₁-C₁₀ éventuellement ramifié, halogénoalkyle en C₁-C₄, cycloalkyle en C₃-C₆, halogéncyclooalkyle en C₃-C₆, cycloalkyl(C₃-C₆)-alkyle(C₁-C₄), alcoxy(C₁-C₄)-alkyle-(C₁-C₄), alkylthio(C₁-C₄-alkyle(C₁-C₄), arylthio-alkyle-(C₁-C₄), alcényle en C₂-C₆, halogénoalcényle en C₂-C₅, cycloalcényle en C₃-C₆, halogénocycloalcényle en C₃-C₆, alcynyle en C₂-C₆, alcoxy en C₁-C₆, halogénoalcoxy en C₁-C₆, alkylthio en C₁-C₄, benzylthio, alkyl(C₁-C₄)-carbonyle, alcoxy(C₁-C₄)-carbonyle, phénylcarbonyle éventuellement substitué, benzylcarbonyle éventuellement substitué, phénoxycarbonyle éventuellement substitué, benzyloxycarbonyle éventuellement substitué, aryle éventuellement substitué, aryloxy éventuellement substitué, arylthio éventuellement substitué, aryl-alkyle(C₁-C₄) éventuellement substitué, aryl-alcényle-(C₂-C₄) éventuellement substitué, aryloxy-alkyle(C₁-C₄) éventuellement substitué, arylthio-alkyle(C₁)C₄) éventuellement substitué, hétéroaryle éventuellement substitué, hétéroaryloxy éventuellement substitué, hétéroarylthio éventuellement substitué, hétéroaryl-alkyle(C₁)C₄) éventuellement substitué, hétéroaryl-alcényle(C₂-C₄) éventuellement substitué, hétéroarylalcoxy(C₁-C₄) éventuellement substitué, hétérocyclyle éventuellement substitué, hétérocyclyloxy éventuellement substitué,
N(R⁶)₂ tandis que les significations de R⁶ sont identiques ou différentes,
CON(R⁷)₂ tandis que les significations de R⁷ sont identiques ou différentes,
R³ et R⁴ forment ensemble
un noyau carbocyclique ou hétérocyclique qui peut être substitué par les restes substituants indiqués sous l'expression "éventuellement substitué", ou
l'un des restes R³ ou R⁴ représente un halogène,
R⁶ représente l'hydrogène, un alkyle en C₁-C₆ ou un phényle éventuellement substitué,
R⁷ représente l'hydrogène ou un alkyle en C₁-C₄,
"éventuellement substitué" indique, outre l'hydrogène, les restes halogéno, cyano, nitro, alkyle en C₁-C₄, alcoxy en C₁-C₄, halogénoalkyle en C₁-C₄, halogénoalcoxy en C₁-C₄, alcoxy(C₁-C₁₀)-iminoalkyle(C₁-C₂), aryle, aryloxy, benzyloxy, hétéroaryle, hétéroaryloxy, cycloalkyle en C₃-C₆, hétérocyclyle ou hétérocyclyloxy,
à l'exception des composés dans lesquels
X représente un CH-alkylthio en C₁-C₂ ou un N-alcoxy en C₁-C₂,
Y désigne l'oxygène,
Z¹, Z² et R² représentent l'hydrogène,
R¹ désigne un alkyle en C₁-C₄,
R³ désigne l'hydrogène ou un groupe alkyle en C₁-C₄, halogénoalkyle en C₁-C₄ ou cycloalkyle en C₃-C₆,
R⁴ représente alkyle en C₁-C₆, aryl-alkyle(C₁-C₄) éventuellement substitué, hétéroaryl-alkyle(C₁-C₄) éventuellement substitué, alcényle en C₂-C₆, hétéroaryl-alcényle(C₂-C₄) éventuellement substitué, cycloalkyle en C₃-C₆, aryle éventuellement substitué, hétéroaryle éventuellement substitué, alkyl(C₁-C₄)-carbonyle, phénylcarbonyle éventuellement substitué, ou
R³ et R⁴ forment conjointement avec l'atome de carbone auquel ils sont liés, un cycle saturé ayant quatre à sept chaînons, éventuellement substitué, contenant éventuellement un atome d'oxygène ou de soufre, qui peut présenter en outre un cycle benzénique condensé éventuellement substitué,
tandis que sont exclus des composés exceptés, les composés particuliers de formule Ia où 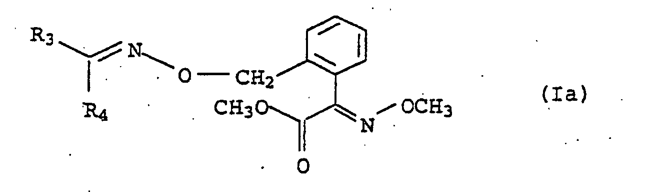
| | R³ | R⁴ |
|---|---|---|
| 8 | CCl₃ | Phényle |
| 9 | CH₂Cl | Phényle |
| 10 | CF₂CF₃ | phényle |
| 11 | CF₂Cl | Phényle |
| 12 | CHCl₂ | Phényle |
| 13 | Cyclopropyle | Cyclopropyle |
| 18 | Cyclopropyle | 4-Ethoxyphényle |
| 20 | Cyclopropyle | Pentachlorphényle |
| 21 | Cyc-lopropyle | Pentafluorphényle |
| 22 | Cyclopentyle | Phényle |
| 23 | Cyclohexyle | Phényle |
| 497 | CH₃ | Méthyle |
| 498 | CH₃ | Ethyle |
| 499 | CH₃ | n-Propyle |
| 500 | CH₃ | iso-Propyle |
| 501 | CH₃ | n-Butyle |
| 502 | CH₃ | iso-Butyle |
| 503 | CH₃ | sec.-Butyle |
| 504 | CH₃ | t-ert.-Butyle |
| 505 | CH₃ | n-Hexyle |
| 507 | CH₃ | Cyclopropyle |
| 508 | CH₃ | Cyclohexyle |
| 542 | CH₃ | Acetyle |
| 543 | CH₃ | Propion-1-yle |
| 544 | CH₃ | Butyr-1-yle |
| 545 | CH₃ | iso-Butyr-1-yle |
| 546 | CH₃ | Pivaloyle |
| 572 | CH₃ | Pentafluorphényle |
| 576 | CH₃ | Pentachlorphényle |
| 583 | CH₃ | 2,3,4-Trichlorphényle |
| 584 | CH₃ | 2,3,5-Trichlorphényle |
| 585 | CH₃ | 2,3,6-Trichlorphényle |
| 586 | CH₃ | 2,4,5-Trichlorphényle |
| 587 | CH₃ | 2,4,6-Trichlorphényle |
| 588 | CH₃ | 3,4,5-Trichlorphényle |
| 589 | CH₃ | 2,3,4,6-Tetrachlorphányle |
| 590 | CH₃ | 2-,3,5,6-Tétrachlorphényle |
| 609 | CH₃ | 2-Cyanophenyle |
| 610 | CH₃ | 3-Cyanophényle |
| 611 | CH₃ | 4-Cyanophényle |
| 618 | CH₃ | 2,4-Diméthylphényle |
| 619 | CH₃ | 2,6-Diméthylphényle |
| 620 | CH₃ | 3,4-Diméthylphényle |
| 621 | CH₃ | 3,5-Diméthylphényle |
| 622 | CH₃ | 2,3,4-Triméthylphényle |
| 623 | CH₃ | 2,3,5-Triméthylphényle |
| 624 | CH₃ | 2,3,6-Triméthylphényle |
| 625 | CH₃ | 2,4,5-Triméthlphényle |
| 62 6 | CH₃ | 2,4,6-Triméthylphényle |
| 627 | CH₃ | 3,4,5-Triméthylphényle |
| 628 | CH₃ | Pentaméthylphényle |
| 629. | CH₃ | 2-Ethylphényle |
| 630 | CH₃ | 3-Ethylphényle |
| 631 | CH₃ | 4-ethylphényle |
| 632 | CH₃ | 3,5-Diethylphényle |
| 633 | CH₃ | 2-n-Propylphényle |
| 634 | CH₃ | 3-n-Propylphényle |
| 635 | CH₃ | 4-n-Propylphényle |
| 636 | CH₃ | 2-iso-Propylphényle |
| 637 | CH₃ | 3-iso-Propylphényle |
| 638 | CH₃ | 4-iso-Propylphényle |
| 639 | CH₃ | 2,4-Di-iso-Propylphenyle |
| 640 | CH₃ | 3,5-Di-iso-Propylphényle |
| 641 | CH₃ | 4-n-Butylphényle |
| 642 | CH₃ | 4-sec.-Butylphényle |
| 643 | CH₃ | 4-iso-Butylphényle |
| 644 | CH₃ | 4-tert.-Butylphényle |
| 645 | CH₃ | 3-tert.-Butylphényle |
| 646 | CH₃ | 2-tert.-Butylphényle |
| 647 | CH₃ | 2,4-Di-tert.-butylphényle |
| 648 | CH₃ | 3,5-Di-tert.-butylphényle |
| 650 | CH₃ | 4-n-Dodécylphényle |
| 651 | CH₃ | 2-Méthyl-4-tert.-butylphényle |
| 652 | CH₃ | 2-Méthyl-6-tert.-butylphényle |
| 653 | CH₃ | 2-Méthyl-4-iso-propylphényle |
| 654 | CH₃ | 2-Méthyl-4-cycloh,exylphényle |
| 655 | CH₃ | 2-Methyl-4-phenylphenyle |
| 656 | CH₃ | 2-Méthyl-4-benzylphényle |
| 657 | CH₃ | 2-Méthyl-4-phénoxyphényle |
| 658 | CH₃ | 2-Méthyl-4-benzyloxyphényle |
| 666 | CH₃ | 2-Méthyl-3-méthoxyphényle |
| 667 | CH₃ | 2-Méthyl-4-méthoxyphényle |
| 668 | CH₃ | 2-Méthyl-5-méthoxyphényle |
| 669 | CH₃ | 2-Méthyl-6-méthoxyphényle |
| 670 | CH₃ | 2-Méthyl-4-iso-propoxyphényle |
| 671 | CH₃ | 2-Méthyl-2,5-dimethoxyphényle |
| 688 | CH₃ | 2-Ethoxyphényle |
| 689 | CH₃ | 3-Ethoxyphényle |
| 690 | CH₃ | 4-Ethoxyphényle |
| 691 | CH₃ | 2-iso-Propoxyphényle |
| 692. | CH₃ | 3-iso-Propoxyphényle |
| 693 | CH₃ | 4-iso-Propoxyphényle |
| 694 | CH₃ | 3-tert.-Butoxyphényle |
| 695 | CH₃ | 4-tert.-Butoxyphényle |
| 699 | CH₃ | 3-(1',1',2',2',-Tétrafluor)éthoxyphényle |
| 700 | CH₃ | 4-(1',1',2',2'-Tétrafluor)éthoxyphényle |
| 701 | CH₃ | 2-Chlorméthylphényle |
| 702 | CH₃ | 3-Chlorméthylphényle |
| 703 | CH₃ | 4-Chlorméthylphényle |
| 707 | CH₃ | 2-(Méthoxyiminométhyl)ghényle |
| 708 | CH₃ | 3-(Méthoxyiminomethyl)phényle |
| 709 | CH₃ | 4-(Méthoxyiminométhyl)phényle |
| 710 | CH₃ | 2-(Ethoxyiminométhyl) phényle |
| 711 | CH₃ | 3-(Ethoxyiminométhyl)phényle |
| 712 | CH₃ | 4-(Ethoxyiminométhyl)phényle |
| 713 | CH₃ | 2-(n-Propoxyiminomethyl)phenyle |
| 714 | CH₃ | 3-(n-Propoxyiminométhyl)phényle |
| 715 | CH₃ | 4-(n-Propoxyiminométhyl)phényle |
| 716 | CH₃ | 2-(iso-Propdxyiminométhyl)phényle |
| 717 | CH₃ | 3-(iso-Propoxyiminométhyl)phényle |
| 718 | CH₃ | 4-(iso-Propoxyiminomèthyl)phenyle |
| 719 | CH₃ | 2-(n-Butoxyiminométhyl)phenyle |
| 720 | CH₃ | 3-(n-Butoxyiminomethyl)phényle |
| 721 | CH₃ | 4-(n-Butoxyiminométhyl)phényle |
| 722 | CH₃ | 2-(iso-Butoxyiminométhyl)phényle |
| 723 | CH₃ | 3-(iso-Butoxyiminométhyl)phényle |
| 724 | CH₃ | 4-(iso-Butoxyiminométhyl)phenyle |
| 725 | CH₃ | 2-(tert.-Butoxyiminométhyl)phényle |
| 726 | CH₃ | 3-(tert.-Butoxyiminomethyl)phényle |
| 727 | CH₃ | 4-(tert.-Butoxyiminométhyl)phényle |
| 728 | CH₃ | 2-(n-Pentoxyiminométhyl)phényle |
| 729 | CH₃ | 3-(n-Pentoxyiminométhyl)phényle |
| 730 | CH₃ | 4-(n-Pentoxyiminométhyl)phényle |
| 731 | CH₃ | 2-(n-Hexoxyiminométhyl)phényle |
| 732 | CH₃ | 3-(n-Hexoxyiminométhyl)phényle |
| 733 | CH₃ | 4-(n-Hexoxyiminométhyl)phényle |
| 40 | CH₃ | 2-(Méthoxyimino-1'-éthyl)phényle |
| 741 | CH₃ | 3-(Méthoxyimino-1'-éthyl)phényle |
| 742 | CH₃ | 4-(Methoxyimino-1'-éthyl)phényle |
| 743 | CH₃ | 2-(Ethoxyimino-1'-éthyl)phényle |
| 744 | CH₃ | 3-(Ethoxyimino-1'-éthyl)phényle |
| 745 | CH₃ | 4-(Ethoxyimino-1'-éthyl)phényle |
| 746 | CH₃ | 2-(n-Progoxyimino-1'-éthyl)phényle |
| 747 | CH₃ | 3-(n-Propoxyimino-1'-éthyl)phényle |
| 748 | CH₃ | 4-(n-Propoxyimino-1'-éthyl)phényle |
| 749 | CH₃ | 2-(n-Butoxyamino-1'-éthyl)phényle |
| 750 | CH₃ | 3-(n-Butoxyamino-1'-éthyl)phényl)phényle |
| 751 | CH₃ | 4-(n-Butoxyamino-1'-ethyl)phényle |
| 752 | CH₃ | 2-(n-Pentoxyimino-1'-ethyl)phényle |
| 753 | CH₃ | 3-(n-Pentoxyimino-1'-ethyl ) phényle |
| 754 | CH₃ | 4-(n-Pentoxyimino-1'-ethyl)phényle |
| 755 | CH₃ | 2-(n-Hexoxyimino-1'-ethyl)phényle |
| 756 | CH₃ | 3-(n-Hexoxyimino-1'-ethyl)phenyle |
| 757 | CH₃ | 4-(n-Hexoxyimino-1'-ethyl)phényle |
| 764 | CH₃ | 2-Phénylphényle |
| 765 | CH₃ | 3-Phénylphényle |
| 766 | CH₃ | 4-Phénylphényle |
| 767 | CH₃ | 2-Phénoxyphényle |
| 768 | CH₃ | 3-Phénoxyphényle |
| 769 | CH₃ | 4-Phénoxyphényle |
| 770 | CH₃ | 2-Benzyloxyphényle |
| 771 | CH₃ | 3-Benzyloxyphényle |
| 772 | CH₃ | 4-Henzyloxyphényle |
| 773 | CH₃ | 4-(Imidazol-1'-yl)phényle |
| 774 | CH₃ | 4-(Piperazin-1'-yl)phényle |
| 777 | CH₃ | 4-(Pyridyl-2'-oxy)phényle |
| 778 | CH₃ | 2-Cyclopropylphényle |
| 779 | CH₃ | 3-Cyclopropylphényle |
| 780 | CH₃ | 4-Cyclopropylphényle |
| 781 | CH₃ | 3-Cyclohexylphényle |
| 782 | CH₃ | 4-Cyclohexylphényle |
| 783 | CH₃ | 4-Oxiranylphényle |
| 784 | CH₃ | 4-(1',3'-Dioxan-2'-yl)phényle |
| 785 | CH₃ | 4-(Tétrahydropyran-2-yloxy) phényle |
| 788 | CH₃ | 9-Anthryle |
| 804 | CH₃ | Benzyle |
| 805 | CH₃ | 2-Méthylbenzyle |
| 806 | CH₃ | 3-Méthylbenzyle |
| 807 | CH₃ | 4-Methylbenzyle |
| 808 | CH₃ | 4-tert-Butylbenzyle |
| 818 | CH₃ | 2-Méthoxybenzyle |
| 819 | CH₃ | 4-Methoxybenzyle |
| 820 | CH₃ | 4-tert-Butoxybenzyle |
| 821 | CH₃ | 4-Phénoxybenzyle |
| 822 | CH₃ | 1-Phenethyle |
| 823 | CH₃ | 2-Phénéthyle |
| 824 | CH₃ | 1-Phénylpropyle |
| 825 | CH₃ | 2-Phénylpropyle |
| 826 | CH₃ | 3-Phénylpropye |
| 827 | CH₃ | 2-Méthyl-2-phénylpropyle |
| 828 | CH₃ | 2-Méthyl-3-phénlpropyle |
| 829 | CH₃ | 4-Phénylbutyle |
| 830 | CH₃ | 2-Phényl-1-éthényle |
| 831 | CH₃ | 1-Phényl-1-éthényle |
| 832 | CH₃ | 1-Phényl-1-propényle |
| 833 | CH₃ | 1-Phényl-1-propen-2-yle |
| 839 | CH₃ | 2,6-Pyrimidinyle |
| 840 | CH₃ | 1,5-Pyrimidinyle |
| 845 | CH₃ | 1-Pyrrolyle |
| 846 | CH₃ | 1-Imidazolyle |
| 847 | CH₃ | 1,2,4-Triazolyle |
| 848 | CH₃ | 1,3,4-Triazolyle |
| 849 | CH₃ | 4-Thiazolyle |
| 857 | CH₃ | 2-Pyridylméthyle |
| 858 | CH₃ | 3-Pyridylméthyle |
| 864 | CH₃ | 2'-Furyl-2-éthényle |
| 865 | CH₃ | 2'-Thiényl-2-éthényle |
| 866 | CH₃ | 3'-Pyridyl-2-éthényle |
| 891 | CH₃ | Cyclopropyle |
| 892 | CH₃ | Cyclobutyle |
| 893 | CH₃ | Cyclopentyle |
| 894 | CH₃ | Cyclohexyle |
| 903 | CH₃ | Ethényle |
| 904 | CH₃ | 1-Propényle |
| 905 | CH₃ | 2-Méthyl-1-propényle |
| 906 | CH₃ | 4-Méthylpent-3-en-1-yle |
| 907 | CH₃ | 2-Propényle |
| 908 | CH₃ | 2-Butényle |
| 909 | CH₃ | 1-Méthyl-2-propényle |
| 910 | CH₃ | 3-Méthyl-2-butényle |
| 948 | Ethyle | Ethyle |
| 949 | Ethyle | n-Propyle |
| 950 | Ethyle | iso-Propyle |
| 951 | Ethyle | n-Butyle |
| 952 | Ethyle | iso-Butyle |
| 953 | Ethyle | 2-Méthyl-butyle |
| 954 | Ethyle | Benzyle |
| 955 | n-Propyle | n-Propyle |
| 956 | iso-Propyle | iso-Propyle |
| 957 | n-Butyle | n-Butyle |
| 958 | iso-Butyle | iso-Butyle |
| 959 | tert. -Butyle | tert. -Butyle |
| 966 | n-Butyle | Phényle |
| | R³ | R⁴ |
|---|---|---|
| 17 | H | Méthyle |
| 18 | H | Ethyle |
| 19 | H | n-Propyle |
| 20 | H | iso-Propyle |
| 21 | H | n-Butyle |
| 22 | H | iso-Butyle |
| 23 | H | sec.-Butyle |
| 24 | H | tert.-Butyle |
| 25 | H | n-gexyle |
| 27 | H | Cyclopropyle |
| 28 | H | Cyclohexyle |
| 62 | H | Acétyle |
| 63 | H | Proption-1-yle |
| 64 | H | Butyr-1-yle |
| 65 | H | iso-Butyr-1-yle |
| 66 | H | Pivaloyle |
| 88 | H | Phényle |
| 89 | H | 2-Fluorphényle |
| 90 | H | 3-Fluorphényle |
| 91 | H | 4-Fluorphényle |
| 92 | H | Pentafluorphényle |
| 93 | H | 2-Chlorphényle |
| 94 | H | 3-Chlorphényle |
| 95 | H | 4-Chlorphényle |
| 97 | H | 2,3-Dichlorphényle |
| 98 | H | 2,4-Dichlorphényle |
| 99 | H | 2,5-Dichlorphényle |
| 100 | H | 2,6-Dichlorphényle |
| 101 | H | 3,4-Dichlorphényle |
| 102 | H | 3,5-Dichlorphényle |
| 103 | H | 2,3,4-Trichlorphényle |
| 104 | H | 2,3,5-Trichlorphényle |
| 105 | H | 2,3,6-Trichlorphényle |
| 106 | H | 2,4,5-Trichlorphényle |
| 107 | H | 2,9,6-Trichlorphényle |
| 108 | H | 3,4,5-Trichlorphényle |
| 111 | H | 2-Bromphényle |
| 112 | H | 3-Bromphényle |
| 113 | H | 4-Bromphényle |
| 114 | H | 2,4-Dibromphényle |
| 115 | H | 3-Brom-4-fluarphényle |
| 116 | H | 3-Brom-4-méthoxyphényle |
| 117 | H | 2-Jodphényle |
| 118 | H | 3-Jodphényle |
| 119 | H | 4-Jodphényle |
| 120 | H | 2-Chlor-4-fluorphényle |
| 121 | H | 2-Chlor-5-fluorphényle |
| 122 | H | 2-Chlor-6-fluorphényle |
| 123 | H | 2-Chlor-4-bromphényle |
| 124 | H | 2-Brom-4-chlorphényle |
| 125 | H | 2-Brom-4-fluorphényle |
| 126 | H | 3-Brom-4-chlorphényle |
| 127 | H | 3-Chlor-4-fluorphényle |
| 128 | H | 3-Fluor-4-chlorphenyle |
| 129 | H | 2-Cyanophényle |
| 130 | H | 3-Cyanophényle |
| 131 | H | 4-Cyanaphényle |
| 132 | H | 2-Nitrophényle |
| 133 | H | 3-Nitrophényle |
| 134 | H | 4-Nitrophényle |
| 135 | H | 2-Méthylphényle |
| 136 | H | 3-Méthylphényle |
| 137 | H | 4-Méthylphényle |
| 138 | H | 2,4-Diméthylphényle |
| 139 | H | 2,6-Diméthylphényle |
| 140 | H | 3,4-Diméthylphényle |
| 141 | H | 3,5-Diméthylphényle |
| 142 | H | 2,3,4-Triméthylphényle |
| 143 | H | 2,3,5-Triméthylphényle |
| 144 | H | 2,3,6-Triméthylphényle |
| 145 | H | 2,4,5-Triméthylphényle |
| 146 | H | 2,4,6-Triméthylphényle |
| 147 | H | 3,4,5-Triméthylphényle |
| 148 | H | Pentaméthylphényle |
| 149 | H | 2-Ethylphényle |
| 150 | H | 3-Ethylphényle |
| 151 | H | 4-Ethylphényle |
| 152 | H | 3,5-Diethylphényle |
| 153 | H | 2-n-Propylphényle |
| 154 | H | 3-n-Prbpylphényle |
| 155 | H | 4-n-Propylphényle |
| 156 | H | 2-iso-Propylphényle |
| 157 | H | 3-iso-Propylphényle |
| 158 | H | 4-iso-Propylphényle |
| 159 | H. | 2,4-Di-iso-propylphényle |
| 160 | H | 3,5-Di-iso-propylphényle |
| 161 | H | 4-n-Butylphényle |
| 162 | H | 4-sec. -Butylphényle |
| 163 | H | 4-iso-Hutylghényle |
| 164 | H | 4-tert.-Butylphényle |
| 165 | H | 3-tert.-Butylphényle |
| 166 | H | 2-tert.-Butylphényle |
| 167 | H | 2,4-Di-tert.-butylphényle |
| 168 | H | 3,5-Di-tert.-butylphényle |
| 171 | H | 2-Methyl-4-tert.-butylphényle |
| 172 | H | 2-Méthyl-6-tert.-butylphényle |
| 173 | H | 2-Méthyl-4-iso-propylphényle |
| 174 | H | 2-Méthyl-4-cyclohexylphényle |
| 175 | H | 2-Méthyl-4-phénylphényle |
| 177 | H | 2-Méthyl-4-phénoxyphéuyle |
| 178 | H | 2-Méthyl-4-benzyloxyphényle |
| 179 | H | 2-Méthyl-3-chlorphényle |
| 180 | H | 2-Méthyl-4-chlorphényle |
| 181 | H | 2-Méthyl-5-chlorphényle |
| 182 | H | 2-Méthyl-6-chlorphényle |
| 183 | H | 2-Méthyl-4-fluorphényle |
| 184 | H | 2-Méthyl-3-bromphényle |
| 185 | H | 2-Méthyl-4-bromphényle |
| 186 | H | 2-Méthyl-3-méthoxyphényle |
| 187 | H | 2-Méthyl-4-méthoxyphényle |
| 188 | H | 2-Méthyl-5-méthoxyphényle |
| 189 | H | 2-Méthyl-6-méthoxyphényle |
| 190 | H | 2-Méthyl-4-iso-propoxyphényle |
| 191 | H | 2-Méthyl-2,5-diméthoxyphényle |
| 192 | H | 2-Méthoxyphényle |
| 193 | H | 3-Méthoxyphényle |
| 194 | H | 4-Méthoxyphényle |
| 195 | H | 2,3-Diméthoxyphényle |
| 196 | H | 2,4-Diméthoxyphényle |
| 197 | H | 2,5-Diméthokyphànyle |
| 198 | H | 2,6-Diméthoxyphényle |
| 199 | H | 3,4-Diméthoxyphényle |
| 200 | H | 3,5-Dimethoxyphényle |
| 201 | H | 3,6-Diméthoxyphényle |
| 202 | H | 2,3,4-Triméthoxyphényle |
| 203 | H | 2,3,5-Trimethoxyphenyle |
| 204, | H | 2,3,6-Triméthoxyphényle |
| 205 | H | 2,4,5-Triméthoxyphényle |
| 206 | H | 2,4,6-Triméthoxyphényle |
| 207 | H | 3,4,5-Triméthoxyphényle |
| 208 | H | 2-Ethoxyphényle |
| 209 | H | 3-Ethoxyphényle |
| 210 | H | 4-Ethoxyphényle |
| 211 | H | 2-iso-Propoxyphényle |
| 212 | H | 3-iso-Propoxyphényle |
| 213 | H | 4-iso-Propoxyphényle |
| 214 | H | 3-tert.-Butoxyphényle |
| 215 | H | 4-tert.-Butoxyphényle |
| 216 | H | 2-Trifluorméthoxyphényle |
| 217 | H | 3-Trifluorméthoxyphényle |
| 218 | H | 4-Trifluorméthoxyphányle |
| 219 | H | 3-(1',1',2',2'-Tétrafluor)éthoxyphényle |
| 220 | H | 4-(1',1',2',2'-Tetrafluor)éthoxyphányle |
| 221 | H | 2-Chlorméthylphényle |
| 222 | H | 3-Chlorméthylphényle |
| 223 | H | 4-Chlorméthylphényle |
| 224 | H | 2-Trifluorméthylphényle |
| 225 | H | 3-Trifluorméthylphényle |
| 226 | H | 4-Trifluorméthylphényle |
| 227 | H | 2-(Méthoxyiminométhyl)phényle |
| 228 | H | 3-(Méthoxyiminométhyl)phényle |
| 229 | H | 4-(Méthoxyiminométhyl)phényle |
| 230 | H | 2-(Ethoxyiminométhyl)phényle |
| 231 | H | 3-(Ethoxyiminométhyl)phényle |
| 232 | H | 4-(Ethoxyiminométhyl)phényle |
| 233 | H | 2-(n-Propoxyiminométhyl)phényle |
| 234 | H | 3-(n-Propoxyiminométhyl)phényle |
| 235 | H | 4- (n-Propoxyiminométhyl)phényle |
| 236 | H | 2-(iso-Propoxyiminométhyl)phényle |
| 237 | H | 3-(iso-Propoxyiminométhyl)phényle |
| 238 | H | 4-(iso-Propoxyiminométhyl)phényle |
| 239 | H | 2-(n-Butoxyiminométhyl)phényle |
| 240 | H | 3-(n-Butoxyiminométhyl)phényle |
| 241 | H | 4-(n-Butoxyiminométhyl)phényle |
| 242 | H | 2-(iso-Butoxyiminométhyl)phényle |
| 243 | H | 3-(iso-Butoxyiminomethyl)phenyle |
| 244 | H | 4- (iso-Butoxyiminométhyl)phényle |
| 245 | H | 2-(tert. -Butoxyiminométhyl)phényle |
| 246 | H | 3-(tert.-Butoxyiminométhyl)phényle |
| 247 | H | 4-(tert.-Butoxyiminométhyl)phényle |
| 248 | H | 2-(n-Pentoxyimonométhyl)phényle |
| 249 | H | 3-(n-Pentoxyimonométhyl)phényle |
| 250 | H | 4-(n-pentoxyimonométhyl)phényle |
| 251 | H | 2-(n-He-xoxyimonbméthyl)phényle |
| 252 | H | 3-(n-Hexoxyimonométhyl)phényle |
| 253 | H | 4-(n-Hexoxyimonométhyl)phényle |
| 260 | H | 2-(Methoxyimino-1'-éthyl)phényle |
| 261 | H | 3-(Méthoxyimino-1'-éthyl)phényle |
| 262 | H | 4-(Méthoxyimino-1'-éthyl)phényle |
| 263 | H | 2-(Ethoxyimino-1'-éthyl)phényle |
| 264 | H | 3-(Ethoxyimino-1'-éthyl)phényle |
| 265 | H | 4-(Ethoxyimino-1'-éthyl)phényle |
| 266 | H | 2-(n-Propoxyimino-1'-éthyl)phényle |
| 267 | H | 3-(n-Propoxyimino-1'-éthyl)phényle |
| 268 | H | 4-(n-Propoxyimino-1-éthyl)phényle |
| 269 | H | 2-(n-Butoxyamino-1'-éthyl)phényle |
| 270. | H | 3-(n-Butoxyamino-1'-éthyl)phényle |
| 271 | H | 4-(n-Butoxyamino-1'-éthyl)phényle |
| 272 | H | 2-(n-Pentoxyimino-1'-éthyl)phényle |
| 273 | H | 3-(n-Pentoxyimino-1'-éthyl)phényle |
| 274 | H | 4-(n-Pentoxyimino-1'-éthyl)phényle |
| 275 | H | 2-(n-Hexoxyimino-1'-éthyl)phényle |
| 276 | H | 3-(n-Hexoxyimino-1'-éthyl)phényle |
| 277 | H | 4-(n-Hexoxyimino-1'-éthyl)phényle |
| 284 | H | 2-Phénylphényle |
| 285 | H | 3-Phénlphényle |
| 286 | H | 4-Phénylphényle |
| 287 | H | 2-Phénoxyphényle |
| 288 | H | 3-Phénoxyphényle |
| 289 | H | 4-Phénoxyphényle |
| 293 | H | 4-(Imidazol-1'-yl)phényle |
| 294 | H | 4-(Piperazin-1'-yl)phényle |
| 295 | H | 4-(Morpholin-1'-yl)phényle |
| 296 | H | 4-(Piperidin-1'-yl)phényle |
| 297 | H | 4-(Pyridyl-2'-oxy)phényle |
| 298 | H | 2-Cyclopropylphényle |
| 299 | H | 3-Cyclopropylphényle |
| 300 | H | 4-Cyclopropylphényle |
| 301 | H | 3-Cyclohexylphényle |
| 302 | H | 4-Cyclohexylphényle |
| 303 | H | 4-Oxiranylphényle |
| 304 | H | 4-(1',3'-Dioxan-2'-yl)phenyle |
| 305 | H | 4- (Tétrahydropyran-2yloxy)phényle |
| 306 | H | 1-Naphthyle |
| 307 | H | 2-Naphthyle |
| 308 | H | 9-Anthryle |
| 324 | H | Benzyle- |
| 325 | H | 2-Méthylbenzyle |
| 326 | H | 3-Méthylbenzyle |
| 327 | H | 4-Méthylbenzyle |
| 328 | H | 4-tert.-Butylbenzyle |
| 329 | H | 2-Chlorbenzyle |
| 330 | H | 3-Chlorbenzyle |
| 331 | H | 4-Chlorbenzyle |
| 332 | H | 2,4-Dichlorbenzyle |
| 333 | H | 2,6-Dichlorbenzyle |
| 334 | H | 2,4,6-Zrichlorbenzyle |
| 335 | H | 2-Trifluormethylbenzyle |
| 336 | H | 3-Trifluorméthylbenzyle |
| 337 | H | 4-Trifluorméthylbenzyle |
| 338 | H | 2-Méthcxybenzyle |
| 339 | H | 4-Méthoxybenzyle |
| 340 | H | 4-tert.-Butoxybenzyle |
| 341 | H | 4-Phénoxybenzyle |
| 342 | H | 1-Phénéthyle |
| 343 | H | 2-Phénéthyle |
| 344 | H | 1-Phénylpropyle |
| 345 | H | 2-Phénylpropyle |
| 346 | H | 3-Phénylpropyle |
| 347 | H | 2-Méthyl-2-phénylpropyle |
| 348 | H | 2-Méthyl-3-phénylpropyle |
| 349 | H | 4-Phénylbutyle |
| 350 | H | 2-Phényl-1-éthéryle |
| 351 | H | 1-Phényl-1-éthényle |
| 352 | H | 1-Phényl-1-propényle |
| 353 | H | 1-Phényl-1-Dropén-2-yle |
| 356. | H | 2-Pyridyle |
| 357 | H | 3-Pyridyle |
| 358 | H | 4-Pyridyle |
| 359 | H | 2,6-Pyrimidinyle |
| 360 | H | 1,5-Pyrimidinyle |
| 361 | H | 2-Thiéryle |
| 362 | H | 3-Thiényle |
| 363 | H | 2-Furyle |
| 364 | H | 3-Furyle |
| 365 | H | 1-Pyrrolyle |
| 366 | H | 1-Imidazolyle |
| 367 | H | 1,2,4-Triazolyle |
| 368 | H | 1,3,4-Triazolyle |
| 369 | H | 4-Thiazolyle |
| 370 | H | 2-Benzothiazolyle |
| 377 | H | 2-Pyridylméthyle |
| 378 | H | 3-Pyridylmethyle |
| 384 | H | 2'-Furyl-2-éthényle |
| 385 | H | 2'-Thiényl-2-éthényle |
| 386 | H | 3'-Pyridyl-2-éthényle |
| 411 | H | Cyclopropyle |
| 412 | H | Cyclobutyle |
| 413 | H | Cyclopentyle |
| 414 | H | Cyclohexyle |
| 423 | H | Ethényle |
| 424 | H | 1-Propényle |
| 425 | H | 2-Methyl-1-propényle |
| 426 | H | 4-Méthylpent-3-en-1-yle |
| 427 | H | 2-Propényle |
| 428 | H | 2-Butényle |
| 429 | H | 1-Méthyl-2-propényle |
| 430 | H | 3-Méthyl-2-butényle |
et les compoés de formule Ib 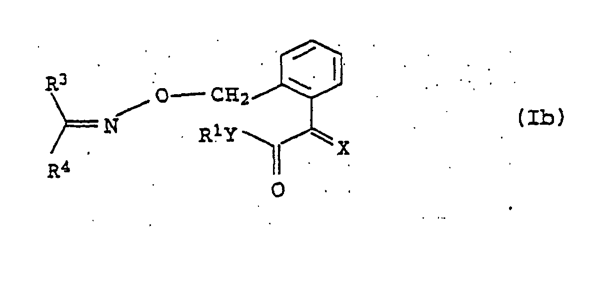 dans lesquels on a
| No. | X | Y | R¹ | R³ | R⁴ |
|---|---|---|---|---|---|
| 5 | CHS-CH₃ | O | CH₃ | Cyclopropyle | 4-tert.-Butylphenyle |
| 25 | CHS-CH₃ | O | CH₃ | CH₃ | Benzyle |
| 26 | CHS-CH₃ | O | CH₃ | CH₃ | tert.-Butyle |
| 27 | CHS-CH₃ | O | CH₃ | CH₃ | iso-Propyle |
| 28 | CHS-CH₃ | O | CH₃ | CH₃ | 2-Méthylbutyle |
| 29 | CHS-CH₃ | O | CH₃ | CH₃ | iso-Butyle |
| 30 | CHS-CH₃, | O | CH₃ | CH₃ | Phenyle |
| 36 | CHS-CH₃ | O | CH₃ | iso-Butyle | iso-Hutyle |
| 38 | CHS-CH₃ | O | CH₃ | CH₂CH₂CH₂CH₂ | |
| 39 | CHS-CH₃ | O | CH₃ | CH₂CH₂CH₂CH₂CH₂ | |
| 196 | N-OCH₃ | O | C₂H₅ | CH₃ | Phényle |
| 198 | N-OCH₃ | O | n-C₃H₅ | CH₃ | Phényle |
| 205 | CHS-CH₃ | O | CH₃ | H | Phényle |
b)
X CH-alcoxy en C₁-C₄,
Y l'oxygène ou le soufre,
R¹,R² indépendamment l'un de l'autre, l'hydrogène ou un alkyle en C₁-C₄,
Z¹, Z² indépendamment l'un de l'autre, l'hydrogène, un halogène, ou un groupe méthyle, méthoxy ou cyano,
R⁴ représente un groupe phényle substitué par un alcoxy(C₁-C₁₀)imino-alkyle(C₁-C₂), et
R³ représente l'hydrogène ou un groupe cyano, alkyle en C₁-C₁₀ éventuellement ramifié, halogénoalkyle en C₁-C₄, cycloalkyle en C₃-C₆, halogénocycloalkyle en C₃-C₆, cycloalkyl(C₃-C₆)-alkyle(C₁-C₄), alcoxy(C₁-C₄)-alkyle-(C₁-C₄), alkylthio(C₁-C₄-alkyle(C₁-C₄), arylthio-alkyle-(C₁-C₄), alcényle en C₂-C₆, halogénoalcényle en C₂-C₅, cycloalcényle en C₃-C₆, halogénocycloalcényle en C₃-C₆, alcynyle en C₂-C₆, alcoxy en C₁-C₆, halogénoalcoxy en C₁-C₆, alkylthio en C₁-C₄, benzylthio, alkyl(C₁-C₄)-carbonyle, alcoxy(C₁-C₄)-carbonyle, phénylcarbonyle éventuellement substitué, benzylcarbonyle éventuellement substitué, phénoxycarbonyle éventuellement substitué, benzyloxycarbonyle éventuellement substitué, aryle éventuellement substitué, aryloxy éventuellement substitué, arylthio éventuellement substitué, arylalkyle(C₁-C₄) éventuellement substitué, arylalcényle(C₂-C₄) éventuellement substitué, aryloxyalkyle(C₁-C₄) éventuellement substitué, arylthioalkyle(C₁)C₄) éventuellement substitué, hétéroaryle éventuellement substitué, hétéroaryloxy éventuellement substitué, hétéroarylthio éventuellement substitué, hétéroaryl-alkyle(C₁)C₄) éventuellement substitué, hétéroaryl-alcényle(C₂-C₄) éventuellement substitué, hétéroaryl-alcoxy(C₁-C₄) éventuellement substitué, hétérocyclyle éventuellement substitué, hétérocyclyloxy éventuellement substitué,
N(R⁶)₂ tandis que les significations de R⁶ sont identiques ou différentes, ou
CON(R⁷)₂ tandis que les significations de R⁷ sont identiques ou différentes,
R⁶ représente l'hydrogène, un alkyle en C₁-C₆ ou un phényle éventuellement substitué,
R⁷ représente l'hydrogène ou un alkyle en C₁-C₄,
"éventuellement substitué" indique, outre l'hydrogène, les restes halogéno, cyano, nitro, alkyle en C₁-C₄, alcoxy en C₁-C₄, halogénoalkyle en C₁-C₄, halogénoalcoxy en C₁-C₄, alcoxy(C₁-C₁₀)-iminoalkyle(C₁-C₂), aryle, aryloxy, benzyloxy, hétéroaryle, hétéroaryloxy, cycloalkyle en C₃-C₆, hétérocyclyle ou hétérocyclyloxy.

2. Ethers O-benzyliques d'oximes de formule II 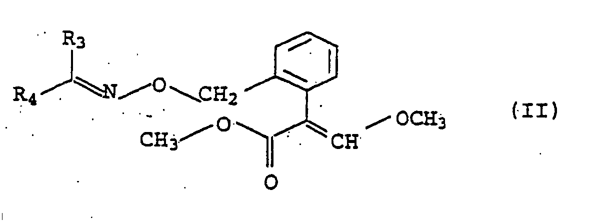 avec les définitions des restes R³ à R⁷ indiquées dans la revendication 1.

3. Ethers O-benzyliques d'oximes de formule III 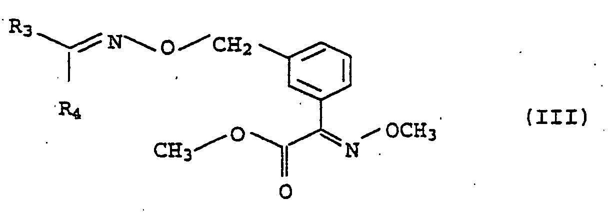 avec les définitions des restes R³ à R⁷ indiquées dans la revendication 1.

4. Procédé pour la préparation d'éthers O-benzyliques d'oximes de formule I selon la revendication 1, **caractérisé par le fait que**
a) on fait réagir un dérivé benzylique de formule 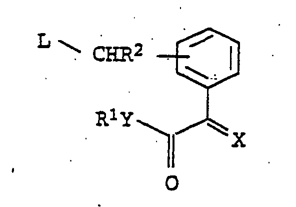 dans laquelle R¹, R², X et Y ont les significations indiquées dans la revendication 1 et L désigne un groupe éliminable, avec le N-hydroxyphtalimide de formule 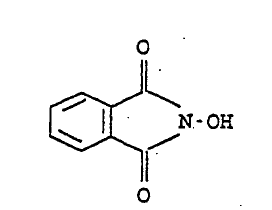 et le cas échéant
b) on convertit le composé ainsi obtenu répondant à la formule 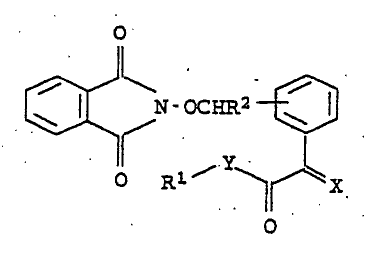 avec un acide minéral ou avec des bases, en l'hydroxylamine O-substituée de formule 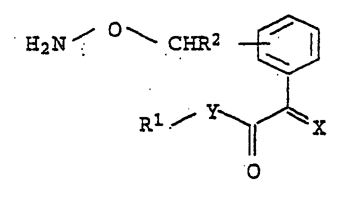 et on fait réagir celle-ci avec un aldéhyde ou une cétone de formule 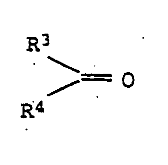 dans laquelle R³ et R⁴ ont les significations indiquées dans la revendication 1, ou bien
c) on fait réagir le dérivé benzylique de formule 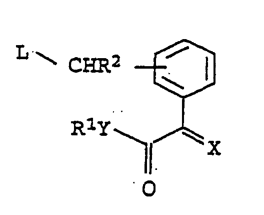 avec une oxime de formule 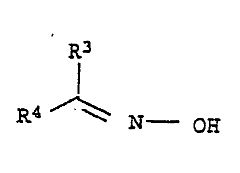 dans laquelle R³ et R⁴ ont les significations indiquées dans la revendication 1.

5. Composés de formule I selon la revendication 1, dans laquelle les substituants ont la signification suivante:
X NOCH₃ ou NOCH₂CH₃,
Y l'oxygène,
R¹ un alkyle en C₁-C₄,
R² l'hydrogène ou un alkyle en C₁-C₂,
Z¹, Z² l'hydrogène,
R³, R⁴ indépendamment l'un de l'autre, hydrogène, cyano, alkyle en C₁-C₁₀ éventuellement ramifié, halogénoalkyle en C₁-C₄, cycloalkyle en C₃-C₆, halogéncyclooalkyle en C₃-C₆, cycloalkyl(C₃-C₆)-alkyle(C₁-C₄), alcoxy(C₁-C₄)-alkyle(C₁-C₄), alkylthio(C₁-C₄-alkyle(C₁-C₄), arylthioalkyle(C₁-C₄), alcényle en C₂-C₆, halogénoalcényle en C₂-C₄, alcynyle en C₂-C₆, alcoxy en C₁-C₆, alkylthio en C₁-C₄, alkyl(C₁-C₄)-carbonyle, alcoxy(C₁-C₄)-carbonyle, aryle éventuellement substitué, aryloxy éventuellement substitué, arylthio éventuellement substitué, arylalkyle(C₁-C₄) éventuellement substitué, aryl-alcényle-(C₂-C₄) éventuellement substitué, hétéroaryle éventuellement substitué, hétéroaryloxy éventuellement substitué, hétéroarylthio éventuellement substitué, hétérocyclyle éventuellement substitué,
N(R⁶)₂ tandis que les significations de R⁶ sont identiques ou différentes,
CON(R⁷)₂ tandis que les significations de R⁷ sont identiques ou différentes,
R³ et R⁴ forment ensemble un noyau carbocyclique ou hétérocyclique qui peut être substitué par les restes substituants indiqués sous l'expression "éventuellement substitué", ou
l'un des restes R³ ou R⁴ représente un halogène,
R⁶ représente l'hydrogène, un alkyle en C₁-C₆ ou un phényle éventuellement substitué,
R⁷ représente l'hydrogène ou un alkyle en C₁-C₄,
"éventuellement substitué" indique, outre l'hydrogène, les restes halogéno, cyano, nitro, alkyle en C₁-C₄, alcoxy en C₁-C₄, halogénoalkyle en C₁-C₄, halogénoalcoxy en C₁-C₄, alcoxy(C₁-C₁₀)-iminoalkyle(C₁-C₂), aryle, aryloxy, benzyloxy, hétéroaryle, hétéroaryloxy, cycloalkyle en C₃-C₆, hétérocyclyle ou hétérocyclyloxy,
à l'exception des composés exclus dans la revendication 1.

6. Fongicide contenant un support inerte et une quantité à efficacité fongicide d'un éther O-benzylique d'oxime de formule I selon la revendication 1.

7. Procédé pour la lutte contre les champignons, **caractérisé par le fait qu'**on traite les champignons ou les matériaux, les végétaux, les plantes, les semences ou le sol menacés d'une attaque par les champignons avec une quantité à efficacité fongicide d'un composé de formule I selon la revendication 1.

8. Composés de formules

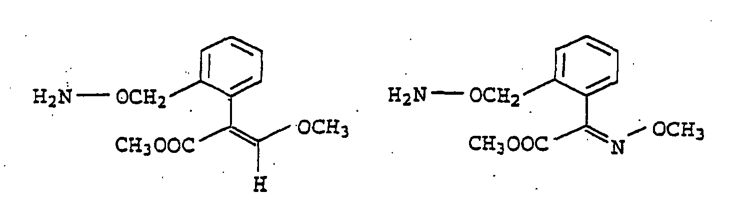

9. Composés de formule I selon la revendication 1, dans laquelle R¹ représente le méthyle, R² l'hydrogène, R³ un cyano, X NOCH₃, Y l'oxygène, et Z¹ et Z² l'hydrogène.

10. Composés de formule I selon la revendication 1, dans laquelle R¹ représente le méthyle, R² l'hydrogène, R³ l'hydrogène ou le méthyle, R⁴ un phényle substitué par un alcoxy(C₁-C₄)imino-alkyle(C₁-C₂), X un CH-alcoxy(C₁-C₄) et Y l'oxygène.
